# EUROPEAN PATENT APPLICATION

(11) **EP 3 623 472 A1**
(43) Date of publication of application: **18.03.2020**
(21) Application number: 18798161.8
(22) Date of filing: 11.05.2018
(51) Int. Cl.: C12N 15/09, C12N 1/21, C12P 17/12

(54) **RECOMBINANT MICROORGANISM, PRODUCTION METHOD OF PYRIDOXAMINE, OR SALT THEREOF, USING RECOMBINANT MICROORGANISM, AND PRODUCTION METHOD OF PYRIDOXAL, OR SALT THEREOF, USING RECOMBINANT MICROORGANISM**

(30) Priority: 12.05.2017 JP 2017095571; 12.05.2017 JP 2017095573
(71) Applicant: Mitsui Chemicals, Inc., Minato-ku Tokyo 105-7122 (JP); Renascience Co., Ltd., Tokyo 105-7117 (JP)
(72) Inventor: TATENO, Toshihiro, Mobara-shi Chiba 297-0017 (JP); YOKOTA, Motoi, Mobara-shi Chiba 297-0017 (JP); HIDESAKI, Tomonori, Omuta-shi Fukuoka 836-8610 (JP); ARAKI, Tadashi, Mobara-shi Chiba 297-0017 (JP); SHINDO, Atsunori, Tokyo 105-7122 (JP); MATSUMOTO, Yoshiko, Mobara-shi Chiba 297-0017 (JP); MIYATA, Toshio, Nagoya-shi Aichi 464-0827 (JP); HONJO, Masaru, Mobara-shi Chiba 297-0017 (JP)
(74) Representative: Mewburn Ellis LLP
(86) International application number: PCT/JP2018/018413
(87) International publication number: WO 2018/207931

(57) **Abstract**

A recombinant microorganism includes a gene encoding a pyridoxine dehydrogenase, a gene encoding a pyridoxamine synthetase having an enzymatic activity of synthesizing pyridoxamine from pyridoxal, and a gene encoding an amino acid regeneration enzyme having an enzymatic activity of regenerating an amino acid consumed by the pyridoxamine synthetase, in which at least two of the gene encoding the pyridoxine dehydrogenase, the gene encoding the pyridoxamine synthetase, or the gene encoding the amino acid regeneration enzyme, are introduced from outside of a bacterial cell, or are endogenous to the bacterial cell and have an enhanced expression. In addition, a recombinant microorganism into which a gene encoding a pyridoxine dehydrogenase is introduced is provided.

## Description

### Technical Field

The present disclosure relates to a recombinant microorganism that can produce pyridoxamine or a salt thereof, a method of producing pyridoxamine or a salt thereof using the recombinant microorganism, a recombinant microorganism that can produce pyridoxal or a salt thereof, and a method of producing pyridoxal or a salt thereof using the recombinant microorganism.

Pyridoxamine and a salt thereof belong to vitamin B₆ family, and are known to have a glycation reaction inhibitory action. Pyridoxamine and a salt thereof, for example, inhibit accumulation of advanced glycation end products (AGEs) in the body which is involved in various aging processes. AGE is a generic term for substances produced by glycation of proteins, and AGE accumulation is believed to aggravate a disease such as diabetes mellitus, atherosclerosis, chronic renal failure, or Alzheimer's dementia. For this reason, pyridoxamine and a salt thereof are promising substances that are expected to make it possible to prevent or treat such a disease by preventing AGE accumulation.

Pyridoxamine and a salt thereof are also known to have activity as a schizophrenia drug, and various studies for their practical use have been made. In addition, development of health foods and cosmetics utilizing various physiological activities of pyridoxamine and a salt thereof is also in progress.

Pyridoxamine and a salt thereof can be synthesized chemically. For example, International Publication No. WO 2006/066806 discloses a method of chemically synthesizing pyridoxamine dihydrochloride using alanine and formic acid as starting materials. WO 2005/077902 discloses a method of chemically synthesizing pyridoxamine from pyridoxine.

On the other hand, biological synthesis of pyridoxamine is also being studied. WO 2007/142222 discloses a method of obtaining pyridoxamine from pyridoxal using a specific microorganism such as *Achromobacter.* WO 2007/142222 also discloses an experiment in which, by culturing *Acremonium fusidioides* in the presence of pyridoxine, a certain amount of the pyridoxine was converted to pyridoxal.

Japanese Patent Application Laid-Open (JP-A) No. H09-107985 discloses a method of producing vitamin B₆, in which a microorganism belonging to the genus *Rhizobium* and capable of producing vitamin B₆ is cultured under aerobic conditions in a culture medium, and produced vitamin B₆ is obtained from a culture solution.

As a study on vitamin B₆ synthesis, WO 2004/035010 discloses a method of producing vitamin B₆ by culturing an organism in which at least one of the activity of yaaD or yaaE of *Bacillus subtilis* is enhanced compared to the parent organism. Japanese Patent Application Laid-Open (JP-A) No. 2000-23690 describes a production of a vitamin B₆ mixture by using cell-free extract derived from *Rhizobium meliloti* IFO 14782. Journal of Molecular Catalysis B: Enzymatic, 2010, vol. 67, p. 104-110 discloses a pyridoxamine-pyruvate aminotransferase (PPAT) that is enabled to use L-glutamate by modifying its sequence. It is described that pyridoxamine was able to be produced by incubating *E. coli* expressing PPAT having such an modified sequence in the presence of a saturated amount of pyridoxal.

Pyridoxal is also one form of vitamin B₆, and a nutrient rcred for the survival of organisms. Vitamin B₆ is not only used in food and food additives but also has wide applications such as raw materials for pharmaceutical products and cosmetics. Pyridoxal has a useful application as a single component. For example, pyridoxal phosphate is used as an active vitamin B₆ agent, and pyridoxal is a material that can be an intermediate product for producing pyridoxal phosphate. Pyridoxal is useful for pharmaceutical products, cosmetics, food, or intermediate products for producing the pharmaceutical products, the cosmetics, and the food. Japanese Patent Publication (JP-B) No. S43-5712 discloses a method of producing a pyridoxal-carbonyl derivative, the method including adding a carbonyl reagent into a reaction liquid in a converting enzyme reaction from pyridoxine to pyridoxal by pyridoxine oxidase. The Journal of Biological Chemistry, 1999, Vol. 274, No. 33, Issue of August 13, pp. 23185-23190 also describes such a converting enzyme.

### SUMMARY OF INVENTION

### Technical Problem

However, high reaction yields have not been achieved by pyridoxamine synthesis by chemical synthesis. For example, in a method described in WO 2006/066806, pyridoxamine dihydrochloride is synthesized through many chemical reactions, which results in lower reaction yields. In a method described in WO 2005/077902, a dimer and a trimer of pyridoxamine are produced, and the reaction yield is not significantly high.

On the other hand, in a method described in WO 2007/142222, in which a specific kind of microorganism is used, the conversion efficiency from pyridoxal to pyridoxamine is varied widely among microbial species and is not significantly high in all. In a culture experiment of *Acremonium fusidioides* in the presence of pyridoxine described in WO 2007/142222, most of a product is pyridoxal rather than pyridoxamine. JP-A No. H09-107985 describes that most of produced vitamin B₆ was pyridoxol (pyridoxine), and production of pyridoxamine was slight.

WO 2004/035010 describes that vitamin B₆ is obtained by culturing in a culture medium a microorganism highly expressing the genes for yaaD and yaaE; however, the product is a mixture of pyridoxine, pyridoxal, pyridoxamine, pyridoxamine phosphate, and the like and pyridoxamine is not selectively obtained.

As described above, chemical synthesis of pyridoxamine or a salt thereof (such as WO 2006/066806 or WO 2005/077902) involves many reaction and purification steps, and high yields have not been achieved. Although some microorganisms have pyridoxamine-synthesizing ability (for example, WO 2007/142222 and JP-ANo. H09-107985), it takes time and effort to newly discover such microorganisms. In addition, selective synthesis of pyridoxamine or a salt thereof among the vitamin B₆ family has not been realized and high pyridoxamine production efficiency has not been achieved. The approach of screening specific microbial species from naturally occurring microbial species as described above does not provide molecular biological information as to which enzyme or gene is important for a high production of vitamin B₆.

For example, a method of producing vitamin B₆ using recombinant microorganisms produced by genetic modification technology is described (WO 2004/035010 and Journal of Molecular Catalysis B: Enzymatic, 2010, vol. 67, p. 104-110). However, in production of substances, vitamin B₆ is non-selectively obtained using a common culture medium (WO 2004/035010), or alternatively, a saturated amount of pyridoxal, which is an expensive raw material, is used to produce pyridoxamine (Journal of Molecular Catalysis B: Enzymatic, 2010, vol. 67, p. 104-110). Therefore, inexpensive and selective production of pyridoxamine or a salt thereof has not been achieved.

In view of the above, according to a first aspect of the disclosure, a recombinant microorganism capable of inexpensively producing pyridoxamine or a salt thereof from pyridoxine or a salt thereof at high production efficiency, and a method of inexpensively producing pyridoxamine or a salt thereof from pyridoxine or a salt thereof at high production efficiency using the recombinant microorganism are provided.

An efficient enzymatic production method that can be used in industrial production has not yet been put to practical use in the production of pyridoxal. In the method described in WO 2004/035010, and the method described in JP-A No. 2000-23690, a mixture of pyridoxine, pyridoxal, and pyridoxamine is obtained by an enzymatic method; however, these methods are not methods in which pyridoxal can be selectively obtained, i.e., can be obtained as pyridoxal with high purity rather than as a mixture of pyridoxine and pyridoxamine.

It is essential to add a carbonyl reagent to a reaction system in the method described in JP-B No. S43-5712. The carbonyl reagent converts produced pyridoxal into a carbonyl derivative. However, a method in which a carbonyl derivative is produced is not often suitable for subjecting pyridoxal as an intermediate product to a subsequent reaction.

From the viewpoint of industrial production of pyridoxal, the present inventors attempted production of an industrially available recombinant microorganism into which a gene encoding a pyridoxine dehydrogenase is introduced. A host cell for such a genetically modified microorganism is preferably a prokaryote from the viewpoint of a cost and the easiness of production. However, for example, in a case in which the introduced gene is a eukaryotic gene, there are many difficulties in introducing, into a prokaryote, a gene encoding a protein that is expressed in a eukaryote, and in expressing the gene, and it is often impossible to express the protein maintaining activity. Thus, a second aspect of the disclosure is to provide a recombinant microorganism that includes and expresses an introduced pyridoxine dehydrogenase.

### Solution to Problem

A first aspect of the disclosure includes the following.
<1> A recombinant microorganism, including:
   a gene encoding a pyridoxine dehydrogenase, a gene encoding a pyridoxamine synthetase having an enzymatic activity of synthesizing pyridoxamine from pyridoxal, and a gene encoding an amino acid regeneration enzyme having an enzymatic activity of regenerating an amino acid consumed by the pyridoxamine synthetase,
   wherein at least two of the gene encoding the pyridoxine dehydrogenase, the gene encoding the pyridoxamine synthetase, or the gene encoding the amino acid regeneration enzyme, are introduced from outside of a bacterial cell, or are endogenous to the bacterial cell and have an enhanced expression.
<2> The recombinant microorganism according to <1>, wherein the pyridoxamine synthetase is a pyridoxamine-pyruvate transaminase, a pyridoxamine-oxaloacetate transaminase, an aspartate transaminase, or a pyridoxamine phosphate transaminase.
<3> The recombinant microorganism according to <1> or <2>, wherein the pyridoxine dehydrogenase includes at least one of the following partial amino acid sequence (a) or partial amino acid sequence (b), and has pyridoxine dehydrogenase activity:
   (a) NX₁X₂EX₃YG (SEQ ID NO:97)
      wherein X₁ represents V, C, I, A, M, S, G, or L,
      X₂ represents G or A, and
      X₃ represents F or L; and
   (b) X₄X₅X₆KGX₇ (SEQ ID NO:98)
      wherein X₄ represents I, V, F, or L,
      X₅ represents S, T, N, C, or M,
      X₆ represents C, V, A, I, W, or F, and
      X₇ represents G, A, S, or C.
<4> The recombinant microorganism according to any one of <1> to <3>, wherein the pyridoxine dehydrogenase is represented by enzyme number EC1.1.1.65.
<5> The recombinant microorganism according to any one of <1> to <4>, wherein the gene encoding the pyridoxine dehydrogenase is derived from *Saccharomyces cerevisiae.*
<6> The recombinant microorganism according to any one of <1> to <5>, wherein the gene encoding the pyridoxine dehydrogenase has:
   a nucleotide sequence of any one of SEQ ID NO:7 or SEQ ID NO:53 to SEQ ID NO:59,
   a region between an 18th nucleotide and a 3' end in a nucleotide sequence of any one of SEQ ID NO:13 or SEQ ID NO:75 to SEQ ID NO:81, or
   a nucleotide sequence that hybridizes with DNA having a nucleotide sequence complementary to a nucleotide sequence of any one of SEQ ID NO:7 or SEQ ID NO:53 to SEQ ID NO:59, or with DNA having a nucleotide sequence complementary to a region between an 18th nucleotide and a 3' end in a nucleotide sequence of any one of SEQ ID NO:13 or SEQ ID NO:75 to SEQ ID NO:81 under a stringent condition, and that encodes a protein having pyridoxine dehydrogenase activity.
<7> The recombinant microorganism according to any one of <1> to <6>, wherein the gene encoding the pyridoxine dehydrogenase has a nucleotide sequence of SEQ ID NO:7, or has a nucleotide sequence that hybridizes with DNA having a nucleotide sequence complementary to the nucleotide sequence of SEQ ID NO:7 under a stringent condition, and that encodes a protein having pyridoxine dehydrogenase activity.
<8> The recombinant microorganism according to any one of <1> to <4>, wherein the gene encoding the pyridoxine dehydrogenase is derived from *Schizosaccharomyces pombe.*
<9> The recombinant microorganism according to any one of <1> to <4> and <8>, wherein the gene encoding the pyridoxine dehydrogenase has a nucleotide sequence of SEQ ID NO:8, or has a nucleotide sequence that hybridizes with DNA having a nucleotide sequence complementary to the nucleotide sequence of SEQ ID NO:8 under a stringent condition, and that encodes a protein having pyridoxine dehydrogenase activity.
<10> The recombinant microorganism according to any one of <1> to <9>, wherein the gene encoding the pyridoxine dehydrogenase has a nucleotide sequence encoding an amino acid sequence of any one of SEQ ID NO:1, SEQ ID NO:2, or SEQ ID NO:22 to SEQ ID NO:28, or an amino acid sequence that has 80% or more sequence identity with at least one amino acid sequence selected from the group consisting of SEQ ID NO:1, SEQ ID NO:2, and SEQ ID NO:22 to SEQ ID NO:28, and that has pyridoxine dehydrogenase activity.
<11> The recombinant microorganism according to any one of <1> to <10>, wherein the gene encoding the pyridoxine dehydrogenase has a nucleotide sequence encoding an amino acid sequence of SEQ ID NO:1 or SEQ ID NO:2, or an amino acid sequence that has 80% or more sequence identity with at least one of the amino acid sequence of SEQ ID NO:1 or the amino acid sequence of SEQ ID NO:2, and that has pyridoxine dehydrogenase activity.
<12> The recombinant microorganism according to any one of <1> to <11>, wherein the pyridoxamine synthetase includes at least one of the following partial amino acid sequence (c), partial amino acid sequence (d), partial amino acid sequence (e), partial amino acid sequence (f), partial amino acid sequence (g), or partial amino acid sequence (h), and has an enzymatic activity of synthesizing pyridoxamine from pyridoxal:
   (c) X₈X₉X₁₀X₁₁X₁₂X₁₃ (SEQ ID NO:99)
      wherein X₈ represents L, M, I, or V,
      X₉ represents H or Q,
      X₁₀ represents G, C, or A,
      X₁₁ represents E or D,
      X₁₂ represents P or A, and
      X₁₃ represents V, I, L, or A;
   (d) X₁₄X₁₅TPSGTX₁₆X₁₇ (SEQ ID NO:100)
      wherein X₁₄ represents H or S,
      X₁₅ represents D or E,
      X₁₆ represents I, V, or L, and
      X₁₇ represents N or T;
   (e) X₁₈DX₁₉VSX₂₀X₂₁ (SEQ ID NO:101)
      wherein X₁₈ represents V, I, or A,
      X₁₉ represents A, T, or S,
      X₂₀ represents S, A, or G, and
      X₂₁ represents F, W, or V;
   (f) X₂₂X₂₃X₂₄KCX₂₅GX₂₆X₂₇P (SEQ ID NO:102)
      wherein X₂₂ represents G or S,
      X₂₃ represents P, S, or A,
      X₂₄ represents N, G, S, A, or Q,
      X₂₅ represents L or M,
      X₂₆ represents A, S, C, or G, and
      X₂₇ represents P, T, S, or A;
   (g) X₂₈X₂₉X₃₀X₃₁SX₃₂GX₃₃X₃₄ (SEQ ID NO:103)
      wherein X₂₈ represents G or D,
      X₂₉ represents V or I,
      X₃₀ represents V, T, A, S, M, I, or L,
      X₃₁ represents F, M, L, I, or V,
      X₃₂ represents S, G, A, T, I, L, or H,
      X₃₃ represents R, M, or Q, and
      X₃₄ represents G, R, A, D, H, or K; and
   (h) X₃₅X₃₆RX₃₇X₃₈HMGX₃₉X₄₀A (SEQ ID NO:104)
      wherein X₃₅ represents L or V,
      X₃₆ represents T, I, V, or L,
      X₃₇ represents I, V, or L,
      X₃₈ represents G or S,
      X₃₉ represents P, A, or R, and
      X₄₀ represents T, V, or S.
<13> The recombinant microorganism according to any one of <1> to <12>, wherein the pyridoxamine synthetase is represented by enzyme number EC2.6.1.30.
<14> The recombinant microorganism according to any one of <1> to <13>, wherein the gene encoding the pyridoxamine synthetase is derived from *Mesorhizobium loti.*
<15> The recombinant microorganism according to any one of <1> to <14>, wherein the gene encoding the pyridoxamine synthetase has:
   a nucleotide sequence of any one of SEQ ID NO:9 or SEQ ID NO:60 to SEQ ID NO:66,
   a region between an 18th nucleotide and a 3' end in a nucleotide sequence of SEQ ID NO:14, or a region between an 18th nucleotide and a 3' end in a nucleotide sequence of any one of SEQ ID NO:82 to SEQ ID NO:88, or
   a nucleotide sequence that hybridizes with DNA having a nucleotide sequence complementary to a nucleotide sequence of any one of SEQ ID NO:9 or SEQ ID NO:60 to SEQ ID NO:66, or with DNA having a nucleotide sequence complementary to the region between an 18th nucleotide and a 3' end in the nucleotide sequence of SEQ ID NO:14, or a region between an 18th nucleotide and a 3' end in a nucleotide sequence of any one of SEQ ID NO:82 to SEQ ID NO:88, under a stringent condition, and that encodes a protein having an enzymatic activity of synthesizing pyridoxamine from pyridoxal.
<16> The recombinant microorganism according to any one of <1> to <15>, wherein the gene encoding the pyridoxamine synthetase has a nucleotide sequence of SEQ ID NO:9, or has a nucleotide sequence that hybridizes with DNA having a nucleotide sequence complementary to the nucleotide sequence of SEQ ID NO:9 under a stringent condition, and that encodes a protein having an enzymatic activity of synthesizing pyridoxamine from pyridoxal.
<17> The recombinant microorganism according to any one of <1> to <16>, wherein the gene encoding the pyridoxamine synthetase has a nucleotide sequence encoding an amino acid sequence of any one of SEQ ID NO:3 or SEQ ID NO:29 to SEQ ID NO:35, or an amino acid sequence that has 80% or more sequence identity with at least one amino acid sequence selected from the group consisting of SEQ ID NO:3 and SEQ ID NO:29 to SEQ ID NO:35, and that has an enzymatic activity of synthesizing pyridoxamine from pyridoxal.
<18> The recombinant microorganism according to any one of <1> to <17>, wherein the gene encoding the pyridoxamine synthetase has a nucleotide sequence encoding an amino acid sequence of SEQ ID NO:3, or an amino acid sequence that has 80% or more sequence identity with the amino acid sequence of SEQ ID NO:3, and that has an enzymatic activity of synthesizing pyridoxamine from pyridoxal.
<19> The recombinant microorganism according to any one of <12> to <18>, wherein the amino acid regeneration enzyme is an alanine dehydrogenase.
<20> The recombinant microorganism according to <19>, wherein the alanine dehydrogenase is enabled to use NADPH as a coenzyme in a reaction in which L-alanine is produced from pyruvic acid and NH₃.
<21> The recombinant microorganism according to <19> or <20>, wherein the alanine dehydrogenase includes at least one of the following partial amino acid sequence (i), partial amino acid sequence (j), partial amino acid sequence (k), or partial amino acid sequence (1), and has an activity of producing L-alanine from pyruvic acid and NH₃:
   (i) EX₄₁KX₄₂X₄₃EX₄₄RX₄₅X₄₆ (SEQ ID NO:105)
      wherein X₄₁ represents I, T, S, F, N, or V,
      X₄₂ represents N, M, A, V, L, T, or D,
      X₄₃ represents H, N, L, or Q,
      X₄₄ represents Y, N, or F,
      X₄₅ represents V or I, and
      X₄₆ represents G or A;
   (j) X₄₇X₄₈X₄₉KVKEPX₅₀ (SEQ ID NO:106)
      wherein X₄₇ represents M or L,
      X₄₈ represents I, L, or V,
      X₄₉ represents V, L, I, or M, and
      X₅₀ represents Q, L, V, N, or I;
   (k) LX₅₁TYLHLA (SEQ ID NO:107)
      wherein X₅₁ represents F or Y; and
   (1) X₅₂DX₅₃AX₅₄DQGG (SEQ ID NO:108)
      wherein X₅₂ represents V or A,
      X₅₃ represents V or I, and
      X₅₄ represents I or V.
<22> The recombinant microorganism according to any one of <19> to <21>, wherein the gene encoding the amino acid regeneration enzyme is derived from *Shewanella sp.* AC10.
<23> The recombinant microorganism according to any one of <19> to <22>, wherein the gene encoding the amino acid regeneration enzyme has:
   a nucleotide sequence of any one of SEQ ID NO:11 or SEQ ID NO:67 to SEQ ID NO:74,
   a region between an 18th nucleotide and a 3' end in a nucleotide sequence of any one of SEQ ID NO:15 or SEQ ID NO:89 to SEQ ID NO:96, or
   a nucleotide sequence that hybridizes with DNA having a nucleotide sequence complementary to a nucleotide sequence of any one of SEQ ID NO:11 or SEQ ID NO:67 to SEQ ID NO:74, or with DNA having a nucleotide sequence complementary to a region between an 18th nucleotide and a 3' end in a nucleotide sequence of any one of SEQ ID NO:15 or SEQ ID NO:89 to SEQ ID NO:96 under a stringent condition, and that encodes a protein having alanine regeneration enzymatic activity.
<24> The recombinant microorganism according to any one of <19> to <23>, wherein the gene encoding the amino acid regeneration enzyme has a nucleotide sequence of SEQ ID NO:11, or has a nucleotide sequence that hybridizes with DNA having a nucleotide sequence complementary to the nucleotide sequence of SEQ ID NO:11 under a stringent condition, and that encodes a protein having alanine regeneration enzymatic activity.
<25> The recombinant microorganism according to any one of <19> to <24>, wherein the gene encoding the amino acid regeneration enzyme has a nucleotide sequence encoding an amino acid sequence of any one of SEQ ID:NO 5 or SEQ ID NO:45 to SEQ ID NO:52, or an amino acid sequence that has 80% or more sequence identity with at least one amino acid sequence selected from the group consisting of SEQ ID NO:5 and SEQ ID NO:45 to SEQ ID NO:52, and that has alanine regeneration enzymatic activity.
<26> The recombinant microorganism according to any one of <19> to <25>, wherein the gene encoding the amino acid regeneration enzyme has a nucleotide sequence encoding an amino acid sequence of SEQ ID NO:5, or an amino acid sequence that has 80% or more sequence identity with the amino acid sequence of SEQ ID NO:5, and that has alanine regeneration enzymatic activity.
<27> The recombinant microorganism according to any one of <1> to <11>, wherein the pyridoxamine synthetase is represented by enzyme number EC2.6.1.31 or EC2.6.1.1.
<28> The recombinant microorganism according to any one of <1> to <11> or <27>, wherein the gene encoding the pyridoxamine synthetase is derived from *Escherichia coli.*
<29> The recombinant microorganism according to any one of <1> to <11>, <27>, or <28>, wherein the gene encoding the pyridoxamine synthetase has a nucleotide sequence of SEQ ID NO:10, or has a nucleotide sequence that hybridizes with DNA having a nucleotide sequence complementary to the nucleotide sequence of SEQ ID NO:10 under a stringent condition, and that encodes a protein having an enzymatic activity of synthesizing pyridoxamine from pyridoxal.
<30> The recombinant microorganism according to any one of <1> to <11>, or <27> to <29>, wherein the gene encoding the pyridoxamine synthetase has a nucleotide sequence encoding an amino acid sequence of SEQ ID NO:4, or an amino acid sequence that has 80% or more sequence identity with the amino acid sequence of SEQ ID NO:4, and that has an enzymatic activity of synthesizing pyridoxamine from pyridoxal.
<31> The recombinant microorganism according to any one of <27> to <30>, wherein the amino acid regeneration enzyme is a glutamate dehydrogenase.
<32> The recombinant microorganism according to <31>, wherein the gene encoding the amino acid regeneration enzyme is derived from *Escherichia coli.*
<33> The recombinant microorganism according to <31> or <32>, wherein the gene encoding the amino acid regeneration enzyme has a nucleotide sequence of SEQ ID NO:12, or has a nucleotide sequence that hybridizes with DNA having a nucleotide sequence complementary to the nucleotide sequence of SEQ ID NO:12 under a stringent condition, and that encodes a protein having glutamate regeneration enzymatic activity.
<34> The recombinant microorganism according to any one of <31> to <33>, wherein the gene encoding the amino acid regeneration enzyme has a nucleotide sequence encoding an amino acid sequence of SEQ ID NO:6, or an amino acid sequence that has 80% or more sequence identity with the amino acid sequence of SEQ ID NO:6, and that has glutamate regeneration enzymatic activity.
<35> The recombinant microorganism according to any one of <1> to <34>, the recombinant microorganism being recombinant *E. coli.*
<36> A method of producing pyridoxamine or a salt thereof, the method including bringing the recombinant microorganism according to any one of <1> to <35>, a culture of the recombinant microorganism, or a treated product of the recombinant microorganism or the culture, into contact with pyridoxine or a salt thereof to produce pyridoxamine or a salt thereof.
<37> The production method according to <36>, wherein the recombinant microorganism, the culture of the recombinant microorganism, or the treated product of the recombinant microorganism or the culture, includes the pyridoxine dehydrogenase, the pyridoxamine synthetase, and the amino acid regeneration enzyme.
<38> The production method according to <36> or <37>, wherein the treated product of the recombinant microorganism or the treated product of the culture of the recombinant microorganism is treated by treatment including one or more selected from the group consisting of heat treatment, cooling treatment, mechanical destruction of a cell, ultrasonic treatment, freeze-thaw treatment, drying treatment, pressurized or reduced pressure treatment, osmotic pressure treatment, cell autolysis, surfactant treatment, enzyme treatment, cell separation treatment, purification treatment, and extraction treatment.
   A second aspect of the disclosure includes the following.
<39> A recombinant microorganism, into which at least one polynucleotide selected from the group consisting of the following (1) to (7) is introduced:
   (1) a polynucleotide that encodes a protein having an amino acid sequence of SEQ ID NO:1;
   (2) a polynucleotide that encodes a protein having an amino acid sequence in which from 1 to 50 amino acids are deleted, added, or substituted in the amino acid sequence of SEQ ID NO:1, the protein having an activity of synthesizing pyridoxine or a salt thereof, or pyridoxal or a salt thereof;
   (3) a polynucleotide that encodes a protein having an amino acid sequence having 60% or more sequence identity with the amino acid sequence of SEQ ID NO:1, the protein having an activity of synthesizing pyridoxine or a salt thereof, or pyridoxal or a salt thereof;
   (4) a polynucleotide having a nucleotide sequence of SEQ ID NO:13;
   (5) a polynucleotide having a nucleotide sequence in which from 1 to 150 nucleotides are deleted, added, or substituted in the nucleotide sequence of SEQ ID NO:13, the polynucleotide encoding a protein having an activity of synthesizing pyridoxine or a salt thereof, or pyridoxal or a salt thereof;
   (6) a polynucleotide having a nucleotide sequence having 60% or more sequence identity with the nucleotide sequence of SEQ ID NO:13, the polynucleotide encoding a protein having an activity of synthesizing pyridoxine or a salt thereof, or pyridoxal or a salt thereof; and
   (7) a polynucleotide that hybridizes with a polynucleotide having a nucleotide sequence complementary to the nucleotide sequence of SEQ ID NO:13 under a stringent condition, the polynucleotide encoding a protein having an activity of synthesizing pyridoxine or a salt thereof, or pyridoxal or a salt thereof.
<40> The recombinant microorganism according to <39>, wherein the recombinant microorganism is a recombinant bacterium.
<41> The recombinant microorganism according to <40>, wherein the recombinant microorganism is recombinant *E. coli.*
<42> The recombinant microorganism according to any one of <39> to <41>, wherein the protein is an enzyme classified under EC number 1.1.1.65.
<43> The recombinant microorganism according to any one of <39> to <42>, wherein the protein is a pyridoxine dehydrogenase derived from *Saccharomyces cerevisiae.*
<44> A method of producing pyridoxal or a salt thereof, the method including bringing the recombinant microorganism according to any one of <39> to <43> into contact with pyridoxine or a salt thereof.
<45> A method of producing pyridoxal or a salt thereof, the method including bringing a culture obtained by culturing the recombinant microorganism according to any one of <39> to <43>, or a treated product of the culture, into contact with pyridoxine or a salt thereof.

### Advantageous Effects of Invention

In accordance with the first aspect of the disclosure, a recombinant microorganism capable of inexpensively producing pyridoxamine or a salt thereof from pyridoxine or a salt thereof at high production efficiency, and a method of inexpensively producing pyridoxamine or a salt thereof from pyridoxine or a salt thereof at high production efficiency using the recombinant microorganism are provided.

In accordance with the second aspect of the disclosure, a recombinant microorganism that includes and expresses an introduced pyridoxine dehydrogenase is provided.

### BRIEF DESCRIPTION OF DRAWINGS

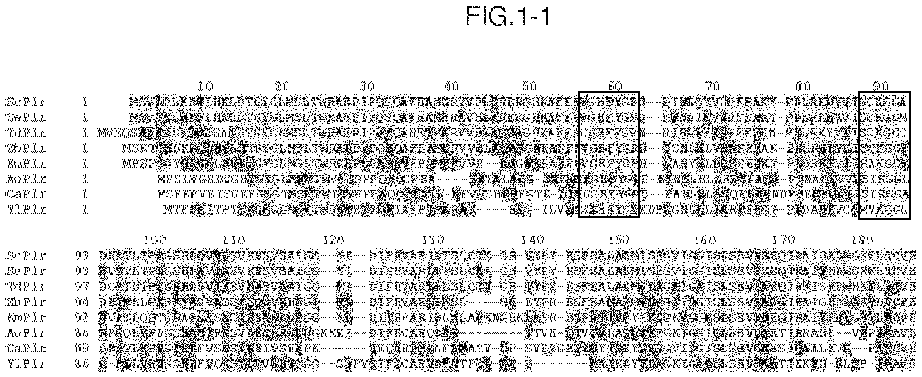

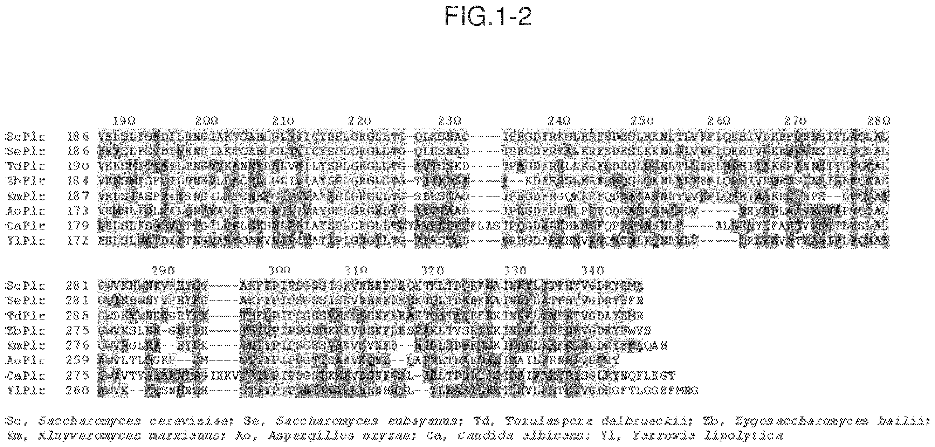

Fig. 1-1 illustrates the alignment of the sequences of SEQ ID NO:1 and SEQ ID NO:22 to SEQ ID NO:28.
Fig. 1-2 illustrates the alignment of the sequences of SEQ ID NO:1 and SEQ ID NO:22 to SEQ ID NO:28.
Fig. 2-1 illustrates the alignment of the sequences of SEQ ID NO:3 and SEQ ID NO:29 to SEQ ID NO:35.
Fig. 2-2 illustrates the alignment of the sequences of SEQ ID NO:3 and SEQ ID NO:29 to SEQ ID NO:35.
Fig. 3-1 illustrates the alignment of the sequences of SEQ ID NO:36 to SEQ ID NO:44.
Fig. 3-2 illustrates the alignment of the sequences of SEQ ID NO:36 to SEQ ID NO:44.

### DESCRIPTION OF EMBODIMENTS

A first aspect of the disclosure (hereinafter, simply referred to as "first aspect") is to provide a recombinant microorganism (hereinafter referred to as "recombinant microorganism according to first aspect") including a gene encoding a pyridoxine dehydrogenase, a gene encoding a pyridoxamine synthetase that has an enzymatic activity of synthesizing pyridoxamine from pyridoxal, and a gene encoding an amino acid regeneration enzyme that has an enzymatic activity of regenerating an amino acid consumed by the pyridoxamine synthetase,
wherein at least two of the gene encoding the pyridoxine dehydrogenase, the gene encoding the pyridoxamine synthetase, or the gene encoding the amino acid regeneration enzyme, are introduced from outside of a bacterial cell, or are originally endogenous to the bacterial cell and have an enhanced expression. A second aspect of the disclosure (hereinafter, simply referred to as "second aspect") is to provide a recombinant microorganism into which (1) a polynucleotide that encodes a protein having an amino acid sequence of SEQ ID NO:1, (2) a polynucleotide that encodes a protein having an amino acid sequence in which from 1 to 50 amino acids are deleted, added, or substituted in the amino acid sequence of SEQ ID NO:1, (3) a polynucleotide that encodes a protein having an amino acid sequence having 60% or more sequence identity with the amino acid sequence of SEQ ID NO:1, (4) a polynucleotide having the nucleotide sequence of SEQ ID NO:13, (5) a polynucleotide having a nucleotide sequence in which from 1 to 150 nucleotides are deleted, added, or substituted in the nucleotide sequence of SEQ ID NO:13, (6) a polynucleotide having a nucleotide sequence having 60% or more sequence identity with the nucleotide sequence of SEQ ID NO:13, or (7) a polynucleotide that hybridizes with a polynucleotide having a nucleotide sequence complementary to the nucleotide sequence of SEQ ID NO:13 under a stringent condition is introduced. A description of "the disclosure" is used so as to be intended to encompass the first aspect and the second aspect. In the disclosure, "introduction" refers to introduction that allows expression in a bacterial cell. The term "recombinant" is used for the same meaning as "gene recombination".

### <First Aspect>

Until now, it has not been known a method for inexpensively producing pyridoxamine or a salt thereof from pyridoxine or a salt thereof at high production efficiency either by a chemical method or a biological method. The structure of pyridoxamine is shown below.

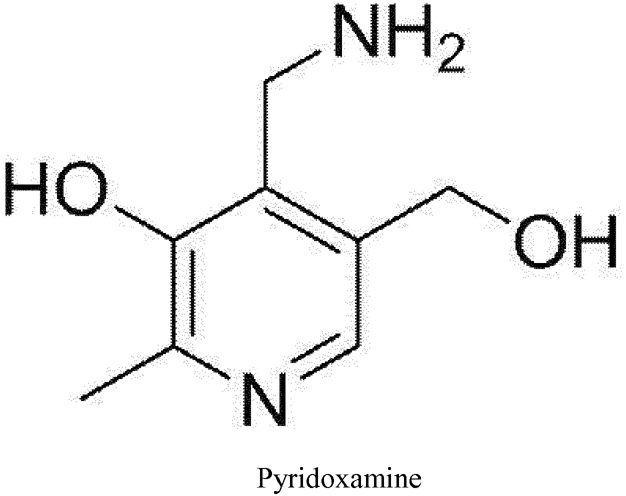

However, the present inventors have surprisingly found that pyridoxamine or a salt thereof can be inexpensively produced from pyridoxine or a salt thereof at high production efficiency by using a recombinant microorganism having above described constitution or a culture of the recombinant microorganism, or a treated product of the recombinant microorganism or the culture. Although the reason for this is not necessarily clear, it is assumed that in a case in which the above three kinds of enzymes exist in combination, due to the cooperative action of the above three kinds of enzymes, the equilibrium of each reaction in a process of producing pyridoxamine or a salt thereof from pyridoxine or a salt thereof and the amount of a by-product or the like produced in this process or the amount of a raw material or the like consumed in this process advantageously affect the production of pyridoxamine or a salt thereof from pyridoxine or a salt thereof. Further, it is assumed that since each of the at least two of the genes encoding the above three kinds of enzymes is introduced from outside of a bacterial cell, or is endogenous to a bacterial cell and has an enhanced expression, the enzymes can be expressed at a high expression level from the introduced or enhanced gene and the production efficiency of pyridoxamine or a salt thereof can be further improved.

Such a cooperative action of the above-described three kinds of enzymes, which has not been found so far, allows the production of pyridoxamine or a salt thereof with high production efficiency by using pyridoxine or a salt thereof, without the need to carry out a multi-step reaction as in a chemical synthesis method. Moreover, by using the recombinant microorganism according to the first aspect, it is possible to avoid by-production of large amounts of other substances included in the vitamin B₆ complex in. Pyridoxine is a raw material which is industrially available at a low cost compared to pyridoxal. By using pyridoxine as a starting material, pyridoxamine or a salt thereof can be inexpensively produced.

### <Pyridoxine Dehydrogenase>

The pyridoxine dehydrogenase is an enzyme also called pyridoxal reductase or pyridoxine-4-dehydrogenase. The pyridoxine dehydrogenase may be an enzyme represented by the enzyme number EC1.1.1.65 or may be an enzyme represented by other enzyme number. Pyridoxine dehydrogenase is an enzyme having an enzymatic activity that catalyzes a reaction of converting pyridoxal into pyridoxine by adding hydrogen, and a reaction reverse thereof. Although pyridoxal or pyridoxine may also exist as a salt depending on a surrounding environment; however, in the disclosure, a description of enzymatic activity is given with the omission of the mention of a salt in order to simplify an expression. Pyridoxine dehydrogenase consumes NADH (reduced nicotinamide adenine dinucleotide) or NADPH (reduced nicotinamide adenine dinucleotide phosphate) as coenzymes when hydrogen is added to pyridoxal; and consumes NAD+ (oxidized nicotinamide adenine dinucleotide) or NADP+ (oxidized nicotinamide adenine dinucleotide phosphate) as a coenzyme when hydrogen is abstracted from pyridoxine. Under general conditions, since the equilibrium of a reaction catalyzed by this enzyme is greatly shifted in favor of the pyridoxine generation, it has been considered that it is impossible to achieve a high yield when pyridoxine dehydrogenase is used in a reaction in which pyridoxine is consumed. In the first aspect of the disclosure, an unexpected effect of achieving inexpensive production of pyridoxamine or a salt thereof from pyridoxine or a salt thereof at high production efficiency can be obtained when pyridoxine dehydrogenase is used in combination with a specific enzyme(s) as described above.

The pyridoxine dehydrogenase is preferably derived from, for example, a microorganism belonging to the division *Ascomycota,* and may be derived from a microorganism belonging to the subdivision *Saccharomycotina* or the subdivision *Pezizomycotina.* More specifically, the pyridoxine dehydrogenase may be, for example, a pyridoxine dehydrogenase (having the amino acid sequence of SEQ ID NO:1) derived from *Saccharomyces cerevisiae,* a pyridoxine dehydrogenase (having the amino acid sequence of SEQ ID NO:22) derived from *Saccharomyces eubayanus,* a pyridoxine dehydrogenase (having the amino acid sequence of SEQ ID NO:23) derived from *Torulaspora delbrueckii,* a pyridoxine dehydrogenase (having the amino acid sequence of SEQ ID NO:24) derived from *Zygosaccharomyces bailii,* a pyridoxine dehydrogenase (having the amino acid sequence of SEQ ID NO:26) derived from *Kluyveromyces marxianus,* a pyridoxine dehydrogenase (having the amino acid sequence of SEQ ID NO:25) derived from *Aspergillus oryzae,* a pyridoxine dehydrogenase (having the amino acid sequence of SEQ ID NO:27) derived from *Candida albicans,* a pyridoxine dehydrogenase (having the amino acid sequence of SEQ ID NO:28) derived from *Yarrowia lipolytica,* or the like. Among these microorganisms, *Aspergillus oryzae* belongs to the subphylum *Pezizomycotina,* and the others belong to the subphylum *Saccharomycotina.*

The pyridoxine dehydrogenase of EC1.1.1.65 may be a pyridoxine dehydrogenase derived from, for example, *Saccharomyces cerevisiae, Schizosaccharomyces pombe,* or the like. Here, the pyridoxine dehydrogenase from *Saccharomyces cerevisiae* has the amino acid sequence of SEQ ID NO:1, and the pyridoxine dehydrogenase from *Schizosaccharomyces pombe* has the amino acid sequence of SEQ ID NO:2.

### <Pyridoxamine Synthetase>

The pyridoxamine synthetase used in the first aspect of the disclosure refers to any enzyme having an enzymatic activity of synthesizing pyridoxamine from pyridoxal. Pyridoxal or pyridoxamine may also exist as a salt depending on a surrounding environment; however, in the disclosure, a description of enzymatic activity is given with the omission of the mention of a salt in order to simplify an expression. Examples of the pyridoxamine synthetase include, for example, a pyridoxamine-pyruvate transaminase (such as an enzyme represented by the enzyme number EC 2.6.1.30), an aspartate transaminase (such as an enzyme represented by EC 2.6.1.1), a pyridoxamine-oxaloacetate transaminase (such as an enzyme represented by EC 2.6.1.31), and a pyridoxamine phosphate transaminase (such as an enzyme represented by EC 2.6.1.54).

The aspartate transaminase represented by EC 2.6.1.1 is a holoenzyme using pyridoxal phosphate as a coenzyme, and has an enzymatic activity of produce glutamic acid and oxaloacetic acid by transferring the amino group of aspartic acid to 2-oxoglutaric acid. The aspartate transaminase in a state of an apoenzyme not bound to pyridoxal phosphate is known synthesize pyridoxamine by transferring the amino group of glutamic acid or aspartic acid to pyridoxal (Journal of Biological Chemistry, January 1962, Vol. 237, No. 1, p. 127-132). In other words, an apoenzyme form of the aspartate transaminase represented by EC 2.6.1.1 is the pyridoxamine-oxaloacetate transaminase of EC 2.6.1.31. For this reason, aspartate transaminase exists as an apoenzyme when a coenzyme pyridoxal phosphate is absent or present in a small amount, and synthesizes pyridoxamine.

The pyridoxamine synthetase has an enzymatic activity of producing pyridoxamine or a salt thereof by oxidizing an amino group moiety of a specific amino acid to (=O) and transferring the amino group in the case of synthesizing pyridoxamine or a salt thereof from pyridoxal or a salt thereof. For example, the pyridoxamine-pyruvate transaminase can use both L-alanine and D-alanine, each of the pyridoxamine-oxaloacetate transaminase and the aspartate transaminase in an apoenzyme state can use D-aspartic acid, L-aspartic acid, D-glutamic acid, and L-glutamic acid, and the pyridoxamine phosphate transaminase can use D-glutamic acid.

The pyridoxamine-pyruvate transaminase may be derived from, for example, a microorganism belonging to the phylum *Proteobacteria,* a microorganism belonging to the phylum *Actinobacteria,* a microorganism belonging to the phylum *Spirocheta,* or a microorganism belonging to the phylum *Filmictes.* The pyridoxamine-pyruvate transaminase may be a pyridoxamine-pyruvate transaminase derived from, for example, *Mesorhizobium loti, Ochrobactrum anthropi,* or the genus *Pseudomonas* (such as *Pseudomonas* sp. MA-1). Here, the pyridoxamine-pyruvate transaminase from *Mesorhizobium loti* has the amino acid sequence of SEQ ID NO:3, for example

The pyridoxamine-pyruvate transaminase may also be, for example, a pyridoxamine-pyruvate transaminase (having the amino acid sequence of SEQ ID NO:29) derived from *Mesorhizobium sp.* YR577, a pyridoxamine-pyruvate transaminase (having the amino acid sequence of SEQ ID NO:30) derived from *Pseudaminobacter salicylatoxidans,* a pyridoxamine-pyruvate transaminase (having the amino acid sequence of SEQ ID NO:31) derived from *Bauldia litoralis,* a pyridoxamine-pyruvate transaminase (having the amino acid sequence of SEQ ID NO:32) derived from *Skermanella stibiiresistens,* a pyridoxamine-pyruvate transaminase (having the amino acid sequence of SEQ ID NO:33) derived from *Rhizobium* sp. AC44/96, a pyridoxamine-pyruvate transaminase (having the amino acid sequence of SEQ ID NO:34) derived from *Erwinia toletana,* or a pyridoxamine-pyruvate transaminase (having the amino acid sequence of SEQ ID NO:35) derived from *Herbiconiux ginsengi.*

The pyridoxamine-oxaloacetate transaminase may be, for example, a pyridoxamine-oxaloacetate transaminase derived from *Escherichia coli, Oryctolagus cuniculus,* or *Rattus norvegicus.* For example, the pyridoxamine-oxaloacetate transaminase from *Escherichia coli* has the amino acid sequence of SEQ ID NO:4. The aspartate transaminase may be an aspartate transaminase derived from, for example, *Escherichia coli, Trichoderma viride,* or the like. The pyridoxamine phosphate transaminase may be a pyridoxamine phosphate transaminase derived from, for example, *Clostridium butyricum.*

### <Amino Acid Regeneration Enzyme>

The amino acid regeneration enzyme used in the first aspect refers to any enzyme having an enzymatic activity of regenerating an amino acid consumed by the pyridoxamine synthetase. Pyridoxamine has an amino group, and an amino acid is considered to be consumed as a source providing an amino group in the case of synthesizing pyridoxamine. An amino acid may also exist as a salt depending on a surrounding environment; however, in the disclosure, a description of enzymatic activity is given with the omission of the mention of a salt in order to simplify an expression.

For example, in a case in which the pyridoxamine synthetase consumes L-alanine, an enzyme capable of regenerating L-alanine can be used as the amino acid regeneration enzyme. In a case in which the pyridoxamine synthetase consumes L-glutamic acid or L-aspartic acid, an enzyme capable of regenerating L-glutamic acid or L-aspartic acid can be used as the amino acid regeneration enzyme.

Examples of the amino acid regeneration enzymes include an alanine dehydrogenase (for example, an enzyme represented by enzyme number 1.4.1.1), a glutamate dehydrogenase (for example, an enzyme represented by enzyme number 1.4.1.2, 1.4.1.3, or 1.4.1.4), and a modified alanine dehydrogenase that is enabled to use NADP⁺/NADPH as a coenzyme by modifying an amino acid sequence. In the first aspect, an enzymatic activity of producing L-alanine from pyruvic acid and NH₃ using NADH or NADPH as a coenzyme may be referred to as "alanine regeneration enzymatic activity", and an enzymatic activity of producing L-glutamic acid from 2-oxoglutaric acid and NH₃ using NADH or NADPH as a coenzyme may be referred to as "glutamic acid regeneration enzymatic activity".

Examples of preferred combinations of the pyridoxamine synthetase and the amino acid regeneration enzyme include a combination of a pyridoxamine-pyruvate transaminase as the pyridoxamine synthetase and an alanine dehydrogenase as the amino acid regeneration enzyme, and a combination of a pyridoxamine-oxaloacetate transaminase or an aspartate transaminase as the pyridoxamine synthetase and a glutamate dehydrogenase as the amino acid regeneration enzyme. The amino acid regeneration enzyme preferably has an enzymatic activity of regenerating an amino acid consumed by the pyridoxamine synthetase encoded by a gene that is introduced from outside of a bacterial cell, or is endogenous to a bacterial cell and has an enhanced expression.

The amino acid regeneration enzyme may be, for example, an alanine dehydrogenase (having the amino acid sequence of SEQ ID NO:36) derived from *Shewanella sp.* Ac10, an alanine dehydrogenase (having the amino acid sequence of SEQ ID NO:37) derived from *Aeromonas hydrophila,* an alanine dehydrogenase (having the amino acid sequence of SEQ ID NO:38) derived from *Rhizobium sp.* LPU83, an alanine dehydrogenase (having the amino acid sequence of SEQ ID NO:39) derived from *Pseudomonas mendocina,* an alanine dehydrogenase (having the amino acid sequence of SEQ ID NO:40, or SEQ ID NO:41) derived from *Bradyrhizobium japonicum,* an alanine dehydrogenase (having the amino acid sequence of SEQ ID NO:42) derived from *Streptomyces aureofaciens,* an alanine dehydrogenase (having the amino acid sequence of SEQ ID NO:43) derived from *Anabaena cylindrica,* an alanine dehydrogenase (having the amino acid sequence of SEQ ID NO:44) derived from *Bacillus subtilis,* or the like. Alternatively, the amino acid regeneration enzyme may be, for example, a glutamate dehydrogenase derived from *Escherichia coli* or the like. It is preferable that the amino acid regeneration enzyme (for example, an alanine dehydrogenase or a glutamate dehydrogenase) can use NADPH (reduced nicotinamide adenine dinucleotide phosphate) as a coenzyme in a reaction in which an amino acid is regenerated.

For example, the alanine dehydrogenase is preferably an alanine dehydrogenase that can use NADPH as a coenzyme in a reaction in which L-alanine is produced from pyruvic acid and NH₃. In the case of using an amino acid regeneration enzyme that can use NADPH as a coenzyme, a NADPH/NADP+ ratio is varied due to a consumption of NADPH, the equilibrium and rate of a reaction catalyzed by a pyridoxine dehydrogenase are influenced, and the production efficiency of pyridoxamine or a salt thereof can be further enhanced. Most alanine dehydrogenases can use NADH (reduced nicotinamide adenine dinucleotide) as a coenzyme, but are incapable of using NADPH. In the case of using an alanine dehydrogenase that can use NADPH as a coenzyme, a NADPH/NADP⁺ ratio is varied due to a consumption of NADPH, the equilibrium and rate of a reaction catalyzed by a pyridoxine dehydrogenase are influenced, and the production efficiency of pyridoxamine or a salt thereof can be further enhanced, as described above. It is also preferable that the amino acid regeneration enzyme (for example, an alanine dehydrogenase or a glutamate dehydrogenase) can use both NADH (reduced nicotinamide adenine dinucleotide) and NADPH (reduced nicotinamide adenine dinucleotide phosphate) as coenzymes in a reaction in which an amino acid is regenerated. Examples of such alanine dehydrogenases include, but are not limited to, a protein having the amino acid sequence of any one of SEQ ID NO:5 or SEQ ID NO:45 to SEQ ID NO:52 described later.

Each of the pyridoxine dehydrogenase, the pyridoxamine synthetase, and the amino acid regeneration enzyme may be a protein that has a known amino acid sequence (such as an amino acid sequence encoded by a gene naturally occurring in an organism, or an amino acid sequence encoded by a gene possessed by a naturally occurring microorganism such as the above-described microorganism) having the enzymatic activity and that is unmodified, or may be a protein that has an amino acid sequence obtained by modifying such an amino acid sequence as long as the activity of the enzymatic activity (such as the above-described enzymatic activity) is not lost. Examples of the modification include insertion, deletion, substitution of an amino acid residue, and addition of an additional amino acid residue to either or both of the N-terminus and the C-terminus of an amino acid sequence. In a case in which there is one or more of the insertion, deletion, and substitution of the amino acid residue, the number of each of insertion, deletion, and substitution, if present, may be, for example, from 1 to 30 amino acid residues, from 1 to 20 amino acid residues, from 1 to 10 amino acid residues, or from 1 to 5 amino acid residues; and the total number of insertion, deletion, and substitution of the amino acid residue may be, for example, from 1 to 50 amino acid residues, from 1 to 30 amino acid residues, from 1 to 10 amino acid residues, or from 1 to 5 amino acid residues. The number of amino acid residue added to the terminus, if present, may be, for example, from 1 to 50 amino acid residues, from 1 to 30 amino acid residues, from 1 to 10 amino acid residues, or from 1 to 5 amino acid residues per one terminus. The additional amino acid residue may form a signal sequence for extracellular secretion or the like. Examples of the signal sequence include an *E. coli* OmpA signal sequence.

Alternatively, each of the enzymes may be a protein having a known amino acid sequence (such as an amino acid sequence encoded by a gene naturally occurring in an organism) having the enzymatic activity per se, or a protein having an amino acid sequence that has 80% or more, 85% or more, 90% or more, or 95% or more sequence identity with a known amino acid sequence (such as an amino acid sequence encoded by a gene naturally occurring in an organism) having the enzymatic activity and that has a desired enzymatic activity (such as the above-mentioned enzymatic activity). Here, the sequence identity can be evaluated using, for example, a BLAST (registered trademark, National Library of Medicine) program with default parameters.

For example, the pyridoxine dehydrogenase may be, for example, a protein having the amino acid sequence of SEQ ID NO:1 or SEQ ID NO:2, or may be a protein having an amino acid sequence in which one or more of substitution, deletion, or insertion of an amino acid residue, or addition of an additional amino acid residue to either or both of the N-terminus and C-terminus of the amino acid sequence are performed in the amino acid sequence of SEQ ID NO:1 or SEQ ID NO:2. Examples of the degree of the substitution, deletion, and insertion of an amino acid residue, and the addition of an additional amino acid residue to either or both of the N-terminus and the C-terminus of the amino acid sequence are as described above.

Alternatively, the pyridoxine dehydrogenase may be a protein having the amino acid sequence of SEQ ID NO:1 or SEQ ID NO:2, or may be a protein having an amino acid sequence that has, for example, 80% or more, 85% or more, 90% or more, or 95% or more sequence identity with the amino acid sequence of SEQ ID NO:1 or SEQ ID NO:2.

In a case of using such a protein having an amino acid sequence similar to at least one of the amino acid sequence of SEQ ID NO:1 or SEQ ID NO:2 as described above, it is necessary that the protein has an activity for a pyridoxine dehydrogenase. The pyridoxine dehydrogenase activity can be measured by, for example, adding a protein to be tested to an aqueous solution including pyridoxine as a substrate and a necessary NAD⁺ or NADP⁺, and quantifying the amount of produced pyridoxal by high performance liquid chromatography, or forming a Schiff base between produced pyridoxal and an amine such as trishydroxymethylaminomethane and quantifying the amount of the Schiff base with absorbance measurement at 415 nm or the like.

Alternatively, the pyridoxine dehydrogenase may be a protein having the amino acid sequence of any one of SEQ ID NO:1, SEQ ID NO:2 or SEQ ID NO:22 to SEQ ID NO:28, or a protein having an amino acid sequence that has, for example, 80% or more, 85% or more, 90% or more, or 95% or more sequence identity with at least one of the amino acid sequence of SEQ ID NO:1, the amino acid sequence of SEQ ID NO:2, the amino acid sequence of SEQ ID NO:22, the amino acid sequence of SEQ ID NO:23, the amino acid sequence of SEQ ID NO:24, the amino acid sequence of SEQ ID NO:25, the amino acid sequence of SEQ ID NO:26, the amino acid sequence of SEQ ID NO:27, or the amino acid sequence of SEQ ID NO:28. It is necessary that the protein has an activity for a pyridoxine dehydrogenase.

Alternatively, the pyridoxine dehydrogenase may be a pyridoxine dehydrogenase including at least one of the following partial amino acid sequence (a) or partial amino acid sequence (b), and having pyridoxine dehydrogenase activity:
(a) NX₁X₂EX₃YG (SEQ ID NO:97)
   wherein X₁ represents V, C, I, A, M, S, G, or L,
   X₂ represents G or A, and
   X₃ represents F or L; and
(b) X₄X₅X₆KGX₇ (SEQ ID NO:98)
   wherein X₄ represents I, V, F, or L,
   X₅ represents S, T, N, C, or M,
   X₆ represents C, V, A, I, W, or F, and
   X₇ represents G, A, S, or C. A protein having an amino acid sequence is also acceptable.

Fig. 1-1 and Fig. 1-2 illustrate the alignment of the sequences of SEQ ID NO:1 and SEQ ID NO:22 to SEQ ID NO:28. In Fig. 1-1 and Fig. 1-2, ScPlr represents a pyridoxine dehydrogenase from *Saccharomyces cerevisiae,* SePlr represents a pyridoxine dehydrogenase from *Saccharomyces eubayanus,* TdPlr represents a pyridoxine dehydrogenase from *Torulaspora delbrueckii,* ZbPlr represents a pyridoxine dehydrogenase from *Zygosaccharomyces bailii,* KmPlr represents a pyridoxine dehydrogenase from *Kluyveromyces marxianus,* AoPlr represents a pyridoxine dehydrogenase from *Aspergillus oryzae,* CaPlr represents a pyridoxine dehydrogenase from *Candida albicans,* and YlPlr represents a pyridoxine dehydrogenase from *Yarrowia lipolytica.* The partial amino acid sequence (a) corresponds to amino acid residues corresponding to the 55th to 61st amino acid residues from the N-terminus of the pyridoxine dehydrogenase from *Saccharomyces cerevisiae* on the alignment, and the partial amino acid sequence (b) corresponds to amino acid residues corresponding to the 86th to 91st amino acid residues from the N-terminus of the pyridoxine dehydrogenase from *Saccharomyces cerevisiae* on the alignment. In the disclosure, the alignment of sequences can be performed using, for example, a BLAST (registered trademark, National Library of Medicine) program with default parameters. The partial amino acid sequence (a) preferably exists in the region of the 45th to 71st amino acid residues from the N-terminus of the protein, and more preferably exists in the region of the 46th to 66th amino acid residues from the N-terminus. The partial amino acid sequence (b) preferably exists in the region of the 76th to 101st amino acid residues from the N-terminus of the protein, and more preferably exists in the region of the 79th to 96th amino acid residues from the N-terminus.

In the disclosure, "amino acid residue corresponding to Xth amino acid residue from N-terminus of enzyme A" in the amino acid sequence of enzyme B refers to an amino acid residue on the amino acid sequence of the enzyme B, corresponding to the Xth amino acid residue from the N-terminus of the amino acid sequence of the enzyme A, in a case in which the amino acid sequence of the enzyme A is aligned with the amino acid sequence of the enzyme B.

As is clear from Fig. 1-1 and Fig. 1-2, the partial amino acid sequence (a) and the partial amino acid sequence (b) are regions highly conserved in a group of pyridoxine dehydrogenases. Accordingly, a variation of the pyridoxine dehydrogenase including at least one of the partial amino acid sequence (a) or the partial amino acid sequence (b) is considered to be highly probable to achieve functioning as the pyridoxine dehydrogenase in the disclosure. Both an amino acid residue corresponding to the 60th tyrosyl residue from the N-terminus of the pyridoxine dehydrogenase from *Saccharomyces cerevisiae* on the alignment and an amino acid residue corresponding to the 89th lysine residue from the N-terminus of the pyridoxine dehydrogenase from *Saccharomyces cerevisiae* on the alignment are considered to be NADPH-binding residues (Journal of Biological Chemistry 1990, 274(33), 23185-23190), and are important residues in view of the function of the pyridoxine dehydrogenase. In a case in which these residues are conserved, it is considered to be highly probable to achieve functioning as the pyridoxine dehydrogenase in the disclosure.

Examples of other expressions of a region including the amino acid residue corresponding to the 60th tyrosyl residue from the N-terminus of the pyridoxine dehydrogenase from *Saccharomyces cerevisiae* on the alignment include the following partial amino acid sequence (a-1). Instead of the partial amino acid sequence (a), the pyridoxine dehydrogenase may include the partial amino acid sequence (a-1):
(a-1) X₁X₂NX₃X₄EX₅YGX₆X₇ (SEQ ID NO:109)
wherein X₁ represents F, L, I, M, Y, or V,
X₂ represents F, I, Y, L, W, or V,
X₃ represents V, C, I, A, M, S, G, or L,
X₄ represents G or A,
X₅ represents F or L,
X₆ represents P, K, R, G, E, T, A, N, or S, and
X₇ represents D, N, H, K, E, P, L, or I.

The partial amino acid sequence (a-1) corresponds to amino acid residues corresponding to the 53rd to 63rd amino acid residues from the N-terminus of the pyridoxine dehydrogenase from *Saccharomyces cerevisiae* on the alignment. The partial amino acid sequence (a-1) preferably exists in the region of the 43rd to 73rd amino acid residues from the N-terminus of the protein, and more preferably exists in the region of the 45th to 68th amino acid residues from the N-terminus.

Examples of other expressions of a region including the amino acid residue corresponding to the 89th lysine residue from the N-terminus of the pyridoxine dehydrogenase from *Saccharomyces cerevisiae* on the alignment include the following partial amino acid sequence (b-1). Instead of the partial amino acid sequence (b), the pyridoxine dehydrogenase may include the partial amino acid sequence (b-1):
(b-1) X₁X₂X₃X₄X₅X₆X₇X₈KGX₉ (SEQ ID NO:110)
wherein X₁ represents R, K, A, S, or N,
X₂ represents K, S, E, Q, D, or A,
X₃ represents D, H, N, Y, E, K, C, Q, or R,
X₄ represents V, T, I, M, or L,
X₅ represents V, I, L, F, M, or T,
X₆ represents I, V, F, or L,
X₇ represents S, T, N, C, or M,
X₈ represents C, V, A, I, W, or F, and
X₉ represents G, A, S, or C.

The partial amino acid sequence (b-1) corresponds to amino acid residues corresponding to the 81st to 91st amino acid residues from the N-terminus of the pyridoxine dehydrogenase from *Saccharomyces cerevisiae* on the alignment. The partial amino acid sequence (a-1) preferably exists in the region of the 71st to 101st amino acid residues from the N-terminus of the protein, and more preferably exists in the region of the 74th to 96th amino acid residues from the N-terminus.

The pyridoxamine synthetase may be, for example, a protein having the amino acid sequence of SEQ ID NO:3, or a protein having an amino acid sequence in which one or more of substitution, deletion, or insertion of an amino acid residue, or addition of an additional amino acid residue to either or both of the N-terminus and C-terminus of the amino acid sequence are performed in the amino acid sequence of SEQ ID NO:3. Examples of the degrees of the substitution, deletion, and insertion of the amino acid residue, and the addition of an additional amino acid residue to either or both of the N-terminus and C-terminus of the amino acid sequence are as described above.

Alternatively, the pyridoxamine synthetase may be a protein having the amino acid sequence of SEQ ID NO:3, or may be a protein having an amino acid sequence having, for example, 80% or more, 85% or more, 90% or more, or 95% or more sequence identity with the amino acid sequence of SEQ ID NO:3.

In the case of using such a protein having an amino acid sequence similar to the amino acid sequence of SEQ ID NO:3 as described above, it is necessary that the protein has an activity for a pyridoxamine synthetase (an enzymatic activity of synthesizing pyridoxamine from pyridoxal, also referred to as pyridoxamine synthetase activity in the first aspect). The enzymatic activity of synthesizing pyridoxamine from pyridoxal can be measured by, for example, adding a protein to be tested to an aqueous solution including pyridoxal as a substrate, and a necessary amino acid (for example, L-alanine in the case of a protein having an amino acid sequence similar to the amino acid sequence of SEQ ID NO:3), and quantifying the amount of produced pyridoxamine by high performance liquid chromatography or the like.

Alternatively, the pyridoxamine synthetase may be a protein having an amino acid sequence of any one of SEQ ID NO:3 or SEQ ID NO:29 to SEQ ID NO:35, or may be a protein having an amino acid sequence having, for example, 80% or more, 85% or more, 90% or more, or 95% or more sequence identity with at least one of the amino acid sequence of SEQ ID NO:3, the amino acid sequence of SEQ ID NO:29, the amino acid sequence of SEQ ID NO:30, the amino acid sequence of SEQ ID NO:31, the amino acid sequence of SEQ ID NO:32, the amino acid sequence of SEQ ID NO:33, the amino acid sequence of SEQ ID NO:34, or the among amino acid sequence of SEQ ID NO:35. It is necessary that the protein also has an activity for a pyridoxamine synthetase. In this case, the enzymatic activity of synthesizing pyridoxamine from pyridoxal can be measured by, for example, adding a protein to be tested to an aqueous solution including pyridoxal as a substrate, and L-alanine, and quantifying the amount of produced pyridoxamine by high performance liquid chromatography or the like.

Alternatively, the pyridoxamine synthetase may be a pyridoxamine synthetase including at least one of the following partial amino acid sequence (c), partial amino acid sequence (d), partial amino acid sequence (e), partial amino acid sequence (f), partial amino acid sequence (g), or partial amino acid sequence (h), and having an enzymatic activity of synthesizing pyridoxamine from pyridoxal. In this case, the enzymatic activity of synthesizing pyridoxamine from pyridoxal can be measured by, for example, adding a protein to be tested to an aqueous solution including pyridoxal as a substrate, and L-alanine, and quantifying the amount of produced pyridoxamine by high performance liquid chromatography or the like.
(c) X₈X₉X₁₀X₁₁X₁₂X₁₃ (SEQ ID NO:99)
   wherein X₈ represents L, M, I, or V,
   X₉ represents H or Q,
   X₁₀ represents G, C, or A,
   X₁₁ represents E or D,
   X₁₂ represents P or A, and
   X₁₃ represents V, I, L, or A;
(d) X₁₄X₁₅TPSGTX₁₆X₁₇ (SEQ ID NO:100)
   wherein X₁₄ represents H or S,
   X₁₅ represents D or E,
   X₁₆ represents I, V, or L, and
   X₁₇ represents N or T;
(e) X₁₈DX₁₉VSX₂₀X₂₁ (SEQ ID NO:101)
   wherein X₁₈ represents V, I, or A,
   X₁₉ represents A, T, or S,
   X₂₀ represents S, A, or G, and
   X₂₁ represents F, W, or V;
(f) X₂₂X₂₃X₂₄KCX₂₅GX₂₆X₂₇P (SEQ ID NO:102)
   wherein X₂₂ represents G or S,
   X₂₃ represents P, S, or A,
   X₂₄ represents N, G, S, A, or Q,
   X₂₅ represents L or M,
   X₂₆ represents A, S, C, or G, and
   X₂₇ represents P, T, S, or A;
(g) X₂₈X₂₉X₃₀X₃₁SX₃₂GX₃₃X₃₄ (SEQ ID NO:103)
   wherein X₂₈ represents G or D,
   X₂₉ represents V or I,
   X₃₀ represents V, T, A, S, M, I or L,
   X₃₁ represents F, M, L, I, or V,
   X₃₂ represents S, G, A, T, I, L, or H,
   X₃₃ represents R, M, or Q, and
   X₃₄ represents G, R, A, D, H, or K;
(h) X₃₅X₃₆RX₃₇X₃₈HMGX₃₉X₄₀A (SEQ ID NO:104)
   wherein X₃₅ represents L or V,
   X₃₆ represents T, I, V, or L,
   X₃₇ represents I, V, or L,
   X₃₈ represents G or S,
   X₃₉ represents P, A, or R, and
   X₄₀ represents T, V, or S.

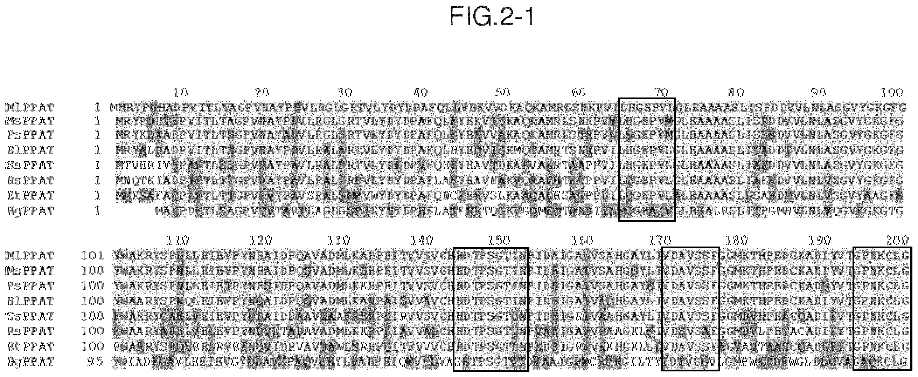

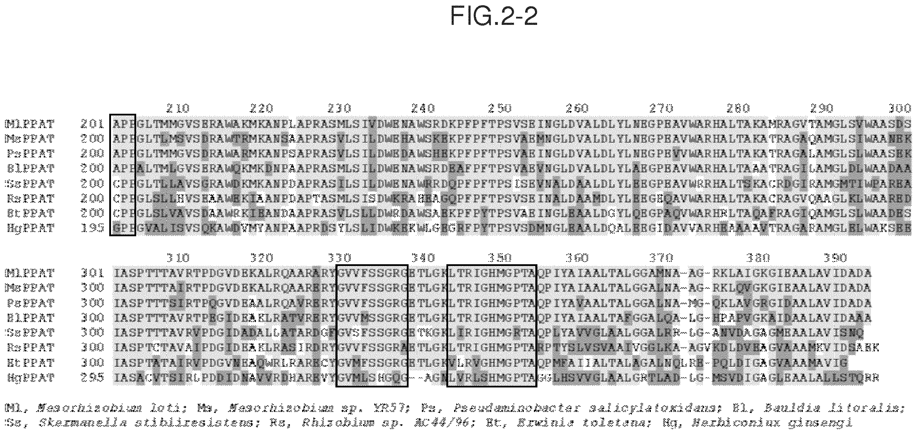

Fig. 2-1 and Fig. 2-2 illustrate the alignment of the sequences of SEQ ID NO:3 and SEQ ID NO:29 to SEQ ID NO:35. In Fig. 2-1 and Fig. 2-2, MlPPAT represents a pyridoxamine-pyruvate transaminase from *Mesorhizobium loti,* MsPPAT represents a pyridoxamine-pyruvate transaminase from *Mesorhizobium sp.* YR577, PsPPAT represents a pyridoxamine-pyruvate transaminase from *Pseudaminobacter salicylatoxidans,* BlPPAT represents a pyridoxamine-pyruvate transaminase from *Bauldia litoralis,* SsPPAT represents a pyridoxamine-pyruvate transaminase from *Skermanella stibiiresistens,* RsPPAT represents a pyridoxamine-pyruvate transaminase from *Rhizobium sp.* AC44/96, EtPPAT represents a pyridoxamine-pyruvate transaminase from *Erwinia toletana,* and HgPPAT represents a pyridoxamine-pyruvate transaminase from *Herbiconiux ginsengi.* The partial amino acid sequence (c) corresponds to amino acid residues corresponding to the 65th to 70th amino acid residues from the N-terminus of the pyridoxamine-pyruvate transaminase from *Mesorhizobium loti* on the alignment, the partial amino acid sequence (d) corresponds to amino acid residues corresponding to the 144th to 152nd amino acid residues from the N-terminus of the pyridoxamine-pyruvate transaminase from *Mesorhizobium loti* on the alignment, the partial amino acid sequence (e) corresponds to amino acid residues corresponding to the 170th to 176th amino acid residues from the N-terminus of the pyridoxamine-pyruvate transaminase from *Mesorhizobium loti* on the alignment, the partial amino acid sequence (f) corresponds to amino acid residues corresponding to the 194th to 203rd amino acid residues from the N-terminus of the pyridoxamine-pyruvate transaminase from *Mesorhizobium loti* on the alignment, the partial amino acid sequence (g) corresponds to amino acid residues corresponding to the 329th to 337th amino acid residues from the N-terminus of the pyridoxamine-pyruvate transaminase from *Mesorhizobium loti* on the alignment, and the partial amino acid sequence (h) corresponds to amino acid residues corresponding to the 343rd to 353rd amino acid residues from the N-terminus of the pyridoxamine-pyruvate transaminase from *Mesorhizobium loti* on the alignment. The partial amino acid sequence (c) preferably exists in the region of the 55th to 80th amino acid residues from the N-terminus of the protein, and more preferably exists in the region of the 56th to 75th amino acid residues from the N-terminus. The partial amino acid sequence (d) preferably exists in the region of the 134th to 162nd amino acid residues from the N-terminus of the protein, and more preferably exists in the region of the 139th to 157th amino acid residues from the N-terminus. The partial amino acid sequence (e) preferably exists in the region of the 160th to 186th amino acid residues from the N-terminus of the protein, and more preferably exists in the region of the 165th to 181st amino acid residues from the N-terminus. The partial amino acid sequence (f) preferably exists in the region of the 184th to 213th amino acid residues from the N-terminus of the protein, and more preferably exists in the region of the 189th to 208th amino acid residues from the N-terminus. The partial amino acid sequence (g) preferably exists in the region of the 319th to 347th amino acid residues from the N-terminus of the protein, and more preferably exists in the region of the 324th to 342nd amino acid residues from the N-terminus. The partial amino acid sequence (h) preferably exists in the region of the 333rd to 363rd amino acid residues from the N-terminus of the protein, and more preferably exists in the region of the 338th to 358th amino acid residues from the N-terminus.

As is clear from Fig. 2-1 and Fig. 2-2, the partial amino acid sequence (c), the partial amino acid sequence (d), the partial amino acid sequence (e), the partial amino acid sequence (f), the partial amino acid sequence (g), and the partial amino acid sequence (h) are regions highly conserved in a group of pyridoxamine-pyruvate transaminases. Accordingly, a variation of the pyridoxamine-pyruvate transaminase including at least one of the partial amino acid sequence (c), the partial amino acid sequence (d), the partial amino acid sequence (e), the partial amino acid sequence (f), the partial amino acid sequence (g), or the partial amino acid sequence (h) is considered to have a high probability of functioning as the pyridoxamine synthetase in the disclosure. It is considered that an amino acid residue corresponding to the 197th lysine residue from the N-terminus of the pyridoxamine-pyruvate transaminase from *Mesorhizobium loti* on the alignment is important for binding to pyridoxal, an amino acid residue corresponding to the 68th glutamate residue from the N-terminus on the alignment is important for catalytic activity, an amino acid residue corresponding to the 171st aspartate residue from the N-terminus on the alignment and an amino acid residue corresponding to the 146th threonine residue from the N-terminus on the alignment assist binding to pyridoxal, and an amino acid residue corresponding to the 336th arginine residue from the N-terminus on the alignment and an amino acid residue corresponding to the 345th arginine residue from the N-terminus on the alignment are important for recognizing an amino acid (Journal of Biological Chemistry, 2008, vol. 283, No. 2 pp1120-1127), and the amino acid residues are important residues in view of the function of the pyridoxine synthetase. In a case in which these residues are conserved, it is considered to be highly probable to achieve functioning as the pyridoxine synthetase in the disclosure. However, an amino acid residue corresponding to the 68th glutamate residue from the N-terminus of the pyridoxamine-pyruvate transaminase from *Mesorhizobium loti* on the alignment may be not only glutamate but also aspartate. An amino acid residue corresponding to the 336th arginine residue from the N-terminus of the pyridoxamine-pyruvate transaminase from *Mesorhizobium loti* on the alignment may be not only arginine but also methionine or glutamine.

Examples of other expressions of a region including the amino acid residue corresponding to the 68th glutamate residue from the N-terminus of the pyridoxamine-pyruvate transaminase from *Mesorhizobium loti* on the alignment include the following partial amino acid sequence (c-1). Instead of the partial amino acid sequence (c), the pyridoxamine synthetase may include the partial amino acid sequence (c-1).
(c-1) X₁X₂X₃X₄X₅X₆X₇X₈X₉X₁₀X₁₁X₁₂X₁₃X₁₄X₁₅X₁₆X₁₇X₁₈X₁₉ (SEQ ID NO:111)
wherein X₁ represents V, L, I, or M,
X₂ represents I, L, or V,
X₃ represents L, M, I, or V,
X₄ represents H or Q,
X₅ represents G, C, or A,
X₆ represents E or D,
X₇ represents P or A,
X₈ represents V, I, A, or L,
X₉ represents L, M, P, or V,
X₁₀ represents G or A,
X₁₁ represents L or I,
X₁₂ represents E or Q,
X₁₃ represents A or G,
X₁₄ represents A or V,
X₁₅ represents A or L,
X₁₆ represents A, L, H, or Y,
X₁₇ represents S, G, or A,
X₁₈ represents L, F, V, or A, and
X₁₉ represents I, F, V, or L.

The partial amino acid sequence (c-1) corresponds to amino acid residues corresponding to the 63rd to 81st amino acid residues from the N-terminus of the pyridoxamine-pyruvate transaminase from *Mesorhizobium loti* on the alignment. The partial amino acid sequence (c-1) preferably exists in the region of the 53rd to 91st amino acid residues from the N-terminus of a protein, and more preferably exists in the region of the 58th to 86th amino acid residues from the N-terminus.

Examples of other expressions of a region including the amino acid residue corresponding to the 146th threonine residue from the N-terminus of a pyridoxamine-pyruvate transaminase from *Mesorhizobium loti* on the alignment include the following partial amino acid sequence (d-1). Instead of the partial amino acid sequence (d), the pyridoxamine synthetase may include the partial amino acid sequence (d-1).
(d-1) X₁X₂X₃X₄X₅X₆X₇X₈TPSGTX₉X₁₀X₁₁X₁₂X₁₃X₁₄X₁₅X₁₆ (SEQ ID NO:112)
wherein X₁ represents V, I, L, or M,
X₂ represents V or I,
X₃ represents S, A, V, C, or F,
X₄ represents V, I, A, L, or T,
X₅ represents C or V,
X₆ represents H, N, or A,
X₇ represents H or S,
X₈ represents D or E,
X₉ represents I, V, or L,
X₁₀ represents N or T,
X₁₁ represents P or D,
X₁₂ represents I, V, L, or A,
X₁₃ represents D, N, E, A, G, Q, V, R, or P,
X₁₄ represents A, E, Q, or D,
X₁₅ represents I or L, and
X₁₆ represents G or A.

The partial amino acid sequence (d-1) corresponds to the amino acid residues corresponding to the 138th to 158th amino acid residues from the N-terminus of the pyridoxamine-pyruvate transaminase from *Mesorhizobium loti* on the alignment. The partial amino acid sequence (d-1) preferably exists in the region of the 128th to 168th amino acid residues from the N-terminus of a protein, and more preferably exists in the region of the 133rd to 163rd amino acid residues from the N-terminus.

Examples of other expressions of a region including the amino acid residue corresponding to the 171st aspartate residue from the N-terminus of a pyridoxamine-pyruvate transaminase from *Mesorhizobium loti* include the following partial amino acid sequence (e-1). Instead of the partial amino acid sequence (e), the pyridoxamine synthetase may include the partial amino acid sequence (e-1).
(e-1) X₁X₂X₃X₄X₅X₆DX₇VSX₈X₉X₁₀X₁₁X₁₂ (SEQ ID NO:113)
wherein X₁ represents G, D, or A,
X₂ represents A, G, K, T, Q, R, or E,
X₃ represents Y, N, L, or F,
X₄ represents L, F, M, or V,
X₅ represents I, L, or Y,
X₆ represents V, A, or I,
X₇ represents A, S, or T,
X₈ represents S, A, or G,
X₉ represents F, W, or V,
X₁₀ represents G, A, or L,
X₁₁ represents G or S, and
X₁₂ represents M, V, or L.

The partial amino acid sequence (e-1) corresponds to the amino acid residues corresponding to the 165th to 179th amino acid residues from the N-terminus of the pyridoxamine-pyruvate transaminase from *Mesorhizobium loti* on the alignment. The partial amino acid sequence (e-1) preferably exists in the region of the 155th to 189th amino acid residues from the N-terminus of the protein, and more preferably exists in the region of the 160th to 184th amino acid residues from the N-terminus.

Examples of other expressions of a region including the amino acid residue corresponding to the 197th lysine residue from the N-terminus of a pyridoxamine-pyruvate transaminase from *Mesorhizobium loti* on the alignment include the following partial amino acid sequence (f-1). Instead of the partial amino acid sequence (f), the pyridoxamine synthetase may include the partial amino acid sequence (f-1).
(f-1) X₁X₂X₃X₄X₅X₆X₇X₈X₉KCX₁₀GX₁₁X₁₂PX₁₃X₁₄X₁₅X₁₆X₁₇X₁₈X₁₉S (SEQ ID NO:114)
wherein X₁ represents A, S, V, or I,
X₂ represents D, G, or A,
X₃ represents I, L, F, V, or M,
X₄ represents Y, F, L, or C,
X₅ represents V or I,
X₆ represents T or A,
X₇ represents G or S,
X₈ represents P, S, or A,
X₉ represents N, G, S, Q, or A,
X₁₀ represents L or M,
X₁₁ represents A, S, C, or G,
X₁₂ represents P, T, S, or A,
X₁₃ represents G, A, or S,
X₁₄ represents L or V,
X₁₅ represents T, S, or A,
X₁₆ represents M, I, L, V, or F,
X₁₇ represents M, L, V, A, or I,
X₁₈ represents G, A, H, or S, and
X₁₉ represents V, I, or A.

The partial amino acid sequence (f-1) corresponds to the amino acid residues corresponding to the 188th to 211st amino acid residues from the N-terminus of the pyridoxamine-pyruvate transaminase of *Mesorhizobium loti* on the alignment. The partial amino acid sequence (f-1) preferably exists in the region of from the 178th to 221st amino acid residues from the N-terminus of the protein, and more preferably exists in the region of from the 183rd to 216th amino acid residues from the N-terminus.

Examples of other expressions of a region including the amino acid residue corresponding to the 336th arginine residue from the N-terminus of the pyridoxamine-pyruvate transaminase from *Mesorhizobium loti* on the alignment include the following partial amino acid sequence (g-1). Instead of the partial amino acid sequence (g), the pyridoxamine synthetase may include the partial amino acid sequence (g-1).
(g-1) X₁X₂X₃X₄X₅SX₆GX₇X₈ (SEQ ID NO:115)
wherein X₁ represents Y, F, H, or S,
X₂ represents G or D,
X₃ represents V or I,
X₄ represents V, T, A, S, M, I, or L,
X₅ represents F, M, L, I, or V,
X₆ represents S, G, A, T, I, L, or H,
X₇ represents R, M, or Q, and
X₈ represents G, R, A, D, H, or K.

The partial amino acid sequence (g-1) corresponds to the amino acid residues corresponding to the 328th to 337th amino acid residues from the N-terminus of the pyridoxamine-pyruvate transaminase from *Mesorhizobium loti* on the alignment. The partial amino acid sequence (g-1) preferably exists in the region of the 318th to 347th amino acid residues from the N-terminus of the protein, and more preferably exists in the region of the 323rd to 342nd amino acid residues from the N-terminus.

Examples of other expressions of a region including the amino acid residue corresponding to the 345th arginine residue from the N-terminus of the pyridoxamine-pyruvate transaminase from *Mesorhizobium loti* on the alignment include the following partial amino acid sequence (h-1). Instead of the partial amino acid sequence (h), the pyridoxamine synthetase may include the partial amino acid sequence (h-1).
(h-1) X₁X₂X₃X₄X₅RX₆X₇HMGX₈X₉AX₁₀X₁₁ (SEQ ID NO:116)
wherein X₁ represents L, Q, K, A, F, Y, or W,
X₂ represents G, N, H, or D,
X₃ represents K, R, or N,
X₄ represents L or V,
X₅ represents T, I, V, or L,
X₆ represents I, V, or L,
X₇ represents G or S,
X₈ represents P, A, or R,
X₉ represents T, V, or S,
X₁₀ represents Q, R, E, K, H, Y, or G, and
X₁₁ represents P or G.

The partial amino acid sequence (h-1) corresponds to the amino acid residues corresponding to the 340th to 355th amino acid residues from the N-terminus of the pyridoxamine-pyruvate transaminase from *Mesorhizobium loti* on the alignment. The partial amino acid sequence (h-1) preferably exists in the region of the 330th to 365th amino acid residues from the N-terminus of a protein, and more preferably exists in the region of the 335th to 360th amino acid residues from the N-terminus.

The pyridoxamine synthetase may be, for example, a protein having the amino acid sequence of SEQ ID NO:4, or may be a protein having an amino acid sequence in which one or more of substitution, deletion, or insertion of an amino acid residue, or addition of an additional amino acid residue to either or both of the N-terminus and C-terminus of the amino acid sequence are performed in the amino acid sequence of SEQ ID NO:4. Examples of the degrees of the substitution, deletion, and insertion of the amino acid residue, and the addition of an additional amino acid residue to either or both of the N-terminus and the C-terminus of the amino acid sequence are as described above.

Alternatively, the pyridoxamine synthetase may be a protein having the amino acid sequence of SEQ ID NO:4, or may be a protein having an amino acid sequence having, for example, 80% or more, 85% or more, 90% or more, or 95% or more sequence identity with the amino acid sequence of SEQ ID NO:4.

In the case of using such a protein having an amino acid sequence similar to the amino acid sequence of SEQ ID NO:4 as described above, it is necessary that the protein has an activity for pyridoxamine synthetase (an enzymatic activity of synthesizing pyridoxamine from pyridoxal). The enzymatic activity of synthesizing pyridoxamine from pyridoxal can be measured by, for example, adding a protein to be tested to an aqueous solution including pyridoxal as a substrate, and necessary amino acid (for example, L-glutamic acid, L-aspartic acid, or a salt thereof in the case of a protein having an amino acid sequence similar to the amino acid sequence of SEQ ID NO:4), and quantifying the amount of produced pyridoxamine by high performance liquid chromatography or the like.

As the amino acid regeneration enzyme, for example, a protein having an amino acid sequence (SEQ ID NO:5) in which an amino acid of D198A is substituted in the amino acid sequence (SEQ ID NO:36) of an amino acid regeneration enzyme derived from *Shewanella sp.* AC10 can be used. The protein is an alanine dehydrogenase represented by enzyme number 1.4.1.1. Not only such an alanine dehydrogenase but also an alanine dehydrogenase in which an amino acid substitution to use NADP⁺ is introduced in the alanine dehydrogenase represented by the enzyme number 1.4.1.1 can be used. In most alanine dehydrogenases, it is possible to use NAD⁺ (oxidized nicotinamide adenine dinucleotide) as a coenzyme, but it is impossible to use NADP⁺ (oxidized nicotinamide adenine dinucleotide phosphate), as described in Journal of Molecular Catalysis B: Enzymatic 30 (2004) 173-176. However, both NAD⁺ and NADP⁺ can be used as coenzymes in the protein having the amino acid sequence of SEQ ID NO:5, in which the amino acid of D198A is substituted (see the document described above). Here, the protein having the amino acid sequence of SEQ ID NO:5 can use both NADH and NADPH in reverse reaction (regeneration of L-alanine from pyruvic acid and NH₃).

Similar amino acid substitution can be performed in the alanine dehydrogenases other than the alanine dehydrogenase derived from *Shewanella sp.* AC10. Herein, substitution of an alanine residue for a residue corresponding to the 198th aspartate residue from the N-terminus of the amino acid sequence of SEQ ID NO:36 on alignment in the amino acid sequences of an alanine dehydrogenase is referred to as "amino acid substitution to use NADP⁺". The alanine dehydrogenase as the amino acid regeneration enzyme may be a protein having an amino acid sequence (SEQ ID NO:45) obtained by amino acid substitution to use NADP⁺ (D198A in this case) of an alanine dehydrogenase derived from *Aeromonas hydrophila,* a protein having an amino acid sequence (SEQ ID NO:46) obtained by amino acid substitution to use NADP⁺ (D198 A in this case) of an alanine dehydrogenase derived from *Rhizobium sp.* LPU83, a protein having an amino acid sequence (SEQ ID NO:47) obtained by amino acid substitution to use NADP⁺ (D198A in this case) of an alanine dehydrogenase derived from *Pseudomonas mendocina,* a protein having an amino acid sequence (SEQ ID NO:48 or SEQ ID NO:49) obtained by amino acid substitution to use NADP⁺ (D198A in this case) of an alanine dehydrogenase derived from *Bradyrhizobium japonicum,* a protein having an amino acid sequence (SEQ ID NO:50) obtained by amino acid substitution to use NADP⁺ (D198A in this case) of an alanine dehydrogenase derived from *Streptomyces aureofaciens,* a protein having an amino acid sequence (SEQ ID NO:51) obtained by amino acid substitution to use NADP⁺ (D198A in this case) of an alanine dehydrogenase derived from *Anabaena cylindrica,* a protein having an amino acid sequence (SEQ ID NO:52) obtained by amino acid substitution to use NADP⁺ (D196A in this case) of an alanine dehydrogenase derived from *Bacillus subtilis,* or the like.

Under reaction conditions in which NAD⁺ is available, the amino acid regeneration enzyme can be used without amino acid substitution to use NADP⁺, and, in addition, the alanine dehydrogenase represented by the enzyme number 1.4.1.1 can be commonly used.

The amino acid regeneration enzyme may be, for example, a glutamate dehydrogenase having the amino acid sequence of SEQ ID NO:6 and derived from *E. coli.* The glutamate dehydrogenase has an enzymatic activity of producing L-glutamic acid from 2-oxoglutaric acid and NH₃ using NADH or NADPH as a coenzyme.

The amino acid regeneration enzyme may be, for example, a protein having the amino acid sequence of SEQ ID NO:5, or a protein having an amino acid sequence in which one or more of substitution, deletion, or insertion of an amino acid residue, or addition of an additional amino acid residue to either or both of the N-terminus and C-terminus of the amino acid sequence are performed in the amino acid sequence of SEQ ID NO:5 Examples of the degrees of the substitution, deletion, and insertion of the amino acid residue, and the addition of an additional amino acid residue to either or both of the N-terminus and C-terminus of the amino acid sequence are as described above.

Alternatively, the amino acid regeneration enzyme may be a protein having the amino acid sequence of SEQ ID NO:5, or may be a protein having an amino acid sequence having, for example, 80% or more, 85% or more, 90% or more, 95% or more sequence identity with the amino acid sequence of SEQ ID NO:5.

In the case of using such a protein having an amino acid sequence similar to the amino acid sequence of SEQ ID NO:5 as described above, it is necessary that the protein has alanine regeneration enzymatic activity. It is preferable to have the 198th Ala residue from the N-terminus of the amino acid sequence of SEQ ID NO:5 or an Ala residue corresponding thereto on sequence alignment.

Alternatively, the amino acid regeneration enzyme may be a protein having an amino acid sequence of any one of SEQ ID NO:5 or SEQ ID NO:45 to SEQ ID NO:52, or may be a protein having an amino acid sequence having, for example, 80% or more, 85% or more, 90% or more, or 95% or more sequence identity with the amino acid sequence of SEQ ID NO:5, SEQ ID NO:45, SEQ ID NO:46, SEQ ID NO:47, SEQ ID NO:48, SEQ ID NO:49, SEQ ID NO:50, SEQ ID NO:51, or SEQ ID NO:52.

In the case of using such a protein having an amino acid sequence similar to the amino acid sequence of SEQ ID NO:5, SEQ ID NO:45, SEQ ID NO:46, SEQ ID NO:47, SEQ ID NO:48, SEQ ID NO:49, SEQ ID NO:50, SEQ ID NO:51, or SEQ ID NO:52 as described above, it is necessary that the protein has alanine regeneration enzymatic activity. It is preferable to have the 198th Ala residue from the N-terminus of the amino acid sequence of SEQ ID NO:5 or an Ala residue corresponding thereto on sequence alignment.

Alternatively, the amino acid regeneration enzyme may be, for example, a protein having the amino acid sequence of SEQ ID NO:6, or a protein having an amino acid sequence in which one or more of substitution, deletion, or insertion of an amino acid residue in the amino acid sequence of SEQ ID NO:6, or addition of an additional amino acid residue to either or both of the N-terminus and C-terminus of the amino acid sequence are performed. Examples of the degrees of the substitution, deletion, and insertion of the amino acid residue, and the addition of an additional amino acid residue to either or both of the N-terminus and C-terminus of the amino acid sequence are as described above.

Alternatively, the amino acid regeneration enzyme may be a protein having the amino acid sequence of SEQ ID NO:6, or may be a protein having an amino acid sequence having, for example, 80% or more, 85% or more, 90% or more, or 95% or more sequence identity with the amino acid sequence of SEQ ID NO:6.

In the case of using such a protein having an amino acid sequence similar to the amino acid sequence of SEQ ID NO:6 as described above, it is necessary that the protein has glutamic acid regeneration enzymatic activity.

An amino acid regeneration enzymatic activity can be measured by, for example, adding a protein to be tested to an aqueous solution including a necessary substrate and NADP or NADPH, if necessary, and quantifying the amount of desired amino acid as a reaction product by high performance liquid chromatography or the like. For example, the alanine regeneration enzymatic activity can be measured by, for example, adding a protein to be tested to an aqueous solution including pyruvic acid, NH₃, and NADH or NADPH, and quantifying the amount of L-alanine as a reaction product by high performance liquid chromatography or the like. The glutamic acid regeneration enzymatic activity can be measured by, for example, adding a protein to be tested to an aqueous solution including 2-oxoglutaric acid, NH₃, and NADH or NADPH, and quantifying the amount of L-glutamic acid as a reaction product by high performance liquid chromatography or the like.

Alternatively, the alanine dehydrogenase may be an enzyme including at least one of a partial amino acid sequence (i), a partial amino acid sequence (j), a partial amino acid sequence (k), or a partial amino acid sequence (1), and having an activity of producing L-alanine from pyruvic acid and NH₃. In such an alanine dehydrogenase, substitution of an alanine residue for an amino acid residue corresponding to the 198th aspartate residue from the N-terminus of *Shewanella sp.* AC10 on alignment can enable both of NAD⁺ and NADP⁺ to be used as coenzymes.
(i) EX₄₁KX₄₂X₄₃EX₄₄RX₄₅X₄₆ (SEQ ID NO:105)
   wherein X₄₁ represents I, T, S, F, N, or V,
   X₄₂ represents N, M, A, V, L, T, or D,
   X₄₃ represents H, N, L, or Q,
   X₄₄ represents Y, N, or F,
   X₄₅ represents V or I, and
   X₄₆ represents G or A;
(j) X₄₇X₄₈X₄₉KVKEPX₅₀ (SEQ ID NO:106)
   wherein X₄₇ represents M or L,
   X₄₈ represents I, L, or V,
   X₄₉ represents V, L, I, or M, and
   X₅₀ represents Q, L, V, N, or I;
(k) LX₅₁TYLHLA (SEQ ID NO:107)
   wherein X₅₁ represents F or Y; and
(1) X₅₂DX₅₃AX₅₄DQGG (SEQ ID NO:108)
   wherein X₅₂ represents V or A,
   X₅₃ represents V or I, and
   X₅₄ represents I or V.

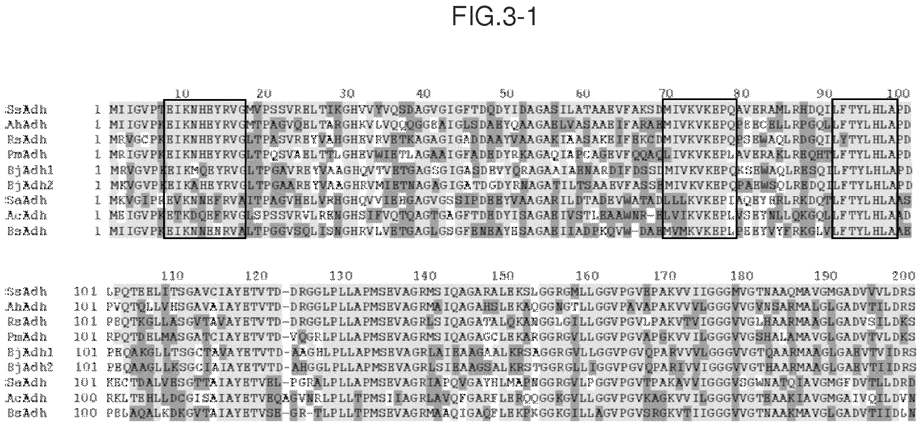

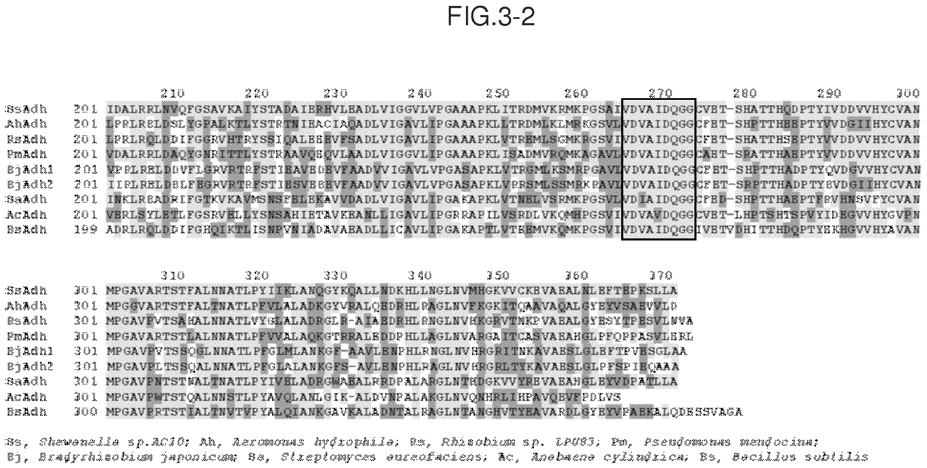

Fig. 3-1 and Fig. 3 -2 illustrate the alignment of the sequences of SEQ ID NO:36 to SEQ ID NO:44. In Fig. 3-1 and Fig. 3-2, SsAdh represents an alanine dehydrogenase from *Shewanella sp.* AC10, AhAdh represents an alanine dehydrogenase from *Aeromonas hydrophila,* RsAdh represents an alanine dehydrogenase from *Rhizobium sp.* LPU83, PmAdh represents an alanine dehydrogenase from *Pseudomonas mendocina,* BjAdh1 represents one of alanine dehydrogenases from *Bradyrhizobium japonicum,* BjAdh2 represents another alanine dehydrogenase from *Bradyrhizobium japonicum,* SaAdh represents an alanine dehydrogenase from *Streptomyces aureofaciens,* AcAdh represents an alanine dehydrogenase from *Anabaena cylindrica,* and BsAdh represents an alanine dehydrogenase from *Bacillus subtilis.* The partial amino acid sequence (i) corresponds to amino acid residues corresponding to the 8hth to 17th amino acid residues from the N-terminus of the alanine dehydrogenase from *Shewanella sp.* AC10 on the alignment, the partial amino acid sequence (j) corresponds to amino acid residues corresponding to the 70th to 78th amino acid residues from the N-terminus of the alanine dehydrogenase from *Shewanella sp.* AC10 on the alignment, the partial amino acid sequence (k) corresponds to amino acid residues corresponding to the 91st to 98th amino acid residues from the N-terminus of the alanine dehydrogenase from *Shewanella sp.* AC10 on the alignment, and the partial amino acid sequence (1) corresponds to amino acid residues corresponding to the 265th to 273rd amino acid residues from the N-terminus of the alanine dehydrogenase from *Shewanella sp.* AC10 on the alignment. The partial amino acid sequence (i) preferably exists in the region of the 2nd to 27th amino acid residues from the N-terminus of the protein, and more preferably exists in the region of the 3rd to 22nd amino acid residues from the N-terminus. The partial amino acid sequence (j) preferably exists in the region of the 60th to 88th amino acid residues from the N-terminus of the protein, and more preferably exists in the region of the 65th to 83rd amino acid residues from the N-terminus. The partial amino acid sequence (k) preferably exists in the region of the 81st to 108th amino acid residues from the N-terminus of the protein, and more preferably exists in the region in the 86th to 103rd amino acid residues from the N-terminus. The partial amino acid sequence (1) preferably exists in the region of the 255th to 283rd amino acid residues from the N-terminus of the protein, and more preferably exists in the region of the 260th to 278th amino acid residues from the N-terminus.

As is clear from Fig. 3-1 and Fig. 3-2, the partial amino acid sequence (i), the partial amino acid sequence (j), the partial amino acid sequence (k), and the partial amino acid sequence (1) are regions highly conserved in a group of alanine dehydrogenases. Accordingly, a variation of the alanine dehydrogenase including at least one of the partial amino acid sequence (i), the partial amino acid sequence (j), the partial amino acid sequence (k),or the partial amino acid sequence (1) is considered to have a high probability of functioning as the amino acid regeneration enzyme in the disclosure. An amino acid residue corresponding to the 15th arginine residue from the N-terminus of the alanine dehydrogenase from *Shewanella sp.* AC 10 on the alignment and an amino acid residue corresponding to the 75th lysine residue from the N-terminus on the alignment are important for binding to NADH, and amino acid residues corresponding to the 96th histidine residue from the N-terminus and the 270th aspartate residue from the N-terminus on the alignment are considered to be important for forming the structure of a protein (J. Mol. Biol., 2008, 377, 1161-1173, Enzyme and Microbial Technology, 2018, 110, 61-68), and are important residues in view of the function of the alanine dehydrogenase. In a case in which these residues are conserved, it is considered to be highly probable to achieve functioning as the amino acid regeneration enzyme in the disclosure.

Examples of other expressions of a region including an amino acid residue corresponding to the 15th arginine residue from the N-terminus of the alanine dehydrogenase from *Shewanella sp.* AC 10 on the alignment include the following partial amino acid sequence (i-1). The alanine dehydrogenase may include the partial amino acid sequence (i-1) instead of the partial amino acid sequence (i).
(i-1) GX₁PX₂EX₃KX₄X₅EX₆RX₇X₈X₉X₁₀PX₁₁X₁₂X₁₃X₁₄X₁₅X₁₆ (SEQ ID NO:117)
wherein X₁ represents V, I, L, or C,
X₂ represents T, K, or R,
X₃ represents I, V, T, S, F, or N,
X₄ represents N, M, A, D, V, L, or T,
X₅ represents H, N, Q, or L,
X₆ represents Y, N, or F,
X₇ represents V or I,
X₈ represents G or A,
X₉ represents M, L, or I,
X₁₀ represents V, T, I, or S,
X₁₁ represents S, A, T, Q, H, N, L, or G,
X₁₂ represents S, A, N, G, or V,
X₁₃ represents V or A,
X₁₄ represents R, K, N, Q, S, A, L, or H,
X₁₅ represents E, Q, D, V, or A, and
X₁₆ represents L, A, V, F, or Y.

The partial amino acid sequence (i-1) corresponds to amino acid residues corresponding to the 4th to 26th amino acid residues from the N-terminus of the alanine dehydrogenase from *Shewanella sp.* AC10 on the alignment. The partial amino acid sequence (i-1) preferably exists in the region of the 2nd to 36th amino acid residues from the N-terminus of the protein, and more preferably exists in the region of the 3d to 31st amino acid residues from the N-terminus.

Examples of other expressions of a region including an amino acid residue corresponding to the 75th lysine residue from the N-terminus of the alanine dehydrogenase from *Shewanella sp.* AC 10 on the alignment include the following partial amino acid sequence (j-1). The alanine dehydrogenase may include the partial amino acid sequence (j-1) instead of the partial amino acid sequence (j).
(j-1) X₁X₂X₃X₄KVKEPX₅X₆X₇EX₈X₉X₁₀ (SEQ ID NO:118)
wherein X₁ represents D, E, Q, or K,
X₂ represents M or L,
X₃ represents I, L, or V,
X₄ represents V, L, I, or M,
X₅ represents Q, L, I, V, or N,
X₆ represents A, T, S, P, M, K, R, Q, V, I, or E,
X₇ represents V, I, E, N, Q, T, A, D, H, M, N, V, A, S, I, D, Q W, or K,
X₈ represents R, C, Y, or W,
X₉ represents A, E, R, Q, K, T, S, M, N, V, P, G, C, or H, and
X₁₀ represents M, L, K, R, Q, E, W, F, or Y.

The partial amino acid sequence (j-1) corresponds to amino acid residues corresponding to the 69th to 84th amino acid residues from the N-terminus of the alanine dehydrogenase from *Shewanella sp.* AC10 on the alignment. The partial amino acid sequence (j-1) preferably exists in the region of the 59th to 94th amino acid residues from the N-terminus of the protein, and more preferably exists in the region of the 64th to 89th amino acid residues from the N-terminus.

Examples of other expressions of a region including an amino acid residue corresponding to the 96th histidine residue from the N-terminus of the alanine dehydrogenase from *Shewanella sp.* AC 10 on the alignment include the following partial amino acid sequence (k-1). The alanine dehydrogenase may include the partial amino acid sequence (k-1) instead of the partial amino acid sequence (k).
(k-1) X₁X₂X₃X₄X₅X₆LX₇TYLHLAX₈X₉X₁₀X₁₁X₁₂X₁₃X₁₄X₁₅LX₁₆X₁₇X₁₈X₁₉ (SEQ ID NO:119)
wherein X₁ represents L or F,
X₂ represents R, K, C, S, H, G, or Q,
X₃ represents H, E, P, S, D, R, K, Q, or A,
X₄ represents D, G, Q, H, E, S, N, or M,
X₅ represents Q or H,
X₆ represents I, L, V, T, C, or A,
X₇ represents F or Y,
X₈ represents P or A,
X₉ represents D, S, N, H, or E,
X₁₀ represents L, M, P, R, V, Q, E, or K,
X₁₁ represents P, A, V, Q, K, E, D, T, N, R, S, or M,
X₁₂ represents Q, C, or L,
X₁₃ represents T, A, or V,
X₁₄ represents E, I, Q, A, R, K, T, D, or N,
X₁₅ represents E, D, L, A, G, H, Y, or S,
X₁₆ represents I, M, V, L, T, or K,
X₁₇ represents T, K, S, D, H, E, A, N, R, G, or Q,
X₁₈ represents S, G, C, A, or K, and
X₁₉ represents G, K, R, Q, or D.

The partial amino acid sequence (k-1) corresponds to amino acid residues corresponding to the 85th to 111th amino acid residues from the N-terminus of the alanine dehydrogenase from *Shewanella sp.* AC10 on the alignment. The partial amino acid sequence (k-1) preferably exists in the region of the 75th to 121st amino acid residues from the N-terminus of the protein, and more preferably exists in the region of the 80th to 116th amino acid residues from the N-terminus.

Examples of other expressions of a region including an amino acid residue corresponding to the 270th aspartate residue from the N-terminus of the alanine dehydrogenase from *Shewanella sp.* AC10 on the alignment include the following partial amino acid sequence (1-1). The alanine dehydrogenase may include the partial amino acid sequence (1-1) instead of the partial amino acid sequence (1).
(1-1) X₁X₂X₃X₄X₅DX₆AX₇DQGGX₈X₉X₁₀X₁₁ (SEQ ID NO:120)
wherein X₁ represents G, R, or S,
X₂ represents S, A, or G,
X₃ represents A or V,
X₄ represents I, L, M, or V,
X₅ represents V or A,
X₆ represents V or I,
X₇ represents I or V,
X₈ represents C or I,
X₉ represents V, I, A, F, S, or C,
X₁₀ represents E or A, and
X₁₁ represents T or D.

The partial amino acid sequence (1-1) corresponds to amino acid residues corresponding to the 261st to 277th amino acid residues from the N-terminus of the alanine dehydrogenase from *Shewanella sp.* AC10 on the alignment. The partial amino acid sequence (1-1) preferably exists in the region of the 251st to 287th amino acid residues from the N-terminus of the protein, and more preferably exists in the region of the 256th to 282nd amino acid residues from the N-terminus.

### <Gene Encoding Pyridoxine Dehydrogenase, Gene Encoding Pyridoxamine Synthetase, and Gene Encoding Amino Acid Regeneration Enzyme>

The gene encoding the pyridoxine dehydrogenase may be any gene encoding the pyridoxine dehydrogenase described above. The gene encoding the pyridoxamine synthetase may be any gene encoding the pyridoxamine synthetase described above. The gene encoding the amino acid regeneration enzyme may be any gene encoding the amino acid regeneration enzyme described above. The enzymes encoded by these genes are not limited to known amino acid sequences having the enzymatic activities (for example, an amino acid sequence encoded by a gene naturally occurring in an organism), and may be enzymes having modified amino acid sequences different from the known amino acid sequences.

Such a gene may be a known gene such as a gene naturally possessed by the microorganism exemplified above as the microorganism (microorganism from which the enzyme is derived) having each enzyme, or a gene in which a nucleotide sequence is modified so that the nucleotide sequence encodes a modified amino acid sequence that is modified from the known amino acid sequence of the enzyme as described above as long as the desired enzymatic activity is obtained. Examples of the modified amino acid sequence include an amino acid sequence similar to any one of the amino acid sequences of SEQ ID NO:1 to SEQ ID NO:6 as described above. The nucleotide sequence of a gene encoding a particular amino acid sequence can be varied within degeneracy of a codon. In this case, it is preferable to use a codon frequently used in a microorganism used as a host of the recombinant microorganism from the viewpoint of gene expression efficiency.

It is also possible to design a nucleotide sequence of a gene based on an amino acid sequence to be encoded using the codon table. The designed nucleotide sequence may be obtained by modifying a known nucleotide sequence using genetic recombination technology, or may be obtained by chemically synthesizing the nucleotide sequence.

Examples of the method of modifying a nucleotide sequence include site-directed mutagenesis (Kramer, W. and frita, H.J., Methods in Enzymology, vol.154, P.350 (1987)), recombinant PCR (PCR Technology, Stockton Press (1989)), a method of chemically synthesizing a specific part of DNA, a method of treating a gene with hydroxyamine, a method of treating a strain carrying a gene with ultraviolet irradiation or a chemical agent such as nitrosoguanidine or nitrous acid, and a method of using a commercially available mutagenesis kit.

For example, each of the gene encoding the pyridoxine dehydrogenase, the gene encoding the pyridoxamine synthetase, and the gene encoding the amino acid regeneration enzyme may be DNA having an unmodified nucleotide sequence that encodes a polynucleotide having the enzymatic activity (for example, a nucleotide sequence possessed by a gene naturally occurring in an organism such as the above-described microorganism), or DNA having a nucleotide sequence in which a modification is made in the above nucleotide sequence as long as the enzymatic activity (the above-described enzymatic activity) of an enzyme encoded by the nucleotide sequence is not lost. Examples of the modification include insertion, deletion, and substitution of a nucleotide, and addition of an additional nucleotide to either or both of the 5' end and 3' end of the nucleotide sequence. In a case in which there is one or more of the insertion, deletion, and substitution of the nucleotide, the number of each of the insertion, deletion, and substitution, if present, may be, for example, from 1 to 90 nucleotides, from 1 to 60 nucleotides, from 1 to 30 amino acid residues, from 1 to 20 amino acid residues, from 1 to 15 nucleotides, from 1 to 10 nucleotides, or from 1 to 5 nucleotides; and the total number of the insertion, deletion, and substitution of the nucleotide may be, for example from 1 to 100 nucleotides, from 1 to 50 nucleotides, from 1 to 30 nucleotides, from 1 to 10 nucleotides, or from 1 to 5 nucleotides. Insertion or deletion of a nucleotide may occur locally, and it is preferable that the entire nucleotide sequence does not have a large frame shift. The number of nucleotide added to the end if present, may be, for example, from 1 to 150 nucleotides, from 1 to 100 nucleotides, from 1 to 50 nucleotides, from 1 to 30 nucleotides, from 1 to 10 nucleotides, or from 1 to 5 nucleotides per end. Such an additional nucleotide may encode a signal sequence for extracellular secretion or the like.

Alternatively, the gene encoding each of the enzymes may be DNA having a known nucleotide sequence (for example, a nucleotide sequence of a gene naturally occurring in an organism) that encodes a polynucleotide having the enzymatic activity per se, or DNA that has a nucleotide sequence having 80% or more, 85% or more, 90% or more, or 95% or more sequence identity with the known nucleotide sequence and that encodes an enzyme having a desired enzymatic activity (such as the above-mentioned enzymatic activity). Here, the sequence identity can be evaluated by using, for example, a BLAST (registered trademark, National Library of Medicine) program with default parameters.

Alternatively, the gene encoding each of the enzymes may be DNA having a known nucleotide sequence (for example, a nucleotide sequence of a gene naturally occurring in an organism) that encodes a polynucleotide having the enzymatic activity per se, or DNA having a nucleotide sequence that hybridizes with DNA having a nucleotide sequence complementary to the known nucleotide sequence under a stringent condition, and that encodes an enzyme having a desired enzymatic activity (such as the above-described enzymatic activity). Hybridization under a stringent condition can be performed as follows.

The hybridization is performed on DNA of interest using DNA consisting of a nucleotide sequence complementary to a reference nucleotide sequence or a partial sequence thereof as a probe. After washing the resultant under a stringent condition, the significance of the hybridization of the probe to the nucleic acid of interest is confirmed. The length of the probe can be, for example, 20 or more continuous nucleotides, preferably 50 or more nucleotides, more preferably 100 or more nucleotides, and further preferably 200 or more nucleotides. It is also preferable to use DNA as a probe that has the same nucleotide length as the reference nucleotide sequence and that is complementary to the reference nucleotide over the entire length. Examples of the conditions for hybridization may include conditions commonly used by those skilled in the art for detecting a specific hybridization signal. Such conditions preferably mean stringent hybridization conditions and stringent wash conditions. Examples thereof include conditions that a temperature of 55°C is maintained overnight, together with a probe, in a solution including 6 × SSC (saline sodium citrate) (composition of 1 × SSC: 0.15 M NaCl, 0.015 M sodium citrate, pH 7.0), 0.5% SDS, 5 × Denhardt, and 100 mg/ mL herring sperm DNA. Examples thereof may include subsequent washing of a filter in 0.2 × SSC at 42°C. Examples of the stringent conditions may include conditions of 0.1 × SSC and 50°C in the step of washing a filter, and, in addition, examples of the stringent conditions can include 0.1 × SSC and 65°C in the same step.

For example, the gene encoding the pyridoxine dehydrogenase may be, for example, DNA having the nucleotide sequence of SEQ ID NO:7 (the nucleotide sequence of a gene encoding a pyridoxine dehydrogenase from *Saccharomyces cerevisiae*), or DNA having a nucleotide sequence that hybridizes with DNA having a nucleotide sequence complementary to the nucleotide sequence of SEQ ID NO:7 under a stringent condition, and that encodes a protein having pyridoxine dehydrogenase activity.

Alternatively, the gene encoding the pyridoxine dehydrogenase may be, for example, DNA having the nucleotide sequence of SEQ ID NO:7, or DNA having a nucleotide sequence in which one or more of the substitution, deletion, or insertion of a nucleotide, or the addition of an additional nucleotide to either or both of the 5' end and 3' end of the nucleotide sequence are performed in the nucleotide sequence of SEQ ID NO:7, and which encodes a protein having pyridoxine dehydrogenase activity. Examples of the degrees of the substitution, deletion, and insertion of a nucleotide, and the addition of an additional nucleotide to either or both of the N-terminus and C-terminus of the nucleotide sequence are as described above.

Alternatively, the pyridoxine dehydrogenase may be DNA having the nucleotide sequence of SEQ ID NO:7, or may be DNA having a nucleotide sequence that has, for example, 80% or more, 85% or more, 90% or more, or 95% or more sequence identity with the nucleotide sequence of SEQ ID NO:7, and that encodes a protein having pyridoxine dehydrogenase activity.

Alternatively, the gene encoding the pyridoxine dehydrogenase may be, for example, DNA having the nucleotide sequence of SEQ ID NO:8 (the nucleotide sequence of a gene encoding a pyridoxine dehydrogenase from *Schizosaccharomyces pombe*), or DNA having a nucleotide sequence that hybridizes with DNA having a nucleotide sequence complementary to the nucleotide sequence of SEQ ID NO:8 under a stringent condition, and that encodes a protein having pyridoxine dehydrogenase activity.

Alternatively, the gene encoding the pyridoxine dehydrogenase may be, for example, DNA having the nucleotide sequence of SEQ ID NO:8, or DNA having a nucleotide sequence in which one or more of the substitution, deletion, or insertion of a nucleotide, or the addition of an additional nucleotide to either or both of the 5' end and 3' end of the nucleotide sequence are performed in the nucleotide sequence of SEQ ID NO:8, and which encodes a protein having pyridoxine dehydrogenase activity. Examples of the degrees of the substitution, deletion, and insertion of a nucleotide, and the addition of an additional nucleotide to either or both of the N-terminus and C-terminus of a nucleotide sequence are as described above.

Alternatively, the pyridoxine dehydrogenase may be DNA having the nucleotide sequence of SEQ ID NO:8, or may be DNA having a nucleotide sequence that has, for example, 80% or more, 85% or more, 90%, or 95% or more sequence identity with the nucleotide sequence of SEQ ID NO:8, and that encodes a protein having pyridoxine dehydrogenase activity.

Alternatively, the gene encoding the pyridoxine dehydrogenase may be, for example, DNA having the nucleotide sequence of any one of SEQ ID NO:7 or SEQ ID NO:53 to SEQ ID NO:59, or DNA having a nucleotide sequence that hybridizes with DNA having a nucleotide sequence complementary to the nucleotide sequence of any one of SEQ ID NO:7 or SEQ ID NO:53 to SEQ ID NO:59 under a stringent condition, and that encodes a protein having pyridoxine dehydrogenase activity.

Alternatively, the gene encoding the pyridoxine dehydrogenase may be, for example, DNA having the nucleotide sequence of any one of SEQ ID NO:7 or SEQ ID NO:53 to SEQ ID NO:59, or DNA having a nucleotide sequence in which one or more of the substitution, deletion, or insertion of a nucleotide, or the addition of an additional nucleotide to either or both of the 5' end and 3' end of the nucleotide sequence are performed in the nucleotide sequence of any one of SEQ ID NO:7 or SEQ ID NO:53 to SEQ ID NO:59, and which encodes a protein having pyridoxine dehydrogenase activity. Examples of the degrees of the substitution, deletion, and insertion of a nucleotide, and the addition of an additional nucleotide to either or both of the N-terminus and C-terminus of a nucleotide sequence are as described above.

Alternatively, the pyridoxine dehydrogenase may be DNA having the nucleotide sequence of any one of SEQ ID NO:7 or SEQ ID NO:53 to SEQ ID NO:59, or may be DNA having a nucleotide sequence that has, for example, 80% or more, 85% or more, 90% or more, or 95% or more sequence identity with at least one of the nucleotide sequence of SEQ ID NO:7, the nucleotide sequence of SEQ ID NO:53 (the nucleotide sequence of a gene encoding a pyridoxine dehydrogenase from *Saccharomyces eubayanus*), the nucleotide sequence of SEQ ID NO:54 (the nucleotide sequence of a gene encoding a pyridoxine dehydrogenase from *Torulaspora delbrueckii*), the nucleotide sequence of SEQ ID NO:55 (the nucleotide sequence of a gene encoding a pyridoxine dehydrogenase from *Zygosaccharomyces bailii*), the nucleotide sequence of SEQ ID NO:57 (the nucleotide sequence of a gene encoding a pyridoxine dehydrogenase from *Kluyveromyces marxianus*), the nucleotide sequence of SEQ ID NO:56 (the nucleotide sequence of a gene encoding a pyridoxine dehydrogenase from *Aspergillus oryzae*), the nucleotide sequence of SEQ ID NO:58 (the nucleotide sequence of a gene encoding a pyridoxine dehydrogenase from *Candida albicans*), or the nucleotide sequence of SEQ ID NO:59 (the nucleotide sequence of a gene encoding a pyridoxine dehydrogenase from *Yarrowia lipolytica*), and that encodes a protein having pyridoxine dehydrogenase activity.

In the case of expression in a recombinant microorganism in which a prokaryote such as *E. coli* is used as a host, a codon may be optimized to facilitate the expression. For example, a nucleotide sequence may be modified so that a codon, which is most frequently used, of codons that encode respective amino acids in a prokaryote as a host is highly frequently used as a codon for the amino acid. From such a viewpoint, for example, DNA having the region between the 18th nucleotide and the 3' end of the nucleotide sequence of any one of SEQ ID NO:13 or SEQ ID NO:75 to SEQ ID NO:81 may be used, or DNA having a nucleotide sequence that hybridizes with DNA having a nucleotide sequence complementary to the region between the 18th nucleotide and the 3' end of the nucleotide sequence of any one of SEQ ID NO:13 or SEQ ID NO:75 to SEQ ID NO:81 under a stringent condition, and that encodes a protein having pyridoxine dehydrogenase activity may be used, as DNA including a gene encoding the pyridoxine dehydrogenase. Seventeen nucleotides from the 5' end of each of the nucleotide sequences of SEQ ID NO:13 and SEQ ID NO:75 to SEQ ID NO:81 are included in an upstream region, and an initiation codon is included in the 18th nucleotide to the 20th nucleotide. Therefore, the region between the 18th nucleotide and the 3' end of each of these nucleotide sequences may be used as a gene region encoding a pyridoxine dehydrogenase.

Alternatively, the gene encoding the pyridoxine dehydrogenase may be, for example, DNA having the region between the 18th nucleotide to the 3' end of the nucleotide sequence of any one of SEQ ID NO:13 or SEQ ID NO:75 to SEQ ID NO:81, or DNA having a nucleotide sequence in which one or more of the substitution, deletion, or insertion of a nucleotide, or the addition of an additional nucleotide to either or both of the 5' end and 3' end of the nucleotide sequence are performed in the region between the 18th nucleotide and 3' end of the nucleotide sequence of any one of SEQ ID NO:13 or SEQ ID NO:75 to SEQ ID NO:81, and which encodes a protein having pyridoxine dehydrogenase activity. Examples of the degrees of the substitution, deletion, and insertion of a nucleotide, and the addition of an additional nucleotide to either or both of the N-terminus and C-terminus of a nucleotide sequence are as described above.

Alternatively, the pyridoxine dehydrogenase may be DNA having the region between the 18th nucleotide and 3' end of the nucleotide sequence of any one of SEQ ID NO:13 or SEQ ID NO:75 to SEQ ID NO:81, or may be DNA having a nucleotide sequence that has, for example, 80% or more, 85% or more, 90% or more, or 95% or more sequence identity with at least one of the region between the 18th nucleotide and 3' end of the nucleotide sequence of SEQ ID NO:13 (codon optimized nucleotide sequence of a gene encoding a pyridoxine dehydrogenase from *Saccharomyces cerevisiae*), the region between the 18th nucleotide and 3' end of the nucleotide sequence of SEQ ID NO:75 (codon optimized nucleotide sequence of a gene encoding a pyridoxine dehydrogenase from *Saccharomyces eubayanus*), the region between the 18th nucleotide and 3' end of the nucleotide sequence of SEQ ID NO:76 (codon optimized nucleotide sequence of a gene encoding a pyridoxine dehydrogenase from *Torulaspora delbrueckii*), the region between the 18th nucleotide and 3' end of the nucleotide sequence of SEQ ID NO:77 (codon optimized nucleotide sequence of a gene encoding a pyridoxine dehydrogenase from *Zygosaccharomyces bailii*), the region between the 18th nucleotide and 3' end of the nucleotide sequence of SEQ ID NO:79 (codon optimized nucleotide sequence of a gene encoding a pyridoxine dehydrogenase from *Kluyveromyces marxianus*), the region between the 18th nucleotide and 3' end of the nucleotide sequence of SEQ ID NO:78 (codon optimized nucleotide sequence of a gene encoding a pyridoxine dehydrogenase from *Aspergillus oryzae*), the region between the 18th nucleotide and 3' end of the nucleotide sequence of SEQ ID NO:80 (codon optimized nucleotide sequence of a gene encoding a pyridoxine dehydrogenase from *Candida albicans*), or the region between the 18th nucleotide and 3' end of the nucleotide sequence of SEQ ID NO:81 (codon optimized nucleotide sequence of a gene encoding a pyridoxine dehydrogenase from *Yarrowia lipolytica*), and that encodes a protein having pyridoxine dehydrogenase activity.

The gene encoding the pyridoxamine synthetase may be, for example, DNA having the nucleotide sequence of SEQ ID NO:9 (nucleotide sequence of pyridoxamine-pyruvate transaminase gene from *Mesorhizobium loti*), or may be DNA having a nucleotide sequence that hybridizes with DNA having a nucleotide sequence complementary to the nucleotide sequence of SEQ ID NO:9 under a stringent condition, and that encodes a protein having pyridoxamine synthetase activity.

Alternatively, the gene encoding the pyridoxamine synthetase may be, for example, DNA having the nucleotide sequence of SEQ ID NO:9, or DNA having a nucleotide sequence in which one or more of the substitution, deletion, or insertion of a nucleotide, or the addition of an additional nucleotide to either or both of the 5' end and the 3' end of the nucleotide sequence are performed in the nucleotide sequence of SEQ ID NO:9, and which encodes a protein having pyridoxamine synthetase activity. Examples of the degrees of the substitution, deletion, and insertion of the nucleotide, and the addition of an additional nucleotide to either or both of the N-terminus and C-terminus of the nucleotide sequence are as described above.

Alternatively, the pyridoxamine synthetase may be DNA having the nucleotide sequence of SEQ ID NO:9, or may be DNA having a nucleotide sequence that has, for example, 80% or more, 85% or more, 90% or more, or 95% or more sequence identity with the nucleotide sequence of SEQ ID NO:9, and that encodes a protein having pyridoxamine synthetase activity.

The gene encoding the pyridoxamine synthetase may be, for example, DNA having the nucleotide sequence of SEQ ID NO:10 (nucleotide sequence of pyridoxamine-oxaloacetate transaminase gene from *Escherichia coli*), or may be DNA having a nucleotide sequence that hybridizes with DNA having a nucleotide sequence complementary to the nucleotide sequence of SEQ ID NO:10 under a stringent condition, and that encodes a protein having pyridoxamine synthetase activity.

Alternatively, the gene encoding the pyridoxamine synthetase may be, for example, DNA having the nucleotide sequence of SEQ ID NO:10, or DNA having a nucleotide sequence in which one or more of the substitution, deletion, or insertion of a nucleotide, or the addition of an additional nucleotide to either or both of the 5' end and the 3' end of the nucleotide sequence are performed in the nucleotide sequence of SEQ ID NO:10, and which encodes a protein having pyridoxamine synthetase activity. Examples of the degrees of the substitution, deletion, and insertion of the nucleotide, and the addition of an additional nucleotide to either or both of the N-terminus and C-terminus of the nucleotide sequence are as described above.

Alternatively, the pyridoxamine synthetase may be DNA having the nucleotide sequence of SEQ ID NO:9, or may be DNA having a nucleotide sequence that has, for example, 80% or more, 85% or more, 90% or more, or 95% or more sequence identity with the nucleotide sequence of SEQ ID NO:9, and that encodes a protein having pyridoxamine synthetase activity.

Alternatively, the gene encoding the pyridoxamine synthetase may be, for example, DNA having the nucleotide sequence of any one of SEQ ID NO:9 or SEQ ID NO:60 to SEQ ID NO:66, or may be DNA having a nucleotide sequence that hybridizes with DNA having a nucleotide sequence complementary to the nucleotide sequence of any one of SEQ ID NO:9 or SEQ ID NO:60 to SEQ ID NO:66 under a stringent condition, and that encodes a protein having pyridoxamine synthetase activity.

Alternatively, the gene encoding the pyridoxamine synthetase may be, for example, DNA having the nucleotide sequence of any one of SEQ ID NO:9 or SEQ ID NO:60 to SEQ ID NO:66, or DNA having a nucleotide sequence in which one or more of the substitution, deletion, or insertion of a nucleotide, or the addition of an additional nucleotide to either or both of the 5' end and the 3' end of the nucleotide sequence are performed in the nucleotide sequence of any one of SEQ ID NO:9 or SEQ ID NO:60 to SEQ ID NO:66, and which encodes a protein having pyridoxamine synthetase activity. Examples of the degrees of the substitution, deletion, and insertion of a nucleotide, and the addition of an additional nucleotide either or both of the N-terminus and C-terminus of a nucleotide sequence are as described above.

Alternatively, the pyridoxamine synthetase may be DNA having the nucleotide sequence of any one of SEQ ID NO:9 or SEQ ID NO:60 to SEQ ID NO:66, or may DNA having a nucleotide sequence that has, for example, 80% or more, 85% or more, 90% or more, or 95% or more sequence identity with at least one of the nucleotide sequence of SEQ ID NO:9, the nucleotide sequence of SEQ ID NO:60 (nucleotide sequence of a gene encoding pyridoxamine-pyruvate transaminase from *Mesorhizobium sp.* YR577), the nucleotide sequence of SEQ ID NO:61 (nucleotide sequence of a gene encoding pyridoxamine-pyruvate transaminase from *Pseudaminobacter salicylatoxidans*), the nucleotide sequence of SEQ ID NO:62 (nucleotide sequence of a gene encoding pyridoxamine-pyruvate transaminase from *Bauldia litoralis*), the nucleotide sequence of SEQ ID NO:63 (nucleotide sequence of a gene encoding pyridoxamine-pyruvate transaminase from *Skermanella stibiiresistens*), the nucleotide sequence of SEQ ID NO:64 (nucleotide sequence of gene encoding pyridoxamine-pyruvate transaminase from *Rhizobium sp.* AC44/96), the nucleotide sequence of SEQ ID NO:65 (nucleotide sequence of a gene encoding pyridoxamine-pyruvate transaminase from *Erwinia toletana*), or the nucleotide sequence of SEQ ID NO:66 (nucleotide sequence of a gene encoding pyridoxamine-pyruvate transaminase from *Herbiconiux ginsengi*), and that encodes a protein having pyridoxamine synthetase activity.

In the case of expression in a recombinant microorganism in which prokaryote such as *E. coli* is used as a host, a codon may be optimized, as described above, to facilitate expression. From such a viewpoint, as DNA including a gene encoding the pyridoxamine synthetase, for example, DNA having the region between the 18th nucleotide and the 3' end of the nucleotide sequence of SEQ ID NO:14 or the region between the 18th nucleotide and the 3' end of the nucleotide sequence of any one of SEQ ID NO:82 to SEQ ID NO:88 may be used, or DNA having a nucleotide sequence that hybridizes with DNA having a nucleotide sequence complementary to the region between 18th nucleotide and the 3' end in the nucleotide sequence of SEQ ID NO:14 or the region between the 18th nucleotide and the 3' end in the nucleotide sequence of any one of SEQ ID NO:82 to SEQ ID NO:88 under a stringent condition, and that encodes a protein having pyridoxamine synthetase activity may be used. Seventeen nucleotides from the 5' end of the nucleotide sequence of SEQ ID NO:14 are included in an upstream region, and an initiation codon is included in the 18th nucleotide to the 20th nucleotide. Therefore, the region between the 18th nucleotide and the 3' end of this nucleotide sequence may be used as a gene region encoding a pyridoxamine synthetase. Similarly, seventeen nucleotides from the 5' end of each of the nucleotide sequences of SEQ ID NO:82 to SEQ ID NO:88 are included in an upstream, and an initiation codon is included in the 18th nucleotide to the 20th nucleotide. Therefore, the region between the 18th nucleotide and the 3' end of these nucleotide sequences may be used as a gene region encoding a pyridoxamine synthetase.

Alternatively, the gene encoding the pyridoxamine synthetase may be, for example, DNA having the region between the 18th nucleotide and the 3' end of the nucleotide sequence of SEQ ID NO:14 or the region between the 18th nucleotide and the 3' end of the nucleotide sequence of any one of SEQ ID NO:82 to SEQ ID NO:88, or DNA having a nucleotide sequence in which one or more of the substitution, deletion, or insertion of a nucleotide, or the addition of an additional nucleotide to either or both of the 5' end and the 3' end of the are performed in the region between the 18th nucleotide and the 3' end of the nucleotide sequence of the nucleotide sequence of SEQ ID NO:14 or the region between the 18th nucleotide and the 3' end of the nucleotide sequence of any one of SEQ ID NO:82 to SEQ ID NO:88, and which encodes a protein having pyridoxamine synthetase activity. Examples of the degrees of the substitution, deletion, and insertion of a nucleotide, and the addition of an additional nucleotide to either or both of the N-terminus and C-terminus of the nucleotide sequence are as described above.

Alternatively, the pyridoxamine synthetase may be DNA having the region between the 18th nucleotide and the 3' end of the nucleotide sequence of SEQ ID NO:14 or the region between the 18th nucleotide and the 3' end of any one of nucleotide sequences of SEQ ID NO:82 to SEQ ID NO:88, or may be DNA having a nucleotide sequence that has, for example, 80% or more, 85% or more, 90% or more, or 95% or more sequence identity with at least one of the region between the 18th nucleotide and the 3' end of the nucleotide sequence of SEQ ID NO:14 (codon-optimized nucleotide sequence of a gene encoding pyridoxamine-pyruvate transaminase from *Mesorhizobium loti*), the region between the 18th nucleotide and the 3' end of the nucleotide sequence of SEQ ID NO:82 (codon-optimized nucleotide sequence of a gene encoding pyridoxamine-pyruvate transaminase from *Mesorhizobium sp.* YR577), the region between the 18th nucleotide and the 3' end of the nucleotide sequence of SEQ ID NO:83 (codon-optimized nucleotide sequence of a gene encoding pyridoxamine-pyruvate transaminase from *Pseudaminobacter salicylatoxidans*), the region between the 18th nucleotide and the 3' end of the nucleotide sequence of SEQ ID NO:84 (codon-optimized nucleotide sequence of a gene encoding pyridoxamine-pyruvate transaminase from *Bauldia litoralis*), the region between the 18th nucleotide and the 3' end of the nucleotide sequence of SEQ ID NO:85 (codon-optimized nucleotide sequence of a gene encoding pyridoxamine-pyruvate transaminase from *Skermanella stibiiresistens*), the region between the 18th nucleotide and the 3' end of the nucleotide sequence of SEQ ID NO:86 (codon-optimized nucleotide sequence of gene encoding pyridoxamine-pyruvate transaminase from *Rhizobium sp.* AC44/96), the region between the 18th nucleotide and the 3' end of the nucleotide sequence of SEQ ID NO:87 (codon-optimized nucleotide sequence of a gene encoding pyridoxamine-pyruvate transaminase from *Erwinia toletana*), or the region between the 18th nucleotide and the 3' end of the nucleotide sequence of SEQ ID NO:88 (codon-optimized nucleotide sequence of a gene encoding pyridoxamine-pyruvate transaminase from *Herbiconiux ginsengi*), and that encodes a protein having pyridoxamine synthetase activity.

The gene encoding the amino acid regeneration enzyme may be, for example, DNA having the nucleotide sequence of SEQ ID NO:11 (the nucleotide sequence of a gene encoding an alanine dehydrogenase from *Shewanella sp.* Ac10), or DNA having a nucleotide sequence that hybridizes with DNA having a nucleotide sequence complementary to the nucleotide sequence of SEQ ID NO:11 under a stringent condition, and that encodes a protein having alanine regeneration enzymatic activity.

Alternatively, the gene encoding the amino acid regeneration enzyme may be, for example, DNA having the nucleotide sequence of SEQ ID NO:11, or DNA having a nucleotide sequence in which one or more of the substitution, deletion, or insertion of a nucleotide, or the addition of an additional nucleotide to either or both of the 5' end and 3' end of the nucleotide sequence are performed in the nucleotide sequence of SEQ ID NO:11, and which encodes a protein having alanine regeneration enzymatic activity. Examples of the degrees of the substitution, deletion, and insertion of a nucleotide, and the addition of an additional nucleotide to either or both of the N-terminus and C-terminus of a nucleotide sequence are as described above.

Alternatively, the amino acid regeneration enzyme may be DNA having the nucleotide sequence of SEQ ID NO:11, or DNA having a nucleotide sequence that has, for example, 80% or more, 85% or more, 90% or more, or 95% or more sequence identity with the nucleotide sequence of SEQ ID NO:11, and that encodes a protein having alanine regeneration enzymatic activity.

Alternatively, the gene encoding the amino acid regeneration enzyme may be, for example, DNA having the nucleotide sequence of SEQ ID NO:12 (the nucleotide sequence of a gene encoding a glutamate dehydrogenase from *Escherichia coli*), or DNA having a nucleotide sequence that hybridizes with DNA having a nucleotide sequence complementary to the nucleotide sequence of SEQ ID NO:12 under a stringent condition, and that encodes a protein having glutamic acid regeneration enzymatic activity.

Alternatively, the gene encoding the amino acid regeneration enzyme may be, for example, DNA having the nucleotide sequence of SEQ ID NO:12, or DNA having a nucleotide sequence in which one or more of the substitution, deletion, or insertion of a nucleotide, or the addition of an additional nucleotide to either or both of the 5' end and 3' end of the nucleotide sequence are performed in the nucleotide sequence of SEQ ID NO:12, and which encodes a protein having glutamic acid regeneration enzymatic activity. Examples of the degrees of the substitution, deletion, and insertion of a nucleotide, and the addition of an additional nucleotide to either or both of the N-terminus and C-terminus of a nucleotide sequence are as described above.

Alternatively, the amino acid regeneration enzyme may be DNA having the nucleotide sequence of SEQ ID NO:12, or DNA having a nucleotide sequence that has, for example, 80% or more, 85% or more, 90% or more, or 95% or more a sequence identity with the nucleotide sequence of SEQ ID NO:12, and that encodes a protein having glutamic acid regeneration enzymatic activity.

The gene encoding the amino acid regeneration enzyme may be, for example, DNA having the nucleotide sequence of any one of SEQ ID NO:11 or SEQ ID NO:67 to SEQ ID NO:74, or DNA having a nucleotide sequence that hybridizes with DNA having a nucleotide sequence complementary to the nucleotide sequence of any one of SEQ ID NO:11 or SEQ ID NO:67 to SEQ ID NO:74 under s stringent condition, and that encodes a protein having alanine regeneration enzymatic activity.

Alternatively, the gene encoding the amino acid regeneration enzyme may be, for example, DNA having the nucleotide sequence of any one of SEQ ID NO:11 or SEQ ID NO:67 to SEQ ID NO:74, or DNA having a nucleotide sequence in which one or more of the substitution, deletion, or insertion of a nucleotide, or the addition of an additional nucleotide to either or both of the 5' end and 3' end of the nucleotide sequence are performed in the nucleotide sequence of any one of SEQ ID NO:11 or SEQ ID NO:67 to SEQ ID NO:74, and which encodes a protein having alanine regeneration enzymatic activity. Examples of the degrees of the substitution, deletion, and insertion of a nucleotide, and the addition of an additional nucleotide to either or both of the N-terminus and C-terminus of a nucleotide sequence are as described above.

Alternatively, the amino acid regeneration enzyme may be DNA having the nucleotide sequence of any one of SEQ ID NO:11 or SEQ ID NO:67 to SEQ ID NO:74, or may be DNA having a nucleotide sequence that has, for example, 80% or more, 85% or more, 90% or more, or 95% or more sequence identity with at least one of the nucleotide sequence of SEQ ID NO:11, the nucleotide sequence of SEQ ID NO:67 (the nucleotide sequence of a gene encoding an alanine dehydrogenase from *Aeromonas hydrophila*), the nucleotide sequence of SEQ ID NO:68 (the nucleotide sequence of a gene encoding an alanine dehydrogenase from *Rhizobium sp.* LPU83), the nucleotide sequence of SEQ ID NO:69 (the nucleotide sequence of a gene encoding an alanine dehydrogenase from *Pseudomonas mendocina*), the nucleotide sequence of SEQ ID NO:70 (the nucleotide sequence of a gene encoding one of alanine dehydrogenases from *Bradyrhizobium japonicum*), the nucleotide sequence of SEQ ID NO:71 (the nucleotide sequence of a gene encoding another alanine dehydrogenase from *Bradyrhizobium japonicum*), the nucleotide sequence of SEQ ID NO:72 (the nucleotide sequence of a gene encoding an alanine dehydrogenase from *Streptomyces aureofaciens*), the nucleotide sequence of SEQ ID NO:73 (the nucleotide sequence of a gene encoding an alanine dehydrogenase from *Anabaena cylindrica*), or the nucleotide sequence of SEQ ID NO:74 (the nucleotide sequence of a gene encoding an alanine dehydrogenase from *Bacillus subtilis*), and that encodes a protein having alanine regeneration enzymatic activity.

In the case of expression in a recombinant microorganism in which a prokaryote such as *E. coli* is used as a host, a codon may be optimized, as described above, to facilitate the expression. From such a viewpoint, as DNA including a gene encoding the amino acid regeneration enzyme, for example, DNA having the region between the 18th nucleotide and 3' end of the nucleotide sequence of any one of SEQ ID NO:15 or SEQ ID NO:89 to SEQ ID NO:96 may be used, or DNA having a nucleotide sequence that hybridizes with DNA having a nucleotide sequence complementary to the region between the 18th nucleotide and 3' end of the nucleotide sequence of any one of SEQ ID NO:15 or SEQ ID NO:89 to SEQ ID NO:96 under a stringent condition, and that encodes a protein having alanine regeneration enzymatic activity may be used. Seventeen nucleotides from the 5' end of each of the nucleotide sequences of SEQ ID NO:15 and SEQ ID NO:89 to SEQ ID NO:96 are included in an upstream region, and an initiation codon is included in the 18th nucleotide to the 20th nucleotide. Therefore, the region between the 18th nucleotide and 3' end of each of these nucleotide sequences may be used as a gene region encoding an alanine regeneration enzyme.

Alternatively, the gene encoding the amino acid regeneration enzyme may be, for example, DNA having the region between the 18th nucleotide to 3' end of the nucleotide sequence of any one of SEQ ID NO:15 or SEQ ID NO:89 to SEQ ID NO:96, or DNA having a nucleotide sequence in which one or more of the substitution, deletion, or insertion of a nucleotide, or the addition of an additional nucleotide to either or both of the 5' end and 3' end of the nucleotide sequence are performed in the region between the 18th nucleotide and 3' end of the nucleotide sequence of any one of SEQ ID NO:15 or SEQ ID NO:89 to SEQ ID NO:96, and which encodes a protein having alanine regeneration enzymatic activity. Examples of the degrees of the substitution, deletion, and insertion of a nucleotide, and the addition of an additional nucleotide to either or both of the N-terminus and C-terminus of a nucleotide sequence are as described above.

Alternatively, the amino acid regeneration enzyme may be DNA having the region between the 18th nucleotide and 3' end of the nucleotide sequence of any one of SEQ ID NO:15 or SEQ ID NO:89 to SEQ ID NO:96, or may be DNA having a nucleotide sequence that has, for example, 80% or more, 85% or more, 90% or more, or 95% or more sequence identity with at least one of the region between the 18th nucleotide and 3' end of the nucleotide sequence of SEQ ID NO:15 (codon optimized nucleotide sequence encoding an amino acid sequence in which the amino acid substitution of D198A is performed in the amino acid sequence of an alanine dehydrogenase from *Shewanella sp.* AC10), the region between the 18th nucleotide and 3' end of the nucleotide sequence of SEQ ID NO:89 (codon optimized nucleotide sequence of a gene encoding an amino acid sequence in which amino acid substitution to use NADP⁺ is performed in an alanine dehydrogenase from *Aeromonas hydrophila*), the region between the 18th nucleotide and 3' end of the nucleotide sequence of SEQ ID NO:90 (codon optimized nucleotide sequence of a gene encoding an amino acid sequence in which amino acid substitution to use NADP⁺ is performed in an alanine dehydrogenase from *Rhizobium sp.* LPU83), the region between the 18th nucleotide and 3' end of the nucleotide sequence of SEQ ID NO:91 (codon optimized nucleotide sequence of a gene encoding an amino acid sequence in which amino acid substitution to use NADP⁺ is performed in an alanine dehydrogenase from *Pseudomonas mendocina*), the region between the 18th nucleotide and 3' end of the nucleotide sequence of SEQ ID NO:92 (codon optimized nucleotide sequence of a gene encoding an amino acid sequence in which amino acid substitution to use NADP⁺ is performed in one of alanine dehydrogenases from *Bradyrhizobium japonicum*), the region between the 18th nucleotide and 3' end of the nucleotide sequence of SEQ ID NO:93 (codon optimized nucleotide sequence of a gene encoding an amino acid sequence in which amino acid substitution to use NADP⁺ is performed in another alanine dehydrogenase from *Bradyrhizobium japonicum*), the region between the 18th nucleotide and 3' end of the nucleotide sequence of SEQ ID NO:94 (codon optimized nucleotide sequence of a gene encoding an amino acid sequence in which amino acid substitution to use NADP⁺ is performed in an alanine dehydrogenase from *Streptomyces aureofaciens*), the region between the 18th nucleotide and 3' end of the nucleotide sequence of SEQ ID NO:95 (codon optimized nucleotide sequence of a gene encoding an amino acid sequence in which amino acid substitution to use NADP⁺ is performed in an alanine dehydrogenase from *Anabaena cylindrica*), or the region between the 18th nucleotide and 3' end of the nucleotide sequence of SEQ ID NO:96 (codon optimized nucleotide sequence of a gene encoding an amino acid sequence in which amino acid substitution to use NADP⁺ is performed in an alanine dehydrogenase from *Bacillus subtilis*), and that encodes a protein having alanine regeneration enzymatic activity.

### <Recombinant Microorganism Having Gene Encoding Pyridoxine Dehydrogenase, Gene Encoding Pyridoxamine Synthetase, and Gene Encoding Amino Acid Regeneration Enzyme>

In the recombinant microorganism according to the disclosure, each of a gene encoding a pyridoxine dehydrogenase, a gene encoding a pyridoxamine synthetase, and a gene encoding an amino acid regeneration enzyme having an enzymatic activity of regenerating an amino acid consumed by the pyridoxamine synthetase may be endogenous to a bacterial cell, endogenous to a bacterial cell and has an enhanced expression, or introduced from outside of a bacterial cell into the bacterial cell. However, at least two of the gene encoding a pyridoxine dehydrogenase, the gene encoding a pyridoxamine synthetase, or the gene encoding an amino acid regeneration enzyme having an enzymatic activity of regenerating an amino acid consumed by the pyridoxamine synthetase, are introduced from outside of a bacterial cell, or are endogenous to a bacterial cell and have an enhanced expression.

In other words, each of the gene encoding a pyridoxine dehydrogenase, the gene encoding a pyridoxamine synthetase, and the gene encoding an amino acid regeneration enzyme having an enzymatic activity of regenerating an amino acid consumed by the pyridoxamine synthetase may be endogenous to the genome of a host microorganism prior to recombination, endogenous to the genome of a host microorganism and has an enhanced expression by an operation such as the substitution of a promoter, or introduced from outside of a bacterial cell into the bacterial cell using a vector such as a plasmid. In the first aspect, a pyridoxamine production capacity due to a combination of the three enzymes is exhibited, and each of at least two of the gene encoding a pyridoxine dehydrogenase, the gene encoding a pyridoxamine synthetase, or the gene encoding an amino acid regeneration enzyme having an enzymatic activity of regenerating an amino acid consumed by the pyridoxamine synthetase, is introduced from outside of a cell in the host microorganism, or has an enhanced expression of the gene endogenous to the cell in the host microorganism by the substitution of a promoter, or the like, whereby the expression of the gene is increased, and the pyridoxamine production capability due to the combination of the three enzymes is further enhanced. In the case of performing the introduction or expression enhancement of each of the gene encoding a pyridoxine dehydrogenase, the gene encoding a pyridoxamine synthetase, and a gene encoding an amino acid regeneration enzyme having an enzymatic activity of regenerating an amino acid consumed by the pyridoxamine synthetase, either or both of the introduction from outside of a cell in a host microorganism and the enhancement of the gene expression by the substitution of a promoter, or the like may be performed.

It is impossible to obtain a sufficient production capability for highly producing pyridoxamine or a salt thereof unless such increase in gene expression as described above is performed in at least two of the gene encoding a pyridoxine dehydrogenase, the gene encoding a pyridoxamine synthetase, or the gene encoding an amino acid regeneration enzyme having an enzymatic activity of regenerating an amino acid consumed by the pyridoxamine synthetase. Since the amino acid regeneration enzyme described herein refers to an amino acid regeneration enzyme having an enzymatic activity of regenerating a specific kind of an amino acid consumed by the pyridoxamine synthetase, the pyridoxamine synthetase and the amino acid regeneration enzyme can also be considered to be in a pair. Since such gene expression as described above is increased in at least two of the gene encoding a pyridoxine dehydrogenase, the gene encoding a pyridoxamine synthetase, or the gene encoding an amino acid regeneration enzyme having an enzymatic activity of regenerating an amino acid consumed by the pyridoxamine synthetase, the pair exists in a bacterial cell in a recombinant microorganism, and the gene expression is increased in at least either of the pyridoxamine synthetase and the amino acid regeneration enzyme in the pair.

Targets in which at least one of the introduction from outside of a bacterial cell or the enhancement of the expression of a gene endogenous to the bacterial cell is performed, may be only two of the gene encoding a pyridoxine dehydrogenase, the gene encoding a pyridoxamine synthetase, or the gene encoding an amino acid regeneration enzyme having an enzymatic activity of regenerating an amino acid consumed by the pyridoxamine synthetase. In a case in which at least one of the introduction from outside of a bacterial cell or the enhancement of the expression of a gene endogenous to the bacterial cell is performed in each of two of the gene encoding a pyridoxine dehydrogenase, the gene encoding a pyridoxamine synthetase, or the gene encoding an amino acid regeneration enzyme having an enzymatic activity of regenerating an amino acid consumed by the pyridoxamine synthetase, at least one of the introduction from outside of a bacterial cell or the enhancement of the expression of a gene endogenous to the bacterial cell may be performed in each of the gene encoding a pyridoxine dehydrogenase and the gene encoding a pyridoxamine synthetase, at least one of the introduction from outside of a bacterial cell or the enhancement of the expression of a gene endogenous to the bacterial cell may be performed in each of the gene encoding a pyridoxine dehydrogenase and the gene encoding the amino acid regeneration enzyme, and at least one of the introduction from outside of a bacterial cell or the enhancement of the expression of a gene endogenous to the bacterial cell may be performed in each of the gene encoding a pyridoxamine synthetase and the gene encoding the amino acid regeneration enzyme.

From the view point of increasing the production efficiency in the production of pyridoxamine or a salt thereof, it is preferable that at least one of the introduction from outside of a bacterial cell or the enhancement of the expression of a gene endogenous to the bacterial cell is performed in all of the gene encoding a pyridoxine dehydrogenase, the gene encoding a pyridoxamine synthetase, and the gene encoding an amino acid regeneration enzyme. From the view point of increasing the production efficiency in the production of pyridoxamine or a salt thereof, it is preferable that at least one of the introduction from outside of a bacterial cell or the enhancement of the expression of a gene endogenous to the bacterial cell is performed in the gene encoding an amino acid regeneration enzyme. The introduction of an enzyme gene from outside of a bacterial cell is not necessarily limited to the introduction for compensating an enzyme gene which is not present in a host microorganism, and the introduction may be performed for the purpose of increasing the expression of an enzyme gene endogenous to a host microorganism. An enzyme gene which is not present in a host microorganism can be easily confirmed using an enzyme database such as KEGG or BRENDA.

In a case in which the expression of a gene endogenous to a host microorganism is enhanced by the substitution of a promoter of the gene with another promoter, the another promoter (newly introduced promoter) is not particularly limited as long as the promoter can enhance the gene expression (more than before promoter substitution) in the host microorganism, and may be a constitutive promoter or an inducible promoter. Substitution of a promoter can be performed using a general genetic modification technology. The sequence of a promoter endogenous to a host microorganism may be partially or completely left unless the sequence adversely affects expression by a newly introduced promoter in practical use.

When the host microorganism is, for example, a prokaryote, examples of the promoter that can be used as the newly introduced promoter include a trp promoter, a lac promoter, and a GAPDH promoter from *E. coli*, a PL promoter and a PR promoter from lambda phage, and a gluconate synthetase promoter (gnt), an alkaline protease promoter (apr), a neutral protease promoter (npr), and an α-amylase promoter (amy) from *Bacillus subtilis.* An originally modified or designed promoter such as a tac promoter can also be used.

When the host microorganism is, for example, a filamentous fungus, examples of the promoter that can be used as the newly introduced promoter include a cellobiohydrolase (cbh) promoter, an endoglucanase (egl) promoter, a xylanase III (xyn3) promoter, a U6 promoter, and an α-amylase (amy) promoter.

When the host microorganism is, for example, yeast, examples of the promoter that can be used as the newly introduced promoter include an alcohol dehydrogenase (ADH1) promoter, a phosphoglycerate kinase (PGK1) promoter, a peptide chain elongation factor (TEF) promoter, a glycerol 3-phosphate dehydrogenase (GPD) promoter, a galactokinase (GAL1) promoter, a metallothionein (CUP1) promoter, a repressive acid phosphatase (PHO5) promoter, and a glyceraldehyde 3-phosphate dehydrogenase (GAPDH) promoter. The sequences of the above-described promoters are not limited to those derived from yeast used as a host microorganism. An exogenous promoter such as a cytomegalovirus (CMV) promoter may also be used.

In the case of introducing a gene (heterogenous gene) from outside of a host microorganism, the method is not particularly limited as long as a gene can be introduced into the inside of a bacterial cell (inside a cell) and an enzyme encoded by the gene can be expressed, and examples thereof include transformation with a plasmid carrying an enzyme gene, introduction of an enzyme gene into a genome, and a combination thereof. When introducing a gene, an expression vector into which the gene is integrated may be introduced into a bacterial cell. The expression vector is not particularly limited as long as the vector is a vector into which the nucleotide sequence of the gene is integrated, and the expression vector is more preferably a plasmid vector or a phage vector having the following constitution, from the viewpoint of improving transformation efficiency or translation efficiency.

The expression vector is not particularly limited as long as the vector includes the nucleotide sequence of the gene and can transform the host microorganism. If necessary, in addition to such a nucleotide sequence, a nucleotide sequence (hereinafter, also simply referred to as "another region") constituting another region may be included. Examples of such a region include a control region required to the production of a desired enzyme by a recombinant microorganism obtained by transformation, and a region required for autonomous replication.

From the viewpoint of facilitating selection of the recombinant microorganism, the vector may further include a nucleotide sequence encoding a selection gene which can be used as a selection marker.

Examples of a control region required to the production of a desired enzyme include a promoter sequence (including an operator sequence for controlling transcription), a ribosome-binding sequence (SD sequence), and a transcription termination sequence.

In the case of using yeast as a host microorganism, from the viewpoint of expression efficiency of the gene, an expression vector that can be used preferably includes a promoter sequence in addition to the nucleotide sequence of the above gene. Any promoter sequence may be used as long as the sequence can express the gene in a transformant obtained using yeast as a host microorganism. Examples of the promoter sequence include the promoters described above as examples of the promoter that can be used as the newly introduced promoter in the case of using yeast as a host microorganism.

The expression vector may include a secretion signal. This allows to, when a recombinant microorganism produces a desired enzyme, extracellular secretion of the enzyme.

The secretion signal is not particularly limited as long as a desired enzyme can be secreted from yeast as a host microorganism. From the viewpoint of secretion efficiency, it is preferable to use an α factor signal sequence, an invertase signal sequence, an acid phosphatase signal sequence, a glucoamylase signal sequence, or the like.

Specific examples of the expression vector having the promoter sequence and the secretion signal as described above include pRS423, pRS424, and YEplac195.

From the viewpoint of expression efficiency of the gene, an expression vector that can be used when a filamentous fungus is used as a host microorganism preferably includes a promoter sequence in addition to the nucleotide sequence of the above gene. Any promoter sequence may be used as long as the sequence can express the gene in a transformant obtained by using a filamentous fungus as a host microorganism. Examples of the promoter include the promoters described above as examples of the promoter that can be used as the newly introduced promoter in the case of using a filamentous fungus as the host microorganism.

Suitable expression vectors for filamentous fungi are described in van den Hondel, C.A.M. J.J.et al.(1991) In: Bennett, J.W. and Lasure, L.L.(eds.) More Gene Manipulations in Fungi. Academic Press, pp.396-428.

Another commonly used expression vector such as pUC18, pBR322, pUC100, pSL1180 (manufactured by Pharmacia Inc.), pFB6 and *Aspergillus* pRAX, or *Trichoderma* pTEX can also be used.

From the viewpoint of the expression efficiency of the gene, an expression vector which can be used when a prokaryote such as *E. coli*, *Bacillus subtilis*, or Actinomycetes is used as a host microorganism preferably includes a promoter sequence in addition to the nucleotide sequence of the above gene. The expression vector may include a ribosome-binding sequence, a transcription termination sequence, or the like other than a promoter sequence.

Examples of the promoter sequence include the promoters described above as examples of the promoter that can be used as the newly introduced promoter in the case of using a prokaryote as the host microorganism.

Examples of the ribosome-binding sequence include a sequence derived from *E. coli* or *B. subtilis*, and the ribosome-binding sequence is not particularly limited thereto as long as the sequence is a sequence that functions in a desired host microorganism such as *E. coli* or *B. subtilis.*

Examples of the ribosome-binding sequence include a sequence in which a consensus sequence having four or more consecutive nucleotides, of sequences complementary to the 3' end region of 16S ribosomal RNA is produced by DNA synthesis.

The transcription termination sequence is not necessarily required, and a ρ-factor independent transcription termination, for example, as a lipoprotein terminator, a trp operon terminator, or the like can be used.

The order of these sequences on the expression vector of the control region is not particularly limited, and the promoter sequence, the ribosome-binding sequence, the gene encoding a target enzyme, and the transcription termination sequence are desirably arranged in this order from the side upstream of the 5'-end in consideration of transcription efficiency.

Specific examples of the expression vector that can be used when a host microorganism is a prokaryote include pBR322, pUC18, Bluescript II SK (+), pKK223-3, or pSC101 including a region that can be autonomously replicated in E. coli, or pUB 110, pTZ4, pC194, ρ11, ϕ1, or ϕ105 including a region that can be autonomously replicated in *Bacillus subtilis.*

Examples of the vector that can be autonomously replicated in two or more kinds of host microorganisms include pHV14, TRp7, YEp7, and pBS7.

In the case of introducing a gene from outside of a cell in the host microorganism, the gene may be a gene having a nucleotide sequence that is not present in the genome of the host microorganism, and may be a gene having a nucleotide sequence that is present in the genome of the host microorganism. Even when the same gene originally exists in the genome of a host microorganism, the introduction of a gene from outside the bacterial cell causes stronger gene expression, and the enzymatic activity is enhanced and the production efficiency of pyridoxamine or a salt thereof can be further enhanced.

Conventional methods well known to those skilled in the art can be used as methods necessary for introducing a gene from outside of a bacterial cell (extracellular) into the bacterial cell (intracellular), such as preparation of a genomic DNA, cleavage and ligation of DNA, transformation, PCR (Polymerase Chain Reaction), and design and synthesis of an oligonucleotide used as a primer. These methods are described in Sambrook, J., et al., "Molecular Cloning A Laboratory Manual, Second Edition", Cold Spring Harbor Laboratory Press, (1989) and the like. For example, a method using competent cells and a method using electroporation are described.

The host microorganism is not particularly limited as long as the microorganism is a microorganism capable of expressing, if present, a gene encoding the pyridoxine dehydrogenase, a gene encoding the pyridoxamine synthetase, and a gene encoding the amino acid regeneration enzyme. Examples of the host microorganism include yeasts, filamentous fungi, and prokaryotes. Examples of the yeasts include a yeast belonging to the genus *Saccharomyces*, such as *Saccharomyces cerevisiae*, a yeast belonging to the genus *Schizosaccharomyces*, such as *Schizosaccharomyces pombe*, a yeast belonging to the genus *Hansenula*, and a yeast belonging to the genus *Pichia.* Examples of the filamentous fungi include a filamentous fungus belonging to the genus *Trichoderma*, such as *Trichoderma reesei* or *viride*, a filamentous fungus belonging to the genus *Aspergillus*, such as *Aspergillus niger* or *oryzae*, a filamentous fungus belonging to the genus *Humicola*, such as *Humicola insolens*, and a filamentous fungus belonging to the genus *Acremonium*, such as *Acremonium cellulolyticus* or *fusidioides.* Examples of the prokaryotes include a bacterium belonging to the genus *Escherichia*, such as *Escherichia coli*, a bacterium belonging to the genus *Schewanella*, such as *Schewanella* sp. AC10, a bacterium belonging to the genus *Mesorhizobium*, such as *Mesorhizobium loti*, a bacterium belonging to the genus *Rhizobium*, such as *Rhizobium meliloti*, a *Bacillus* bacterium belonging to the genus *Bacillus*, such as *Bacillus subtilis,* and an actinomycete such as an actinomycete belonging to the genus *Streptomyces*, such as *Streptomyces lividans.*

A desired gene can be introduced into a host microorganism by, for example, introducing (transforming) into these host microorganisms an expression vector including the desired gene. The introduced gene can be highly expressed in the obtained recombinant microorganism, for example, by the activity of a promoter included in the expression vector.

In a case in which the host microorganism is *E. coli*, for example, a competent cell method by calcium treatment, an electroporation method, or the like can be used as a method of introducing the recombinant DNA such as an expression vector into a cell of the host microorganism. The enzyme encoded by the introduced gene can be stably produced at a high expression level by culturing a recombinant microorganism obtained in such a manner.

The DNA encoding the enzyme can be isolated from the recombinant microorganism, and can be transferred into another microorganism. Using this DNA as a template, the DNA encoding the enzyme can be easily introduced into another host microorganism, by amplifying a DNA fragment encoding an enzyme by PCR, treating the resultant with a restriction enzyme or the like, and then the obtained fragment is ligated with another vector DNA fragment.

### <Method of Producing Pyridoxamine or Salt Thereof>

In one embodiment, a method of producing pyridoxamine or a salt thereof includes bringing the recombinant microorganism, the culture of the recombinant microorganism, or the treated product of the recombinant microorganism or the culture according to the first aspect, into contact with pyridoxine or a salt thereof to produce pyridoxamine or a salt thereof.

Examples of a salt of pyridoxine include a salt of pyridoxine and an acid. Examples of the acid include hydrochloric acid, sulfuric acid, nitric acid, and phosphoric acid. Examples of the salt of pyridoxine include pyridoxine hydrochloride.

Examples of the salt of pyridoxamine include a salt of pyridoxamine with an acid. Examples of the acid include hydrochloric acid, sulfuric acid, nitric acid, acetic acid, and phosphoric acid. From the viewpoint of use as a medicament, the salt of pyridoxamine is preferably pyridoxamine dihydrochloride, which is most advanced for medical applications.

Such a method of producing pyridoxamine or a salt thereof using the recombinant microorganism, the culture of the recombinant microorganism, or the treated product of the recombinant microorganism or the culture is also simply referred to as "method of producing pyridoxamine or a salt thereof using a recombinant microorganism".

The culture of the recombinant microorganism refers to a product obtained by culturing the recombinant microorganism, which is composed of a bacterial cell, a surrounding medium, and the like. The use of the culture is not necessarily required. For example, a dried or frozen recombinant microorganism cell prepared in advance may be added directly to a reaction system.

When culturing a recombinant microorganism, any of a synthetic medium or a natural medium can be used as a culture medium, as long as the culture medium is a culture medium including adequate amounts of a carbon source, a nitrogen source, an inorganic substance, and another nutrient. The culture can be performed using a general culture method, such as shaking culture, aeration stirring culture, continuous culture, or fed-batch culture in a liquid medium including the culture components.

More specifically, the culture condition for the recombinant microorganism is the same as the culture condition for the original host microorganism, and known conditions can be used.

Known components can be used as the components used in the culture medium. For example, organic nutrient sources such as a meat extract, a yeast extract, a malt extract, peptone, NZ amine, and potato, carbon sources such as glucose, maltose, sucrose, starch, and an organic acid, nitrogen sources such as ammonium sulfate, urea, and ammonium chloride, inorganic nutrient sourced such as phosphate, magnesium, potassium, and iron, and vitamins can be used in combination, if appropriate.

In culture of a recombinant microorganism transformed by the expression vector including the selection marker, for example, a culture medium including an agent against the drug resistance is used in a case in which the selection marker has drug resistance, and a culture medium that does not include a nutrient is used in a case in which the selection marker has a requirement for the nutrient.

The culture conditions may be selected, if appropriate, depending on the kinds of the recombinant microorganism, a culture medium, and a culture method, and are not particularly limited as long as the culture conditions are conditions under which the recombinant microorganism grows and can produce a pyridoxine dehydrogenase, a pyridoxamine synthetase, and an amino acid regeneration enzyme.

The pH of the culture medium may be selected, for example, in a range of from pH 4 to pH 8, and may be in a range of from pH 5 to pH 8.

The culture temperature is, for example, from 20°C to 45°C, and preferably from 24°C to 37°C. The culture may be performed aerobically or anaerobically depending on the kinds of a microorganism.

The culture period is, for example, from 1 day to 7 days. The culture period may be set to maximize the production amount of the target enzyme.

The treated product of the recombinant microorganism refers to a product obtained by any treatment of the recombinant microorganism as long as the activities of the pyridoxine dehydrogenase, the pyridoxamine synthetase, and the amino acid regeneration enzyme produced by the recombinant microorganism are not lost. Examples of such treatment include one or more selected from the group consisting of heat treatment, cooling treatment, mechanical destruction, ultrasonic treatment, freeze-thaw treatment, drying treatment, pressurized or reduced pressure treatment, osmotic pressure treatment, autolysis, surfactant treatment, and enzyme treatment (for example, cell lysis treatment). Even in a case in which the recombinant microorganism per se is killed by such treatment, such a treated product can be used for a reaction as long as the activity of the enzyme produced by the microorganism remains.

The treated product of the culture refers to a product obtained by any treatment of the culture of the recombinant microorganism as long as the activities of the pyridoxine dehydrogenase, the pyridoxamine synthetase, and the amino acid regeneration enzyme produced by the recombinant microorganism are not lost. Examples of such treatment include one or more selected from the group consisting of heat treatment, cooling treatment, mechanical destruction of a cell, ultrasonic treatment, freeze-thaw treatment, drying treatment, pressurized or reduced pressure treatment, osmotic pressure treatment, cell autolysis, surfactant treatment, enzyme treatment (for example, cell destruction treatment), cell separation treatment, purification treatment, and extraction treatment. For example, a cell of the recombinant microorganism may be separated from a culture medium or the like, and the separated cell may be added to a reaction system. Means such as filtration or centrifugation can be used for such separation. Alternatively, purification treatment for separating, from a contaminant, the pyridoxine dehydrogenase, the pyridoxamine synthetase, and the amino acid regeneration enzyme may be performed, and a solution including an enzyme obtained by the purification treatment may be added to the reaction system. Alternatively, an extract obtained by extracting the culture using an organic solvent such as methanol or acetonitrile, or a mixed solvent of an organic solvent and water may be added to the reaction system. It is also acceptable that such a purified product or extract does not include a cell of a recombinant microorganism. Even in a case in which the cell of the microorganism does not exist, such a purified product or extract can be used for a reaction as long as the enzymatic activity remains.

Such disruption or lysis treatment of a cell as described above can be performed by destructing the cell membrane of the recombinant microorganism according to a known method such as lysozyme treatment, freeze-thawing, or ultrasonic disruption.

It is preferable that the recombinant microorganism, the culture of the recombinant microorganism, or the treated product of the recombinant microorganism or the culture according to the first aspect, is brought into contact with pyridoxine or a salt thereof under the following conditions.

The contact is preferably performed in a solution including pyridoxine or a salt thereof as a substrate. The pH of the solution is not particularly limited as long as the enzymatic activities of the pyridoxine dehydrogenase, the pyridoxamine synthetase, and the amino acid regeneration enzyme are maintained, and is preferably from pH 6.0 to pH 9.0, and more preferably from pH 7.0 to pH 8.5. The temperature of the solution is also not particularly limited as long as the enzymatic activities of the pyridoxine dehydrogenase, the pyridoxamine synthetase, and the amino acid regeneration enzyme are maintained, and is preferably from 20°C to 70°C, and more preferably from 25°C to 50°C.

As a medium for the solution, water or an aqueous medium, an organic solvent, or a mixed solvent of an organic solvent and water or an aqueous medium may be used. Examples of the aqueous medium include buffers such as a phosphate buffer, a HEPES (N-2-hydroxyethylpiperazine-N-ethanesulfonic acid) buffer, and a tris[tris(hydroxymethyl)aminomethane]hydrochloride buffer. Any organic solvent may be used as long as the solvent does not inhibit a reaction, and examples thereof include acetone, ethyl acetate, dimethyl sulfoxide, xylene, methanol, ethanol, and butanol.

It is preferable that the recombinant microorganism, the culture of the recombinant microorganism, or the treated product of the recombinant microorganism or the culture according to the first aspect, is brought into contact with pyridoxine or a salt thereof under a shaking or stirring condition. For example, such contact can be performed in a solution. For example, pyridoxine or a salt thereof in the form of a substrate solution or in the form of a solid may be added to a solution including the recombinant microorganism, the culture of the recombinant microorganism, or the treated product of the recombinant microorganism or the culture. An amino acid to be consumed by a pyridoxamine synthetase may be further added to a solution including the recombinant microorganism, the culture of the recombinant microorganism, or the treated product of the recombinant microorganism or the culture. The amino acid may be included in a substrate solution including pyridoxine or a salt thereof, and may be added to a solution including the recombinant microorganism, the culture of the recombinant microorganism, or the treated product of the recombinant microorganism or the culture together with the pyridoxine or a salt thereof. Alternatively, pyridoxine or a salt thereof included in a substrate solution other than the substrate solution, or included in the form of a solid may be added to a solution including the recombinant microorganism, the culture of the recombinant microorganism, or the treated product of the recombinant microorganism or the culture.

An acid or an alkali may be added to maintain the pH of a reaction liquid in an appropriate range at the initiation of the reaction or during the reaction. Examples of the alkali that can be added to the reaction liquid include an alkali metal hydroxide such as lithium hydroxide, sodium hydroxide, or potassium hydroxide; and one such as ammonium hydroxide, calcium hydroxide, dipotassium phosphate, disodium phosphate, potassium pyrophosphate, or ammonia which is dissolved in water to make the liquid basic. Examples of the acid that can be added to a reaction liquid include hydrochloric acid, sulfuric acid, nitric acid, acetic acid, and phosphoric acid.

The contact may be performed, for example, under an air atmosphere or under a partial deoxygenation atmosphere. The deoxidizing atmosphere can be achieved by substitution with an inert gas, pressure reduction, boiling, or a combination thereof. It is preferable at least to replace with an inert gas, or to use an inert gas atmosphere. Examples of the inert gas include nitrogen gas, helium gas, argon gas, and carbon dioxide, and nitrogen gas is preferable.

In a preferable embodiment, the recombinant microorganism, the culture of the recombinant microorganism, or the treated product of the recombinant microorganism or the culture to be used includes the pyridoxine dehydrogenase, the pyridoxamine synthetase, and the amino acid regeneration enzyme having an enzymatic activity of regenerating an amino acid consumed by the pyridoxamine synthetase. For this reason, the pyridoxine dehydrogenase, the pyridoxamine synthetase, and the amino acid regeneration enzyme having an enzymatic activity of regenerating an amino acid consumed by the pyridoxamine synthetase, which are present together in the reaction liquid, act cooperatively when contacting, and pyridoxamine or a salt thereof is produced with high production efficiency. Although it is not essential that the treated product of a recombinant microorganism or the treated product of a culture includes the recombinant microorganism in a living state, from the viewpoint of continuously supplying the substance involved in the reaction by metabolism, it is preferable to include the recombinant microorganism in a living state.

Regarding the timing of addition, the recombinant microorganism, the culture of the recombinant microorganism, or the treated product of the recombinant microorganism or the culture, may be added all at once at the initiation of a reaction, or may be added in batches or continuously during a reaction. Similarly, pyridoxamine, which is a raw material, may be added all at once at the initiation of a reaction, or may be added in batches or continuously during a reaction.

The reaction liquid may include an amino acid to be consumed in the production of pyridoxamine or a salt thereof by a pyridoxamine synthetase. This amino acid may also be added all at once at the initiation of a reaction, or may be added in batches or continuously during a reaction. For example, in a case in which a pyridoxamine-pyruvate transaminase is used as a pyridoxamine synthetase, the reaction liquid may include one or more of L-alanine and D-alanine. In a case in which a pyridoxamine-oxaloacetate transaminase or an aspartate transaminase is used as a pyridoxamine synthetase, the reaction liquid may include at least one of L-aspartic acid, D-aspartic acid, L-glutamic acid, or D-glutamic acid. An amino acid may also exist as a salt depending on a surrounding environment; however, in the disclosure, a description of amino acid is given with inclusion of the mention of a salt. For example, the description of L-glutamic acid includes not only L-glutamic acid which has not formed salt, but also L-glutamic acid which has formed salt (for example, monosodium L-glutamate monohydrate). Examples of counter ions in the case of forming a salt include cations such as sodium ion and potassium ion, and anions such as chloride ion, acetate ion, and nitrate ion.

The concentration of pyridoxine or a salt thereof in the reaction liquid may be, for example, from 10 mM to 1 M, or may be from 50 mM to 800 mM, from 70 mM to 500 mM, or from 0.8 mM to 50 mM. The concentration of the amino acid (the amino acid to be consumed in producing pyridoxamine or a salt thereof by a pyridoxamine synthetase) may be, for example, from 0.1 mM to 2 M, or may be from 1 mM to 1 M, from 2 mM to 500 mM, from 2 mM to 400 mM, from 5 mM to 150 mM, or from 10 mM to 100 mM. Alternatively, the concentration of the amino acid may be from 5 mM to 800 mM, from 10 mM to 400 mM, or from 10 mM to 200 mM.

In the method of producing pyridoxamine or a salt thereof according to the first aspect, pyridoxamine or a salt thereof can be produced with high production efficiency even when the concentration of the amino acid in the reaction liquid is relatively low. It is a surprising effect that pyridoxamine or a salt thereof can be produced with high production efficiency even when relatively low concentration of the amino acid in used. Until now, for the production of pyridoxamine from pyridoxal, a relatively high concentration of glutamic acid was used in Journal of Molecular Catalysis B: Enzymatic, 2010, vol. 67, p. 104-110, and a relatively high concentration of alanine and glutamic acid was used in the wording example of WO 2007/142222. Pyridoxamine has an amino group, and an amino acid is considered to be consumed as a source providing the amino group. Therefore, it is considered that, in Journal of Molecular Catalysis B: Enzymatic, 2010, vol. 67, p. 104-110 and WO 2007/142222, a high concentration of amino acid was included in a reaction liquid in consideration of amino acid consumption. Although the reason that the above effect can be obtained in the method of producing pyridoxamine or a salt thereof according to the first aspect is not necessarily clear, it is assumed that an amino acid can be regenerated due to the presence of the amino acid regeneration enzyme in the recombinant microorganism according to the first aspect, whereby the production of pyridoxamine or a salt thereof is not arrested even when the amino acid concentration is not high, and pyridoxamine or a salt thereof can be produced at high production efficiency. As a result, according to the method of producing pyridoxamine or a salt thereof according to the first aspect, a cost of adding a large amount of amino acid can be saved, and time for performing an operation to maintain a high amino acid concentration by monitoring the concentration of an amino acid during a reaction can be save.

Examples of the method of bringing the recombinant microorganism, the culture of the recombinant microorganism, or the treated product of the recombinant microorganism or the culture, into contact with pyridoxine or a salt thereof include: a method of adding the recombinant microorganism, the culture of the recombinant microorganism, or the treated product of the recombinant microorganism or the culture, to a solution including pyridoxine or a salt thereof and allowing a reaction to proceed while stirring; a method of adding the recombinant microorganism, the culture of the recombinant microorganism, or the treated product of the recombinant microorganism or the culture, to a solution including pyridoxine or a salt thereof and allowing a reaction to proceed while shaking; and a method of mixing the recombinant microorganism, the culture of the recombinant microorganism, or the treated product of the recombinant microorganism or the culture and pyridoxine or a salt thereof thoroughly in a solution, then allowing the mixture to stand still for allowing a reaction to proceed. From the viewpoint of reaction efficiency, preferable examples thereof include a method of adding the recombinant microorganism, the culture of the recombinant microorganism, or the treated product of the recombinant microorganism or the culture, to a solution including pyridoxine or a salt thereof and allowing a reaction to proceed while stirring.

A reaction vessel that can be used for a reaction is not particularly limited. The reaction vessel is preferably a vessel in which the recombinant microorganism, the culture of the recombinant microorganism, or the treated product of the recombinant microorganism or the culture, and the solution including pyridoxine or a salt thereof added thereto can be stirred enough to mix them sufficiently, and which has a temperature control function of keeping the temperature within an optimum temperature range for a pyridoxine dehydrogenase, a pyridoxamine synthetase, and an amino acid regeneration enzyme.

The contact time (reaction time) of the recombinant microorganism, the culture of the recombinant microorganism, or the treated product of the recombinant microorganism or the culture, with pyridoxine or a salt thereof is not particularly limited as long as the enzymatic activities of the pyridoxine dehydrogenase, the pyridoxamine synthetase, and the amino acid regeneration enzyme are maintained. For example, the contact time may be from 30 minutes to 100 hours, and may be from 2 hours to 50 hours. The reaction may be performed with a batch method, may be performed with a semi-batch method in which either or both of a substrate, and the microorganism, the culture or the treated product are added in batches during the reaction, or may be performed with a continuous method. In the case of the semi-batch method or the continuous method, the upper limit of the reaction time is not particularly limited since an operation such as supplying either or both of a new raw material, and the recombinant microorganism, the culture, or the treated product is conducted.

In the above method, the use of the recombinant microorganism, the culture of the recombinant microorganism, or the treated product of the recombinant microorganism or the culture according to the first aspect, allows the production of pyridoxamine or a salt thereof inexpensively with high production efficiency by using pyridoxine or a salt thereof as a raw material, without the need to carry out a complicated step as in a chemical synthesis method. Pyridoxamine or a salt thereof obtained by the above-described method can be used, for example, for producing a product that utilizes its physiological activity. For example, the product can be used for an application such as prevention or treatment of a disease or schizophrenia caused by AGE accumulation such as diabetes mellitus, atherosclerosis, chronic renal failure, or Alzheimer's dementia, a health food, or a cosmetic.

### <Second Aspect>

A recombinant microorganism selectively producing pyridoxal with high efficiency using pyridoxine as a substrate has not been known until now.

The structures of pyridoxine and pyridoxal are illustrated below. In the disclosure, the words "pyridoxine" and "pyridoxal" are used so as to be intended to encompass each salt thereof unless it is clearly described that the salt is particularly excluded. Examples of salts of pyridoxine include pyridoxine hydrochloride, pyridoxine sulfate, pyridoxine nitrate, and pyridoxine phosphate. Examples of salts of pyridoxal include pyridoxal hydrochloride, pyridoxal sulfate, pyridoxal nitrate, and pyridoxal phosphate.

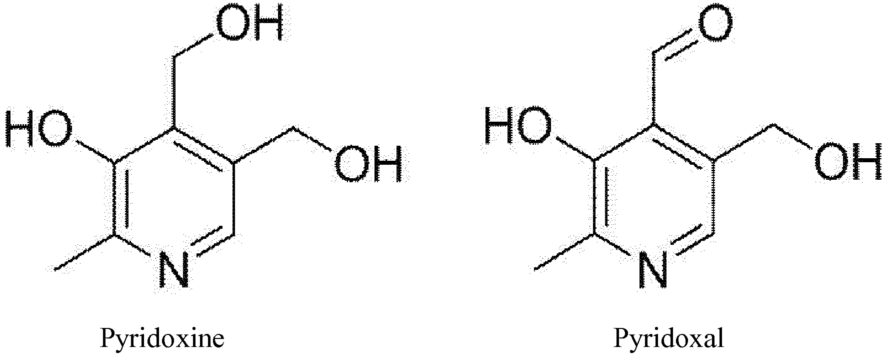

### <Recombinant Microorganism according to Second Aspect>

### [Amino Acid Sequence of SEQ ID NO:1]

The amino acid sequence of SEQ ID NO:1 is a pyridoxine dehydrogenase derived from *Saccharomyces cerevisiae.* The pyridoxine dehydrogenase is an oxidoreductase that catalyzes a reaction of producing pyridoxal, NADPH, and H⁺ from pyridoxine and NADP⁺ and a reaction reverse thereto, as described below. The pyridoxine dehydrogenase derived from *Saccharomyces cerevisiae* is classified under enzyme number EC 1.1.1.65 according to the report of the enzyme commission of the International Union of Biochemistry (I.U.B.).

Pyridoxine + NADP⁺ ⇆ Pyridoxal + NADPH + H⁺

In the recombinant microorganism according to the second aspect, a polynucleotide introduced into a host microorganism is at least one polynucleotide selected from the group consisting of the following (1) to (7).

### (1) Polynucleotide Encoding Protein Having Amino Acid Sequence of SEQ ID NO:1

In the recombinant microorganism according to the second aspect, the polynucleotide introduced into a host microorganism is preferably a polynucleotide encoding a protein having the amino acid sequence of SEQ ID NO:1. In the case of the polynucleotide that encodes a protein including the amino acid sequence of SEQ ID NO:1, any codon may be used. Preferably, a polynucleotide having the nucleotide sequence of SEQ ID NO:13 is used.

### (2) Polynucleotide Encoding Protein Having Amino Acid Sequence in Which from 1 to 50 Amino Acids Are Deleted, Added, or Substituted in Amino Acid Sequence of SEQ ID NO:1

In the recombinant microorganism according to the second aspect, the polynucleotide introduced into a host microorganism may be a polynucleotide that encodes a protein having an amino acid sequence in which from 1 to 50 amino acids are deleted, added, or substituted in the amino acid sequence of SEQ ID NO:1. The protein has an activity of synthesizing pyridoxine or pyridoxal. The protein preferably has an enzymatic activity classified under EC1.1.1.65.

In the second aspect, the activity of synthesizing pyridoxine refers to a capability of synthesizing pyridoxine using pyridoxal as a substrate, and the activity of synthesizing pyridoxal refers to a capability of synthesizing pyridoxal using pyridoxine as a substrate.

The number of amino acid residues deleted, added, or substituted in the amino acid sequence of SEQ ID NO:1 is preferably from 1 to 40, or from 1 to 30, still more preferably from 1 to 20, or from 1 to 15, and particularly preferably from 1 to 10, or from 1 to 5.

The activity of synthesizing pyridoxine can be confirmed by, for example, bringing a protein encoded by the polynucleotide of any one of the above (1) to (7) into contact with pyridoxal and NADPH under an appropriate environment such as phosphate buffered saline, and detecting the production of pyridoxine. The activity of synthesizing pyridoxal can be confirmed by bringing a protein encoded by the polynucleotide of any one of the above (1) to (7) into contact with pyridoxine and NADP⁺ under an appropriate environment such as phosphate buffered saline, and detecting the production of pyridoxal.

The detection of pyridoxal or pyridoxine can be performed by a known method, for example, high performance liquid chromatography (HPLC).

### (3) Polynucleotide Encoding Protein Having Amino Acid Sequence Having 60% or More Sequence Identity with Amino Acid Sequence of SEQ ID NO:1

In the recombinant microorganism according to the second aspect, the polynucleotide introduced into a host microorganism may be a polynucleotide that encodes a protein having at least 60% sequence identity with the amino acid sequence of SEQ ID NO 1, and having an activity of synthesizing pyridoxine or pyridoxal. The protein preferably has an enzymatic activity classified under EC1.1.1.65.

The sequence identity is preferably at least 70%, more preferably at least 80%, or at least 85% or more, and still more preferably at least 90%, at least 91%, at least 92%, at least 93%, at least 94%, at least 95%, at least 96%, at least 97%, at least 98%, at least 99%, or 100%.

### (4) Polynucleotide Having Nucleotide Sequence of SEQ ID NO:13

In the recombinant microorganism according to the second aspect, the polynucleotide introduced into a host microorganism is preferably a polynucleotide having the nucleotide sequence of SEQ ID NO:13.

The polynucleotide having the nucleotide sequence of SEQ ID NO:13 encodes a pyridoxine dehydrogenase from *Saccharomyces cerevisiae*, which is a protein having the amino acid sequence of SEQ ID NO:1.

### (5) Polynucleotide Having Nucleotide Sequence in Which from 1 to 150 Nucleotides Are Deleted, Added, or Substituted in Nucleotide Sequence of SEQ ID NO:13

In the recombinant microorganism according to the second aspect, the polynucleotide introduced into a host microorganism may be a polynucleotide having a nucleotide sequence in which from 1 to 150 nucleotides are deleted, added, or substituted in the nucleotide sequence of SEQ ID NO:13. The protein encoded by the polynucleotide has an activity of synthesizing pyridoxine or pyridoxal, and preferably has an enzymatic activity classified under EC1.1.1.65.

The number of nucleotides deleted, added, or substituted in the nucleotide sequence of SEQ ID NO:13 is preferably from 1 to 140, from 1 to 120, from 1 to 100, from 1 to 90, from 1 to 80, from 1 to 70, from 1 to 60, or from 1 to 50, more preferably from 1 to 40, from 1 to 30, from 1 to 20, or from 1 to 15, and still more preferably from 1 to 10, or from 1 to 5.

### (6) Polynucleotide Having Nucleotide Sequence Having 60%or More Sequence Identity with Nucleotide Sequence of SEQ ID NO:13

In the recombinant microorganism according to the second aspect, the polynucleotide introduced into a host microorganism may be a polynucleotide including a nucleotide sequence having 60% or more sequence identity with the nucleotide sequence of SEQ ID NO:13. A protein encoded by the polynucleotide has an activity of synthesizing pyridoxine or pyridoxal, and the protein preferably has an enzymatic activity classified under EC1.1.1.65.

The sequence identity is preferably at least 70%, more preferably at least 80%, or at least 85% or more, and still more preferably at least 90%, at least 91%, at least 92%, at least 93%, at least 94%, at least 95%, at least 96%, at least 97%, at least 98%, at least 99%, or 100%.

### (7) Polynucleotide that Hybridizes with Polynucleotide Having Nucleotide Sequence Complementary to Nucleotide Sequence of SEQ ID NO:13 under Stringent Condition

In the recombinant microorganism according to the second aspect, the polynucleotide introduced into a host microorganism may be a polynucleotide that hybridizes with a polynucleotide having a nucleotide sequence complementary to the nucleotide sequence of SEQ ID NO:13 under a stringent condition. A protein encoded by the polynucleotide has an activity of synthesizing pyridoxine or pyridoxal, and the protein preferably has an enzymatic activity classified under EC1.1.1.65.

The stringent conditions refer to conditions under which a specific hybrid is formed, and a nonspecific hybrid is not formed. As the stringent conditions, conditions according to a sequence to be compared can be set, if appropriate, on the basis of the description of Molecular Cloning 3rd (J. Sambrook et al., Cold Spring Harbor Lab. Press, 2001). This document is incorporated herein by reference.

Typical stringent conditions mean, for example, stringent hybridization conditions and stringent washing conditions. Examples thereof include conditions that a temperature of 55°C is maintained overnight, together with a probe, in a solution including 6 × SSC (composition of 1 × SSC: 0.15 M NaCl, 0.015 M sodium citrate, pH 7.0), 0.5% SDS, 5 × Denhardt, and 100 mg/mL herring sperm DNA. Examples thereof may include subsequent washing of a filter in 0.2 × SSC at 42°C. Examples of the stringent conditions may include conditions of 0.1 × SSC and 50°C in a step of washing a filter, and, in addition, examples of the stringent conditions can include conditions of 0.1 × SSC and 65°C in the same step.

The sequence identity with the amino acid sequence of SEQ ID NO:1 is expressed as a percentage obtained by multiplying, by 100, a value obtained by dividing the number of identical amino acids, in the case of performing optimal alignment to an amino acid sequence to be compared, by 345 which is the number of the amino acid residues of SEQ ID NO:1.

The sequence identity with the polynucleotide sequence of SEQ ID NO:13 is expressed as a percentage obtained by multiplying, by 100, a value obtained by dividing the number of identical nucleotides, in the case of performing optimal alignment to a polynucleotide sequence to be compared, by 1055 which is the number of the nucleotides of the polynucleotide of SEQ ID NO:13.

The optimal alignment refers to alignment in which the number of amino acids or nucleotides matching between two sequences is the greatest. The optimal alignment between sequences can be performed by using a known method, for example, a BLAST (registered trademark, National Library of Medicine) program while turning off mask filtering in the default parameters of the program.

The less number of amino acid residues or nucleotides deleted, added, or substituted in the amino acid sequence of SEQ ID NO:1 or the nucleotide sequence of SEQ ID NO:13 in the polynucleotide described in any one of the (2) (3), (5), or (6) described above is preferred, and a higher identity with the amino acid sequence of SEQ ID NO:1 or the nucleotide sequence of SEQ ID NO:13 is preferred. However, in the case of a variation that has no or less influence on enzymatic activity, the effect caused by the recombinant microorganism according to the second aspect can be exhibited even in a case in which the number of deleted, added, or substituted amino acid residues or nucleotides is large. For example, it is well known to those skilled in the art that substitution between amino acids having similar properties has little influence on the function of a protein. As another example, it is well known to those skilled in the art that there are plural kinds of codons that encode one amino acid, and even in the case of polynucleotides that encode an identical amino acid sequence, differences between used codons may result in many differences from the nucleotide sequence of SEQ ID NO:13.

In the recombinant microorganism according to the second aspect, a protein having a capability of synthesizing pyridoxal using pyridoxine as a substrate, or a protein having a capability of synthesizing pyridoxine using pyridoxal as a substrate is expressed from an introduced gene, and a protein having an enzymatic activity classified under EC1.1.1.65 is preferably expressed.

The expression of the protein having a capability of synthesizing pyridoxal using pyridoxine as a substrate or the protein having a capability of synthesizing pyridoxine using pyridoxal as a substrate from an introduced gene in the recombinant microorganism according to the second aspect may be confirmed by a known method. The expression of the protein can be confirmed by bringing the recombinant microorganism, into which a gene is introduced, into contact with pyridoxine or pyridoxal as a substrate, and detecting the production of pyridoxine or pyridoxal as the resultant of enzyme reaction. In the case of a recombinant microorganism in which different reactions occur before and after the synthesis of pyridoxine or pyridoxal, the production of pyridoxine or pyridoxal as the resultant of enzyme reaction from pyridoxine or pyridoxal as a substrate can be theoretically confirmed in consideration of the reactions before and after the synthesis.

### <Preparation of Recombinant Microorganism according to Second Aspect>

### [Host]

As the microorganism used as a host in the case of preparing the recombinant microorganism according to the second aspect, a microorganism used in the expression of a protein can be commonly used as the host. Examples of the host microorganism include yeasts, filamentous fungi, and prokaryotes. Examples of the yeasts include a yeast belonging to the genus *Saccharomyces*, such as *Saccharomyces cerevisiae*, a yeast belonging to the genus *Schizosaccharomyces*, such as *Schizosaccharomyces pombe*, a yeast belonging to the genus *Hansenula*, and a yeast belonging to the genus *Pichia.* A microorganism capable of selectively and efficiently producing pyridoxal can be obtained by introducing the polynucleotide of any one of the (1) to (7) described above as well as an endogenous pyridoxine dehydrogenase in a case in which *Saccharomyces cerevisiae* is used as the host.

Examples of the filamentous fungi include a filamentous fungus belonging to the genus *Trichoderma*, such as *Trichoderma reesei* or *viride*, a filamentous fungus belonging to the genus *Aspergillus*, such as *Aspergillus niger* or *oryzae*, a filamentous fungus belonging to the genus *Humicola*, such as *Humicola insolens*, and a filamentous fungus belonging to the genus *Acremonium*, such as *Acremonium cellulolyticus* or *fusidioides.*

Examples of bacteria include an *Escherichia* bacterium, a *Bacillus* bacterium, a bacterium belonging to the genus *Corynebacterium*, and the genus *Rhodococcus. E. coli* (*Escherichia coli*) which has been industrially used in many cases is particularly preferably used. Examples of hosts other than *E. coli* include *Bacillus brevis*, *Bacillus megaterium*, *Brevibacillus choshinensis*, *Corynebacterium glutamicum*, and *Rhodococcus erythropolis.*

### [Introduction into Host of Polynucleotide]

A method of introducing a polynucleotide into a host is known. A target polynucleotide can be introduced into a host by, for example, a common method, described in "Molecular Cloning 3rd Edition" (J. Sambrook et al.; Cold Spring Harbor Laboratory Press, 2001) and the like, and known in the fields of molecular biology, biotechnology, and genetic engineering. For example, a recombinant microorganism can be obtained by preparing an expression vector including the polynucleotides described in (1) to (6), and transforming any host microorganism by the expression vector.

In a case in which the host is bacterium, a competent cell method by calcium treatment, an electroporation method, or the like can be used as a method of introducing the recombinant DNA of an expression vector or the like into a cell of the host bacterium. A large amount of pyridoxine dehydrogenase can be stably expressed by culturing a recombinant bacterium obtained in such a manner.

The expression vector is not particularly limited as long as the expression vector includes the polynucleotide described in any one of the (1) to (7) as above. A common expression vector known in the field of genetic engineering can be adopted as the expression vector. The expression vector may include an element, if necessary, such as a control region necessary for the expression of the polynucleotide, or a region necessary for autonomous replication, enabling a protein to be produced by a recombinant microorganism. Examples of the control region necessary for the expression include a promoter sequence (including an operator sequence that controls transcription), a ribosome-binding sequence (SD sequence), a transcription termination sequence, a restriction enzyme cleavage sequence, or other genes. The other genes may be, for example, a protein having an action of converting produced pyridoxine or pyridoxal as an intermediate product into another material.

Examples of the promoter sequence include the trp promoter of a tryptophan operon and the lac promoter of a lactose operon derived from *E. coli*, a PL promoter and a PR promoter derived from lambda phage, and a gluconate synthase promoter (gnt), a glyceraldehyde 3-phosphate dehydrogenase (GAPDH) promoter, a glutamate decarboxylase A (gadA) promoter, a serine hydroxymethyltransferase (glyA) promoter, an alkaline protease promoter (apr), a neutral proteinase promoter (npr), or an α-amylase promoter (amy) derived from *Bacillus subtilis.*

An originally modified or designed promoter sequence such as a tac promoter can also be used.

Examples of the ribosome-binding sequence include a sequence derived from *E. coli* or *Bacillus subtilis,* and the ribosome-binding sequence is not particularly limited as long as the ribosome-binding sequence is a sequence that functions in a desired host cell such as *E*. *coli* or *Bacillus subtilis.*

Examples of the ribosome-binding sequence include a sequence in which a consensus sequence having four or more consecutive nucleotides, of sequences complementary to the 3' end region of a 16S ribosomal RNA, is produced by DNA synthesis.

The transcription termination sequence is not necessarily required, and a ρ-factor independent terminator, for example, a lipoprotein terminator, a trp operon terminator, or the like can be used.

The order of these sequences on the expression vector of the control region is not particularly limited, and the promoter sequence, the ribosome-binding sequence, a gene encoding a target protein, and the transcription termination sequence are desirably aligned in the order mentioned above from the upstream (5'-end side) in consideration of transcription efficacy.

Specific examples of the expression vector herein include pBR322, pUC18, Bluescript II SK (+), pKK223-3, or pSC101 including a region that can be autonomously replicated in *E*. *coli*, or pUB110, pTZ4, pC194, ρ11, ϕ1, or ϕ105 including a region that can be autonomously replicated in *Bacillus subtilis*, which can be used as the expression vector.

Examples of expression vectors that can be autonomously replicated in two or more kinds of hosts include pHV14, TRp7, YEp7, and pBS7, which can be used as the expression vectors.

### <Method of Producing Pyridoxal>

A method of producing pyridoxal according to the second aspect includes bringing the recombinant microorganism according to the second aspect into contact with pyridoxine. The recombinant microorganism obtained by introducing the expression vector described above into a desired host microorganism produces a protein encoded by a polynucleotide included in the expression vector. The produced protein generates pyridoxal using pyridoxine as a substrate.

The recombinant microorganism according to the second aspect may be proliferated by culturing the recombinant microorganism. Culture conditions are similar to the conditions under which the host cell is cultured, and known conditions can be used as the culture conditions.

Any of a synthetic medium and a natural medium can be used as a culture medium as long as the culture medium is a culture medium including adequate amounts of a carbon source, a nitrogen source, an inorganic substance, and another nutrient. Known components can be used as the components used in the culture medium. For example, organic nutrient sources such as a meat extract, a yeast extract, a malt extract, peptone, NZ amine, and potato, carbon sources such as glucose, maltose, sucrose, starch, and an organic acid, nitrogen sources such as ammonium sulfate, urea, and ammonium chloride, inorganic nutrient sources such as a phosphate, magnesium, potassium, and iron, and vitamins can be used in combination, if appropriate.

In culture of a recombinant microorganism transformed by the expression vector including the selection marker, for example, a culture medium including an agent against the drug resistance is used in a case in which the selection marker has the drug resistance, and a culture medium that does not include a nutrient is used in a case in which the selection marker has a requirement for the nutrient. The pH of the culture medium may be selected in a range of from pH 4 to pH 8.

The culture of the recombinant microorganism can be performed using a usual culture method such as shaking culture, aeration stirring culture, continuous culture, or fed-batch culture in a liquid medium containing the culture medium.

The culture conditions may be selected, if appropriate, depending on the kinds of a host, a culture medium, and a culture method, and are not particularly limited as long as the culture conditions are conditions under which a recombinant microorganism grows, and the recombinant microorganism according to the second aspect can be produced.

The culture is aerobically performed at a culture temperature of from 20°C to 45°C, preferably from 24°C to 37°C.

For example, in a case in which the host is *E*. *coli*, an LB (Lysogeny Broth) culture medium, an M9 culture medium, or the like is commonly used as a culture medium in which recombinant *E*. *coli* is cultured, and a culture medium in which such a culture medium is allowed to contain 0.1 µg/mL or more of Fe ions and Co ions as components is more preferred. The recombinant *E*. *coli* may be inoculated into the culture medium, and then grown at an appropriate culture temperature (of commonly from 20°C to 50°C).

A culture obtained by culturing the recombinant microorganism according to the second aspect, or a treated product of the culture may be brought into contact with pyridoxine as a substrate in order to produce pyridoxal. A recombinant protein produced by the recombinant microorganism according to the second aspect can produce pyridoxal using pyridoxine as the substrate.

The culture obtained by culturing the recombinant microorganism is not particularly limited as long as the culture is obtained by culturing a recombinant microorganism. The culture may be a bacterial cell recovered from a culture medium, or may be a protein encoded by the polynucleotide that is recovered from a culture medium and described in any one of the (1) to (7) described above. Alternatively, a culture medium including a bacterial cell, or a culture medium that does not include a bacterial cell but includes a recombinant protein produced by a recombinant microorganism may be used as it is.

The treated product of the culture refers to a product obtained by any treatment of the culture of the recombinant microorganism according to the second aspect as long as the activity of the recombinant protein produced by the recombinant microorganism is not lost. Examples of such treatment include treatment including one or more selected from the group consisting of heat treatment, cooling treatment, mechanical destruction of a cell, ultrasonic treatment, freeze-thaw treatment, drying treatment, pressurized or reduced pressure treatment, osmotic pressure treatment, cell autolysis, surfactant treatment, enzyme treatment (for example, cell destruction treatment), cell separation treatment, purification treatment, and extraction treatment. For example, a cell of the recombinant microorganism may be separated from a culture medium or the like, and the separated cell may be added to a reaction system. Means such as filtration or centrifugation can be used for such separation. Alternatively, purification treatment for separating, from a contaminant, the recombinant protein produced by the recombinant microorganism according to the second aspect may be performed, and a solution containing an enzyme obtained by the purification treatment may be added to the reaction system. Alternatively, an extract obtained by extracting the culture using an organic solvent such as methanol or acetonitrile, or a mixed solvent of an organic solvent and water may be added to the reaction system. It is also acceptable that such a purified product or extract does not include a bacterial cell of a recombinant microorganism. Even in a case in which the bacterial cell does not exist, such a purified product or extract can be used in production of pyridoxine or pyridoxal as long as the activity of the recombinant protein produced by the recombinant microorganism according to the second aspect remains.

Such disruption or lysis treatment of a cell as described above can be performed by destructing the cell membrane of the recombinant microorganism according to a known method such as lysozyme treatment, freeze-thawing, or ultrasonic disruption.

In the second aspect, the treated product of the culture may be obtained by, for example, immobilizing a bacterial cell or a protein encoded by the polynucleotide described in any one of the (1) to (7) as above on a solid phase such as a bead or a membrane. A conventionally known method can be used as a method of immobilizing the bacterial cell or protein on the solid phase.

During culturing the recombinant microorganism according to the second aspect, the recombinant microorganism and pyridoxine as a substrate may be brought into contact with each other in order to produce pyridoxal. For example, pyridoxine may be added to a culture medium in order to bring the recombinant microorganism and pyridoxine into contact with each other. Pyridoxine may be included in the culture medium from the beginning of culture, or may be added after, for example, the proliferation period of the microorganism starts following the start of the culture.

In the method of producing pyridoxal according to the second aspect, produced pyridoxal may be recovered from the cultured recombinant microorganism per se, the culture medium, and others, or may be directly subjected to a desired subsequent step without recovering the pyridoxal. A method commonly used in this field can be used as a method of recovering pyridoxal. The subsequent step is not particularly limited as long as pyridoxal is used in the subsequent step. Examples of the subsequent step include a step of phosphorylating pyridoxal.

Pyridoxal can be selectively produced with high efficiency by the methods of producing the recombinant microorganism and pyridoxal according to the second aspect, as described above.

The term "step" as used herein includes not only a separate step but also a step that is not clearly distinguished from other steps as long as the desired purpose of the step is achieved therefrom. As used herein, the notation "to" expressing a numerical range including the numerical values before and after "to", as the minimum value and the maximum value, respectively.

Herein, the amount of a component of a composition, when plural substances corresponding to the same component exist in the composition, the total amount of the component in the composition refers to a total amount of the plural substances in the composition, unless otherwise specified.

### EXAMPLES

The present embodiment is described more specifically with reference to the following examples. However, the disclosure is not limited at all to these examples. In addition, "%" indicating the amounts of components contained in compositions in examples is based on mass standard unless otherwise specified.

### <Analysis Conditions>

Pyridoxine hydrochloride, pyridoxal hydrochloride, and pyridoxamine dihydrochloride were quantified by high performance liquid chromatography. The analysis conditions thereof are as follows.
Column: Shodex (registered trademark) Asahipak ODP-50 6E (SHOWA DENKO K.K.)
Guard column: Shodex (registered trademark) Asahipak ODP-50G 6A (SHOWA DENKO K.K.)
Column temperature: 30°C
Pump flow rate: 1.0 mL/min
Eluent: 50 mM phosphate buffer (pH 2.0)
Detection: UV 254 nm

### Comparative Example 1: Production of ppat Expression Strain

A synthetic DNA having the nucleotide sequence of SEQ ID NO:14 was obtained by custom synthesis of the nucleotide sequence of a pyridoxamine-pyruvate aminotransferase gene derived from *Mesorhizobium loti* MAFF303099, codon-optimized for *E. coli*, from GenScript. A DNA fragment including a target gene was amplified by PCR with an oligonucleotide having the nucleotide sequence of SEQ ID NO:18 and an oligonucleotide having the nucleotide sequence of SEQ ID NO:19 as primers using the synthetic DNA as a template. The amplified DNA fragment was treated with EcoRI and BamHI, and the obtained DNA fragment and a treated product of pUC18 (manufactured by Takara Bio Inc.) with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:14 according to a usual method of determining a nucleotide sequence. The obtained plasmid was named pUC 18-ppat. Here, ppat is the abbreviated name of a pyridoxamine-pyruvate aminotransferase gene.

### Comparative Example 2: Production of ppat-plr Expression Strain

A synthetic DNA having the nucleotide sequence of SEQ ID NO:13 was obtained by custom synthesis of the nucleotide sequence of a pyridoxal reductase gene derived from *Saccharomyces cerevisiae*, codon-optimized for *E*. *coli*, from GenScript. A DNA fragment including a target gene was amplified by PCR with an oligonucleotide having the nucleotide sequence of SEQ ID NO:16 and an oligonucleotide having the nucleotide sequence of SEQ ID NO:17 as primers using the synthetic DNA as a template. The amplified DNA fragment was treated with SalI and HindIII, and the obtained DNA fragment and a treated product obtained by treating the pUC18-ppat produced in Comparative Example 1 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:13 according to a usual method of determining a nucleotide sequence. The obtained plasmid was named pUC 18-ppat-plr. Here, plr is the abbreviated name of a pyridoxal reductase gene (corresponding to pyridoxine dehydrogenase). *E*. *coli* DH5α does not originally have a gene of a pyridoxine dehydrogenase. Further, *E*. *coli* DH5α does not originally have a gene of an alanine regeneration enzyme capable of regenerating L-alanine consumed by pyridoxamine-pyruvate aminotransferase. Accordingly, a ppat-plr expression strain obtained in such a case corresponds to a comparative example because the ppat-plr expression strain does not have a gene encoding an amino acid regeneration enzyme having an enzymatic activity of regenerating an amino acid consumed by an introduced pyridoxamine synthetase although two of a gene encoding a pyridoxine dehydrogenase, a gene encoding a pyridoxamine synthetase, or a gene encoding an amino acid regeneration enzyme are introduced from outside of a bacterial cell.

### Comparative Example 3: Production of ppat-adh Expression Strain

A synthetic DNA having the nucleotide sequence of SEQ ID NO:15 was obtained by custom synthesis of the nucleotide sequence of an alanine dehydrogenase gene derived from *Shewanella sp.* AC 10, into which a mutation as D198A in an encoded amino acid sequence was introduced, and which was codon-optimized for *E*. *coli*, from GenScript. A DNA fragment including a target gene was amplified by PCR with an oligonucleotide having the nucleotide sequence of SEQ ID NO:20 and an oligonucleotide having the nucleotide sequence of SEQ ID NO:21 as primers using the synthetic DNA as a template. The amplified DNA fragment was treated with BamHI and SalI, and the obtained DNA fragment and a treated product obtained by treating the pUC18-ppat produced in Comparative Example 1 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:15 according to a usual method of determining a nucleotide sequence. The obtained plasmid was named pUC 18-ppat-adh. Here, adh is the abbreviated name of an alanine dehydrogenase gene. As described above, *E*. *coli* DH5α does not originally has a gene of a pyridoxine dehydrogenase, and therefore, the ppat-adh expression strain obtained in such a case corresponds to a comparative example.

### Example 1: Production of ppat-adh-plr Expression Strain

A synthetic DNA having the nucleotide sequence of SEQ ID NO:15 was obtained by custom synthesis of the nucleotide sequence of an alanine dehydrogenase gene derived from *Shewanella sp.* AC 10, into which a mutation as D198A in an encoded amino acid sequence was introduced, and which was codon-optimized for *E*. *coli*, from GenScript. A DNA fragment including a target gene was amplified by PCR with an oligonucleotide having the nucleotide sequence of SEQ ID NO:20 and an oligonucleotide having the nucleotide sequence of SEQ ID NO:21 as primers using the synthetic DNA as a template. The amplified DNA fragment was treated with BamHI and SalI, and the obtained DNA fragment and a treated product obtained by treating the pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:15 according to a usual method of determining a nucleotide sequence. The obtained plasmid was named pUC 18-ppat-adh-plr.

### Test 1: Production of Pyridoxamine Using Pyridoxine as Raw Material

DH5α transformed with each of pUC18 and the plasmids produced in Comparative Examples 1 to 3 and Example 1 was inoculated into 100 mL of LB medium including 100 µg/mL of ampicillin in a 500 mL baffled Erlenmeyer flask and cultured with shaking at 30°C for 24 hours. The culture solution was centrifuged at 8,000 rpm for 20 minutes, and bacterial cells obtained as a precipitate were suspended in 1 mL of water to prepare a bacterial cell suspension.

Pyridoxine hydrochloride and L-alanine were dissolved in water to prepare a substrate solution including pyridoxine hydrochloride (600 mM) and L-alanine (600 mM). The pH of the substrate solution was adjusted to pH 8.0 with 25% aqueous ammonia, and 500 µL of substrate solution and 1,000 µL of bacterial cell suspension were mixed, and allowed to react at 37°C for 24 hours. A part of the reaction liquid was collected, and analyzed under the analysis conditions described above. The results are set forth in Table 1. The yield set forth in Table 1 represents the ratio of the molar amount of obtained pyridoxamine dihydrochloride to the molar amount of pyridoxine hydrochloride in the substrate solution.

**Table 1**

| Introduced Plasmid | Yield | Remarks |
|---|---|---|
| pUC18 | 0% | |
| pUC18-ppat | 0% | Comparative Example 1 |
| pUC18-ppat-plr | 7% | Comparative Example 2 |
| pUC18-ppat-adh | 0% | Comparative Example 3 |
| pUC 18-ppat-adh-plr | 88% | Example 1 |

As set forth in Table 1, pyridoxamine dihydrochloride was produced with high production efficiency in a case in which all of the gene encoding a pyridoxine dehydrogenase, the gene encoding a pyridoxamine synthetase, and the gene encoding an amino acid regeneration enzyme having an enzymatic activity of regenerating an amino acid consumed by a pyridoxamine synthetase were included, and two or more of the genes were extracellularly introduced.

### Test 2: Production of Pyridoxamine Using Pyridoxine as Raw Material (Examination of Concentration of L-alanine)

DH5α transformed with the plasmid (pUC18-ppat-adh-plr) produced in Example 1 was inoculated into 100 mL of LB medium including 100 µg/mL of ampicillin in a 500 mL baffled Erlenmeyer flask and cultured with shaking at 30°C for 24 hours. The culture solution was centrifuged at 8,000 rpm for 20 minutes, and bacterial cells obtained as a precipitate were suspended in 0.5 mL of water to prepare a bacterial cell suspension.

Pyridoxine hydrochloride was dissolved in water, and the solution was adjusted to pH 8.0 with 25% aqueous ammonia to prepare a substrate solution (1) including pyridoxine hydrochloride (600 mM). L-alanine was dissolved in water, and the solution was adjusted to pH 8.0 with 25% aqueous ammonia to prepare a substrate solution (2) including L-alanine (1,200 mM). Mixing of 500 µL of substrate solution (1) and a predetermined amount of substrate solution (2) was performed. To each liquid mixture, 500 µL of bacterial cell suspension was added, and water was further added, to prepare 1,500 µL of reaction liquid, which was allowed to react at 37°C for 24 hours. The amount of substrate solution (2) in such a case was changed so that the concentration of L-alanine in each reaction liquid was 0 mM, 1 mM, 5 mM, 10 mM, 50 mM, 200 mM, 400 mM, or 800 mM. A part of the reaction liquid was collected, and analyzed under the analysis conditions described above, to quantify pyridoxine hydrochloride, pyridoxal hydrochloride, and pyridoxamine dihydrochloride. The results are set forth in Table 2. The yield set forth in Table 2 represents the ratio of the molar amount of obtained pyridoxamine dihydrochloride to the molar amount of pyridoxine hydrochloride in the substrate solution.

**Table 2**

| L-alanine (mM) | Yield |
|---|---|
| 0 | 39% |
| 1 | 46% |
| 5 | 57% |
| 10 | 76% |
| 50 | 91% |
| 200 | 88% |
| 400 | 69% |
| 800 | 61% |

As set forth in Table 2, with regard to the concentration of L-alanine in the substrate liquid mixture, high yield was achieved in a wide range of the concentration of L-alanine. Pyridoxamine of which concentration was not less than the concentration of L-alanine was produced in a case in which the concentration of L-alanine was in a concentration range of from 0 mM to 50 mM. This reveals that L-alanine consumed by reaction for producing pyridoxamine was able to be regenerated by introducing alanine dehydrogenase, and pyridoxamine production reaction was allowed to proceed by the regeneration of L-alanine even in the case of the low concentration of L-alanine.

### Test 3: Production of Pyridoxamine Using Pyridoxine as Raw Material (Examination of Concentration of L-alanine)

DH5α obtained by transforming the plasmid (pUC18-ppat-adh-plr) produced in Example 1 was inoculated into 100 mL of LB medium including 100 µg/mL of ampicillin in a 500 mL baffled Erlenmeyer flask and cultured with shaking at 30°C for 24 hours. The culture solution was centrifuged at 8,000 rpm for 20 minutes, and bacterial cells obtained as a precipitate were suspended in 0.5 mL of water to prepare a bacterial cell suspension.

Pyridoxine hydrochloride was dissolved in water, the solution was adjusted to pH 8.0 with 25% aqueous ammonia to prepare a substrate solution including pyridoxine hydrochloride (600 mM). 500 µL of this substrate solution was mixed with 500 µL of bacterial cell suspension, and a residual amount of water to prepare 1500 µL of reaction liquid, an L-alanine powder was added to achieve saturation solubility, and the resultant was allowed to react at 37°C for 24 hours. A part of the reaction liquid was collected, and analyzed under the analysis conditions described above, to quantify pyridoxine hydrochloride, pyridoxal hydrochloride, and pyridoxamine dihydrochloride. As a result, the reaction yield was 39%. In other words, higher yield was able to be achieved in the case of using L-alanine at lower concentration.

As described above, it is revealed that the method of producing pyridoxamine or a salt thereof according to the first aspect enables pyridoxamine or a salt thereof to be inexpensively produced from pyridoxine or a salt thereof with high production efficiency even in the case of using a relatively small amount of amino acid to be consumed rather than adding a large amount of amino acid to be consumed.

### Example 2: Production of ppat-adh-Seplr Expression Plasmid

A synthetic DNA having the nucleotide sequence of SEQ ID NO:75 was obtained by custom synthesis of a gene in which the nucleotide sequence of a pyridoxal reductase gene derived from *Saccharomyces eubayanus* was designed to be codon-optimized for *E*. *coli*, and in which SalI was introduced into the 5' end and HindIII was introduced into the 3' end, from eurofins. A DNA fragment obtained by treating the synthetic DNA with SalI/HindIII, and a DNA fragment obtained by treating pUC18-ppat-adh produced in Comparative Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:75 according to a usual method of determining a nucleotide sequence. The obtained plasmid was named pUC 18-ppat-adh-Seplr.

### Example 3: Production of ppat-adh-Tdplr Expression Plasmid

A synthetic DNA having the nucleotide sequence of SEQ ID NO:76 was obtained by custom synthesis of a gene in which the nucleotide sequence of a pyridoxal reductase gene derived from *Torulaspora delbrueckii* was designed to be codon-optimized for *E*. *coli*, and in which SalI was introduced into the 5' end and HindIII was introduced into the 3' end, from eurofins. A DNA fragment obtained by treating the synthetic DNA with SalI/HindIII, and a DNA fragment obtained by treating pUC18-ppat-adh produced in Comparative Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:76 according to a usual method of determining a nucleotide sequence. The obtained plasmid was named pUC18-ppat-adh-Tdplr.

### Example 4: Production of ppat-adh-Zbplr Expression Plasmid

A synthetic DNA having the nucleotide sequence of SEQ ID NO:77 was obtained by custom synthesis of a gene in which the nucleotide sequence of a pyridoxal reductase gene derived from *Zygosaccharomyces bailii* was designed to be codon-optimized for *E*. *coli*, and in which SalI was introduced into the 5' end and HindIII was introduced into the 3' end, from GenScript. A DNA fragment obtained by treating the synthetic DNA with SalI/HindIII, and a DNA fragment obtained by treating pUC18-ppat-adh produced in Comparative Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:77 according to a usual method of determining a nucleotide sequence. The obtained plasmid was named pUC 18-ppat-adh-Zbplr.

### Example 5: Production of ppat-adh-Aoplr Expression Plasmid

A synthetic DNA having the nucleotide sequence of SEQ ID NO:78 was obtained by custom synthesis of a gene in which the nucleotide sequence of a pyridoxal reductase gene derived from *Aspergillus oryzae* was designed to be codon-optimized for *E*. *coli*, and in which SalI was introduced into the 5' end and HindIII was introduced into the 3' end was synthesized, from GenScript. A DNA fragment obtained by treating the synthetic DNA with SalI/HindIII, and a DNA fragment obtained by treating pUC18-ppat-adh produced in Comparative Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:78 according to a usual method of determining a nucleotide sequence. The obtained plasmid was named pUC 18-ppat-adh-Aoplr.

### Example 6: Production of ppat-adh-Kmplr Expression Plasmid

A synthetic DNA having the nucleotide sequence of SEQ ID NO:79 was obtained by custom synthesis of a gene in which the nucleotide sequence of a pyridoxal reductase gene derived from *Kluyveromyces marxianus* was designed to be codon-optimized for *E*. *coli*, and in which SalI was introduced into the 5' end and HindIII was introduced into the 3' end, from GenScript. A DNA fragment obtained by treating the synthetic DNA with SalI/HindIII, and a DNA fragment obtained by treating pUC18-ppat-adh produced in Comparative Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:79 according to a usual method of determining a nucleotide sequence. The obtained plasmid was named pUC 1 8-ppat-adh-Kmplr.

### Example 7: Production of ppat-adh-Caplr Expression Plasmid

A synthetic DNA having the nucleotide sequence of SEQ ID NO:80 was obtained by custom synthesis of a gene in which the nucleotide sequence of a pyridoxal reductase gene derived from *Candida albicans* was designed to be codon-optimized for *E*. *coli*, and in which SalI was introduced into the 5' end and HindIII was introduced into the 3' end, from eurofins A DNA fragment obtained by treating the synthetic DNA with SalI/HindIII, and a DNA fragment obtained by treating pUC18-ppat-adh produced in Comparative Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:80 according to a usual method of determining a nucleotide sequence. The obtained plasmid was named pUC 18-ppat-adh-Caplr.

### Example 8: Production of ppat-adh-Ylplr Expression Plasmid

A synthetic DNA having the nucleotide sequence of SEQ ID NO:81 was obtained by custom synthesis of a gene in which the nucleotide sequence of a pyridoxal reductase gene derived from *Yarrowia lipolytica* was designed to be codon-optimized for *E*. *coli*, and in which SalI was introduced into the 5' end and HindIII was introduced into the 3' end, from GenScript. A DNA fragment obtained by treating the synthetic DNA with SalI/HindIII, and a DNA fragment obtained by treating pUC18-ppat-adh produced in Comparative Example 3 with SalI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:81 according to a usual method of determining a nucleotide sequence. The obtained plasmid was named pUC 1 8-ppat-adh-Ylplr.

### Test 4: Production of Pyridoxamine Using Pyridoxine as Raw Material

DH5α transformed with each of the plasmids produced in Examples 2 to 8 was inoculated into 100 mL of LB medium including 100 µg/mL of ampicillin in a 500 mL baffled Erlenmeyer flask and cultured with shaking at 30°C for 24 hours. 40 mL of culture solution was taken, and centrifuged at 5,000 rpm for 5 minutes, and bacterial cells obtained as a precipitate were suspended in 0.9 mL of water to prepare a bacterial cell suspension.

Pyridoxine hydrochloride and L-alanine were dissolved in water to prepare a substrate solution including pyridoxine hydrochloride (500 mM) and L-alanine (500 mM). The pH of the substrate solution was adjusted to pH 8.0 with 25% aqueous ammonia, and 400 µL of substrate solution, 500 µL of water, and 100 µL of bacterial cell suspension were mixed, and allowed to react at 37°C for 1 hour. A part of the reaction liquid was collected, and analyzed under the analysis conditions described above. The results are set forth in Table 3. The amount of produced pyridoxamine set forth in Table 3 is expressed in a relative amount, with the molar amount of pyridoxamine dihydrochloride produced by the ppat-adh-plr expression strain produced in Example 1 set to 100.

**Table 3**

| Introduced Plasmid | Amount of Produced Pyridoxamine (Relative Value) |
|---|---|
| pUC18-ppat-adh-plr | 100 |
| pUC18-ppat-adh-Seplr | 120 |
| pUC18-ppat-adh-Tdplr | 19 |
| pUC18-ppat-adh-Zbplr | 29 |
| pUC18-ppat-adh-Aoplr | 75 |
| pUC18-ppat-adh-Kmplr | 92 |
| pUC18-ppat-adh-Caplr | 112 |
| pUC18-ppat-adh-YlpIr | 53 |

As set forth in Table 3, pyridoxamine dihydrochloride was able to be also produced from pyridoxine hydrochloride by introducing a pyridoxal reductase gene derived from *Saccharomyces eubayanus*, a pyridoxal reductase gene derived from *Torulaspora delbrueckii*, a pyridoxal reductase gene derived from *Zygosaccharomyces bailii*, a pyridoxal reductase gene derived from *Aspergillus oryzae*, a pyridoxal reductase gene derived from *Kluyveromyces marxianus*, a pyridoxal reductase gene derived from *Candida albicans*, or a pyridoxal reductase gene derived from *Yarrowia lipolytica*, as a gene encoding a pyridoxine dehydrogenase, instead of the pyridoxal reductase gene derived from *Saccharomyces cerevisiae* used in Example 1.

### Comparative Example 4: Production of adh-plr Expression Plasmid

A pyridoxal reductase gene (plr) fragment recovered by treating pUC18-ppat-plr produced in Comparative Example 2 with SalI and HindIII, an alanine dehydrogenase gene (adh) fragment recovered by treating pUC-ppat-adh produced in Comparative Example 3 with BamHI and SalI, and a treated product of pUC18 with BamHI and HindIII were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner, and the obtained plasmid was named pUC18-adh-plr.

### Example 9: Production of Msppat-adh-plr Expression Plasmid

A synthetic DNA having the nucleotide sequence of SEQ ID NO:82 was obtained by custom synthesis of a gene in which the nucleotide sequence of a pyridoxamine-pyruvate aminotransferase gene derived from *Mesorhizobium sp.* YR577 was designed to be codon-optimized for *E*. *coli*, and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Comparative Example 4 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:82 according to a usual method of determining a nucleotide sequence. The obtained plasmid was named pUC 1 8-Msppat-adh-plr.

### Example 10: Production of Psppat-adh-plr Expression Plasmid

A synthetic DNA having the nucleotide sequence of SEQ ID NO:83 was obtained by custom synthesis of a gene in which the nucleotide sequence of a pyridoxamine-pyruvate aminotransferase gene derived from *Pseudaminobacter salicylatoxidans* was designed to be codon-optimized for *E*. *coli*, and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Comparative Example 4 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:83 according to a usual method of determining a nucleotide sequence. The obtained plasmid was named pUC 18-Psppat-adh-plr.

### Example 11: Production of Blppat-adh-plr Expression Plasmid

A synthetic DNA having the nucleotide sequence of SEQ ID NO:84 was obtained by custom synthesis of a gene in which the nucleotide sequence of a pyridoxamine-pyruvate aminotransferase gene derived from *Bauldia litoralis* was designed to be codon-optimized for *E*. *coli*, in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Comparative Example 4 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:84 according to a usual method of determining a nucleotide sequence. The obtained plasmid was named pUC18-Blppat-adh-plr.

### Example 12: Production of Ssppat-adh-plr Expression Plasmid

A synthetic DNA having the nucleotide sequence of SEQ ID NO:85 was obtained by custom synthesis of a gene in which the nucleotide sequence of a pyridoxamine-pyruvate aminotransferase gene derived from *Skermanella stibiiresistens* was designed to be codon-optimized for *E*. *coli*, and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Comparative Example 4 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:85 according to a usual method of determining a nucleotide sequence. The obtained plasmid was named pUC 18-Ssppat-adh-plr.

### Example 13: Production of Rsppat-adh-plr Expression Plasmid

A synthetic DNA having the nucleotide sequence of SEQ ID NO:86 was obtained by custom synthesis of a gene in which the nucleotide sequence of a pyridoxamine-pyruvate aminotransferase gene derived from *Rhizobium sp.* AC44/96 was designed to be codon-optimized for *E*. *coli*, and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Comparative Example 4 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:86 according to a usual method of determining a nucleotide sequence. The obtained plasmid was named pUC 1 8-Rsppat-adh-plr.

### Example 14: Production of Etppat-adh-plr Expression Plasmid

A synthetic DNA having the nucleotide sequence of SEQ ID NO:87 was obtained by custom synthesis of a gene in which the nucleotide sequence of a pyridoxamine-pyruvate aminotransferase gene derived from *Erwinia toletana* was designed to be codon-optimized for *E*. *coli*, and in which EcoRI was introduced into the 5' end and BamHI was introduced into the 3' end, from eurofins. A DNA fragment obtained by treating the synthetic DNA with EcoRI/BamHI, and a DNA fragment obtained by treating pUC18-adh-plr produced in Comparative Example 4 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:87 according to a usual method of determining a nucleotide sequence. The obtained plasmid was named pUC 1 8-Etppat-adh-plr.

### Example 15: Production of Hgppat-adh-plr Expression Plasmid

A synthetic DNA having the nucleotide sequence of SEQ ID NO:88 was obtained by custom synthesis of a gene in which the nucleotide sequence of a pyridoxamine-pyruvate aminotransferase gene derived from *Herbiconiux ginsengi* was designed to be codon-optimized for *E*. *coli*, and in which EcoRI was introduced into the 5' end and BamHI was introduced into the 3' end, from eurofins,. A DNA fragment obtained by treating the synthetic DNA with EcoRI/BamHI, and a DNA fragment obtained by treating pUC18-adh-plr produced in Comparative Example 4 with EcoRI and BamHI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:88 according to a usual method of determining a nucleotide sequence. The obtained plasmid was named pUC18-Hgppat-adh-plr.

### Test 5: Production of Pyridoxamine Using Pyridoxine as Raw Material

DH5α transformed with each of the plasmids produced in Examples 9 to 15 was inoculated into 100 mL of LB medium including 100 µg/mL of ampicillin in a 500 mL baffled Erlenmeyer flask and cultured with shaking at 30°C for 24 hours. 40 mL of culture solution was taken, and centrifuged at 5,000 rpm for 5 minutes, and bacterial cells obtained as a precipitate were suspended in 0.9 mL of water to prepare a bacterial cell suspension.

Pyridoxine hydrochloride and L-alanine were dissolved in water to prepare a substrate solution including pyridoxine hydrochloride (500 mM) and L-alanine (500 mM). The pH of the substrate solution was adjusted to pH 8.0 with 25% aqueous ammonia. 400 µL of substrate solution, 500 µL of water, and 100 µL of bacterial cell suspension were mixed, and allowed to react at 37°C for 1 hour. A part of the reaction liquid was collected, and analyzed under the analysis conditions described above. The results are set forth in Table 4. The amount of produced pyridoxamine set forth in Table 4 is expressed in a relative amount, with the molar amount of pyridoxamine dihydrochloride produced by the ppat-adh-plr expression strain produced in Example 1 set to 100.

**Table 4**

| Introduced Plasmid | Amount of Produced Pyridoxamine (Relative Value) |
|---|---|
| pUC18-ppat-adh-plr | 100 |
| pUC18-Msppat-adh-plr | 46 |
| pUC18-Psppat-adh-plr | 113 |
| pUC18-BIppat-adh-plr | 34 |
| pUC18-Ssppat-adh-plr | 77 |
| pUC18-Rsppat-adh-plr | 34 |
| pUC18-Etppat-adh-plr | 109 |
| pUC18-Hgppat-adh-plr | 75 |

As set forth in Table 4, pyridoxamine dihydrochloride was able to be also produced from pyridoxine hydrochloride by introducing a pyridoxamine-pyruvate aminotransferase gene derived from *Mesorhizobium sp.* YR577, a pyridoxamine-pyruvate aminotransferase gene derived from *Pseudaminobacter salicylatoxidans*, a pyridoxamine-pyruvate aminotransferase gene derived from *Bauldia litoralis*, a pyridoxamine-pyruvate aminotransferase gene derived from *Skermanella stibiiresistens*, a pyridoxamine-pyruvate aminotransferase gene derived from *Rhizobium sp.* AC44/96, a pyridoxamine-pyruvate aminotransferase gene derived from *Erwinia toletana*, or a pyridoxamine-pyruvate aminotransferase gene derived from *Herbiconiux ginsengi*, as a gene encoding a pyridoxamine-pyruvate aminotransferase, instead of the pyridoxamine-pyruvate aminotransferase gene derived from *Mesorhizobium loti* MAFF303099 used in Example 1.

### Example 16: Production of ppat-Ahadh-plr Expression Plasmid

A synthetic DNA having the nucleotide sequence of SEQ ID NO:89 was obtained by custom synthesis of the nucleotide sequence of an alanine dehydrogenase gene derived from *Aeromonas hydrophila*, into which a mutation as D198A in an encoded amino acid sequence was introduced, which was designed to be codon-optimized for *E*. *coli*, and in which BamHI was introduced into the 5' end and SalI was introduced into the 3' end, from eurofins. A DNA fragment obtained by treating the synthetic DNA with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:89 according to a usual method of determining a nucleotide sequence. The obtained plasmid was named pUC18-ppat-Ahadh-plr.

### Example 17: Production of ppat-Rsadh-plr Expression Plasmid

A synthetic DNA having the nucleotide sequence of SEQ ID NO:90 was obtained by custom synthesis of the nucleotide sequence of an alanine dehydrogenase gene derived from *Rhizobium sp.* LPU83, into which a mutation as D198A in an encoded amino acid sequence was introduced, which was designed to be codon-optimized for *E*. *coli*, and in which BamHI was introduced into the 5' end and SalI was introduced into the 3' end, from eurofins. A DNA fragment obtained by treating the synthetic DNA with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:90 according to a usual method of determining a nucleotide sequence. The obtained plasmid was named pUC 18-ppat-Rsadh-plr.

### Example 18: Production of ppat-Pmadh-plr Expression Plasmid

A synthetic DNA having the nucleotide sequence of SEQ ID NO:91 was obtained by custom synthesis of the nucleotide sequence of an alanine dehydrogenase gene derived from *Pseudomonas mendocina*, into which a mutation as D198A in an encoded amino acid sequence was introduced, which was designed to be codon-optimized for *E*. *coli*, and in which BamHI was introduced into the 5' end and SalI was introduced into the 3' end, from eurofins. A DNA fragment obtained by treating the synthetic DNA with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:91 according to a usual method of determining a nucleotide sequence. The obtained plasmid was named pUC 18-ppat-Pmadh-plr.

### Example 19: Production of ppat-Bjadh1-plr Expression Plasmid and ppat-Bjadh2-plr Expression Plasmid

Two kinds of alanine dehydrogenase genes derived from *Bradyrhizobium japonicum* exist in *Bradyrhizobium japonicum.* Two kinds of synthetic DNAs having the nucleotide sequences of SEQ ID NO:92 and SEQ ID NO:93 were obtained by custom synthesis of the nucleotide sequences of the two kinds of the alanine dehydrogenase genes derived from *Bradyrhizobium japonicum*, into which a mutation as D198A in an encoded amino acid sequence was introduced, which were designed to be codon-optimized for *E. coli*, and in which BamHI was introduced into the 5' end and SalI was introduced into the 3' end, from eurofins. A DNA fragment obtained by treating the synthetic DNA including SEQ ID NO:92 with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:92 according to a usual method of determining a nucleotide sequence. The obtained plasmid was named pUC18-ppat-Bjadh1-plr.

Similarly, a DNA fragment obtained by treating the synthetic DNA including SEQ ID NO:93 with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:93 according to a usual method of determining a nucleotide sequence. The obtained plasmid was named pUC 18-ppat-Bj adh2-plr.

### Example 20: Production of ppat-Saadh-plr Expression Plasmid

A synthetic DNA having the nucleotide sequence of SEQ ID NO:94 was obtained by custom synthesis of the nucleotide sequence of an alanine dehydrogenase gene derived from *Streptomyces aureofaciens*, into which a mutation as D198A in an encoded amino acid sequence was introduced, which was designed to be codon-optimized for *E. coli*, and in which BamHI was introduced into the 5' end and SalI was introduced into the 3' end, from eurofins. A DNA fragment obtained by treating the synthetic DNA with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:94 according to a usual method of determining a nucleotide sequence. The obtained plasmid was named pUC 18-ppat-Saadh-plr.

### Example 21: Production of ppat-Acadh-plr Expression Plasmid

A synthetic DNA having the nucleotide sequence of SEQ ID NO:95 was obtained by custom synthesis of the nucleotide sequence of an alanine dehydrogenase gene derived from *Anabaena cylindrica*, into which a mutation as D198A in an encoded amino acid sequence was introduced, which was designed to be codon-optimized for *E. coli*, and in which BamHI was introduced into the 5' end and SalI was introduced into the 3' end, from eurofins. A DNA fragment obtained by treating the synthetic DNA with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:95 according to a usual method of determining a nucleotide sequence. The obtained plasmid was named pUC 18-ppat-Acadh-plr.

### Example 22: Production of ppat-Bsadh-plr Expression Plasmid

A synthetic DNA having the nucleotide sequence of SEQ ID NO:96 was obtained by custom synthesis of the nucleotide sequence of an alanine dehydrogenase gene derived from *Bacillus subtilis*, into which a mutation as D196A in an encoded amino acid sequence was introduced, which was designed to be codon-optimized for *E. coli*, and in which BamHI was introduced into the 5' end and SalI was introduced into the 3' end, from eurofins. A DNA fragment obtained by treating the synthetic DNA with BamHI/SalI, and a DNA fragment obtained by treating pUC18-ppat-plr produced in Comparative Example 2 with BamHI and SalI were ligated using Ligation High (manufactured by TOYOBO Co., Ltd.). *Escherichia coli* DH5α (manufactured by TOYOBO Co., Ltd.) was transformed with the ligation product. The transformant was cultured on an LB agar medium, and a strain having a target plasmid was selected from ampicillin resistant strains. The plasmid was extracted from the transformant obtained in such a manner.

The nucleotide sequence of the DNA fragment introduced into the plasmid was confirmed to be the nucleotide sequence of SEQ ID NO:96 according to a usual method of determining a nucleotide sequence. The obtained plasmid was named pUC18-ppat-Bsadh-plr.

### Test 6: Production of Pyridoxamine Using Pyridoxine as Raw Material

DH5α transformed with each of the plasmids produced in Examples 16 to 22 was inoculated into 100 mL of LB medium including 100 µg/mL of ampicillin in a 500 mL baffled Erlenmeyer flask and cultured with shaking at 30°C for 24 hours. 40 mL of culture solution was taken, and centrifuged at 5,000 rpm for 5 minutes, and bacterial cells obtained as a precipitate were suspended in 0.9 mL of water to adjust prepare a bacterial cell suspension.

Pyridoxine hydrochloride and L-alanine were dissolved in water to prepare a substrate solution including pyridoxine hydrochloride (500 mM) and L-alanine (500 mM). The pH of the substrate solution was adjusted to pH 8.0 with 25% aqueous ammonia, and 400 µL of substrate solution, 500 µL of water, and 100 µL of bacterial cell suspension were mixed, and allowed to react at 37°C for 1 hour. A part of the reaction liquid was collected, and analyzed under the analysis conditions described above. The results are set forth in Table 5. The amount of produced pyridoxamine set forth in Table 5 is expressed in a relative amount, with the molar amount of pyridoxamine dihydrochloride produced by the ppat-adh-plr expression strain produced in Example 1 set to 100.

**Table 5**

| Introduced Plasmid | Amount of Produced Pyridoxamine (Relative Value) |
|---|---|
| pUC18-ppat-adh-plr | 100 |
| pUC18-ppat-Ahadh-plr | 79 |
| pUC18-ppat-Rsadh-plr | 66 |
| pUC18-ppat-Pmadh-plr | 113 |
| pUC18-ppat-Bjadh1-plr | 32 |
| pUC18-ppat-Bjadh2-plr | 22 |
| pUC18-ppat-Saadh-plr | 84 |
| pUC18-ppat-Acadh-plr | 24 |
| pUC18-ppat-Bsadh-plr | 105 |

As set forth in Table 5, pyridoxamine dihydrochloride was able to be also produced from pyridoxine hydrochloride by introducing an alanine dehydrogenase gene derived from *Aeromonas hydrophila*, an alanine dehydrogenase gene derived from *Rhizobium sp.* LPU83, an alanine dehydrogenase gene derived from *Pseudomonas mendocina*, an alanine dehydrogenase gene derived from *Bradyrhizobium japonicum* (each of two kinds of alanine dehydrogenase genes from *Bradyrhizobium japonicum*), an alanine dehydrogenase gene derived from *Streptomyces aureofaciens*, an alanine dehydrogenase gene derived from *Anabaena cylindrica*, or an alanine dehydrogenase gene derived from *Bacillus subtilis*, as a gene encoding an alanine dehydrogenase, instead of the alanine dehydrogenase gene derived from *Shewanella sp.* AC 10 used in Example 1.

### Example 23: Production of E. coli Expressing Protein Including Amino Acid Sequence of SEQ ID NO:1

A DNA (SEQ ID NO:13) in which a nucleotide sequence encoding the pyridoxine dehydrogenase gene (SEQ ID NO:1) of *Saccharomyces cerevisiae* was codon-optimized for *E. coli* was obtained by custom synthesis from GenScript. A target gene was amplified by a polymerase chain reaction (PCR) method using, as primers, oligonucleotides shown in SEQ ID NO:18 and SEQ ID NO:17 with the obtained synthetic DNA as a template. A DNA fragment obtained by treating the amplified DNA with EcoRI/HindIII, and an EcoRI/HindIII treated product of pUC18 (manufactured by Takara Bio Inc.) were ligated using Ligation High (manufactured by TOYOBO Co., Ltd.) to obtain an expression vector. *Escherichia coli* DH5α (manufactured by TOYOBO Co., Ltd.) was transformed with the ligation product including the expression vector. The transformant was cultured on an LB agar medium, and a strain having a target plasmid was selected from ampicillin resistant strains.

The plasmid was extracted from the obtained transformant in order to confirm that the selected strain includes a polynucleotide including the nucleotide sequence of SEQ ID NO:13. The nucleotide sequence of the DNA fragment introduced into the plasmid was confirmed to be the nucleotide sequence of SEQ ID NO:13 according to a usual method of determining a nucleotide sequence. The obtained plasmid was named pUC 18-plr.

### Example 24: Synthesis of Pyridoxal Using Pyridoxine as Substrate with Pyridoxine Dehydrogenase Expression Strain

An *E. coli* DH5α strain transformed with pUC18-plr was inoculated into 2 mL of LB liquid medium (containing 100 µg/mL of ampicillin), and the resultant was cultured with shaking in a test tube at 37°C for 24 hours. 1 mL of culture solution was centrifuged at 13,000 rpm for 5 minutes, and obtained bacterial cells were suspended in 1 mL of water to obtain a bacterial cell suspension.

A substrate solution was obtained by dissolving pyridoxine hydrochloride and β-NADP⁺ in water so that each thereof was 100 mM. Mixing of 100 µL of substrate solution, 100 µL of 1 M Tris-HCl (pH 8.0), and 700 µL of water was performed, and 100 µL of bacterial cell suspension was added to the resultant to start reaction. The reaction was conducted at 37°C for 1 hour.

After the end of the reaction, a part of the reaction liquid was collected, and analyzed by high performance liquid chromatography (HPLC).

The reaction conducted for 1 hour allowed 37.2 µg of pyridoxal to be obtained, and the rate of producing pyridoxal per OD 660 was 2.1 × 10⁻³ µmol/min/OD 660.

### • Analysis Conditions of HPLC

Column: Shodex Asahipak ODP-50 6E (trade name, manufactured by SHOWA DENKO K.K.)
Guard column: Shodex Asahipak ODP-50G 6A (trade name, manufactured by SHOWA DENKO K.K.)
Column temperature: 30°C
Pump flow rate: 1.0 mL/min
Eluent: 50 mM phosphate buffer (pH 2.0)
Detection: UV 294 nm.

### Comparative Example 5: Synthesis of Pyridoxal Using Pyridoxine as Substrate with Yeast

*Saccharomyces cerevisiae* X-2180-1A (ATCC26486) was inoculated into 2 mL of YPD liquid medium, and the resultant was cultured with shaking in a test tube at 30°C for 24 hours. 1 mL of culture solution was centrifuged at 13,000 rpm for 5 minutes, and obtained bacterial cells were suspended in 1 mL of water to obtain a bacterial cell suspension.

A substrate solution having the same composition as the composition of the substrate solution in Example 24 was prepared, and reaction was conducted under the same conditions as the reaction conditions in Example 24.

After the end of the reaction, a part of the reaction liquid was collected, and analyzed by high performance liquid chromatography (HPLC) under conditions similar to the conditions in Example 24.

The reaction conducted for 1 hour did not allow pyridoxal to be obtained.

### Example 25: Synthesis of Pyridoxine Using Pyridoxal as Substrate with Pyridoxine Dehydrogenase Expression Strain

A bacterial cell suspension was prepared in a manner similar to the manner of Example 24, and a substrate solution was obtained by dissolving pyridoxal hydrochloride and β-NADPH in water so that each thereof was 100 mM. Mixing of 100 µL of substrate solution, 100 µL of 1 M Tris-HCl (pH 8.0), and 700 µL of water was performed, and 100 µL of bacterial cell suspension was added to the resultant to start reaction.

After the end of the reaction, a part of the reaction liquid was collected, and analyzed by high performance liquid chromatography (HPLC) under conditions similar to the conditions in Example 24.

The reaction conducted for 1 hour allowed 815.5 µg of pyridoxine to be obtained, and the rate of producing pyridoxine per OD 660 was 4.5 × 10⁻² µmol/min/OD 660.

### Comparative Example 6: Synthesis of Pyridoxine Using Pyridoxal as Substrate with Yeast (Rate of Producing Pyridoxine per OD 660)

A bacterial cell suspension was prepared in a manner similar to the manner of Comparative Example 5, a substrate solution having the same composition as the composition of the substrate solution in Example 25 was prepared, and reaction was conducted under the same conditions as the reaction conditions in Example 25.

After the end of the reaction, a part of the reaction liquid was collected, and analyzed by high performance liquid chromatography (HPLC) under conditions similar to the conditions in Example 24.

The reaction conducted for 1 hour allowed 10.9 µg of pyridoxine to be obtained, and the rate of producing pyridoxine per OD 660 was 1.7 × 10⁻⁴ µmol/min/OD 660.

The results of Examples 24 to 25 and Comparative Examples 5 to 6 are set forth in Table 6. Examples 24 and 25 were prominently superior in the yield of pyridoxal or pyridoxine, and the rate of producing pyridoxal or pyridoxine to Comparative Examples 5 and 6. In particular, pyridoxal which was not more than detection sensitivity in Comparative Example 5 was able to be produced in Example 24 in the synthesis of pyridoxal.

**Table 6**

| | Synthesis of Pyridoxal | | Synthesis of Pyridoxine | |
|---|---|---|---|---|
| | Example 2 | Comparative Example 1 | Example 3 | Comparative Example 2 |
| Yield (µg) | 37.2 | Not detected | 815.5 | 10.9 |
| Production Rate (µmol/min/OD 660) | 2.1×10⁻³ | Not detected | 4.5×10⁻² | 1.7×10⁻⁴ |

### Example 26: Synthesis of Pyridoxal Using Pyridoxine as Substrate with Pyridoxine Dehydrogenase Expression Strain

An *E. coli* DH5α strain transformed with pUC18-plr was inoculated into 100 mL of LB liquid medium (containing 100 µg/mL of ampicillin), and the resultant was cultured with shaking in a baffled Erlenmeyer flask at 37°C for 24 hours. 40 mL of culture solution was centrifuged at 8,000 rpm for 20 minutes, and obtained bacterial cells were suspended in 400 µL of water to obtain a bacterial cell suspension. A substrate solution having the same composition as the composition of the substrate solution in Example 24 was prepared, and reaction was conducted under the same conditions as the reaction conditions in Example 24. The reaction was conducted at 37°C for 30 minutes.

After the end of the reaction, a part of the reaction liquid was collected, and analyzed by high performance liquid chromatography (HPLC) under conditions similar to the conditions in Example 24.

The reaction conducted for 30 minutes allowed 262.4 µg of pyridoxal to be obtained, and the rate of producing pyridoxal per dried bacterial cell weight was 5.8 µmol/min/g-dry cell.

### Comparative Example 7: Synthesis of Pyridoxal Using Pyridoxine as Substrate with Dry Yeast

Super Camelia Dry Yeast (trade name, Nisshin Foods Inc.) was suspended in water to achieve 100 mg/mL, and a bacterial cell suspension was obtained.

A substrate solution having the same composition as the composition of the substrate solution in Example 24 was prepared, and reaction was conducted under the same conditions as the reaction conditions in Example 24.

After the end of the reaction, a part of the reaction liquid was collected, and analyzed by high performance liquid chromatography (HPLC) under conditions similar to the conditions in Example 24.

The reaction conducted for 30 minutes allowed 34.1 µg of pyridoxal to be obtained, and the rate of producing pyridoxal per dried bacterial cell weight was 0.9 µmol/min/g-dry cell.

### Example 27: Synthesis of Pyridoxine Using Pyridoxal as Substrate with Pyridoxine Dehydrogenase Expression Strain

A bacterial cell suspension was prepared in a manner similar to the manner of Example 26, a substrate solution having the same composition as the composition of the substrate solution in Example 25 was prepared, and reaction was conducted under the same conditions as the reaction conditions in Example 25.

After the end of the reaction, a part of the reaction liquid was collected, and analyzed by high performance liquid chromatography (HPLC) under conditions similar to the conditions in Example 24.

The reaction conducted for 30 minutes allowed 1457.4 µg of pyridoxine to be obtained, and the rate of producing pyridoxine per dried bacterial cell weight was 33.5 µmol/min/g-dry cell.

### Comparative Example 8: Synthesis of Pyridoxine Using Pyridoxal as Substrate with Dry Yeast (Rate of Producing Pyridoxine Per Dried Bacterial Cell Weight)

A bacterial cell suspension was prepared in a manner similar to the manner of Comparative Example 7, a substrate solution having the same composition as the composition of the substrate solution in Example 25 was prepared, and reaction was conducted under the same conditions as the reaction conditions in Example 25.

After the end of the reaction, a part of the reaction liquid was collected, and analyzed by high performance liquid chromatography (HPLC) under conditions similar to the conditions in Example 24.

The reaction conducted for 30 minutes allowed 325.3 µg of pyridoxine to be obtained, and the rate of producing pyridoxine per dried bacterial cell weight was 13.9 µmol/min/g-dry cell.

The results of Examples 26 to 27 and Comparative Examples 7 to 8 are set forth in Table 7. Examples 26 and 27 were prominently superior in the yield of pyridoxal or pyridoxine, and the rate of producing pyridoxal or pyridoxine to Comparative Examples 7 and 8. In Example 26, the obtained yield of pyridoxal was about 8 times that in Comparative Example 7, and the production rate per gram of dried bacterial cell weight was about 6.5 times. In Example 27, the obtained yield of pyridoxine was about 4.5 times that in Comparative Example 8, and the production rate per gram of dried bacterial cell weight was about 2.5 times.

**Table 7**

| | Synthesis of Pyridoxal | | Synthesis of Pyridoxine | |
|---|---|---|---|---|
| | Example 4 | Comparative Example 3 | Example 5 | Comparative Example 4 |
| Yield (µg) | 262.4 | 34.1 | 1457.4 | 325.3 |
| Production Rate (µmol/min/ g-dry cell) | 5.8 | 0.9 | 33.5 | 13.9 |

The disclosures of Japanese Patent Application No. 2017-095571 filed on May 12, 2017, and Japanese Patent Application No. 2017-095573 filed on May 12, 2017 are incorporated herein by reference in their entirety.

All publications, patent application, and technical standards mentioned in this specification are herein incorporated by reference to the same extent as if each individual publication, patent application, or technical standard was specifically and individually indicated to be incorporated by reference.

## Claims

1. A recombinant microorganism, comprising:
a gene encoding a pyridoxine dehydrogenase, a gene encoding a pyridoxamine synthetase having an enzymatic activity of synthesizing pyridoxamine from pyridoxal, and a gene encoding an amino acid regeneration enzyme having an enzymatic activity of regenerating an amino acid consumed by the pyridoxamine synthetase,
wherein at least two of the gene encoding the pyridoxine dehydrogenase, the gene encoding the pyridoxamine synthetase, or the gene encoding the amino acid regeneration enzyme, are introduced from outside of a bacterial cell, or are endogenous to the bacterial cell and have an enhanced expression.

2. The recombinant microorganism according to claim 1, wherein the pyridoxamine synthetase is a pyridoxamine-pyruvate transaminase, a pyridoxamine-oxaloacetate transaminase, an aspartate transaminase, or a pyridoxamine phosphate transaminase.

3. The recombinant microorganism according to claim 1 or 2, wherein the pyridoxine dehydrogenase comprises at least one of the following partial amino acid sequence (a) or partial amino acid sequence (b), and has pyridoxine dehydrogenase activity:
(a) NX₁X₂EX₃YG (SEQ ID NO:97)
wherein X₁ represents V, C, I, A, M, S, G, or L,
X₂ represents G or A, and
X₃ represents F or L; and
(b) X₄X₅X₆KGX₇ (SEQ ID NO:98)
wherein X₄ represents I, V, F, or L,
X₅ represents S, T, N, C, or M,
X₆ represents C, V, A, I, W, or F, and
X₇ represents G, A, S, or C.

4. The recombinant microorganism according to any one of claims 1 to 3, wherein the pyridoxine dehydrogenase is represented by enzyme number EC1.1.1.65.

5. The recombinant microorganism according to any one of claims 1 to 4, wherein the gene encoding the pyridoxine dehydrogenase is derived from *Saccharomyces cerevisiae.*

6. The recombinant microorganism according to any one of claims 1 to 5, wherein the gene encoding the pyridoxine dehydrogenase has:
a nucleotide sequence of any one of SEQ ID NO:7 or SEQ ID NO:53 to SEQ ID NO:59,
a region between an 18th nucleotide and a 3' end in a nucleotide sequence of any one of SEQ ID NO:13 or SEQ ID NO:75 to SEQ ID NO:81, or
a nucleotide sequence that hybridizes with DNA having a nucleotide sequence complementary to a nucleotide sequence of any one of SEQ ID NO:7 or SEQ ID NO:53 to SEQ ID NO:59, or with DNA having a nucleotide sequence complementary to a region between an 18th nucleotide and a 3' end in a nucleotide sequence of any one of SEQ ID NO:13 or SEQ ID NO:75 to SEQ ID NO:81 under a stringent condition, and that encodes a protein having pyridoxine dehydrogenase activity.

7. The recombinant microorganism according to any one of claims 1 to 4, wherein the gene encoding the pyridoxine dehydrogenase is derived from *Schizosaccharomyces pombe.*

8. The recombinant microorganism according to any one of claims 1 to 4, wherein the gene encoding the pyridoxine dehydrogenase has a nucleotide sequence of SEQ ID NO:8, or has a nucleotide sequence that hybridizes with DNA having a nucleotide sequence complementary to the nucleotide sequence of SEQ ID NO:8 under a stringent condition, and that encodes a protein having pyridoxine dehydrogenase activity.

9. The recombinant microorganism according to any one of claims 1 to 8, wherein the gene encoding the pyridoxine dehydrogenase has a nucleotide sequence encoding an amino acid sequence of any one of SEQ ID NO:1, SEQ ID NO:2, or SEQ ID NO:22 to SEQ ID NO:28, or an amino acid sequence that has 80% or more sequence identity with at least one amino acid sequence selected from the group consisting of SEQ ID NO:1, SEQ ID NO:2, and SEQ ID NO:22 to SEQ ID NO:28, and that has pyridoxine dehydrogenase activity.

10. The recombinant microorganism according to any one of claims 1 to 9, wherein the pyridoxamine synthetase comprises at least one of the following partial amino acid sequence (c), partial amino acid sequence (d), partial amino acid sequence (e), partial amino acid sequence (f), partial amino acid sequence (g), or partial amino acid sequence (h), and has an enzymatic activity of synthesizing pyridoxamine from pyridoxal:
(c) X₈X₉X₁₀X₁₁X₁₂X₁₃ (SEQ ID NO:99)
wherein X₈ represents L, M, I, or V,
X₉ represents H or Q,
X₁₀ represents G, C, or A,
X₁₁ represents E or D,
X₁₂ represents P or A, and
X₁₃ represents V, I, L, or A;
(d) X₁₄X₁₅TPSGTX₁₆X₁₇ (SEQ ID NO:100)
wherein X₁₄ represents H or S,
X₁₅ represents D or E,
X₁₆ represents I, V, or L, and
X₁₇ represents N or T;
(e) X₁₈DX₁₉VSX₂₀X₂₁ (SEQ ID NO:101)
wherein X₁₈ represents V, I, or A,
X₁₉ represents A, T, or S,
X₂₀ represents S, A, or G, and
X₂₁ represents F, W, or V;
(f) X₂₂X₂₃X₂₄KCX₂₅GX₂₆X₂₇P (SEQ ID NO:102)
wherein X₂₂ represents G or S,
X₂₃ represents P, S, or A,
X₂₄ represents N, G, S, A, or Q,
X₂₅ represents L or M,
X₂₆ represents A, S, C, or G, and
X₂₇ represents P, T, S, or A;
(g) X₂₈X₂₉X₃₀X₃₁SX₃₂GX₃₃X₃₄ (SEQ ID NO:103)
wherein X₂₈ represents G or D,
X₂₉ represents V or I,
X₃₀ represents V, T, A, S, M, I, or L,
X₃₁ represents F, M, L, I, or V,
X₃₂ represents S, G, A, T, I, L, or H,
X₃₃ represents R, M, or Q, and
X₃₄ represents G, R, A, D, H, or K; and
(h) X₃₅X₃₆RX₃₇X₃₈HMGX₃₉X₄₀A (SEQ ID NO:104)
wherein X₃₅ represents L or V,
X₃₆ represents T, I, V, or L,
X₃₇ represents I, V, or L,
X₃₈ represents G or S,
X₃₉ represents P, A, or R, and
X₄₀ represents T, V, or S.

11. The recombinant microorganism according to any one of claims 1 to 10, wherein the pyridoxamine synthetase is represented by enzyme number EC2.6.1.30.

12. The recombinant microorganism according to any one of claims 1 to 11, wherein the gene encoding the pyridoxamine synthetase is derived from *Mesorhizobium loti.*

13. The recombinant microorganism according to any one of claims 1 to 12, wherein the gene encoding the pyridoxamine synthetase has:
a nucleotide sequence of any one of SEQ ID NO:9 or SEQ ID NO:60 to SEQ ID NO:66,
a region between an 18th nucleotide and a 3' end in a nucleotide sequence of SEQ ID NO:14, or a region between an 18th nucleotide and a 3' end in a nucleotide sequence of any one of SEQ ID NO:82 to SEQ ID NO:88, or
a nucleotide sequence that hybridizes with DNA having a nucleotide sequence complementary to a nucleotide sequence of any one of SEQ ID NO:9 or SEQ ID NO:60 to SEQ ID NO:66, or with DNA having a nucleotide sequence complementary to the region between a 18th nucleotide and an 3' end in the nucleotide sequence of SEQ ID NO:14, or a region between an 18th nucleotide and a 3' end in a nucleotide sequence of any one of SEQ ID NO:82 to SEQ ID NO:88, under a stringent condition, and that encodes a protein having an enzymatic activity of synthesizing pyridoxamine from pyridoxal.

14. The recombinant microorganism according to any one of claims 1 to 13, wherein the gene encoding the pyridoxamine synthetase has a nucleotide sequence encoding an amino acid sequence of any one of SEQ ID NO:3 or SEQ ID NO:29 to SEQ ID NO:35, or an amino acid sequence that has 80% or more sequence identity with at least one amino acid sequence selected from the group consisting of SEQ ID NO:3 and SEQ ID NO:29 to SEQ ID NO:35, and that has an enzymatic activity of synthesizing pyridoxamine from pyridoxal.

15. The recombinant microorganism according to any one of claims 10 to 14, wherein the amino acid regeneration enzyme is an alanine dehydrogenase.

16. The recombinant microorganism according to claim 15, wherein the alanine dehydrogenase is enabled to use NADPH as a coenzyme in a reaction in which L-alanine is produced from pyruvic acid and NH₃.

17. The recombinant microorganism according to claim 15 or 17, wherein the alanine dehydrogenase comprises at least one of the following partial amino acid sequence (i), partial amino acid sequence (j), partial amino acid sequence (k), or partial amino acid sequence (1), and has an activity of producing L-alanine from pyruvic acid and NH₃:
(i) EX₄₁KX₄₂X₄₃EX₄₄RX₄₅X₄₆ (SEQ ID NO:105)
wherein X₄₁ represents I, T, S, F, N, or V,
X₄₂ represents N, M, A, V, L, T, or D,
X₄₃ represents H, N, L, or Q,
X₄₄ represents Y, N, or F,
X₄₅ represents V or I, and
X₄₆ represents G or A;
(j) X₄₇X₄₈X₄₉KVKEPX₅₀ (SEQ ID NO:106)
wherein X₄₇ represents M or L,
X₄₈ represents I, L, or V,
X₄₉ represents V, L, I, or M, and
X₅₀ represents Q, L, V, N, or I;
(k) LX₅₁TYLHLA (SEQ ID NO:107)
wherein X₅₁ represents F or Y; and
(1) X₅₂DX₅₃AX₅₄DQGG (SEQ ID NO:108)
wherein X₅₂ represents V or A,
X₅₃ represents V or I, and
X₅₄ represents I or V.

18. The recombinant microorganism according to any one of claims 15 to 17, wherein the gene encoding the amino acid regeneration enzyme is derived from *Shewanella sp.* AC10.

19. The recombinant microorganism according to any one of claims 15 to 18, wherein the gene encoding the amino acid regeneration enzyme has:
a nucleotide sequence of any one of SEQ ID NO:11 or SEQ ID NO:67 to SEQ ID NO:74,
a region between an 18th nucleotide and a 3' end in a nucleotide sequence of any one of SEQ ID NO:15 or SEQ ID NO:89 to SEQ ID NO:96, or
a nucleotide sequence that hybridizes with DNA having a nucleotide sequence complementary to a nucleotide sequence of any one of SEQ ID NO:11 or SEQ ID NO:67 to SEQ ID NO:74, or with DNA having a nucleotide sequence complementary to a region between an 18th nucleotide and a 3' end in a nucleotide sequence of any one of SEQ ID NO:15 or SEQ ID NO:89 to SEQ ID NO:96 under a stringent condition, and that encodes a protein having alanine regeneration enzymatic activity.

20. The recombinant microorganism according to any one of claims 15 to 19, wherein the gene encoding the amino acid regeneration enzyme has a nucleotide sequence encoding an amino acid sequence of any one of SEQ ID:NO 5 or SEQ ID NO:45 to SEQ ID NO:52, or an amino acid sequence that has 80% or more sequence identity with at least one amino acid sequence selected from the group consisting of SEQ ID NO:5 and SEQ ID NO:45 to SEQ ID NO:52, and that has alanine regeneration enzymatic activity.

21. The recombinant microorganism according to any one of claims 1 to 9, wherein the pyridoxamine synthetase is represented by enzyme number EC2.6.1.31 or EC2.6.1.1.

22. The recombinant microorganism according to any one of claims 1 to 9 or claim 21, wherein the gene encoding the pyridoxamine synthetase is derived from *Escherichia coli.*

23. The recombinant microorganism according to any one of claims 1 to 9, claim 21, or claim 22, wherein the gene encoding the pyridoxamine synthetase has a nucleotide sequence of SEQ ID NO:10, or has a nucleotide sequence that hybridizes with DNA having a nucleotide sequence complementary to the nucleotide sequence of SEQ ID NO:10 under a stringent condition, and that encodes a protein having an enzymatic activity of synthesizing pyridoxamine from pyridoxal.

24. The recombinant microorganism according to any one of claims 1 to 9, or claims 21 to 23, wherein the gene encoding the pyridoxamine synthetase has a nucleotide sequence encoding an amino acid sequence of SEQ ID NO:4, or an amino acid sequence that has 80% or more sequence identity with the amino acid sequence of SEQ ID NO:4, and that has an enzymatic activity of synthesizing pyridoxamine from pyridoxal.

25. The recombinant microorganism according to any one of claims 21 to 24, wherein the amino acid regeneration enzyme is a glutamate dehydrogenase.

26. The recombinant microorganism according to claim 25, wherein the gene encoding the amino acid regeneration enzyme is derived from *Escherichia coli.*

27. The recombinant microorganism according to claim 25 or 26, wherein the gene encoding the amino acid regeneration enzyme has a nucleotide sequence of SEQ ID NO:12, or has a nucleotide sequence that hybridizes with DNA having a nucleotide sequence complementary to the nucleotide sequence of SEQ ID NO:12 under a stringent condition, and that encodes a protein having glutamate regeneration enzymatic activity.

28. The recombinant microorganism according to any one of claims 25 to 27, wherein the gene encoding the amino acid regeneration enzyme has a nucleotide sequence encoding an amino acid sequence of SEQ ID NO:6, or an amino acid sequence that has 80% or more sequence identity with the amino acid sequence of SEQ ID NO:6, and that has glutamate regeneration enzymatic activity.

29. The recombinant microorganism according to any one of claims 1 to 28, the recombinant microorganism being recombinant *E. coli.*

30. A method of producing pyridoxamine or a salt thereof, the method comprising bringing the recombinant microorganism according to any one of claims 1 to 29, a culture of the recombinant microorganism, or a treated product of the recombinant microorganism or the culture, into contact with pyridoxine or a salt thereof to produce pyridoxamine or a salt thereof.

31. The production method according to claim 30, wherein the recombinant microorganism, the culture of the recombinant microorganism, or the treated product of the recombinant microorganism or the culture, comprises the pyridoxine dehydrogenase, the pyridoxamine synthetase, and the amino acid regeneration enzyme.

32. The production method according to claim 30 or 31, wherein the treated product of the recombinant microorganism or the treated product of the culture of the recombinant microorganism is treated by treatment comprising one or more selected from the group consisting of heat treatment, cooling treatment, mechanical destruction of a cell, ultrasonic treatment, freeze-thaw treatment, drying treatment, pressurized or reduced pressure treatment, osmotic pressure treatment, cell autolysis, surfactant treatment, enzyme treatment, cell separation treatment, purification treatment, and extraction treatment.

33. A recombinant microorganism, into which at least one polynucleotide selected from the group consisting of the following (1) to (7) is introduced:
(1) a polynucleotide that encodes a protein having an amino acid sequence of SEQ ID NO:1;
(2) a polynucleotide that encodes a protein having an amino acid sequence in which from 1 to 50 amino acids are deleted, added, or substituted in the amino acid sequence of SEQ ID NO:1, the protein having an activity of synthesizing pyridoxine or a salt thereof, or pyridoxal or a salt thereof;
(3) a polynucleotide that encodes a protein having an amino acid sequence having 60% or more sequence identity with the amino acid sequence of SEQ ID NO:1, the protein having an activity of synthesizing pyridoxine or a salt thereof, or pyridoxal or a salt thereof;
(4) a polynucleotide having a nucleotide sequence of SEQ ID NO:13;
(5) a polynucleotide having a nucleotide sequence in which from 1 to 150 nucleotides are deleted, added, or substituted in the nucleotide sequence of SEQ ID NO:13, the polynucleotide encoding a protein having an activity of synthesizing pyridoxine or a salt thereof, or pyridoxal or a salt thereof;
(6) a polynucleotide having a nucleotide sequence having 60% or more sequence identity with the nucleotide sequence of SEQ ID NO:13, the polynucleotide encoding a protein having an activity of synthesizing pyridoxine or a salt thereof, or pyridoxal or a salt thereof; and
(7) a polynucleotide that hybridizes with a polynucleotide having a nucleotide sequence complementary to the nucleotide sequence of SEQ ID NO:13 under a stringent condition, the polynucleotide encoding a protein having an activity of synthesizing pyridoxine or a salt thereof, or pyridoxal or a salt thereof.

34. The recombinant microorganism according to claim 33, wherein the recombinant microorganism is a recombinant bacterium.

35. The recombinant microorganism according to claim 34, wherein the recombinant microorganism is recombinant *E. coli.*

36. The recombinant microorganism according to any one of claims 33 to 35, wherein the protein is an enzyme classified under EC number 1.1.1.65.

37. The recombinant microorganism according to any one of claims 33 to 36, wherein the protein is a pyridoxine dehydrogenase derived from *Saccharomyces cerevisiae.*

38. A method of producing pyridoxal or a salt thereof, the method comprising bringing the recombinant microorganism according to any one of claims 33 to 37 into contact with pyridoxine or a salt thereof.

39. A method of producing pyridoxal or a salt thereof, the method comprising bringing a culture obtained by culturing the recombinant microorganism according to any one of claims 33 to 37, or a treated product of the culture, into contact with pyridoxine or a salt thereof.


SEQUENCE LISTING

<110>  Mitsui Chemicals Inc.
       Renascience Co., Ltd

<120>  Recombinant Microorganism, Method of Producing Pyridoxamine or
       Salt Thereof, and Method of Producing Pyridoxal or Salt Thereof

<130>  MT-F03227-00

<150>  JP2017-095571
<151>  2017-05-12

<150>  JP2017-095573
<151>  2017-05-12

<160>  120

<170>  PatentIn version 3.5

<210>  1
<211>  345
<212>  PRT
<213>  Saccharomyces cerevisiae

<400>  1

Met Ser Val Ala Asp Leu Lys Asn Asn Ile His Lys Leu Asp Thr Gly
1               5                   10                  15

Tyr Gly Leu Met Ser Leu Thr Trp Arg Ala Glu Pro Ile Pro Gln Ser
            20                  25                  30

Gln Ala Phe Glu Ala Met His Arg Val Val Glu Leu Ser Arg Glu Arg
        35                  40                  45

Gly His Lys Ala Phe Phe Asn Val Gly Glu Phe Tyr Gly Pro Asp Phe
    50                  55                  60

Ile Asn Leu Ser Tyr Val His Asp Phe Phe Ala Lys Tyr Pro Asp Leu
65                  70                  75                  80

Arg Lys Asp Val Val Ile Ser Cys Lys Gly Gly Ala Asp Asn Ala Thr
                85                  90                  95

Leu Thr Pro Arg Gly Ser His Asp Asp Val Val Gln Ser Val Lys Asn
            100                 105                 110

Ser Val Ser Ala Ile Gly Gly Tyr Ile Asp Ile Phe Glu Val Ala Arg
        115                 120                 125

Ile Asp Thr Ser Leu Cys Thr Lys Gly Glu Val Tyr Pro Tyr Glu Ser
    130                 135                 140

Phe Glu Ala Leu Ala Glu Met Ile Ser Glu Gly Val Ile Gly Gly Ile
145                 150                 155                 160

Ser Leu Ser Glu Val Asn Glu Glu Gln Ile Arg Ala Ile His Lys Asp
                165                 170                 175

Trp Gly Lys Phe Leu Thr Cys Val Glu Val Glu Leu Ser Leu Phe Ser
            180                 185                 190

Asn Asp Ile Leu His Asn Gly Ile Ala Lys Thr Cys Ala Glu Leu Gly
        195                 200                 205

Leu Ser Ile Ile Cys Tyr Ser Pro Leu Gly Arg Gly Leu Leu Thr Gly
    210                 215                 220

Gln Leu Lys Ser Asn Ala Asp Ile Pro Glu Gly Asp Phe Arg Lys Ser
225                 230                 235                 240

Leu Lys Arg Phe Ser Asp Glu Ser Leu Lys Lys Asn Leu Thr Leu Val
                245                 250                 255

Arg Phe Leu Gln Glu Glu Ile Val Asp Lys Arg Pro Gln Asn Asn Ser
            260                 265                 270

Ile Thr Leu Ala Gln Leu Ala Leu Gly Trp Val Lys His Trp Asn Lys
        275                 280                 285

Val Pro Glu Tyr Ser Gly Ala Lys Phe Ile Pro Ile Pro Ser Gly Ser
    290                 295                 300

Ser Ile Ser Lys Val Asn Glu Asn Phe Asp Glu Gln Lys Thr Lys Leu
305                 310                 315                 320

Thr Asp Gln Glu Phe Asn Ala Ile Asn Lys Tyr Leu Thr Thr Phe His
                325                 330                 335

Thr Val Gly Asp Arg Tyr Glu Met Ala
            340                 345

<210>  2
<211>  333
<212>  PRT
<213>  Schizosaccharomyces pombe

<400>  2

Met Pro Ile Val Ser Gly Phe Lys Val Gly Pro Ile Gly Phe Gly Leu
1               5                   10                  15

Met Gly Leu Thr Trp Lys Pro Lys Gln Thr Pro Asp Glu Glu Ala Phe
            20                  25                  30

Glu Val Met Asn Tyr Ala Leu Ser Gln Gly Ser Asn Tyr Trp Asp Ala
        35                  40                  45

Gly Glu Phe Tyr Gly Val Asp Pro Pro Thr Ser Asn Leu Asp Leu Leu
    50                  55                  60

Ala Arg Tyr Phe Glu Lys Tyr Pro Glu Asn Ala Asn Lys Val Phe Leu
65                  70                  75                  80

Ser Val Lys Gly Gly Leu Asp Phe Lys Thr Leu Phe Leu Val Gly Asn
                85                  90                  95

Arg Thr Ser Phe Pro Arg Ser Val Glu Asn Val Ile Ala His Leu Arg
            100                 105                 110

Gly Thr Gln Lys Leu Asp Leu Phe Gln Cys Ala Arg Val Asp Pro Asn
        115                 120                 125

Val Pro Ile Glu Thr Thr Met Lys Thr Leu Lys Gly Phe Val Asp Ser
    130                 135                 140

Gly Lys Ile Ser Cys Val Gly Leu Ser Glu Val Ser Ala Glu Thr Ile
145                 150                 155                 160

Lys Arg Ala His Ala Val Val Pro Ile Ala Ala Val Glu Val Glu Tyr
                165                 170                 175

Ser Leu Phe Ser Arg Asp Ile Glu Thr Asn Gly Ile Met Asp Ile Cys
            180                 185                 190

Arg Lys Leu Ser Ile Pro Ile Ile Ala Tyr Ser Pro Phe Cys Arg Gly
        195                 200                 205

Leu Leu Thr Gly Arg Ile Lys Thr Val Glu Asp Leu Lys Glu Phe Ala
    210                 215                 220

Lys Ser Phe Pro Phe Leu Glu Tyr Leu Asp Arg Phe Ser Pro Asp Val
225                 230                 235                 240

Phe Ala Lys Asn Leu Pro Phe Leu Gln Ala Val Glu Gln Leu Ala Lys
                245                 250                 255

Lys Phe Gly Met Thr Met Pro Glu Phe Ser Leu Leu Phe Ile Met Ala
            260                 265                 270

Ser Gly Asn Gly Leu Val Ile Pro Ile Pro Gly Ser Thr Ser Val Ser
        275                 280                 285

Arg Thr Lys Ser Asn Leu Asn Ala Leu Asn Lys Ser Leu Ser Pro Glu
    290                 295                 300

Gln Phe Lys Glu Ala Lys Glu Val Leu Ser Lys Tyr Pro Ile Tyr Gly
305                 310                 315                 320

Leu Arg Tyr Asn Glu Gln Leu Ala Gly Thr Leu Ser Val
                325                 330

<210>  3
<211>  393
<212>  PRT
<213>  Mesorhizobium loti

<400>  3

Met Met Arg Tyr Pro Glu His Ala Asp Pro Val Ile Thr Leu Thr Ala
1               5                   10                  15

Gly Pro Val Asn Ala Tyr Pro Glu Val Leu Arg Gly Leu Gly Arg Thr
            20                  25                  30

Val Leu Tyr Asp Tyr Asp Pro Ala Phe Gln Leu Leu Tyr Glu Lys Val
        35                  40                  45

Val Asp Lys Ala Gln Lys Ala Met Arg Leu Ser Asn Lys Pro Val Ile
    50                  55                  60

Leu His Gly Glu Pro Val Leu Gly Leu Glu Ala Ala Ala Ala Ser Leu
65                  70                  75                  80

Ile Ser Pro Asp Asp Val Val Leu Asn Leu Ala Ser Gly Val Tyr Gly
                85                  90                  95

Lys Gly Phe Gly Tyr Trp Ala Lys Arg Tyr Ser Pro His Leu Leu Glu
            100                 105                 110

Ile Glu Val Pro Tyr Asn Glu Ala Ile Asp Pro Gln Ala Val Ala Asp
        115                 120                 125

Met Leu Lys Ala His Pro Glu Ile Thr Val Val Ser Val Cys His His
    130                 135                 140

Asp Thr Pro Ser Gly Thr Ile Asn Pro Ile Asp Ala Ile Gly Ala Leu
145                 150                 155                 160

Val Ser Ala His Gly Ala Tyr Leu Ile Val Asp Ala Val Ser Ser Phe
                165                 170                 175

Gly Gly Met Lys Thr His Pro Glu Asp Cys Lys Ala Asp Ile Tyr Val
            180                 185                 190

Thr Gly Pro Asn Lys Cys Leu Gly Ala Pro Pro Gly Leu Thr Met Met
        195                 200                 205

Gly Val Ser Glu Arg Ala Trp Ala Lys Met Lys Ala Asn Pro Leu Ala
    210                 215                 220

Pro Arg Ala Ser Met Leu Ser Ile Val Asp Trp Glu Asn Ala Trp Ser
225                 230                 235                 240

Arg Asp Lys Pro Phe Pro Phe Thr Pro Ser Val Ser Glu Ile Asn Gly
                245                 250                 255

Leu Asp Val Ala Leu Asp Leu Tyr Leu Asn Glu Gly Pro Glu Ala Val
            260                 265                 270

Trp Ala Arg His Ala Leu Thr Ala Lys Ala Met Arg Ala Gly Val Thr
        275                 280                 285

Ala Met Gly Leu Ser Val Trp Ala Ala Ser Asp Ser Ile Ala Ser Pro
    290                 295                 300

Thr Thr Thr Ala Val Arg Thr Pro Asp Gly Val Asp Glu Lys Ala Leu
305                 310                 315                 320

Arg Gln Ala Ala Arg Ala Arg Tyr Gly Val Val Phe Ser Ser Gly Arg
                325                 330                 335

Gly Glu Thr Leu Gly Lys Leu Thr Arg Ile Gly His Met Gly Pro Thr
            340                 345                 350

Ala Gln Pro Ile Tyr Ala Ile Ala Ala Leu Thr Ala Leu Gly Gly Ala
        355                 360                 365

Met Asn Ala Ala Gly Arg Lys Leu Ala Ile Gly Lys Gly Ile Glu Ala
    370                 375                 380

Ala Leu Ala Val Ile Asp Ala Asp Ala
385                 390

<210>  4
<211>  396

<212>  PRT
<213>  Escherichia coli

<400>  4

Met Phe Glu Asn Ile Thr Ala Ala Pro Ala Asp Pro Ile Leu Gly Leu
1               5                   10                  15

Ala Asp Leu Phe Arg Ala Asp Glu Arg Pro Gly Lys Ile Asn Leu Gly
            20                  25                  30

Ile Gly Val Tyr Lys Asp Glu Thr Gly Lys Thr Pro Val Leu Thr Ser
        35                  40                  45

Val Lys Lys Ala Glu Gln Tyr Leu Leu Glu Asn Glu Thr Thr Lys Asn
    50                  55                  60

Tyr Leu Gly Ile Asp Gly Ile Pro Glu Phe Gly Arg Cys Thr Gln Glu
65                  70                  75                  80

Leu Leu Phe Gly Lys Gly Ser Ala Leu Ile Asn Asp Lys Arg Ala Arg
                85                  90                  95

Thr Ala Gln Thr Pro Gly Gly Thr Gly Ala Leu Arg Val Ala Ala Asp
            100                 105                 110

Phe Leu Ala Lys Asn Thr Ser Val Lys Arg Val Trp Val Ser Asn Pro
        115                 120                 125

Ser Trp Pro Asn His Lys Ser Val Phe Asn Ser Ala Gly Leu Glu Val
    130                 135                 140

Arg Glu Tyr Ala Tyr Tyr Asp Ala Glu Asn His Thr Leu Asp Phe Asp
145                 150                 155                 160

Ala Leu Ile Asn Ser Leu Asn Glu Ala Gln Ala Gly Asp Val Val Leu
                165                 170                 175

Phe His Gly Cys Cys His Asn Pro Thr Gly Ile Asp Pro Thr Leu Glu
            180                 185                 190

Gln Trp Gln Thr Leu Ala Gln Leu Ser Val Glu Lys Gly Trp Leu Pro
        195                 200                 205

Leu Phe Asp Phe Ala Tyr Gln Gly Phe Ala Arg Gly Leu Glu Glu Asp
    210                 215                 220

Ala Glu Gly Leu Arg Ala Phe Ala Ala Met His Lys Glu Leu Ile Val
225                 230                 235                 240

Ala Ser Ser Tyr Ser Lys Asn Phe Gly Leu Tyr Asn Glu Arg Val Gly
                245                 250                 255

Ala Cys Thr Leu Val Ala Ala Asp Ser Glu Thr Val Asp Arg Ala Phe
            260                 265                 270

Ser Gln Met Lys Ala Ala Ile Arg Ala Asn Tyr Ser Asn Pro Pro Ala
        275                 280                 285

His Gly Ala Ser Val Val Ala Thr Ile Leu Ser Asn Asp Ala Leu Arg
    290                 295                 300

Ala Ile Trp Glu Gln Glu Leu Thr Asp Met Arg Gln Arg Ile Gln Arg
305                 310                 315                 320

Met Arg Gln Leu Phe Val Asn Thr Leu Gln Glu Lys Gly Ala Asn Arg
                325                 330                 335

Asp Phe Ser Phe Ile Ile Lys Gln Asn Gly Met Phe Ser Phe Ser Gly
            340                 345                 350

Leu Thr Lys Glu Gln Val Leu Arg Leu Arg Glu Glu Phe Gly Val Tyr
        355                 360                 365

Ala Val Ala Ser Gly Arg Val Asn Val Ala Gly Met Thr Pro Asp Asn
    370                 375                 380

Met Ala Pro Leu Cys Glu Ala Ile Val Ala Val Leu
385                 390                 395

<210>  5
<211>  371
<212>  PRT
<213>  Artificial

<220>
<223>  Modified Alanine Dehydrogenase

<400>  5

Met Ile Ile Gly Val Pro Thr Glu Ile Lys Asn His Glu Tyr Arg Val
1               5                   10                  15

Gly Met Val Pro Ser Ser Val Arg Glu Leu Thr Ile Lys Gly His Val
            20                  25                  30

Val Tyr Val Gln Ser Asp Ala Gly Val Gly Ile Gly Phe Thr Asp Gln
        35                  40                  45

Asp Tyr Ile Asp Ala Gly Ala Ser Ile Leu Ala Thr Ala Ala Glu Val
    50                  55                  60

Phe Ala Lys Ser Asp Met Ile Val Lys Val Lys Glu Pro Gln Ala Val
65                  70                  75                  80

Glu Arg Ala Met Leu Arg His Asp Gln Ile Leu Phe Thr Tyr Leu His
                85                  90                  95

Leu Ala Pro Asp Leu Pro Gln Thr Glu Glu Leu Ile Thr Ser Gly Ala
            100                 105                 110

Val Cys Ile Ala Tyr Glu Thr Val Thr Asp Asp Arg Gly Gly Leu Pro
        115                 120                 125

Leu Leu Ala Pro Met Ser Glu Val Ala Gly Arg Met Ser Ile Gln Ala
    130                 135                 140

Gly Ala Arg Ala Leu Glu Lys Ser Leu Gly Gly Arg Gly Met Leu Leu
145                 150                 155                 160

Gly Gly Val Pro Gly Val Glu Pro Ala Lys Val Val Ile Ile Gly Gly
                165                 170                 175

Gly Met Val Gly Thr Asn Ala Ala Gln Met Ala Val Gly Met Gly Ala
            180                 185                 190

Asp Val Val Val Leu Ala Arg Ser Ile Asp Ala Leu Arg Arg Leu Asn
        195                 200                 205

Val Gln Phe Gly Ser Ala Val Lys Ala Ile Tyr Ser Thr Ala Asp Ala
    210                 215                 220

Ile Glu Arg His Val Leu Glu Ala Asp Leu Val Ile Gly Gly Val Leu
225                 230                 235                 240

Val Pro Gly Ala Ala Ala Pro Lys Leu Ile Thr Arg Asp Met Val Lys
                245                 250                 255

Arg Met Lys Pro Gly Ser Ala Ile Val Asp Val Ala Ile Asp Gln Gly
            260                 265                 270

Gly Cys Val Glu Thr Ser His Ala Thr Thr His Gln Asp Pro Thr Tyr
        275                 280                 285

Ile Val Asp Asp Val Val His Tyr Cys Val Ala Asn Met Pro Gly Ala
    290                 295                 300

Val Ala Arg Thr Ser Thr Phe Ala Leu Asn Asn Ala Thr Leu Pro Tyr
305                 310                 315                 320

Ile Ile Lys Leu Ala Asn Gln Gly Tyr Lys Gln Ala Leu Leu Asn Asp
                325                 330                 335

Lys His Leu Leu Asn Gly Leu Asn Val Met His Gly Lys Val Val Cys
            340                 345                 350

Lys Glu Val Ala Glu Ala Leu Asn Leu Glu Phe Thr Glu Pro Lys Ser
        355                 360                 365

Leu Leu Ala
    370

<210>  6
<211>  447
<212>  PRT
<213>  Escherichia coli

<400>  6

Met Asp Gln Thr Tyr Ser Leu Glu Ser Phe Leu Asn His Val Gln Lys
1               5                   10                  15

Arg Asp Pro Asn Gln Thr Glu Phe Ala Gln Ala Val Arg Glu Val Met
            20                  25                  30

Thr Thr Leu Trp Pro Phe Leu Glu Gln Asn Pro Lys Tyr Arg Gln Met
        35                  40                  45

Ser Leu Leu Glu Arg Leu Val Glu Pro Glu Arg Val Ile Gln Phe Arg
    50                  55                  60

Val Val Trp Val Asp Asp Arg Asn Gln Ile Gln Val Asn Arg Ala Trp
65                  70                  75                  80

Arg Val Gln Phe Ser Ser Ala Ile Gly Pro Tyr Lys Gly Gly Met Arg
                85                  90                  95

Phe His Pro Ser Val Asn Leu Ser Ile Leu Lys Phe Leu Gly Phe Glu
            100                 105                 110

Gln Thr Phe Lys Asn Ala Leu Thr Thr Leu Pro Met Gly Gly Gly Lys
        115                 120                 125

Gly Gly Ser Asp Phe Asp Pro Lys Gly Lys Ser Glu Gly Glu Val Met
    130                 135                 140

Arg Phe Cys Gln Ala Leu Met Thr Glu Leu Tyr Arg His Leu Gly Ala
145                 150                 155                 160

Asp Thr Asp Val Pro Ala Gly Asp Ile Gly Val Gly Gly Arg Glu Val
                165                 170                 175

Gly Phe Met Ala Gly Met Met Lys Lys Leu Ser Asn Asn Thr Ala Cys
            180                 185                 190

Val Phe Thr Gly Lys Gly Leu Ser Phe Gly Gly Ser Leu Ile Arg Pro
        195                 200                 205

Glu Ala Thr Gly Tyr Gly Leu Val Tyr Phe Thr Glu Ala Met Leu Lys
    210                 215                 220

Arg His Gly Met Gly Phe Glu Gly Met Arg Val Ser Val Ser Gly Ser
225                 230                 235                 240

Gly Asn Val Ala Gln Tyr Ala Ile Glu Lys Ala Met Glu Phe Gly Ala
                245                 250                 255

Arg Val Ile Thr Ala Ser Asp Ser Ser Gly Thr Val Val Asp Glu Ser
            260                 265                 270

Gly Phe Thr Lys Glu Lys Leu Ala Arg Leu Ile Glu Ile Lys Ala Ser
        275                 280                 285

Arg Asp Gly Arg Val Ala Asp Tyr Ala Lys Glu Phe Gly Leu Val Tyr
    290                 295                 300

Leu Glu Gly Gln Gln Pro Trp Ser Leu Pro Val Asp Ile Ala Leu Pro
305                 310                 315                 320

Cys Ala Thr Gln Asn Glu Leu Asp Val Asp Ala Ala His Gln Leu Ile
                325                 330                 335

Ala Asn Gly Val Lys Ala Val Ala Glu Gly Ala Asn Met Pro Thr Thr
            340                 345                 350

Ile Glu Ala Thr Glu Leu Phe Gln Gln Ala Gly Val Leu Phe Ala Pro
        355                 360                 365

Gly Lys Ala Ala Asn Ala Gly Gly Val Ala Thr Ser Gly Leu Glu Met
    370                 375                 380

Ala Gln Asn Ala Ala Arg Leu Gly Trp Lys Ala Glu Lys Val Asp Ala

385                 390                 395                 400

Arg Leu His His Ile Met Leu Asp Ile His His Ala Cys Val Glu His
                405                 410                 415

Gly Gly Glu Gly Glu Gln Thr Asn Tyr Val Gln Gly Ala Asn Ile Ala
            420                 425                 430

Gly Phe Val Lys Val Ala Asp Ala Met Leu Ala Gln Gly Val Ile
        435                 440                 445

<210>  7
<211>  1038
<212>  DNA
<213>  Saccharomyces cerevisiae

<400>  7
atgtctgtcg ccgatttgaa aaacaacatc cacaagttag atactggcta tggtttaatg      60

agtttgactt ggagagccga gcctatccct cagtcgcagg ctttcgaggc catgcacaga     120

gtggttgagt tatccagaga acgtgggcac aaggcctttt tcaacgttgg tgaattctat     180

ggtcccgatt ttattaattt gtcgtatgtt cacgacttct ttgcgaaata cccagatttg     240

agaaaggatg tggttatcag ttgtaaaggt ggtgcagaca atgctacctt aacccccaga     300

ggcagtcacg atgatgttgt acaaagcgta aagaattcag ttagtgctat tggtggctac     360

atcgacatct tcgaagtcgc aagaatcgac acttccctat gcacgaaagg agaggtctac     420

ccctacgaat cgttcgaagc gcttgctgag atgatctccg aaggcgttat tggcggtatt     480

tcattaagtg aagttaatga agagcaaatt agagctattc acaaggattg gggaaagttt     540

ttgacctgcg ttgaagtgga actttctttg ttcagtaatg acattttaca caacggaatt     600

gctaaaacat gtgctgaatt ggggttgtcc atcatctgct actccccact gggcagagga     660

ttgttgacag gtcaattgaa gtcaaacgct gatatccctg agggtgactt tagaaagtcg     720

ttaaagagat ttagcgacga gtctttgaaa aaaaacctga ccttggtcag gtttctacag     780

gaagaaatag tcgacaagcg cccacaaaac aactccatta ctcttgcaca actggctttg     840

ggatgggtta agcactggaa caaagttccg gaatacagtg gcgccaaatt tatcccaatt     900

ccaagtggct cttctatttc caaggttaat gaaaactttg atgaacagaa aaccaaactt     960

accgatcaag agttcaatgc cattaacaaa tatttgacta ctttccatac tgttggtgac    1020

agatacgaaa tggcgtaa                                                  1038

<210>  8
<211>  1002
<212>  DNA
<213>  Schizosaccharomyces pombe

<400> 8
atgcctatcg ttagcggatt taaagtcgga cctattggct ttggtcttat gggtttaacc 60

tggaagccca agcaaactcc agatgaagaa gcctttgaag taatgaatta tgccttatcc 120

caaggctcca attattggga tgcgggtgaa ttttatggtg tcgaccctcc cacctctaat 180

ttagatttat tagctagata ttttgaaaaa tatcccgaaa acgctaataa ggtgttttta 240

tctgtcaaag gtggtttgga ttttaaaaca ctattcctgg tgggaaatcg gacttcgttt 300

ccaaggagtg ttgaaaatgt gatagctcat ttacgtggga cccaaaagct agatctcttc 360

caatgtgctc gagtcgaccc aaatgttccc attgagacaa ccatgaagac attgaagggg 420

tttgtggact ctggaaagat ttcatgtgtc ggccttagcg aagtttctgc tgaaactatc 480

aaacgtgctc acgctgtggt ccccattgct gcagtcgaag tcgaatactc cttgttttct 540

cgtgatatcg agacaaacgg tattatggat atttgtagga agttatctat ccctattatt 600

gcatactctc ctttctgtcg tggccttttg actggtcgca ttaagactgt cgaagacctt 660

aaagaatttg ctaaatcttt cccattctta gaatattggg acagatttag ccctgatgtt 720

tttgctaaaa atcttccatt cctccaagct gtcgagcaac ttgctaaaaa gtttggtatg 780

actatgcccg agttttcact tctttttata atggcgtctg gaaacggtct ggtcattcct 840

attcctggct ccacatctgt ttctcgtaca aagagcaatc tcaatgcact gaataaatct 900

ttgtctcctg aacaatttaa agaggccaaa gaggtcttga gtaaataccc aatctacggg 960

ttacgttata atgaacagct tgcgggcact ctttccgttt aa 1002


<210> 9
<211> 1182
<212> DNA
<213> Mesorhizobium loti

<400> 9
atgatgcgct atcccgaaca tgccgatccg gtcatcaccc tgaccgcggg gccggtgaac 60

gcctatccag aagtgttgcg cggcctcggc cgtacggtgc tttatgatta cgatcctgca 120

ttccagctcc tttacgagaa ggtggtcgac aaggcgcaga aggcgatgcg gctgtcgaac 180

aagccggtca tcctgcatgg cgagccggtg ctcggcctcg aagcggcggc ggcgtcgctg 240

atctcgcccg acgacgttgt gctcaatctt gcctcgggcg tctacggcaa gggctttggc 300

tattgggcga agcggtactc gccgcatctg ctcgagatcg aagtgcccta taacgaggcg 360

atcgatccgc aggcggtcgc cgacatgctc aaggcgcatc cggagatcac cgttgtgtcg 420

gtctgtcatc acgacacgcc gtcgggcacc atcaatccga tcgacgccat cggcgcgctg 480

gtctcggcgc atggcgccta tctgatcgtc gacgcggtct cgtcctttgg cggcatgaag 540

acgcatcccg aggattgcaa agccgatatt tatgtcaccg gccccaacaa atgcctgggc 600

gcccctcccg gcctcaccat gatgggcgtc agcgagcggg cctgggccaa gatgaaggcc 660

aaccccctgg cgccgcgcgc atcgatgctg agcattgtcg actgggaaaa tgcctggtcg 720

agggacaagc cgtttccgtt cacgccgtcg gtctcggaga tcaacgggct cgacgtcgcg 780

ctcgatcttt acctcaatga gggaccggag gcggtatggg cgcgccatgc gctcaccgcc 840

aaggccatgc gagcgggcgt caccgcgatg ggcctgtcgg tctgggccgc cagcgacagc 900

atcgcgtcgc cgaccaccac cgccgtccgc acccccgatg gtgtcgacga gaaggcgctt 960

cgccaggccg cgcgtgcccg ttacggcgtg gtgttttcgt cggggcgggg cgagacgttg 1020

gggaagctga cgcgcatcgg ccatatgggg ccgaccgccc agccgatcta cgcgatcgcg 1080

gcgctgacgg cacttggcgg cgccatgaac gcggccggcc ggaaactcgc aatcggcaaa 1140

ggcatcgagg cggcgctggc cgtaatcgac gccgacgcct ga 1182

<210> 10
<211> 1191
<212> DNA
<213> Escherichia coli

<400> 10
atgtttgaga acattaccgc cgctcctgcc gacccgattc tgggcctggc cgatctgttt 60

cgtgccgatg aacgtcccgg caaaattaac ctcgggattg gtgtctataa agatgagacg 120

ggcaaaaccc cggtactgac cagcgtgaaa aaggctgaac agtatctgct cgaaaatgaa 180

accaccaaaa attacctcgg cattgacggc atccctgaat ttggtcgctg cactcaggaa 240

ctgctgtttg gtaaaggtag cgccctgatc aatgacaaac gtgctcgcac ggcacagact 300

ccggggggca ctggcgcact acgcgtggct gccgatttcc tggcaaaaaa taccagcgtt 360

aagcgtgtgt gggtgagcaa cccaagctgg ccgaaccata agagcgtctt taactctgca 420

ggtctggaag ttcgtgaata cgcttattat gatgcggaaa atcacactct tgacttcgat 480

gcactgatta acagcctgaa tgaagctcag gctggcgacg tagtgctgtt ccatggctgc 540

tgccataacc caaccggtat cgaccctacg ctggaacaat ggcaaacact ggcacaactc 600

tccgttgaga aaggctggtt accgctgttt gacttcgctt accagggttt tgcccgtggt 660

ctggaagaag atgctgaagg actgcgcgct ttcgcggcta tgcataaaga gctgattgtt 720

gccagttcct actctaaaaa ctttggcctg tacaacgagc gtgttggcgc ttgtactctg 780

gttgctgccg acagtgaaac cgttgatcgc gcattcagcc aaatgaaagc ggcgattcgc 840

gctaactact ctaacccacc agcacacggc gcttctgttg ttgccaccat cctgagcaac 900

gatgcgttac gtgcgatttg ggaacaagag ctgactgata tgcgccagcg tattcagcgt 960

atgcgtcagt tgttcgtcaa tacgctgcag gaaaaaggcg caaaccgcga cttcagcttt 1020

atcatcaaac agaacggcat gttctccttc agtggcctga caaaagaaca agtgctgcgt 1080

ctgcgcgaag agtttggcgt atatgcggtt gcttctggtc gcgtaaatgt ggccgggatg 1140

acaccagata acatggctcc gctgtgcgaa gcgattgtgg cagtgctgta a          1191

<210>  11
<211>  1116
<212>  DNA
<213>  Artificial

<220>
<223>  Modified Nucleotide Sequence for Alanine Dehydrogenase

<400>  11
atgattattg gtgttccaac agaaatcaaa aaccacgaat accgtgtagg catggtccct      60

tcaagcgtac gtgaattaac cataaaaggt cacgttgttt atgttcaatc agatgcgggt     120

gtgggtattg gttttaccga tcaagactat attgatgcgg gcgcctcgat tttagctaca     180

gcagctgaag tgtttgctaa atcagacatg attgtaaaag ttaaagagcc acaagcagtt     240

gaacgtgcaa tgttacgtca cgaccagatt ttattcactt acttgcacct tgcaccagat     300

ttgccacaaa ctgaagaatt aatcaccagt ggcgctgttt gtattgctta cgaaacagtc     360

actgatgatc gcggcggatt accattactt gccccaatgt cagaagttgc tggccgcatg     420

tcaatccaag caggcgcacg cgcacttgaa aaatcattag gcggccgtgg tatgttgctt     480

ggtggtgttc cgggtgttga gccagctaaa gtggtcatca ttggtggcgg tatggttggt     540

acaaacgcag cacaaatggc tgttggtatg ggtgctgatg ttgttgtttt ggctcgcagt     600

atcgatgcat tacgtcgcct aaatgttcaa tttggttcag cggttaaagc tatttactca     660

acagctgatg ctattgagcg tcacgtatta gaagcagacc ttgttattgg cggtgtatta     720

gttccaggtg ctgcagcacc aaagttaatc actcgtgaca tggttaagcg catgaaacct     780

ggcagtgcaa ttgttgacgt tgcaattgac caaggtggtt gtgttgaaac ttcgcacgct     840

accactcacc aagacccaac ttacattgtt gatgacgtag tgcattactg tgtggctaac     900

atgccaggtg cagtagcgcg tacttctacg tttgcattaa acaatgcaac actaccttac     960

attatcaagt tagcgaacca aggctacaaa caagcattat taaatgacaa acatttatta    1020

aatggcttaa acgtaatgca cggtaaagtc gtctgtaaag aagtggctga agcgttgaac    1080

cttgagttca ctgaaccaaa aagcctactt gcttaa                              1116

<210>  12
<211>  1344
<212>  DNA
<213>  Escherichia coli

<400>  12
atggatcaga catattctct ggagtcattc ctcaaccatg tccaaaagcg cgacccgaat      60

caaaccgagt tcgcgcaagc cgttcgtgaa gtaatgacca cactctggcc ttttcttgaa     120

caaaatccaa aatatcgcca gatgtcatta ctggagcgtc tggttgaacc ggagcgcgtg     180

atccagtttc gcgtggtatg ggttgatgat cgcaaccaga tacaggtcaa ccgtgcatgg 240

cgtgtgcagt tcagctctgc catcggcccg tacaaaggcg gtatgcgctt ccatccgtca 300

gttaaccttt ccattctcaa attcctcggc tttgaacaaa ccttcaaaaa tgccctgact 360

actctgccga tgggcggtgg taaaggcggc agcgatttcg atccgaaagg aaaaagcgaa 420

ggtgaagtga tgcgtttttg ccaggcgctg atgactgaac tgtatcgcca cctgggcgcg 480

gataccgacg ttccggcagg tgatatcggg gttggtggtc gtgaagtcgg ctttatggcg 540

gggatgatga aaaagctctc caacaatacc gcctgcgtct tcaccggtaa gggcctttca 600

tttggcggca gtcttattcg cccggaagct accggctacg gtctggttta tttcacagaa 660

gcaatgctaa aacgccacgg tatgggtttt gaagggatgc gcgtttccgt ttctggctcc 720

ggcaacgtcg cccagtacgc tatcgaaaaa gcgatggaat ttggtgctcg tgtgatcact 780

gcgtcagact ccagcggcac tgtagttgat gaaagcggat tcacgaaaga gaaactggca 840

cgtcttatcg aaatcaaagc cagccgcgat ggtcgagtgg cagattacgc caaagaattt 900

ggtctggtct atctcgaagg ccaacagccg tggtctctac cggttgatat cgccctgcct 960

tgcgccaccc agaatgaact ggatgttgac gccgcgcatc agcttatcgc taatggcgtt 1020

aaagccgtcg ccgaaggggc aaatatgccg accaccatcg aagcgactga actgttccag 1080

caggcaggcg tactatttgc accgggtaaa gcggctaatg ctggtggcgt cgctacatcg 1140

ggcctggaaa tggcacaaaa cgctgcgcgc ctgggctgga aagccgagaa agttgacgca 1200

cgtttgcatc acatcatgct ggatatccac catgcctgtg ttgagcatgg tggtgaaggt 1260

gagcaaacca actacgtgca gggcgcgaac attgccggtt ttgtgaaggt tgccgatgcg 1320

atgctggcgc agggtgtgat ttaa 1344

<210> 13
<211> 1055
<212> DNA
<213> Artificial

<220>
<223> Artificial Fragment for Pyridoxine Dehydrogenase

<400> 13
acaaaaagga taaaacaatg tcagtagctg atctaaagaa caacatacac aaactagata 60

caggatatgg gctaatgagc ctgacctggc gtgcggaacc gatcccgcag agccaagcgt 120

tcgaggcgat gcaccgtgtg gttgagctga gccgtgaacg tggtcacaag gcgttcttta 180

acgtgggcga gttctacggc ccggacttta tcaacctgag ctacgttcac gatttctttg 240

cgaagtatcc ggacctgcgt aaagatgtgg ttattagctg caagggtggc gcggacaacg 300

cgaccctgac cccgcgtggc agccacgatg atgtggttca gagcgtgaaa aacagcgtta 360

gcgcgatcgg tggctatatc gacattttcg aggtggcgcg tattgatacc agcctgtgca 420

ccaaaggtga agtttacccg tatgagagct ttgaagcgct ggcggagatg atcagcgaag 480

gcgtgatcgg tggcattagc ctgagcgagg ttaacgagga acaaatccgt gcgattcaca 540

aggactgggg taaattcctg acctgcgttg aggtggaact gagcctgttt agcaacgata 600

tcctgcacaa cggtattgcg aagacctgcg cggaactggg cctgagcatc atttgctaca 660

gcccgctggg tcgtggcctg ctgaccggtc agctgaaaag caacgcggac atcccggagg 720

gcgatttccg taagagcctg aaacgtttta gcgacgaaag cctgaagaaa aacctgaccc 780

tggtgcgttt cctgcaagag gaaatcgttg ataagcgtcc gcagaacaac agcattaccc 840

tggcgcaact ggcgctgggt tgggtgaagc actggaacaa agttccggag tatagcggtg 900

cgaagtttat cccgattccg agcggcagca gcattagcaa agtgaacgag aacttcgacg 960

aacagaagac caaactgacc gatcaagaat ttaatgcgat taacaaatac ctgaccacct 1020

tccacaccgt tggcgaccgc tatgagatgg cgtaa 1055

<210> 14
<211> 1199
<212> DNA
<213> Artificial

<220>
<223> Artificial Fragment for Pyridoxamine Pyruvate Transaminase

<400> 14
acaaaaagga taaaacaatg atgaggtatc ccgaacacgc tgatccagta ataactttaa 60

cagcaggacc cgtaaacgcg tatccggaag ttctgcgtgg tctgggccgt accgttctgt 120

acgactatga tccggcgttc cagctgctgt acgagaaggt ggttgataag gcgcaaaaag 180

cgatgcgtct gagcaacaaa ccggtgattc tgcacggcga accggttctg ggtctggaag 240

cggcggcggc gagcctgatc agcccggacg atgtggttct gaacctggcg agcggcgtgt 300

acggcaaggg ctttggttac tgggcgaaac gttatagccc gcacctgctg gagattgaag 360

tgccgtataa cgaggcgatt gacccgcagg cggttgcgga tatgctgaag gcgcacccgg 420

aaatcaccgt ggttagcgtg tgccaccacg acaccccgag cggtaccatc aacccgattg 480

acgcgattgg tgcgctggtt agcgcgcacg gtgcgtacct gattgtggac gcggttagca 540

gcttcggtgg catgaagacc cacccggagg actgcaaagc ggatatctat gtgaccggtc 600

cgaacaaatg cctgggtgct ccgccgggtc tgaccatgat gggtgttagc gaacgtgcgt 660

gggcgaagat gaaagcgaac ccgctggcgc cgcgtgcgag catgctgagc attgtggact 720

gggagaacgc gtggagccgt gataagccgt tcccgtttac cccgagcgtg agcgaaatca 780

acggcctgga cgttgcgctg gatctgtatc tgaacgaagg tccggaagcg gtttgggcgc 840

gtcatgcgct gaccgcgaag gcgatgcgtg cgggcgtgac cgcgatgggt ctgagcgttt 900

gggcggcgag cgatagcatt gcgagcccga ccaccaccgc ggtgcgtacc ccggatggtg 960

ttgatgaaaa ggcgctgcgt caggcggcgc gtgcgcgtta cggcgtggtt tttagcagcg 1020

gccgtggtga aaccctgggt aaactgaccc gtattggcca catgggtccg accgcgcaac 1080

cgatttatgc gattgcggcg ctgaccgcgc tgggtggcgc gatgaacgcg gcgggtcgta 1140

agctggcgat tggcaaaggt atcgaagcgg cgctggcggt tatcgacgcg gatgcgtga 1199

<210> 15
<211> 1133
<212> DNA
<213> Artificial

<220>
<223> Artificial Fragment for Alanine Dehydrogenase

<400> 15
acaaaaagga taaaacaatg ataataggag tacccacaga aataaagaac cacgaataca 60

gggttgggat ggtaccgagc agcgtgcgtg aactgaccat caagggccac gtggtttacg 120

ttcagagcga tgcgggcgtg ggtattggtt tcaccgacca agattatatc gatgcgggtg 180

cgagcattct ggcgaccgcg gcggaagtgt ttgcgaaaag cgacatgatc gttaaggtga 240

aagaaccgca ggcggtggaa cgtgcgatgc tgcgtcacga ccaaattctg ttcacctacc 300

tgcacctggc gccggatctg ccgcagaccg aggaactgat caccagcggt gcggtttgca 360

ttgcgtatga gaccgtgacc gatgatcgtg gtggcctgcc gctgctggcg ccgatgagcg 420

aggttgcggg tcgtatgagc atccaagcgg gtgcgcgtgc gctggagaag agcctgggtg 480

gtcgtggtat gctgctgggt ggcgttccgg gcgtggaacc ggcgaaggtg gttatcattg 540

gtggcggtat ggttggtacc aacgcggcgc agatggcggt gggtatgggt gcggatgtgg 600

ttgtgctggc gcgtagcatt gatgcgctgc gtcgtctgaa cgttcaattc ggcagcgcgg 660

tgaaagcgat ctacagcacc gcggacgcga ttgagcgtca cgttctggaa gcggatctgg 720

tgatcggcgg tgttctggtt ccgggtgcgg cggcgccgaa gctgattacc cgtgacatgg 780

ttaagcgtat gaaaccgggc agcgcgatcg ttgacgtggc gattgatcag ggcggttgcg 840

ttgaaaccag ccacgcgacc acccaccaag atccgaccta catcgtggac gatgttgtgc 900

actattgcgt tgcgaacatg ccgggtgcgg tggcgcgtac cagcaccttt gcgctgaaca 960

acgcgaccct gccgtacatc attaagctgg cgaaccaggg ctataaacaa gcgctgctga 1020

acgataagca cctgctgaac ggtctgaacg tgatgcacgg caaggttgtg tgcaaggaag 1080

tggcggaggc gctgaacctg gagtttaccg agccgaagag cctgctggcg taa 1133

<210> 16
<211> 34
<212> DNA

<213>  Artificial

<220>
<223>  Primer Sequence

<400>  16
acgcgtcgac acaaaaagga taaaacaatg tcag                                    34


<210>  17
<211>  29
<212>  DNA
<213>  Artificial

<220>
<223>  Primer Sequence

<400>  17
cccaagcttt tacgccatct catagcggt                                          29


<210>  18
<211>  29
<212>  DNA
<213>  Artificial

<220>
<223>  Primer Sequence

<400>  18
ccggaattca caaaaaggat aaaacaatg                                          29


<210>  19
<211>  28
<212>  DNA
<213>  Artificial

<220>
<223>  Primer Sequence

<400>  19
cgcggatcct cacgcatccg cgtcgata                                           28


<210>  20
<211>  29
<212>  DNA
<213>  Artificial

<220>
<223>  Primer Sequence

<400>  20
cgcggatcca caaaaaggat aaaacaatg                                          29


<210>  21
<211>  30
<212>  DNA
<213>  Artificial

<220>

<223>  Primer Sequence

<400>  21
acgcgtcgac ttacgccagc aggctcttcg                                        30


<210>  22
<211>  345
<212>  PRT
<213>  Saccharomyces eubayanus

<400>  22

Met Ser Val Thr Glu Leu Arg Asn Asp Ile His Lys Leu Asp Thr Gly
1               5                   10                  15

Tyr Gly Leu Met Ser Leu Thr Trp Arg Ala Glu Pro Ile Pro Gln Ser
            20                  25                  30

Gln Ala Phe Glu Ala Met His Arg Ala Val Glu Leu Ala Arg Glu Arg
        35                  40                  45

Gly His Lys Ala Phe Phe Asn Val Gly Glu Phe Tyr Gly Pro Asp Phe
    50                  55                  60

Val Asn Leu Ile Phe Val Arg Asp Phe Phe Ala Lys Tyr Pro Asp Leu
65                  70                  75                  80

Arg Lys His Val Val Ile Ser Cys Lys Gly Gly Met Glu Val Ser Thr
                85                  90                  95

Leu Thr Pro Asn Gly Ser His Asp Ala Val Ile Lys Ser Val Lys Asn
            100                 105                 110

Ser Val Ser Ala Ile Gly Gly Tyr Ile Asp Ile Phe Glu Val Ala Arg
        115                 120                 125

Leu Asp Thr Ser Leu Cys Ala Lys Gly Glu Val Tyr Pro Tyr Glu Ser
    130                 135                 140

Phe Glu Ala Leu Ala Glu Met Ile Ser Glu Gly Val Ile Gly Gly Ile
145                 150                 155                 160

Ser Leu Ser Glu Val Thr Glu Glu Gln Ile Arg Ala Ile Tyr Lys Asp
                165                 170                 175

Trp Gly Lys Phe Leu Thr Cys Val Glu Leu Glu Val Ser Leu Phe Ser
            180                 185                 190

Thr Asp Ile Phe His Asn Gly Ile Ala Lys Thr Cys Ala Glu Leu Gly
        195                 200                 205

Leu Thr Val Ile Cys Tyr Ser Pro Leu Gly Arg Gly Leu Leu Thr Gly
    210                 215                 220

Gln Leu Lys Ser Asn Ala Asp Ile Pro Glu Gly Asp Phe Arg Lys Ala
225                 230                 235                 240

Leu Lys Arg Phe Ser Asp Glu Ser Leu Lys Lys Asn Leu Asp Leu Val
                245                 250                 255

Arg Phe Leu Gln Glu Glu Ile Val Gly Lys Arg Ser Lys Asp Asn Ser
            260                 265                 270

Ile Thr Leu Pro Gln Leu Ala Leu Gly Trp Ile Lys His Trp Asn Tyr
        275                 280                 285

Val Pro Glu Tyr Lys Gly Ala Lys Phe Ile Pro Ile Pro Ser Gly Ser
    290                 295                 300

Ser Ile Ser Lys Val Asn Glu Asn Phe Asp Glu Lys Lys Thr Gln Leu
305                 310                 315                 320

Thr Asp Lys Glu Phe Lys Ala Ile Asn Asp Phe Leu Ala Thr Phe His
                325                 330                 335

Thr Val Gly Asp Arg Tyr Glu Phe Asn
            340                 345

<210>  23
<211>  349
<212>  PRT
<213>  Torulaspora delbrueckii

<400>  23

Met Val Glu Gln Ser Ala Ile Asn Lys Leu Lys Gln Asp Leu Ser Ala
1               5                   10                  15

Ile Asp Thr Gly Tyr Gly Leu Met Ser Leu Thr Trp Arg Ala Glu Pro
            20                  25                  30

Ile Pro Glu Thr Gln Ala His Glu Thr Met Lys Arg Val Val Glu Leu
        35                  40                  45

Ala Gln Ser Lys Gly His Lys Ala Phe Phe Asn Cys Gly Glu Phe Tyr
    50                  55                  60

Gly Pro Asn Arg Ile Asn Leu Thr Tyr Ile Arg Asp Phe Phe Val Lys
65                  70                  75                  80

Asn Pro Glu Leu Arg Lys Tyr Val Ile Ile Ser Cys Lys Gly Gly Cys
                85                  90                  95

Asp Cys Glu Thr Leu Thr Pro Lys Gly Lys His Asp Asp Val Ile Lys
            100                 105                 110

Ser Val Glu Ala Ser Val Ala Ala Ile Gly Gly Phe Ile Asp Ile Phe
        115                 120                 125

Glu Val Ala Arg Leu Asp Leu Ser Leu Cys Thr Asn Gly Glu Thr Tyr
    130                 135                 140

Pro Tyr Glu Ser Phe Glu Ala Leu Ala Glu Met Val Asp Asn Gly Ala
145                 150                 155                 160

Ile Gly Ala Ile Ser Leu Ser Glu Val Thr Ala Glu Gln Ile Arg Gly
                165                 170                 175

Ile Ser Lys Asp Trp His Lys Tyr Leu Val Ser Val Glu Val Glu Leu
            180                 185                 190

Ser Met Phe Thr Lys Ala Ile Leu Thr Asn Gly Val Val Lys Ala Asn
        195                 200                 205

Asn Asp Leu Asn Leu Val Thr Ile Leu Tyr Ser Pro Leu Gly Arg Gly
    210                 215                 220

Leu Leu Thr Gly Ala Val Thr Ser Ser Lys Asp Ile Pro Ala Gly Asp
225                 230                 235                 240

Phe Arg Asn Leu Leu Lys Arg Phe Asp Asp Glu Ser Leu Arg Gln Asn
                245                 250                 255

Leu Thr Leu Leu Asp Phe Leu Arg Asp Glu Ile Ile Ala Lys Arg Pro
            260                 265                 270

Ala Asn Asn Glu Ile Thr Leu Pro Gln Val Ala Leu Gly Trp Asp Lys
        275                 280                 285

Tyr Trp Asn Lys Thr Gly Glu Tyr Pro Asn Thr His Phe Leu Pro Ile
    290                 295                 300

Pro Ser Gly Ser Ser Val Lys Lys Leu Glu Glu Asn Phe Asp Glu Ala
305                 310                 315                 320

Lys Thr Gln Ile Thr Ala Glu Glu Phe Arg Lys Ile Asn Asp Phe Leu

325                 330                 335

Lys Asn Phe Lys Thr Val Gly Asp Ala Tyr Glu Met Arg
            340                 345

<210>  24
<211>  341
<212>  PRT
<213>  Zygosaccharomyces bailii

<400>  24

Met Ser Lys Thr Gly Glu Leu Lys Arg Gln Leu Asn Gln Leu His Thr
1               5                   10                  15

Gly Tyr Gly Leu Met Ser Leu Thr Trp Arg Ala Asp Pro Val Pro Gln
            20                  25                  30

Glu Gln Ala Phe Glu Ala Met Glu Arg Val Val Ser Leu Ala Gln Ala
        35                  40                  45

Ser Gly Asn Lys Ala Phe Phe Asn Val Gly Glu Phe Tyr Gly Pro Asp
    50                  55                  60

Tyr Ser Asn Leu Glu Leu Val Lys Ala Phe Phe Glu Ala Lys Pro Glu
65                  70                  75                  80

Leu Arg Glu His Val Leu Ile Ser Cys Lys Gly Gly Val Asp Asn Thr
                85                  90                  95

Lys Leu Leu Pro Lys Gly Lys Tyr Ala Asp Val Leu Ser Ser Ile Glu
            100                 105                 110

Gln Cys Val Lys His Leu Gly Thr His Leu Asp Ile Phe Glu Val Ala
        115                 120                 125

Arg Leu Asp Lys Ser Leu Gly Gly Glu Tyr Pro Arg Glu Ser Phe Glu
    130                 135                 140

Ala Met Ala Ser Met Val Asp Lys Gly Ile Ile Asp Gly Ile Ser Leu
145                 150                 155                 160

Ser Glu Val Thr Ala Asp Glu Ile Arg Ala Ile Gly His Asp Trp Ala
                165                 170                 175

Lys Tyr Leu Val Cys Val Glu Val Glu Phe Ser Met Phe Ser Pro Gln
            180                 185                 190

Ile Leu His Asn Gly Val Leu Asp Ala Cys Asn Asp Leu Gly Leu Ile

195                 200                 205

Val Ile Ala Tyr Ser Pro Leu Gly Arg Gly Leu Leu Thr Gly Thr Ile
    210                 215                 220

Thr Lys Asp Ser Ala Phe Lys Asp Phe Arg Ser Ser Leu Lys Arg Phe
225                 230                 235                 240

Gln Lys Asp Ser Leu Gln Lys Asn Leu Ala Leu Thr Glu Phe Leu Gln
                245                 250                 255

Asp Gln Ile Val Asp Gln Arg Ser Ser Thr Asn Pro Ile Ser Leu Pro
            260                 265                 270

Gln Val Ala Leu Gly Trp Val Lys Ser Leu Asn Asn Gly Lys Tyr Pro
        275                 280                 285

His Thr His Ile Val Pro Ile Pro Ser Gly Ser Asp Lys Arg Lys Val
    290                 295                 300

Glu Glu Asn Phe Asp Glu Ser Arg Ala Lys Leu Thr Val Ser Glu Ile
305                 310                 315                 320

Glu Lys Ile Asn Asp Phe Leu Lys Ser Phe Asn Val Val Gly Asp Arg
                325                 330                 335

Tyr Glu Trp Val Ser
            340

<210>  25
<211>  320
<212>  PRT
<213>  Aspergillus oryzae

<400>  25

Met Pro Ser Leu Val Gly Arg Asp Val Gly His Thr Gly Tyr Gly Leu
1               5                   10                  15

Met Arg Met Thr Trp Val Pro Gln Pro Pro Pro Gln Glu Gln Cys Phe
            20                  25                  30

Glu Ala Leu Asn Thr Ala Leu Ala His Gly Ser Asn Phe Trp Asn Ala
        35                  40                  45

Gly Glu Leu Tyr Gly Thr Pro Glu Tyr Asn Ser Leu His Leu Leu His
    50                  55                  60

Ser Tyr Phe Ala Gln His Pro Glu Asn Ala Asp Lys Val Val Leu Ser

65                  70                  75                  80

Ile Lys Gly Gly Leu Lys Pro Gly Gln Leu Val Pro Asp Gly Ser Glu
                85                  90                  95

Ala Asn Ile Arg Arg Ser Val Asp Glu Cys Leu Arg Val Leu Asp Gly
            100                 105                 110

Lys Lys Lys Ile Asp Ile Phe Glu Cys Ala Arg Gln Asp Pro Lys Thr
        115                 120                 125

Thr Val Glu Gln Thr Val Thr Val Leu Ala Gln Leu Val Lys Glu Gly
    130                 135                 140

Lys Ile Gly Gly Ile Gly Leu Ser Glu Val Asp Ala Glu Thr Ile Arg
145                 150                 155                 160

Arg Ala His Lys Val His Pro Ile Ala Ala Val Glu Val Glu Met Ser
                165                 170                 175

Leu Phe Asp Leu Thr Ile Leu Gln Asn Asp Val Ala Lys Val Cys Ala
            180                 185                 190

Glu Leu Asn Ile Pro Ile Val Ala Tyr Ser Pro Leu Gly Arg Gly Val
        195                 200                 205

Leu Ala Gly Ala Phe Thr Thr Ala Ala Asp Ile Pro Asp Gly Asp Phe
    210                 215                 220

Arg Lys Thr Leu Pro Lys Phe Gln Asp Glu Ala Met Lys Gln Asn Ile
225                 230                 235                 240

Lys Leu Val Asn Glu Val Asn Asp Leu Ala Ala Arg Lys Gly Val Ala
                245                 250                 255

Pro Val Gln Ile Ala Leu Ala Trp Val Leu Thr Leu Ser Gly Lys Pro
            260                 265                 270

Gly Met Pro Thr Ile Ile Pro Ile Pro Gly Gly Thr Thr Ser Ala Lys
        275                 280                 285

Val Ala Gln Asn Leu Gln Ala Pro Arg Leu Thr Asp Ala Glu Met Ala
    290                 295                 300

Glu Ile Asp Ala Ile Leu Lys Arg Asn Glu Ile Val Gly Thr Arg Tyr
305                 310                 315                 320

<210>  26
<211>  343
<212>  PRT
<213>  Kluyveromyces marxianus

<400>  26

Met Pro Ser Pro Ser Asp Tyr Arg Lys Glu Leu Leu Asp Val Glu Val
1               5                   10                  15

Gly Tyr Gly Leu Met Ser Leu Thr Trp Arg Lys Asp Pro Leu Pro Ala
            20                  25                  30

Glu Lys Val Phe Pro Thr Met Lys Lys Val Val Glu Lys Ala Gly Asn
        35                  40                  45

Lys Lys Ala Leu Phe Asn Val Gly Glu Phe Tyr Gly Pro His Leu Ala
    50                  55                  60

Asn Tyr Lys Leu Leu Gln Ser Phe Phe Asp Lys Tyr Pro Glu Asp Arg
65                  70                  75                  80

Lys Lys Val Ile Ile Ser Ala Lys Gly Gly Val Asn Val Glu Thr Leu
                85                  90                  95

Gln Pro Thr Gly Asp Ala Asp Ser Ile Ser Ala Ser Ile Glu Asn Ala
            100                 105                 110

Leu Lys Val Phe Gly Gly Tyr Leu Asp Ile Tyr Glu Pro Ala Arg Ile
        115                 120                 125

Asp Leu Ala Leu Ala Glu Lys Asn Gly Glu Lys Leu Phe Pro Arg Glu
    130                 135                 140

Thr Phe Asp Thr Ile Val Lys Tyr Ile Lys Asp Gly Lys Val Gly Gly
145                 150                 155                 160

Phe Ser Leu Ser Glu Val Thr Asn Glu Gln Ile Arg Ala Ile Tyr Lys
                165                 170                 175

Glu Tyr Gly Glu Tyr Leu Ala Cys Val Glu Val Glu Leu Ser Ile Ala
            180                 185                 190

Ser Pro Glu Ile Ile Ser Asn Gly Ile Leu Asp Thr Cys Asn Glu Phe
        195                 200                 205

Gly Ile Pro Val Val Ala Tyr Ala Pro Leu Gly Arg Gly Leu Leu Thr
    210                 215                 220

Gly Ser Leu Lys Ser Thr Ala Asp Ile Pro Glu Gly Asp Phe Arg Gly
225                 230                 235                 240

Gln Leu Lys Arg Phe Gln Asp Asp Ala Ile Ala His Asn Leu Thr Leu
                245                 250                 255

Val Lys Phe Leu Gln Asp Glu Ile Ala Ala Lys Arg Ser Asp Asn Pro
            260                 265                 270

Ser Leu Pro Gln Val Ala Ile Gly Trp Val Arg Gly Leu Arg Arg Glu
        275                 280                 285

Tyr Pro Lys Thr Asn Ile Ile Pro Ile Pro Ser Gly Ser Ser Val Glu
    290                 295                 300

Lys Val Ser Val Asn Phe Asp His Ile Asp Leu Ser Asp Asp Glu Met
305                 310                 315                 320

Ser Lys Ile Lys Asp Phe Leu Lys Ser Phe Lys Ile Ala Gly Asp Arg
                325                 330                 335

Tyr Glu Phe Ala Gln Ala His
            340

<210>  27
<211>  349
<212>  PRT
<213>  Candida albicans

<400>  27

Met Ser Phe Lys Pro Val Glu Ile Ser Gly Lys Phe Gly Phe Gly Thr
1               5                   10                  15

Met Ser Met Thr Trp Thr Pro Thr Pro Pro Pro Ala Gln Gln Ser Ile
            20                  25                  30

Asp Thr Leu Lys Phe Val Thr Ser His Pro Lys Phe Gly Thr Lys Leu
        35                  40                  45

Ile Asn Gly Gly Glu Phe Tyr Gly Pro Asp Phe Ala Asn Leu Lys Leu
    50                  55                  60

Leu Lys Gln Phe Leu Glu Glu Asn Asp Pro Glu Glu Asn Lys Gln Leu
65                  70                  75                  80

Ile Ile Ser Ile Lys Gly Gly Ala Asp Asn Glu Thr Leu Lys Pro Asn
                85                  90                  95

Gly Thr Lys Glu Phe Val Ser Lys Ser Ile Glu Asn Ile Val Ser Phe
            100                 105                 110

Phe Pro Lys Gln Lys Gln Asn Arg Pro Lys Leu Leu Phe Glu Met Ala
        115                 120                 125

Arg Val Asp Pro Ser Val Pro Tyr Gly Glu Thr Ile Gly Tyr Ile Ser
    130                 135                 140

Glu Tyr Val Lys Ser Gly Val Ile Asp Gly Ile Ser Leu Ser Glu Val
145                 150                 155                 160

Gly Lys Glu Ser Ile Gln Ala Ala Leu Lys Val Phe Pro Ile Ser Cys
                165                 170                 175

Val Glu Leu Glu Leu Ser Leu Phe Ser Gln Glu Val Ile Thr Thr Gly
            180                 185                 190

Ile Leu Glu Glu Leu Ser Lys His Asn Leu Pro Leu Ile Ala Tyr Ser
        195                 200                 205

Pro Leu Cys Arg Gly Leu Leu Thr Asp Tyr Ala Val Glu Asn Ser Asp
    210                 215                 220

Thr Phe Leu Ala Ser Ile Pro Gln Gly Asp Ile Arg His His Leu Asp
225                 230                 235                 240

Lys Phe Gln Pro Asp Thr Phe Asn Lys Asn Leu Pro Ala Leu Lys Glu
                245                 250                 255

Leu Tyr Lys Phe Ala His Glu Val Lys Asn Thr Thr Leu Glu Ser Leu
            260                 265                 270

Ala Leu Ser Trp Ile Val Thr Val Ser Glu Ala Arg Asn Phe Arg Gly
        275                 280                 285

Ile Glu Lys Val Thr Arg Ile Leu Pro Ile Pro Ser Gly Ser Thr Lys
    290                 295                 300

Lys Arg Val Glu Ser Asn Phe Gly Ser Leu Ile Glu Leu Thr Asp Asp
305                 310                 315                 320

Asp Leu Gln Ser Ile Asp Glu Ile Phe Ala Lys Tyr Pro Ile Ser Gly
                325                 330                 335

Leu Arg Tyr Asn Gln Phe Leu Glu Gly Thr Leu Phe Gln
            340                 345

<210>  28
<211>  329
<212>  PRT
<213>  Yarrowia lipolytica

<400>  28

Met Thr Phe Asn Lys Ile Thr Pro Thr Ser Lys Gly Phe Gly Leu Met
1               5                   10                  15

Gly Phe Thr Trp Arg Glu Thr Glu Thr Pro Asp Glu Ile Ala Phe Pro
            20                  25                  30

Thr Met Lys Arg Ala Ile Glu Lys Gly Ile Leu Val Trp Asn Ser Ala
        35                  40                  45

Glu Phe Tyr Gly Thr Lys Asp Pro Leu Gly Asn Leu Lys Leu Ile Arg
    50                  55                  60

Arg Tyr Phe Glu Lys Tyr Pro Glu Asp Ala Asp Lys Val Cys Leu Met
65                  70                  75                  80

Val Lys Gly Gly Leu Gly Pro Asn Leu Val Pro Asn Gly Ser Lys Glu
                85                  90                  95

Phe Val Gln Lys Ser Ile Asp Thr Val Leu Glu Thr Leu Gly Gly Ser
            100                 105                 110

Val Pro Val Ser Ile Phe Gln Cys Ala Arg Val Asp Pro Asn Thr Pro
        115                 120                 125

Ile Glu Glu Thr Val Ala Ala Ile Lys Glu Tyr Val Asp Ala Gly Lys
    130                 135                 140

Ile Gly Ala Leu Gly Leu Ser Glu Val Gly Ala Ala Thr Ile Glu Lys
145                 150                 155                 160

Val His Ser Leu Ser Pro Ile Ala Ala Val Glu Asn Glu Leu Ser Leu
                165                 170                 175

Trp Ala Thr Asp Ile Phe Thr Asn Gly Val Ala Glu Val Cys Ala Lys
            180                 185                 190

Tyr Asn Ile Pro Ile Thr Ala Tyr Ala Pro Leu Gly Ser Gly Val Leu
        195                 200                 205

Thr Gly Arg Phe Lys Ser Thr Gln Asp Val Pro Glu Gly Asp Ala Arg
    210                 215                 220

Lys His Met Val Lys Tyr Gln Glu Glu Asn Leu Lys Gln Asn Leu Val
225                 230                 235                 240

Leu Val Asp Arg Leu Lys Glu Val Ala Thr Lys Ala Gly Ile Pro Leu
                245                 250                 255

Pro Gln Met Ala Ile Ala Trp Val Lys Ala Gln Ser Asn His Asn Gly
            260                 265                 270

His Gly Thr Ile Ile Pro Ile Pro Gly Asn Thr Thr Val Ala Arg Leu
        275                 280                 285

Glu Glu Asn His Asn Asp Leu Thr Leu Ser Ala Glu Thr Leu Lys Glu
    290                 295                 300

Ile Asp Asp Val Leu Lys Ser Thr Lys Ile Val Gly Asp Arg Gly Phe
305                 310                 315                 320

Thr Leu Gly Gly Glu Phe Met Asn Gly
                325

<210>  29
<211>  392
<212>  PRT
<213>  Mesorhizobium sp. YR577

<400>  29

Met Arg Tyr Pro Asp His Thr Glu Pro Val Ile Thr Leu Thr Ala Gly
1               5                   10                  15

Pro Val Asn Ala Tyr Pro Asp Val Leu Arg Gly Leu Gly Arg Thr Val
            20                  25                  30

Leu Tyr Asp Tyr Asp Pro Ala Phe Gln Leu Phe Tyr Glu Lys Val Ile
        35                  40                  45

Gly Lys Ala Gln Lys Ala Met Arg Leu Ser Asn Lys Pro Val Val Leu
    50                  55                  60

His Gly Glu Pro Val Met Gly Leu Glu Ala Ala Ala Ala Ser Leu Ile
65                  70                  75                  80

Ser Arg Asp Asp Val Val Leu Asn Leu Ala Ser Gly Val Tyr Gly Lys
                85                  90                  95

Gly Phe Gly Tyr Trp Ala Lys Arg Tyr Ser Pro Asn Leu Leu Glu Ile
            100                 105                 110

Glu Val Pro Tyr Asn Glu Ala Ile Asp Pro Gln Ser Val Ala Asp Met
        115                 120                 125

Leu Lys Ser His Pro Glu Ile Thr Val Val Ser Val Cys His His Asp
    130                 135                 140

Thr Pro Ser Gly Thr Ile Asn Pro Ile Asp Glu Ile Gly Ala Ile Val
145                 150                 155                 160

Ser Ala His Gly Gly Tyr Leu Ile Val Asp Ala Val Ser Ser Phe Gly
                165                 170                 175

Gly Met Lys Thr His Pro Glu Asp Cys Lys Ala Asp Ile Tyr Val Thr
            180                 185                 190

Gly Pro Asn Lys Cys Leu Gly Ala Pro Pro Gly Leu Thr Leu Met Ser
        195                 200                 205

Val Ser Asp Arg Ala Trp Thr Arg Met Lys Ala Asn Ser Ala Ala Pro
    210                 215                 220

Arg Ala Ser Val Leu Ser Ile Leu Asp Trp Glu His Ala Trp Ser Lys
225                 230                 235                 240

Glu Lys Pro Phe Pro Phe Thr Pro Ser Val Ala Glu Met Asn Gly Leu
                245                 250                 255

Asp Val Ala Leu Asp Leu Tyr Leu Asn Glu Gly Pro Glu Ala Val Trp
            260                 265                 270

Ala Arg His Ala Leu Thr Ala Lys Ala Thr Arg Ala Gly Ala Gln Ala
        275                 280                 285

Met Gly Leu Ser Ile Trp Ala Ala Asn Glu Lys Ile Ala Ser Pro Thr
    290                 295                 300

Thr Thr Ala Ile Arg Thr Pro Asp Gly Val Asp Glu Lys Ala Leu Arg
305                 310                 315                 320

Gln Ala Ala Arg Glu Arg Tyr Gly Val Val Phe Ser Ser Gly Arg Gly
                325                 330                 335

Glu Thr Leu Gly Lys Leu Thr Arg Ile Gly His Met Gly Pro Thr Ala
            340                 345                 350

Gln Pro Ile Tyr Ala Ile Ala Ala Leu Thr Ala Leu Gly Gly Ala Leu
        355                 360                 365

Asn Ala Ala Gly Arg Lys Leu Gln Val Gly Lys Gly Ile Glu Ala Ala
    370                 375                 380

Leu Ala Val Ile Asp Ala Asp Ala
385                 390

<210>  30
<211>  392
<212>  PRT
<213>  Pseudaminobacter salicylatoxidans

<400>  30

Met Arg Tyr Lys Asp Asn Ala Asp Pro Val Ile Thr Leu Thr Ser Gly
1               5                   10                  15

Pro Val Asn Ala Tyr Ala Asp Val Leu Arg Gly Leu Ser Arg Thr Val
            20                  25                  30

Leu Tyr Asp Tyr Asp Pro Ala Phe Gln Leu Phe Tyr Glu Asn Val Val
        35                  40                  45

Ala Lys Ala Gln Lys Ala Met Arg Leu Ser Thr Arg Pro Val Leu Leu
    50                  55                  60

Gln Gly Glu Pro Val Met Gly Leu Glu Ala Ala Ala Ala Ser Leu Ile
65                  70                  75                  80

Ser Ser Glu Asp Val Val Leu Asn Leu Ala Ser Gly Val Tyr Gly Lys
                85                  90                  95

Gly Phe Gly Tyr Trp Ala Lys Arg Tyr Ser Pro Asn Leu Leu Glu Ile
            100                 105                 110

Glu Thr Pro Tyr Asn Glu Ser Ile Asp Pro Gln Ala Val Ala Asp Met
        115                 120                 125

Leu Lys Lys His Pro Glu Ile Thr Val Val Ser Val Cys His His Asp
    130                 135                 140

Thr Pro Ser Gly Thr Ile Asn Pro Ile Asp Glu Ile Gly Ala Ile Val
145                 150                 155                 160

Ser Ala His Gly Ala Tyr Phe Ile Val Asp Ala Val Ser Ser Phe Gly
                165                 170                 175

Gly Met Lys Thr His Pro Glu Asp Cys Lys Ala Asp Leu Tyr Val Thr
            180                 185                 190

Gly Pro Asn Lys Cys Leu Gly Ala Pro Pro Gly Leu Thr Met Met Gly
        195                 200                 205

Val Ser Asp Arg Ala Trp Ala Arg Met Lys Ala Asn Pro Ser Ala Pro
    210                 215                 220

Arg Ala Ser Val Leu Ser Ile Leu Asp Trp Glu Asp Ala Trp Ser His
225                 230                 235                 240

Glu Lys Pro Phe Pro Phe Thr Pro Ser Val Ala Glu Ile Asn Gly Leu
                245                 250                 255

Asp Val Ala Leu Asp Leu Tyr Leu Asn Glu Gly Pro Glu Val Val Trp
            260                 265                 270

Ala Arg His Ala Leu Thr Ala Lys Ala Thr Arg Ala Gly Ala Leu Ala
        275                 280                 285

Met Gly Leu Ser Leu Trp Ala Ala Ser Glu Lys Ile Ala Ser Pro Thr
    290                 295                 300

Thr Thr Ser Ile Arg Thr Pro Gln Gly Val Asp Glu Ala Ala Leu Arg
305                 310                 315                 320

Gln Ala Val Arg Glu Arg Tyr Gly Val Val Phe Ser Ser Gly Arg Gly
                325                 330                 335

Glu Thr Leu Gly Lys Leu Thr Arg Ile Gly His Met Gly Pro Thr Ala
            340                 345                 350

Gln Pro Ile Tyr Ala Val Ala Ala Leu Thr Ala Leu Gly Gly Ala Leu
        355                 360                 365

Asn Ala Met Gly Gln Lys Leu Ala Val Gly Arg Gly Ile Asp Ala Ala
    370                 375                 380

Leu Ala Val Ile Asp Ala Asp Ala
385                 390

<210>  31
<211>  392
<212>  PRT
<213>  Bauldia litoralis

<400>  31

Met Arg Tyr Ala Leu Asp Ala Asp Pro Val Ile Thr Leu Thr Ser Gly
1               5                   10                  15

Pro Val Asn Ala Tyr Pro Asp Val Leu Arg Ala Leu Ala Arg Thr Val
            20                  25                  30

Leu Tyr Asp Tyr Asp Pro Ala Phe Gln Leu His Tyr Glu Gln Val Ile
        35                  40                  45

Gly Lys Met Gln Thr Ala Met Arg Thr Ser Asn Arg Pro Val Ile Leu
    50                  55                  60

His Gly Glu Pro Val Leu Gly Leu Glu Ala Ala Ala Ala Ser Leu Ile
65                  70                  75                  80

Thr Ala Asp Asp Thr Val Leu Asn Leu Ala Ser Gly Val Tyr Gly Lys
                85                  90                  95

Gly Phe Gly Tyr Trp Ala Ala Arg Tyr Ser Pro Asn Gln Leu Glu Ile
            100                 105                 110

Glu Val Pro Tyr Asn Gln Ala Ile Asp Pro Gln Gln Val Ala Asp Met
        115                 120                 125

Leu Lys Ala Asn Pro Ala Ile Ser Val Val Ala Val Cys His His Asp
    130                 135                 140

Thr Pro Ser Gly Thr Ile Asn Pro Ile Asp Glu Ile Gly Ala Ile Val
145                 150                 155                 160

Ala Asp His Gly Ala Tyr Leu Ile Val Asp Ala Val Ser Ser Phe Gly
                165                 170                 175

Gly Met Lys Thr His Pro Glu Asp Cys Lys Ala Asp Ile Tyr Val Thr
            180                 185                 190

Gly Pro Asn Lys Cys Leu Gly Ala Pro Pro Ala Leu Thr Met Leu Gly
        195                 200                 205

Val Ser Glu Arg Ala Trp Gln Lys Met Lys Asp Asn Pro Ala Ala Pro
    210                 215                 220

Arg Ala Ser Met Leu Ser Ile Leu Asp Trp Glu Asn Ala Trp Ser Arg
225                 230                 235                 240

Asp Glu Ala Phe Pro Phe Thr Pro Ser Val Ala Glu Val Asn Gly Leu
                245                 250                 255

Asp Val Ala Leu Asp Leu Tyr Leu Ala Glu Gly Pro Glu Ala Val Trp

260                 265                 270

Ala Arg His Ala Leu Thr Ala Ala Ala Thr Arg Ala Gly Ile Leu Ala
        275                 280                 285

Met Gly Leu Asp Leu Trp Ala Ala Asp Ala Ala Ile Ala Ser Pro Thr
    290                 295                 300

Thr Thr Ala Val Arg Thr Pro Glu Gly Ile Asp Glu Ala Lys Leu Arg
305                 310                 315                 320

Ala Thr Val Arg Glu Arg Tyr Gly Val Val Met Ser Ser Gly Arg Gly
                325                 330                 335

Glu Thr Leu Gly Lys Leu Thr Arg Ile Gly His Met Gly Pro Thr Ala
            340                 345                 350

Gln Pro Ile Tyr Ala Ile Ala Ala Leu Thr Ala Phe Gly Gly Ala Leu
        355                 360                 365

Gln Ala Leu Gly His Pro Ala Pro Val Gly Lys Ala Ile Asp Ala Ala
    370                 375                 380

Leu Ala Val Ile Asp Ala Ala Ala
385                 390

<210>  32
<211>  391
<212>  PRT
<213>  Skermanella stibiiresistens

<400>  32

Met Thr Val Glu Arg Ile Val Glu Pro Ala Phe Thr Leu Ser Ser Gly
1               5                   10                  15

Pro Val Asp Ala Tyr Pro Ala Val Leu Arg Ala Leu Ser Arg Thr Val
            20                  25                  30

Leu Tyr Asp Phe Asp Pro Val Phe Gln His Phe Tyr Glu Ala Val Thr
        35                  40                  45

Asp Lys Ala Lys Val Ala Leu Arg Thr Ala Ala Pro Pro Val Ile Leu
    50                  55                  60

His Gly Glu Pro Val Leu Gly Leu Glu Ala Ala Ala Ala Ser Leu Ile
65                  70                  75                  80

Ala Arg Asp Asp Val Val Leu Asn Leu Ala Ser Gly Val Tyr Gly Lys

                85                  90                  95

Gly Phe Gly Phe Trp Ala Lys Arg Tyr Cys Ala Glu Leu Val Glu Ile
            100                 105                 110

Glu Val Pro Tyr Asp Asp Ala Ile Asp Pro Ala Ala Val Glu Ala Ala
        115                 120                 125

Phe Arg Glu Arg Pro Asp Ile Arg Val Val Ser Val Cys His His Asp
    130                 135                 140

Thr Pro Ser Gly Thr Leu Asn Pro Ile Asp Glu Ile Gly Arg Ile Val
145                 150                 155                 160

Ala Ala His Gly Ala Tyr Leu Ile Val Asp Ala Val Ser Ser Phe Gly
                165                 170                 175

Gly Met Asp Val His Pro Glu Ala Cys Gln Ala Asp Ile Phe Val Thr
            180                 185                 190

Gly Pro Asn Lys Cys Leu Gly Cys Pro Pro Gly Leu Thr Leu Leu Ala
        195                 200                 205

Val Ser Gly Arg Ala Trp Asp Lys Met Lys Ala Asn Pro Asp Ala Pro
    210                 215                 220

Arg Ala Ser Ile Leu Ser Ile Leu Asp Trp Glu Asn Ala Trp Arg Arg
225                 230                 235                 240

Asp Gln Pro Phe Pro Phe Thr Pro Ser Ile Ser Glu Val Asn Ala Leu
                245                 250                 255

Asp Ala Ala Leu Asp Leu Tyr Leu Glu Glu Gly Pro Glu Ala Val Trp
            260                 265                 270

Arg Arg His Ala Leu Thr Ser Lys Ala Cys Arg Asp Gly Ile Arg Ala
        275                 280                 285

Met Gly Met Thr Ile Trp Pro Ala Arg Glu Ala Ile Ala Ser Pro Thr
    290                 295                 300

Thr Thr Ala Val Arg Val Pro Asp Gly Ile Asp Ala Asp Ala Leu Leu
305                 310                 315                 320

Ala Thr Ala Arg Asp Gly Phe Gly Val Ser Phe Ser Ser Gly Arg Gly
                325                 330                 335

Glu Thr Lys Gly Lys Leu Ile Arg Ile Gly His Met Gly Arg Thr Ala
            340                 345                 350

Gln Pro Leu Tyr Ala Val Val Gly Leu Ala Ala Leu Gly Gly Ala Leu
        355                 360                 365

Arg Arg Leu Gly Ala Asn Val Asp Ala Gly Ala Gly Met Glu Ala Ala
    370                 375                 380

Leu Ala Val Ile Ser Asn Gln
385                 390

<210>  33
<211>  394
<212>  PRT
<213>  Rhizobium sp. AC44/96

<400>  33

Met Asn Gln Thr Lys Ile Ala Asp Pro Ile Phe Thr Leu Thr Thr Gly
1               5                   10                  15

Pro Val Asp Ala Tyr Pro Ala Val Leu Arg Ala Leu Ser Arg Pro Val
            20                  25                  30

Leu Tyr Asp Tyr Asp Pro Ala Phe Leu Ala Phe Tyr Glu Ala Val Asn
        35                  40                  45

Ala Lys Val Gln Arg Ala Phe His Thr Lys Thr Pro Pro Val Ile Leu
    50                  55                  60

Gln Gly Glu Pro Val Leu Gly Leu Glu Ala Ala Ala Ala Ser Leu Ile
65                  70                  75                  80

Ala Lys Lys Asp Val Val Leu Asn Leu Val Ser Gly Val Tyr Gly Lys
                85                  90                  95

Gly Phe Gly Phe Trp Ala Ala Arg Tyr Ala Arg Glu Leu Val Glu Leu
            100                 105                 110

Glu Val Pro Tyr Asn Asp Val Leu Thr Ala Asp Ala Val Ala Asp Met
        115                 120                 125

Leu Lys Lys Arg Pro Asp Ile Ala Val Val Ala Leu Cys His His Asp
    130                 135                 140

Thr Pro Ser Gly Thr Val Asn Pro Val Ala Glu Ile Gly Ala Val Val
145                 150                 155                 160

Arg Ala Ala Gly Lys Leu Phe Ile Val Asp Ser Val Ser Ala Phe Gly
                165                 170                 175

Gly Met Asp Val Leu Pro Glu Thr Ala Cys Ala Asp Ile Phe Val Thr
            180                 185                 190

Gly Pro Asn Lys Cys Leu Gly Cys Pro Pro Gly Leu Ser Leu Leu His
        195                 200                 205

Val Ser Glu Ala Ala Trp Glu Lys Ile Ala Ala Asn Pro Asp Ala Pro
    210                 215                 220

Thr Ala Ser Met Leu Ser Ile Ser Asp Trp Lys Arg Ala His Glu Ala
225                 230                 235                 240

Gly Gln Pro Phe Pro Phe Thr Pro Ser Val Ser Glu Ile Asn Ala Leu
                245                 250                 255

Asp Ala Ala Met Asp Leu Tyr Leu Glu Glu Gly Glu Gln Ala Val Trp
            260                 265                 270

Ala Arg His Ala Leu Thr Ala Lys Ala Cys Arg Ala Gly Val Gln Ala
        275                 280                 285

Ala Gly Leu Lys Leu Trp Ala Ala Arg Glu Asp Ile Ala Ser Pro Thr
    290                 295                 300

Cys Thr Ala Val Ala Ile Pro Asp Gly Ile Asp Glu Ala Lys Leu Arg
305                 310                 315                 320

Ala Ser Ile Arg Asp Arg Tyr Gly Val Val Phe Ser Ser Gly Arg Ala
                325                 330                 335

Glu Thr Leu Gly Lys Leu Thr Arg Ile Gly His Met Gly Pro Thr Ala
            340                 345                 350

Arg Pro Thr Tyr Ser Leu Val Ser Val Ala Ala Ile Val Gly Gly Leu
        355                 360                 365

Lys Ala Ala Gly Val Lys Asp Leu Asp Val Glu Ala Gly Val Ala Ala
    370                 375                 380

Ala Met Lys Val Ile Asp Ser Ala Glu Lys
385                 390

<210>  34
<211>  390
<212>  PRT

<213>  Erwinia toletana

<400>  34

Met Met Arg Ser Ala Phe Ala Gln Pro Leu Phe Thr Leu Thr Thr Gly
1               5                   10                  15

Pro Val Asp Val Tyr Pro Ala Val Ser Arg Ala Leu Ser Met Pro Val
            20                  25                  30

Trp Tyr Asp Tyr Asp Pro Ala Phe Gln Asn Cys Phe Glu Arg Val Ser
        35                  40                  45

Leu Lys Ala Ala Gln Ala Leu Glu Ser Ala Thr Pro Pro Leu Ile Leu
    50                  55                  60

Gln Gly Glu Pro Val Leu Ala Leu Glu Ala Ala Ala Ala Ser Leu Leu
65                  70                  75                  80

Ser Ala Glu Asp Met Val Leu Asn Leu Val Ser Gly Val Tyr Ala Ala
                85                  90                  95

Gly Phe Ser Glu Trp Ala Arg Arg Tyr Ser Arg Gln Val Glu Glu Leu
            100                 105                 110

Arg Val Glu Phe Asn Gln Val Ile Asp Pro Val Ala Val Asp Ala Trp
        115                 120                 125

Leu Ser Arg His Pro Gln Ile Thr Val Val Val Val Cys His His Asp
    130                 135                 140

Thr Pro Ser Gly Thr Leu Asn Pro Leu Asp Glu Ile Gly Arg Val Val
145                 150                 155                 160

Lys Lys His Gly Lys Leu Leu Leu Val Asp Ala Val Ser Ser Phe Ala
                165                 170                 175

Gly Val Ala Val Thr Ala Ala Ser Cys Gln Ala Asp Leu Phe Ile Thr
            180                 185                 190

Gly Pro Asn Lys Cys Leu Gly Cys Pro Pro Gly Leu Ser Leu Val Ala
        195                 200                 205

Val Ser Asp Ala Ala Trp Arg Lys Ile Glu Ala Asn Asp Ala Ala Pro
    210                 215                 220

Arg Ala Ser Val Leu Ser Leu Leu Asp Trp Arg Asp Ala Trp Ser Ala
225                 230                 235                 240

Glu Lys Pro Phe Pro Tyr Thr Pro Ser Val Ala Glu Ile Asn Gly Leu
                245                 250                 255

Glu Ala Ala Leu Asp Gly Tyr Leu Gln Glu Gly Pro Ala Gln Val Trp
            260                 265                 270

Ala Arg His Arg Leu Thr Ala Gln Ala Phe Arg Ala Gly Ile Gln Ala
        275                 280                 285

Met Gly Leu Ser Leu Trp Ala Ala Asp Glu Ser Ile Ala Ser Pro Thr
    290                 295                 300

Ala Thr Ala Ile Arg Val Pro Asp Gly Val Asn Glu Ala Gln Trp Arg
305                 310                 315                 320

Leu Arg Ala Arg Glu Cys Tyr Gly Val Met Phe Ser Ser Gly Arg Gly
                325                 330                 335

Glu Thr Leu Gly Lys Val Leu Arg Val Gly His Met Gly Pro Thr Ala
            340                 345                 350

Gln Pro Met Phe Ala Ile Ile Ala Leu Thr Ala Leu Ala Gly Ala Leu
        355                 360                 365

Asn Gln Leu Arg Glu Pro Gln Leu Asp Ile Gly Ala Gly Val Ala Ala
    370                 375                 380

Ala Met Ala Val Ile Gly
385                 390

<210>  35
<211>  386
<212>  PRT
<213>  Herbiconiux ginsengi

<400>  35

Met Ala His Pro Asp Phe Thr Leu Ser Ala Gly Pro Val Thr Val Thr
1               5                   10                  15

Ala Arg Thr Leu Ala Gly Leu Gly Ser Pro Ile Leu Tyr His Tyr Asp
            20                  25                  30

Pro Glu Phe Leu Ala Thr Phe Arg Arg Thr Gln Gly Lys Val Gly Gln
        35                  40                  45

Met Phe Gln Thr Asp Asn Asp Ile Ile Leu Met Gln Gly Glu Ala Ile
    50                  55                  60

Val Gly Leu Glu Gly Ala Leu Arg Ser Leu Ile Thr Pro Gly Met His
65                  70                  75                  80

Val Leu Asn Leu Val Gln Gly Val Phe Gly Lys Gly Thr Gly Tyr Trp
                85                  90                  95

Ile Ala Asp Phe Gly Ala Val Leu His Glu Ile Glu Val Gly Tyr Asp
            100                 105                 110

Asp Ala Val Ser Pro Ala Gln Val Glu Glu Tyr Leu Asp Ala His Pro
        115                 120                 125

Glu Ile Gln Met Val Cys Leu Val Ala Ser Glu Thr Pro Ser Gly Thr
    130                 135                 140

Val Thr Asp Val Ala Ala Ile Gly Pro Met Cys Arg Asp Arg Gly Ile
145                 150                 155                 160

Leu Thr Tyr Ile Asp Thr Val Ser Gly Val Leu Gly Met Pro Trp Lys
                165                 170                 175

Thr Asp Glu Trp Gly Leu Asp Leu Cys Val Ala Gly Ala Gln Lys Cys
            180                 185                 190

Leu Gly Gly Pro Pro Gly Val Ala Leu Ile Ser Val Ser Gln Lys Ala
        195                 200                 205

Trp Asp Val Met Tyr Ala Asn Pro Ala Ala Pro Arg Asp Ser Tyr Leu
    210                 215                 220

Ser Leu Ile Asp Trp Lys Glu Lys Trp Leu Gly Glu Gly Arg Phe Pro
225                 230                 235                 240

Tyr Thr Pro Ser Val Ser Asp Met Asn Gly Leu Glu Ala Ala Leu Asp
                245                 250                 255

Gln Ala Leu Glu Glu Gly Ile Asp Ala Val Val Ala Arg His Glu Ala
            260                 265                 270

Ala Ala Ala Val Thr Arg Ala Gly Ala Arg Ala Met Gly Leu Glu Leu
        275                 280                 285

Trp Ala Lys Ser Glu Glu Ile Ala Ser Ala Cys Val Thr Ser Ile Arg
    290                 295                 300

Leu Pro Asp Asp Ile Asp Asn Ala Val Val Arg Asp His Ala Arg Glu
305                 310                 315                 320

Val Tyr Gly Val Met Leu Ser His Gly Gln Gly Ala Gly Asn Leu Val
                325                 330                 335

Arg Leu Ser His Met Gly Pro Thr Ala Gly Gly Leu His Ser Val Val
            340                 345                 350

Gly Leu Ala Ala Leu Gly Arg Thr Leu Ala Asp Leu Gly Met Ser Val
        355                 360                 365

Asp Ile Gly Ala Gly Leu Glu Ala Ala Leu Ala Leu Leu Ser Thr Gln
    370                 375                 380

Arg Arg
385

<210>  36
<211>  371
<212>  PRT
<213>  Shewanella sp. AC10

<400>  36

Met Ile Ile Gly Val Pro Thr Glu Ile Lys Asn His Glu Tyr Arg Val
1               5                   10                  15

Gly Met Val Pro Ser Ser Val Arg Glu Leu Thr Ile Lys Gly His Val
            20                  25                  30

Val Tyr Val Gln Ser Asp Ala Gly Val Gly Ile Gly Phe Thr Asp Gln
        35                  40                  45

Asp Tyr Ile Asp Ala Gly Ala Ser Ile Leu Ala Thr Ala Ala Glu Val
    50                  55                  60

Phe Ala Lys Ser Asp Met Ile Val Lys Val Lys Glu Pro Gln Ala Val
65                  70                  75                  80

Glu Arg Ala Met Leu Arg His Asp Gln Ile Leu Phe Thr Tyr Leu His
                85                  90                  95

Leu Ala Pro Asp Leu Pro Gln Thr Glu Glu Leu Ile Thr Ser Gly Ala
            100                 105                 110

Val Cys Ile Ala Tyr Glu Thr Val Thr Asp Asp Arg Gly Gly Leu Pro
        115                 120                 125

Leu Leu Ala Pro Met Ser Glu Val Ala Gly Arg Met Ser Ile Gln Ala
    130                 135                 140

Gly Ala Arg Ala Leu Glu Lys Ser Leu Gly Gly Arg Gly Met Leu Leu
145                 150                 155                 160

Gly Gly Val Pro Gly Val Glu Pro Ala Lys Val Val Ile Ile Gly Gly
                165                 170                 175

Gly Met Val Gly Thr Asn Ala Ala Gln Met Ala Val Gly Met Gly Ala
            180                 185                 190

Asp Val Val Val Leu Asp Arg Ser Ile Asp Ala Leu Arg Arg Leu Asn
        195                 200                 205

Val Gln Phe Gly Ser Ala Val Lys Ala Ile Tyr Ser Thr Ala Asp Ala
    210                 215                 220

Ile Glu Arg His Val Leu Glu Ala Asp Leu Val Ile Gly Gly Val Leu
225                 230                 235                 240

Val Pro Gly Ala Ala Ala Pro Lys Leu Ile Thr Arg Asp Met Val Lys
                245                 250                 255

Arg Met Lys Pro Gly Ser Ala Ile Val Asp Val Ala Ile Asp Gln Gly
            260                 265                 270

Gly Cys Val Glu Thr Ser His Ala Thr Thr His Gln Asp Pro Thr Tyr
        275                 280                 285

Ile Val Asp Asp Val Val His Tyr Cys Val Ala Asn Met Pro Gly Ala
    290                 295                 300

Val Ala Arg Thr Ser Thr Phe Ala Leu Asn Asn Ala Thr Leu Pro Tyr
305                 310                 315                 320

Ile Ile Lys Leu Ala Asn Gln Gly Tyr Lys Gln Ala Leu Leu Asn Asp
                325                 330                 335

Lys His Leu Leu Asn Gly Leu Asn Val Met His Gly Lys Val Val Cys
            340                 345                 350

Lys Glu Val Ala Glu Ala Leu Asn Leu Glu Phe Thr Glu Pro Lys Ser
        355                 360                 365

Leu Leu Ala
    370

<210>  37

<211>  371
<212>  PRT
<213>  Aeromonas hydrophila

<400>  37

Met Ile Ile Gly Val Pro Lys Glu Ile Lys Asn His Glu Tyr Arg Val
1               5                   10                  15

Gly Met Thr Pro Ala Gly Val Gln Glu Leu Thr Ala Arg Gly His Lys
            20                  25                  30

Val Leu Val Gln Gln Gln Gly Gly Glu Ala Ile Gly Leu Ser Asp Ala
        35                  40                  45

Glu Tyr Gln Ala Ala Gly Ala Glu Leu Val Ala Ser Ala Ala Glu Ile
    50                  55                  60

Phe Ala Arg Ala Glu Met Ile Val Lys Val Lys Glu Pro Gln Pro Glu
65                  70                  75                  80

Glu Cys Glu Leu Leu Arg Pro Gly Gln Leu Leu Phe Thr Tyr Leu His
                85                  90                  95

Leu Ala Pro Asp Pro Val Gln Thr Gln Leu Leu Val His Ser Gly Ala
            100                 105                 110

Val Ala Ile Ala Tyr Glu Thr Val Thr Asp Asp Arg Gly Gly Leu Pro
        115                 120                 125

Leu Leu Ala Pro Met Ser Glu Val Ala Gly Arg Met Ala Ile Gln Ala
    130                 135                 140

Gly Ala His Ser Leu Glu Lys Ala Gln Gly Gly Asn Gly Thr Leu Leu
145                 150                 155                 160

Gly Gly Val Pro Ala Val Ala Pro Ala Lys Val Val Val Leu Gly Gly
                165                 170                 175

Gly Val Val Gly Val Asn Ser Ala Arg Met Ala Leu Gly Leu Gly Ala
            180                 185                 190

Asp Val Thr Ile Leu Asp Arg Ser Leu Pro Arg Leu Arg Glu Leu Asp
        195                 200                 205

Ser Leu Tyr Gly Pro Ala Leu Lys Thr Leu Tyr Ser Thr Arg Thr Asn
    210                 215                 220

Ile Glu Ala Cys Ile Ala Gln Ala Asp Leu Val Ile Gly Ala Val Leu

225                 230                 235                 240

Ile Pro Gly Ala Ala Ala Pro Lys Leu Leu Thr Arg Asp Met Leu Lys
                245                 250                 255

Leu Met Arg Lys Gly Ser Val Leu Val Asp Val Ala Ile Asp Gln Gly
            260                 265                 270

Gly Cys Phe Glu Thr Ser His Pro Thr Thr His Glu Glu Pro Thr Tyr
        275                 280                 285

Val Val Asp Gly Ile Ile His Tyr Cys Val Ala Asn Met Pro Gly Gly
    290                 295                 300

Val Ala Arg Thr Ser Thr Phe Ala Leu Thr Asn Ala Thr Leu Pro Phe
305                 310                 315                 320

Val Leu Ala Leu Ala Asp Lys Gly Tyr Val Arg Ala Leu Gln Glu Asp
                325                 330                 335

Arg His Leu Arg Ala Gly Leu Asn Val Phe Lys Gly Lys Ile Thr Gln
            340                 345                 350

Ala Ala Val Ala Gln Ala Leu Gly Tyr Glu Tyr Val Ser Ala Glu Val
        355                 360                 365

Val Leu Asp
    370

<210>  38
<211>  372
<212>  PRT
<213>  Rhizobium sp. LPU83

<400>  38

Met Arg Val Gly Cys Pro Lys Glu Ile Lys Asn His Glu Tyr Arg Val
1               5                   10                  15

Gly Leu Thr Pro Ala Ser Val Arg Glu Tyr Val Ala His Gly His Glu
            20                  25                  30

Val Arg Val Glu Thr Lys Ala Gly Ala Gly Ile Gly Ala Asp Asp Ala
        35                  40                  45

Ala Tyr Val Ala Ala Gly Ala Lys Ile Ala Ala Ser Ala Lys Glu Ile
    50                  55                  60

Phe Glu Lys Cys Asp Met Ile Val Lys Val Lys Glu Pro Gln Pro Ser

65                  70                  75                  80

Glu Trp Ala Gln Leu Arg Asp Gly Gln Ile Leu Tyr Thr Tyr Leu His
                85                  90                  95

Leu Ala Pro Asp Pro Glu Gln Thr Lys Gly Leu Leu Ala Ser Gly Val
            100                 105                 110

Thr Ala Val Ala Tyr Glu Thr Val Thr Asp Asp Arg Gly Gly Leu Pro
        115                 120                 125

Leu Leu Ala Pro Met Ser Glu Val Ala Gly Arg Leu Ser Ile Gln Ala
    130                 135                 140

Gly Ala Thr Ala Leu Gln Lys Ala Asn Gly Gly Leu Gly Ile Leu Leu
145                 150                 155                 160

Gly Gly Val Pro Gly Val Leu Pro Ala Lys Val Thr Val Ile Gly Gly
                165                 170                 175

Gly Val Val Gly Leu His Ala Ala Arg Met Ala Ala Gly Leu Gly Ala
            180                 185                 190

Asp Val Ser Ile Leu Asp Lys Ser Leu Pro Arg Leu Arg Gln Leu Asp
        195                 200                 205

Asp Ile Phe Gly Gly Arg Val His Thr Arg Tyr Ser Ser Ile Gln Ala
    210                 215                 220

Leu Glu Glu Glu Val Phe Ser Ala Asp Leu Val Ile Gly Ala Val Leu
225                 230                 235                 240

Ile Pro Gly Ala Ala Ala Pro Lys Leu Val Thr Arg Glu Met Leu Ser
                245                 250                 255

Gly Met Lys Arg Gly Ser Val Ile Val Asp Val Ala Ile Asp Gln Gly
            260                 265                 270

Gly Cys Phe Glu Thr Ser His Ala Thr Thr His Ser Glu Pro Thr Tyr
        275                 280                 285

Val Val Asp Asp Val Val His Tyr Cys Val Ala Asn Met Pro Gly Ala
    290                 295                 300

Val Pro Val Thr Ser Ala His Ala Leu Asn Asn Ala Thr Leu Val Tyr
305                 310                 315                 320

Gly Leu Ala Leu Ala Asp Arg Gly Leu Arg Ala Ile Ala Glu Asp Arg
                325                 330                 335

His Leu Arg Asn Gly Leu Asn Val His Lys Gly Arg Val Thr Asn Lys
            340                 345                 350

Pro Val Ala Glu Ala Leu Gly Tyr Glu Ser Tyr Thr Pro Glu Ser Val
        355                 360                 365

Leu Asn Val Ala
    370

<210>  39
<211>  373
<212>  PRT
<213>  Pseudomonas mendocina

<400>  39

Met Arg Ile Gly Val Pro Lys Glu Ile Lys Asn His Glu Tyr Arg Val
1               5                   10                  15

Gly Leu Thr Pro Gln Ser Val Ala Glu Leu Thr Thr Leu Gly His Glu
            20                  25                  30

Val Trp Ile Glu Thr Leu Ala Gly Ala Ala Ile Gly Phe Ala Asp Glu
        35                  40                  45

Asp Tyr Arg Lys Ala Gly Ala Gln Ile Ala Pro Cys Ala Gly Glu Val
    50                  55                  60

Phe Gln Gln Ala Gln Leu Ile Val Lys Val Lys Glu Pro Leu Ala Val
65                  70                  75                  80

Glu Arg Ala Lys Leu Arg Glu Gln His Thr Leu Phe Thr Tyr Leu His
                85                  90                  95

Leu Ala Pro Asp Arg Pro Gln Thr Asp Glu Leu Met Ala Ser Gly Ala
            100                 105                 110

Thr Cys Ile Ala Tyr Glu Thr Val Thr Asp Val Gln Gly Arg Leu Pro
        115                 120                 125

Leu Leu Ala Pro Met Ser Glu Val Ala Gly Arg Met Ser Ile Gln Ala
    130                 135                 140

Gly Ala Gly Cys Leu Glu Lys Ala Arg Gly Gly Arg Gly Val Leu Leu
145                 150                 155                 160

Gly Gly Val Pro Gly Val Ala Pro Gly Lys Val Val Ile Leu Gly Gly
                165                 170                 175

Gly Val Val Gly Ser His Ala Leu Ala Met Ala Val Gly Leu Gly Ala
            180                 185                 190

Asp Val Thr Val Leu Asp Lys Ser Val Asp Ala Leu Arg Arg Leu Asp
        195                 200                 205

Ala Gln Tyr Gly Asn Arg Ile Thr Thr Leu Tyr Ser Thr Arg Ala Ala
    210                 215                 220

Val Gln Glu Gln Val Leu Ala Ala Asp Leu Val Ile Gly Gly Val Leu
225                 230                 235                 240

Ile Pro Gly Ala Ala Ala Pro Lys Leu Ile Ser Ala Asp Met Val Arg
                245                 250                 255

Gln Met Lys Ala Gly Ala Val Leu Val Asp Val Ala Ile Asp Gln Gly
            260                 265                 270

Gly Cys Ala Glu Thr Ser Arg Ala Thr Thr His Ala Glu Pro Thr Tyr
        275                 280                 285

Val Val Asp Asp Val Val His Tyr Cys Val Ala Asn Met Pro Gly Ala
    290                 295                 300

Val Ala Arg Thr Ser Thr Leu Ala Leu Asn Asn Ala Thr Leu Pro Phe
305                 310                 315                 320

Val Val Ala Leu Ala Gln Lys Gly Thr Arg Arg Ala Leu Glu Asp Asp
                325                 330                 335

Pro His Leu Leu Ala Gly Leu Asn Val Ala Arg Gly Ala Ile Thr Cys
            340                 345                 350

Ala Ser Val Ala Glu Ala His Gly Leu Pro Phe Gln Pro Pro Ala Ser
        355                 360                 365

Val Leu Glu Arg Leu
    370

<210>  40
<211>  371
<212>  PRT
<213>  Bradyrhizobium japonicum

<400>  40

Met Arg Val Gly Val Pro Lys Glu Ile Lys Met Gln Glu Tyr Arg Val
1               5                   10                  15

Gly Leu Thr Pro Gly Ala Val Arg Glu Tyr Val Ala Ala Gly His Gln
            20                  25                  30

Val Thr Val Glu Thr Gly Ala Gly Ser Gly Ile Gly Ala Ser Asp Glu
        35                  40                  45

Val Tyr Gln Arg Ala Gly Ala Ala Ile Ala Glu Asn Ala Arg Asp Ile
    50                  55                  60

Phe Asp Ser Ser Asp Met Ile Val Lys Val Lys Glu Pro Gln Lys Ser
65                  70                  75                  80

Glu Trp Ala Gln Leu Arg Glu Ser Gln Ile Leu Phe Thr Tyr Leu His
                85                  90                  95

Leu Ala Pro Asp Pro Glu Gln Ala Lys Gly Leu Leu Thr Ser Gly Cys
            100                 105                 110

Thr Ala Val Ala Tyr Glu Thr Val Thr Asp Ala Ala Gly His Leu Pro
        115                 120                 125

Leu Leu Ala Pro Met Ser Glu Val Ala Gly Arg Leu Ala Ile Glu Ala
    130                 135                 140

Ala Gly Ala Ala Leu Lys Arg Ser Ala Gly Gly Arg Gly Val Leu Leu
145                 150                 155                 160

Gly Gly Val Pro Gly Val Gln Pro Ala Arg Val Val Val Leu Gly Gly
                165                 170                 175

Gly Val Val Gly Thr Gln Ala Ala Arg Met Ala Ala Gly Leu Gly Ala
            180                 185                 190

Glu Val Thr Val Ile Asp Arg Ser Val Pro Arg Leu Arg Glu Leu Asp
        195                 200                 205

Asp Val Phe Leu Gly Arg Val Arg Thr Arg Phe Ser Thr Ile Glu Ala
    210                 215                 220

Val Glu Asp Glu Val Phe Ala Ala Asp Val Val Ile Gly Ala Val Leu
225                 230                 235                 240

Val Pro Gly Ala Ser Ala Pro Lys Leu Val Thr Arg Gly Met Leu Lys
                245                 250                 255

Ser Met Arg Pro Gly Ala Val Leu Val Asp Val Ala Ile Asp Gln Gly
        260                 265                 270

Gly Cys Phe Glu Thr Ser His Pro Thr Thr His Ala Asp Pro Thr Tyr
        275                 280                 285

Gln Val Asp Gly Val Val His Tyr Cys Val Ala Asn Met Pro Gly Ala
    290                 295                 300

Val Pro Val Thr Ser Ser Gln Gly Leu Asn Asn Ala Thr Leu Pro Phe
305                 310                 315                 320

Gly Leu Met Leu Ala Asn Lys Gly Phe Ala Ala Val Leu Glu Asn Pro
                325                 330                 335

His Leu Arg Asn Gly Leu Asn Val His Arg Gly Arg Ile Thr Asn Lys
            340                 345                 350

Ala Val Ala Glu Ser Leu Gly Leu Glu Phe Thr Pro Val Glu Ser Gly
        355                 360                 365

Leu Ala Ala
    370

<210>  41
<211>  370
<212>  PRT
<213>  Bradyrhizobium japonicum

<400>  41

Met Lys Val Gly Val Pro Lys Glu Ile Lys Ala His Glu Tyr Arg Val
1               5                   10                  15

Gly Leu Thr Pro Gly Ala Ala Arg Glu Tyr Val Ala Ala Gly His Arg
            20                  25                  30

Val Met Ile Glu Thr Asn Ala Gly Ala Gly Ile Gly Ala Thr Asp Gly
        35                  40                  45

Asp Tyr Arg Asn Ala Gly Ala Thr Ile Leu Thr Ser Ala Ala Glu Val
    50                  55                  60

Phe Ala Ser Ser Glu Met Ile Val Lys Val Lys Glu Pro Gln Pro Ala
65                  70                  75                  80

Glu Trp Ser Gln Leu Arg Glu Asp Gln Ile Leu Phe Thr Tyr Leu His
                85                  90                  95

Leu Ala Pro Asp Pro Glu Gln Ala Ala Gly Leu Leu Lys Ser Gly Cys
            100                 105                 110

Ile Ala Ile Ala Tyr Glu Thr Val Thr Asp Ala His Gly Gly Leu Pro
        115                 120                 125

Leu Leu Ala Pro Met Ser Glu Val Ala Gly Arg Leu Ser Ile Glu Ala
    130                 135                 140

Ala Gly Ser Ala Leu Lys Arg Ser Thr Gly Gly Arg Gly Leu Leu Ile
145                 150                 155                 160

Gly Gly Val Pro Gly Val Gln Pro Ala Arg Ile Val Val Ile Gly Gly
                165                 170                 175

Gly Val Val Gly Thr His Ala Ala Arg Met Ala Ala Gly Leu Gly Ala
            180                 185                 190

Glu Val Thr Ile Ile Asp Arg Ser Ile Ile Arg Leu Arg Glu Leu Asp
        195                 200                 205

Glu Leu Phe Glu Gly Arg Val Arg Thr Arg Phe Ser Thr Ile Glu Ser
    210                 215                 220

Val Glu Glu Glu Val Phe Ala Ala Asp Val Val Ile Gly Ala Val Leu
225                 230                 235                 240

Val Pro Gly Ala Ser Ala Pro Lys Leu Val Arg Arg Ser Met Leu Ser
                245                 250                 255

Ser Met Arg Lys Arg Ala Val Leu Val Asp Val Ala Ile Asp Gln Gly
            260                 265                 270

Gly Cys Phe Glu Thr Ser Arg Pro Thr Thr His Ala Asp Pro Thr Tyr
        275                 280                 285

Glu Val Asp Gly Ile Ile His Tyr Cys Val Ala Asn Met Pro Gly Ala
    290                 295                 300

Val Pro Leu Thr Ser Ser Gln Ala Leu Asn Asn Ala Thr Leu Pro Phe
305                 310                 315                 320

Gly Leu Ala Leu Ala Asn Lys Gly Phe Ser Ala Val Leu Glu Asn Pro
                325                 330                 335

His Leu Arg Ala Gly Leu Asn Val His Arg Gly Arg Leu Thr Tyr Lys
            340                 345                 350

Ala Val Ala Glu Ser Leu Gly Leu Pro Phe Ser Pro Ile Glu Gln Ala
        355                 360                 365

Ala Ala
    370

<210>  42
<211>  371
<212>  PRT
<213>  Streptomyces aureofaciens

<400>  42

Met Lys Val Gly Ile Pro Arg Glu Val Lys Asn Asn Glu Phe Arg Val
1               5                   10                  15

Ala Ile Thr Pro Ala Gly Val His Glu Leu Val Arg His Gly His Gln
            20                  25                  30

Val Val Ile Glu His Gly Ala Gly Val Gly Ser Ser Ile Pro Asp Glu
        35                  40                  45

Glu Tyr Val Ala Ala Gly Ala Arg Ile Leu Asp Thr Ala Asp Glu Val
    50                  55                  60

Trp Ala Thr Ala Asp Leu Leu Leu Lys Val Lys Glu Pro Ile Ala Gln
65                  70                  75                  80

Glu Tyr His Arg Leu Arg Lys Asp Gln Thr Leu Phe Thr Tyr Leu His
                85                  90                  95

Leu Ala Ala Ser Lys Glu Cys Thr Asp Ala Leu Val Glu Ser Gly Thr
            100                 105                 110

Thr Ala Ile Ala Tyr Glu Thr Val Glu Leu Pro Gly Arg Ala Leu Pro
        115                 120                 125

Leu Leu Ala Pro Met Ser Glu Val Ala Gly Arg Ile Ala Pro Gln Val
    130                 135                 140

Gly Ala Tyr His Leu Met Ala Pro Asn Gly Gly Arg Gly Val Leu Pro
145                 150                 155                 160

Gly Gly Val Pro Gly Val Thr Pro Ala Lys Ala Val Val Ile Gly Gly
                165                 170                 175

Gly Val Ser Gly Trp Asn Ala Thr Gln Ile Ala Val Gly Met Gly Phe
            180                 185                 190

Asp Val Thr Leu Leu Asp Arg Asp Ile Asn Lys Leu Arg Glu Ala Asp
        195                 200                 205

Arg Ile Phe Gly Thr Lys Val Lys Ala Val Met Ser Asn Ser Phe Glu
    210                 215                 220

Leu Glu Lys Ala Val Val Asp Ala Asp Leu Val Ile Gly Ala Val Leu
225                 230                 235                 240

Ile Pro Gly Ala Lys Ala Pro Lys Leu Val Thr Asn Glu Leu Val Ser
                245                 250                 255

Arg Met Lys Pro Gly Ser Val Leu Val Asp Ile Ala Ile Asp Gln Gly
            260                 265                 270

Gly Cys Phe Glu Asp Ser His Pro Thr Thr His Ala Glu Pro Thr Phe
        275                 280                 285

Arg Val His Asn Ser Val Phe Tyr Cys Val Ala Asn Met Pro Gly Ala
    290                 295                 300

Val Pro Asn Thr Ser Thr Asn Ala Leu Thr Asn Ala Thr Leu Pro Tyr
305                 310                 315                 320

Ile Val Glu Leu Ala Asp Arg Gly Trp Ala Glu Ala Leu Arg Arg Asp
                325                 330                 335

Pro Ala Leu Ala Arg Gly Leu Asn Thr His Asp Gly Lys Val Val Tyr
            340                 345                 350

Arg Glu Val Ala Glu Ala His Gly Leu Glu Tyr Val Asp Pro Ala Thr
        355                 360                 365

Leu Leu Ala
    370

<210>  43
<211>  363
<212>  PRT
<213>  Anabaena cylindrica

<400>  43

Met Glu Ile Gly Val Pro Lys Glu Thr Lys Asp Gln Glu Phe Arg Val
1               5                   10                  15

Gly Leu Ser Pro Ser Ser Val Arg Val Leu Arg Glu Asn Gly His Ser
            20                  25                  30

Ile Phe Val Gln Thr Gln Ala Gly Thr Gly Ala Gly Phe Thr Asp Glu
        35                  40                  45

Asp Tyr Ile Ser Ala Gly Ala Glu Ile Val Ser Thr Leu Glu Ala Ala
    50                  55                  60

Trp Asn Arg Glu Leu Val Ile Lys Val Lys Glu Pro Leu Val Ser Glu
65                  70                  75                  80

Tyr Asn Leu Leu Gln Lys Gly Gln Leu Leu Phe Thr Tyr Leu His Leu
                85                  90                  95

Ala Ala Asp Arg Lys Leu Thr Glu His Leu Leu Asp Cys Gly Ile Ser
            100                 105                 110

Ala Ile Ala Tyr Glu Thr Val Glu Gln Ala Gly Val Asn Arg Leu Pro
        115                 120                 125

Leu Leu Thr Pro Met Ser Ile Ile Ala Gly Arg Leu Ala Val Gln Phe
    130                 135                 140

Gly Ala Arg Phe Leu Glu Arg Gln Gln Gly Gly Lys Gly Val Leu Leu
145                 150                 155                 160

Gly Gly Val Pro Gly Val Lys Ala Gly Lys Val Val Ile Leu Gly Gly
                165                 170                 175

Gly Val Val Gly Thr Glu Ala Ala Lys Ile Ala Val Gly Met Gly Ala
            180                 185                 190

Ile Val Gln Ile Leu Asp Val Asn Val Glu Arg Leu Ser Tyr Leu Glu
        195                 200                 205

Thr Leu Phe Gly Ser Arg Val Glu Leu Leu Tyr Ser Asn Ser Ala His
    210                 215                 220

Ile Glu Thr Ala Val Lys Glu Ala Asn Leu Leu Ile Gly Ala Val Leu
225                 230                 235                 240

Ile Pro Gly Arg Arg Ala Pro Ile Leu Val Ser Arg Asp Leu Val Lys
                245                 250                 255

Gln Met His Pro Gly Ser Val Ile Val Asp Val Ala Val Asp Gln Gly
            260                 265                 270

Gly Cys Val Glu Thr Leu His Pro Thr Ser His Thr Ser Pro Val Tyr

275                 280                 285

Ile Asp Glu Gly Val Val His Tyr Gly Val Pro Asn Met Pro Gly Ala
    290                 295                 300

Val Pro Trp Thr Ser Thr Gln Ala Leu Asn Asn Ser Thr Leu Pro Tyr
305                 310                 315                 320

Ala Val Gln Leu Ala Asn Leu Gly Ile Lys Ala Leu Asp Val Asn Pro
                325                 330                 335

Ala Leu Ala Lys Gly Leu Asn Val Gln Asn His Arg Leu Ile His Pro
            340                 345                 350

Ala Val Gln Glu Val Phe Pro Asp Leu Val Ser
        355                 360

<210>  44
<211>  378
<212>  PRT
<213>  Bacillus subtilis

<400>  44

Met Ile Ile Gly Val Pro Lys Glu Ile Lys Asn Asn Glu Asn Arg Val
1               5                   10                  15

Ala Leu Thr Pro Gly Gly Val Ser Gln Leu Ile Ser Asn Gly His Arg
            20                  25                  30

Val Leu Val Glu Thr Gly Ala Gly Leu Gly Ser Gly Phe Glu Asn Glu
        35                  40                  45

Ala Tyr Glu Ser Ala Gly Ala Glu Ile Ile Ala Asp Pro Lys Gln Val
    50                  55                  60

Trp Asp Ala Glu Met Val Met Lys Val Lys Glu Pro Leu Pro Glu Glu
65                  70                  75                  80

Tyr Val Tyr Phe Arg Lys Gly Leu Val Leu Phe Thr Tyr Leu His Leu
                85                  90                  95

Ala Ala Glu Pro Glu Leu Ala Gln Ala Leu Lys Asp Lys Gly Val Thr
            100                 105                 110

Ala Ile Ala Tyr Glu Thr Val Ser Glu Gly Arg Thr Leu Pro Leu Leu
        115                 120                 125

Thr Pro Met Ser Glu Val Ala Gly Arg Met Ala Ala Gln Ile Gly Ala

130                 135                 140

Gln Phe Leu Glu Lys Pro Lys Gly Gly Lys Gly Ile Leu Leu Ala Gly
145                 150                 155                 160

Val Pro Gly Val Ser Arg Gly Lys Val Thr Ile Ile Gly Gly Gly Val
                165                 170                 175

Val Gly Thr Asn Ala Ala Lys Met Ala Val Gly Leu Gly Ala Asp Val
            180                 185                 190

Thr Ile Ile Asp Leu Asn Ala Asp Arg Leu Arg Gln Leu Asp Asp Ile
        195                 200                 205

Phe Gly His Gln Ile Lys Thr Leu Ile Ser Asn Pro Val Asn Ile Ala
    210                 215                 220

Asp Ala Val Ala Glu Ala Asp Leu Leu Ile Cys Ala Val Leu Ile Pro
225                 230                 235                 240

Gly Ala Lys Ala Pro Thr Leu Val Thr Glu Glu Met Val Lys Gln Met
                245                 250                 255

Lys Pro Gly Ser Val Ile Val Asp Val Ala Ile Asp Gln Gly Gly Ile
            260                 265                 270

Val Glu Thr Val Asp His Ile Thr Thr His Asp Gln Pro Thr Tyr Glu
        275                 280                 285

Lys His Gly Val Val His Tyr Ala Val Ala Asn Met Pro Gly Ala Val
    290                 295                 300

Pro Arg Thr Ser Thr Ile Ala Leu Thr Asn Val Thr Val Pro Tyr Ala
305                 310                 315                 320

Leu Gln Ile Ala Asn Lys Gly Ala Val Lys Ala Leu Ala Asp Asn Thr
                325                 330                 335

Ala Leu Arg Ala Gly Leu Asn Thr Ala Asn Gly His Val Thr Tyr Glu
            340                 345                 350

Ala Val Ala Arg Asp Leu Gly Tyr Glu Tyr Val Pro Ala Glu Lys Ala
        355                 360                 365

Leu Gln Asp Glu Ser Ser Val Ala Gly Ala
    370                 375

<210>  45
<211>  371
<212>  PRT
<213>  Artificial Sequence

<220>
<223>  Modified Alanine Dehydrogenase

<400>  45

Met Ile Ile Gly Val Pro Lys Glu Ile Lys Asn His Glu Tyr Arg Val
1               5                   10                  15

Gly Met Thr Pro Ala Gly Val Gln Glu Leu Thr Ala Arg Gly His Lys
            20                  25                  30

Val Leu Val Gln Gln Gln Gly Gly Glu Ala Ile Gly Leu Ser Asp Ala
        35                  40                  45

Glu Tyr Gln Ala Ala Gly Ala Glu Leu Val Ala Ser Ala Ala Glu Ile
    50                  55                  60

Phe Ala Arg Ala Glu Met Ile Val Lys Val Lys Glu Pro Gln Pro Glu
65                  70                  75                  80

Glu Cys Glu Leu Leu Arg Pro Gly Gln Leu Leu Phe Thr Tyr Leu His
                85                  90                  95

Leu Ala Pro Asp Pro Val Gln Thr Gln Leu Leu Val His Ser Gly Ala
            100                 105                 110

Val Ala Ile Ala Tyr Glu Thr Val Thr Asp Asp Arg Gly Gly Leu Pro
        115                 120                 125

Leu Leu Ala Pro Met Ser Glu Val Ala Gly Arg Met Ala Ile Gln Ala
    130                 135                 140

Gly Ala His Ser Leu Glu Lys Ala Gln Gly Gly Asn Gly Thr Leu Leu
145                 150                 155                 160

Gly Gly Val Pro Ala Val Ala Pro Ala Lys Val Val Val Leu Gly Gly
                165                 170                 175

Gly Val Val Gly Val Asn Ser Ala Arg Met Ala Leu Gly Leu Gly Ala
            180                 185                 190

Asp Val Thr Ile Leu Ala Arg Ser Leu Pro Arg Leu Arg Glu Leu Asp
        195                 200                 205

Ser Leu Tyr Gly Pro Ala Leu Lys Thr Leu Tyr Ser Thr Arg Thr Asn

210                 215                 220

Ile Glu Ala Cys Ile Ala Gln Ala Asp Leu Val Ile Gly Ala Val Leu
225                 230                 235                 240

Ile Pro Gly Ala Ala Ala Pro Lys Leu Leu Thr Arg Asp Met Leu Lys
                245                 250                 255

Leu Met Arg Lys Gly Ser Val Leu Val Asp Val Ala Ile Asp Gln Gly
            260                 265                 270

Gly Cys Phe Glu Thr Ser His Pro Thr Thr His Glu Glu Pro Thr Tyr
        275                 280                 285

Val Val Asp Gly Ile Ile His Tyr Cys Val Ala Asn Met Pro Gly Gly
    290                 295                 300

Val Ala Arg Thr Ser Thr Phe Ala Leu Thr Asn Ala Thr Leu Pro Phe
305                 310                 315                 320

Val Leu Ala Leu Ala Asp Lys Gly Tyr Val Arg Ala Leu Gln Glu Asp
                325                 330                 335

Arg His Leu Arg Ala Gly Leu Asn Val Phe Lys Gly Lys Ile Thr Gln
            340                 345                 350

Ala Ala Val Ala Gln Ala Leu Gly Tyr Glu Tyr Val Ser Ala Glu Val
        355                 360                 365

Val Leu Asp
    370

<210>  46
<211>  372
<212>  PRT
<213>  Artificial  Sequence

<220>
<223>  Modified Alanine Dehydrogenase

<400>  46

Met Arg Val Gly Cys Pro Lys Glu Ile Lys Asn His Glu Tyr Arg Val
1               5                   10                  15

Gly Leu Thr Pro Ala Ser Val Arg Glu Tyr Val Ala His Gly His Glu
            20                  25                  30

Val Arg Val Glu Thr Lys Ala Gly Ala Gly Ile Gly Ala Asp Asp Ala
        35                  40                  45

Ala Tyr Val Ala Ala Gly Ala Lys Ile Ala Ala Ser Ala Lys Glu Ile
    50                  55                  60

Phe Glu Lys Cys Asp Met Ile Val Lys Val Lys Glu Pro Gln Pro Ser
65                  70                  75                  80

Glu Trp Ala Gln Leu Arg Asp Gly Gln Ile Leu Tyr Thr Tyr Leu His
                85                  90                  95

Leu Ala Pro Asp Pro Glu Gln Thr Lys Gly Leu Leu Ala Ser Gly Val
            100                 105                 110

Thr Ala Val Ala Tyr Glu Thr Val Thr Asp Asp Arg Gly Gly Leu Pro
        115                 120                 125

Leu Leu Ala Pro Met Ser Glu Val Ala Gly Arg Leu Ser Ile Gln Ala
    130                 135                 140

Gly Ala Thr Ala Leu Gln Lys Ala Asn Gly Gly Leu Gly Ile Leu Leu
145                 150                 155                 160

Gly Gly Val Pro Gly Val Leu Pro Ala Lys Val Thr Val Ile Gly Gly
                165                 170                 175

Gly Val Val Gly Leu His Ala Ala Arg Met Ala Ala Gly Leu Gly Ala
            180                 185                 190

Asp Val Ser Ile Leu Ala Lys Ser Leu Pro Arg Leu Arg Gln Leu Asp
        195                 200                 205

Asp Ile Phe Gly Gly Arg Val His Thr Arg Tyr Ser Ser Ile Gln Ala
    210                 215                 220

Leu Glu Glu Glu Val Phe Ser Ala Asp Leu Val Ile Gly Ala Val Leu
225                 230                 235                 240

Ile Pro Gly Ala Ala Ala Pro Lys Leu Val Thr Arg Glu Met Leu Ser
                245                 250                 255

Gly Met Lys Arg Gly Ser Val Ile Val Asp Val Ala Ile Asp Gln Gly
            260                 265                 270

Gly Cys Phe Glu Thr Ser His Ala Thr Thr His Ser Glu Pro Thr Tyr
        275                 280                 285

Val Val Asp Asp Val Val His Tyr Cys Val Ala Asn Met Pro Gly Ala

290                 295                 300

Val Pro Val Thr Ser Ala His Ala Leu Asn Asn Ala Thr Leu Val Tyr
305                 310                 315                 320

Gly Leu Ala Leu Ala Asp Arg Gly Leu Arg Ala Ile Ala Glu Asp Arg
                325                 330                 335

His Leu Arg Asn Gly Leu Asn Val His Lys Gly Arg Val Thr Asn Lys
            340                 345                 350

Pro Val Ala Glu Ala Leu Gly Tyr Glu Ser Tyr Thr Pro Glu Ser Val
        355                 360                 365

Leu Asn Val Ala
    370

<210>  47
<211>  373
<212>  PRT
<213>  Artificial Sequence

<220>
<223>  Modified Alanine Dehydrogenase

<400>  47

Met Arg Ile Gly Val Pro Lys Glu Ile Lys Asn His Glu Tyr Arg Val
1               5                   10                  15

Gly Leu Thr Pro Gln Ser Val Ala Glu Leu Thr Thr Leu Gly His Glu
            20                  25                  30

Val Trp Ile Glu Thr Leu Ala Gly Ala Ala Ile Gly Phe Ala Asp Glu
        35                  40                  45

Asp Tyr Arg Lys Ala Gly Ala Gln Ile Ala Pro Cys Ala Gly Glu Val
    50                  55                  60

Phe Gln Gln Ala Gln Leu Ile Val Lys Val Lys Glu Pro Leu Ala Val
65                  70                  75                  80

Glu Arg Ala Lys Leu Arg Glu Gln His Thr Leu Phe Thr Tyr Leu His
                85                  90                  95

Leu Ala Pro Asp Arg Pro Gln Thr Asp Glu Leu Met Ala Ser Gly Ala
            100                 105                 110

Thr Cys Ile Ala Tyr Glu Thr Val Thr Asp Val Gln Gly Arg Leu Pro
        115                 120                 125

Leu Leu Ala Pro Met Ser Glu Val Ala Gly Arg Met Ser Ile Gln Ala
    130                 135                 140

Gly Ala Gly Cys Leu Glu Lys Ala Arg Gly Gly Arg Gly Val Leu Leu
145                 150                 155                 160

Gly Gly Val Pro Gly Val Ala Pro Gly Lys Val Val Ile Leu Gly Gly
                165                 170                 175

Gly Val Val Gly Ser His Ala Leu Ala Met Ala Val Gly Leu Gly Ala
            180                 185                 190

Asp Val Thr Val Leu Ala Lys Ser Val Asp Ala Leu Arg Arg Leu Asp
        195                 200                 205

Ala Gln Tyr Gly Asn Arg Ile Thr Thr Leu Tyr Ser Thr Arg Ala Ala
    210                 215                 220

Val Gln Glu Gln Val Leu Ala Ala Asp Leu Val Ile Gly Gly Val Leu
225                 230                 235                 240

Ile Pro Gly Ala Ala Ala Pro Lys Leu Ile Ser Ala Asp Met Val Arg
                245                 250                 255

Gln Met Lys Ala Gly Ala Val Leu Val Asp Val Ala Ile Asp Gln Gly
            260                 265                 270

Gly Cys Ala Glu Thr Ser Arg Ala Thr Thr His Ala Glu Pro Thr Tyr
        275                 280                 285

Val Val Asp Asp Val Val His Tyr Cys Val Ala Asn Met Pro Gly Ala
    290                 295                 300

Val Ala Arg Thr Ser Thr Leu Ala Leu Asn Asn Ala Thr Leu Pro Phe
305                 310                 315                 320

Val Val Ala Leu Ala Gln Lys Gly Thr Arg Arg Ala Leu Glu Asp Asp
                325                 330                 335

Pro His Leu Leu Ala Gly Leu Asn Val Ala Arg Gly Ala Ile Thr Cys
            340                 345                 350

Ala Ser Val Ala Glu Ala His Gly Leu Pro Phe Gln Pro Pro Ala Ser
        355                 360                 365

Val Leu Glu Arg Leu

    370

<210>  48
<211>  371
<212>  PRT
<213>  Artificial Sequence

<220>
<223>  Modified Alanine Dehydrogenase

<400>  48

Met Arg Val Gly Val Pro Lys Glu Ile Lys Met Gln Glu Tyr Arg Val
1               5                   10                  15

Gly Leu Thr Pro Gly Ala Val Arg Glu Tyr Val Ala Ala Gly His Gln
            20                  25                  30

Val Thr Val Glu Thr Gly Ala Gly Ser Gly Ile Gly Ala Ser Asp Glu
        35                  40                  45

Val Tyr Gln Arg Ala Gly Ala Ala Ile Ala Glu Asn Ala Arg Asp Ile
    50                  55                  60

Phe Asp Ser Ser Asp Met Ile Val Lys Val Lys Glu Pro Gln Lys Ser
65                  70                  75                  80

Glu Trp Ala Gln Leu Arg Glu Ser Gln Ile Leu Phe Thr Tyr Leu His
                85                  90                  95

Leu Ala Pro Asp Pro Glu Gln Ala Lys Gly Leu Leu Thr Ser Gly Cys
            100                 105                 110

Thr Ala Val Ala Tyr Glu Thr Val Thr Asp Ala Ala Gly His Leu Pro
        115                 120                 125

Leu Leu Ala Pro Met Ser Glu Val Ala Gly Arg Leu Ala Ile Glu Ala
    130                 135                 140

Ala Gly Ala Ala Leu Lys Arg Ser Ala Gly Gly Arg Gly Val Leu Leu
145                 150                 155                 160

Gly Gly Val Pro Gly Val Gln Pro Ala Arg Val Val Val Leu Gly Gly
                165                 170                 175

Gly Val Val Gly Thr Gln Ala Ala Arg Met Ala Ala Gly Leu Gly Ala
            180                 185                 190

Glu Val Thr Val Ile Ala Arg Ser Val Pro Arg Leu Arg Glu Leu Asp
        195                 200                 205

Asp Val Phe Leu Gly Arg Val Arg Thr Arg Phe Ser Thr Ile Glu Ala
    210                 215                 220

Val Glu Asp Glu Val Phe Ala Ala Asp Val Val Ile Gly Ala Val Leu
225                 230                 235                 240

Val Pro Gly Ala Ser Ala Pro Lys Leu Val Thr Arg Gly Met Leu Lys
                245                 250                 255

Ser Met Arg Pro Gly Ala Val Leu Val Asp Val Ala Ile Asp Gln Gly
            260                 265                 270

Gly Cys Phe Glu Thr Ser His Pro Thr Thr His Ala Asp Pro Thr Tyr
        275                 280                 285

Gln Val Asp Gly Val Val His Tyr Cys Val Ala Asn Met Pro Gly Ala
    290                 295                 300

Val Pro Val Thr Ser Ser Gln Gly Leu Asn Asn Ala Thr Leu Pro Phe
305                 310                 315                 320

Gly Leu Met Leu Ala Asn Lys Gly Phe Ala Ala Val Leu Glu Asn Pro
                325                 330                 335

His Leu Arg Asn Gly Leu Asn Val His Arg Gly Arg Ile Thr Asn Lys
            340                 345                 350

Ala Val Ala Glu Ser Leu Gly Leu Glu Phe Thr Pro Val Glu Ser Gly
        355                 360                 365

Leu Ala Ala
    370

<210>  49
<211>  370
<212>  PRT
<213>  Artificial Sequence

<220>
<223>  Modified Alanine Dehydrogenase

<400>  49

Met Lys Val Gly Val Pro Lys Glu Ile Lys Ala His Glu Tyr Arg Val
1               5                   10                  15

Gly Leu Thr Pro Gly Ala Ala Arg Glu Tyr Val Ala Ala Gly His Arg
            20                  25                  30

Val Met Ile Glu Thr Asn Ala Gly Ala Gly Ile Gly Ala Thr Asp Gly
        35                  40                  45

Asp Tyr Arg Asn Ala Gly Ala Thr Ile Leu Thr Ser Ala Ala Glu Val
    50                  55                  60

Phe Ala Ser Ser Glu Met Ile Val Lys Val Lys Glu Pro Gln Pro Ala
65                  70                  75                  80

Glu Trp Ser Gln Leu Arg Glu Asp Gln Ile Leu Phe Thr Tyr Leu His
                85                  90                  95

Leu Ala Pro Asp Pro Glu Gln Ala Ala Gly Leu Leu Lys Ser Gly Cys
            100                 105                 110

Ile Ala Ile Ala Tyr Glu Thr Val Thr Asp Ala His Gly Gly Leu Pro
        115                 120                 125

Leu Leu Ala Pro Met Ser Glu Val Ala Gly Arg Leu Ser Ile Glu Ala
    130                 135                 140

Ala Gly Ser Ala Leu Lys Arg Ser Thr Gly Gly Arg Gly Leu Leu Ile
145                 150                 155                 160

Gly Gly Val Pro Gly Val Gln Pro Ala Arg Ile Val Val Ile Gly Gly
                165                 170                 175

Gly Val Val Gly Thr His Ala Ala Arg Met Ala Ala Gly Leu Gly Ala
            180                 185                 190

Glu Val Thr Ile Ile Ala Arg Ser Ile Ile Arg Leu Arg Glu Leu Asp
        195                 200                 205

Glu Leu Phe Glu Gly Arg Val Arg Thr Arg Phe Ser Thr Ile Glu Ser
    210                 215                 220

Val Glu Glu Glu Val Phe Ala Ala Asp Val Val Ile Gly Ala Val Leu
225                 230                 235                 240

Val Pro Gly Ala Ser Ala Pro Lys Leu Val Arg Arg Ser Met Leu Ser
                245                 250                 255

Ser Met Arg Lys Arg Ala Val Leu Val Asp Val Ala Ile Asp Gln Gly
            260                 265                 270

Gly Cys Phe Glu Thr Ser Arg Pro Thr Thr His Ala Asp Pro Thr Tyr
        275                 280                 285

Glu Val Asp Gly Ile Ile His Tyr Cys Val Ala Asn Met Pro Gly Ala
    290                 295                 300

Val Pro Leu Thr Ser Ser Gln Ala Leu Asn Asn Ala Thr Leu Pro Phe
305                 310                 315                 320

Gly Leu Ala Leu Ala Asn Lys Gly Phe Ser Ala Val Leu Glu Asn Pro
                325                 330                 335

His Leu Arg Ala Gly Leu Asn Val His Arg Gly Arg Leu Thr Tyr Lys
            340                 345                 350

Ala Val Ala Glu Ser Leu Gly Leu Pro Phe Ser Pro Ile Glu Gln Ala
        355                 360                 365

Ala Ala
    370

<210>  50
<211>  371
<212>  PRT
<213>  Artificial Sequence

<220>
<223>  Modified Alanine Dehydrogenase

<400>  50

Met Lys Val Gly Ile Pro Arg Glu Val Lys Asn Asn Glu Phe Arg Val
1               5                   10                  15

Ala Ile Thr Pro Ala Gly Val His Glu Leu Val Arg His Gly His Gln
            20                  25                  30

Val Val Ile Glu His Gly Ala Gly Val Gly Ser Ser Ile Pro Asp Glu
        35                  40                  45

Glu Tyr Val Ala Ala Gly Ala Arg Ile Leu Asp Thr Ala Asp Glu Val
    50                  55                  60

Trp Ala Thr Ala Asp Leu Leu Leu Lys Val Lys Glu Pro Ile Ala Gln
65                  70                  75                  80

Glu Tyr His Arg Leu Arg Lys Asp Gln Thr Leu Phe Thr Tyr Leu His
                85                  90                  95

Leu Ala Ala Ser Lys Glu Cys Thr Asp Ala Leu Val Glu Ser Gly Thr
            100                 105                 110

Thr Ala Ile Ala Tyr Glu Thr Val Glu Leu Pro Gly Arg Ala Leu Pro
        115                 120                 125

Leu Leu Ala Pro Met Ser Glu Val Ala Gly Arg Ile Ala Pro Gln Val
    130                 135                 140

Gly Ala Tyr His Leu Met Ala Pro Asn Gly Gly Arg Gly Val Leu Pro
145                 150                 155                 160

Gly Gly Val Pro Gly Val Thr Pro Ala Lys Ala Val Val Ile Gly Gly
                165                 170                 175

Gly Val Ser Gly Trp Asn Ala Thr Gln Ile Ala Val Gly Met Gly Phe
            180                 185                 190

Asp Val Thr Leu Leu Ala Arg Asp Ile Asn Lys Leu Arg Glu Ala Asp
        195                 200                 205

Arg Ile Phe Gly Thr Lys Val Lys Ala Val Met Ser Asn Ser Phe Glu
    210                 215                 220

Leu Glu Lys Ala Val Val Asp Ala Asp Leu Val Ile Gly Ala Val Leu
225                 230                 235                 240

Ile Pro Gly Ala Lys Ala Pro Lys Leu Val Thr Asn Glu Leu Val Ser
                245                 250                 255

Arg Met Lys Pro Gly Ser Val Leu Val Asp Ile Ala Ile Asp Gln Gly
            260                 265                 270

Gly Cys Phe Glu Asp Ser His Pro Thr Thr His Ala Glu Pro Thr Phe
        275                 280                 285

Arg Val His Asn Ser Val Phe Tyr Cys Val Ala Asn Met Pro Gly Ala
    290                 295                 300

Val Pro Asn Thr Ser Thr Asn Ala Leu Thr Asn Ala Thr Leu Pro Tyr
305                 310                 315                 320

Ile Val Glu Leu Ala Asp Arg Gly Trp Ala Glu Ala Leu Arg Arg Asp
                325                 330                 335

Pro Ala Leu Ala Arg Gly Leu Asn Thr His Asp Gly Lys Val Val Tyr
            340                 345                 350

Arg Glu Val Ala Glu Ala His Gly Leu Glu Tyr Val Asp Pro Ala Thr
        355                 360                 365

Leu Leu Ala
    370

<210>  51
<211>  363
<212>  PRT
<213>  Artificial Sequence

<220>
<223>  Modified Alanine Dehydrogenase

<400>  51

Met Glu Ile Gly Val Pro Lys Glu Thr Lys Asp Gln Glu Phe Arg Val
1               5                   10                  15

Gly Leu Ser Pro Ser Ser Val Arg Val Leu Arg Glu Asn Gly His Ser
            20                  25                  30

Ile Phe Val Gln Thr Gln Ala Gly Thr Gly Ala Gly Phe Thr Asp Glu
        35                  40                  45

Asp Tyr Ile Ser Ala Gly Ala Glu Ile Val Ser Thr Leu Glu Ala Ala
    50                  55                  60

Trp Asn Arg Glu Leu Val Ile Lys Val Lys Glu Pro Leu Val Ser Glu
65                  70                  75                  80

Tyr Asn Leu Leu Gln Lys Gly Gln Leu Leu Phe Thr Tyr Leu His Leu
                85                  90                  95

Ala Ala Asp Arg Lys Leu Thr Glu His Leu Leu Asp Cys Gly Ile Ser
            100                 105                 110

Ala Ile Ala Tyr Glu Thr Val Glu Gln Ala Gly Val Asn Arg Leu Pro
        115                 120                 125

Leu Leu Thr Pro Met Ser Ile Ile Ala Gly Arg Leu Ala Val Gln Phe
    130                 135                 140

Gly Ala Arg Phe Leu Glu Arg Gln Gln Gly Gly Lys Gly Val Leu Leu
145                 150                 155                 160

Gly Gly Val Pro Gly Val Lys Ala Gly Lys Val Val Ile Leu Gly Gly
                165                 170                 175

Gly Val Val Gly Thr Glu Ala Ala Lys Ile Ala Val Gly Met Gly Ala
            180                 185                 190

Ile Val Gln Ile Leu Ala Val Asn Val Glu Arg Leu Ser Tyr Leu Glu
        195                 200                 205

Thr Leu Phe Gly Ser Arg Val Glu Leu Leu Tyr Ser Asn Ser Ala His
    210                 215                 220

Ile Glu Thr Ala Val Lys Glu Ala Asn Leu Leu Ile Gly Ala Val Leu
225                 230                 235                 240

Ile Pro Gly Arg Arg Ala Pro Ile Leu Val Ser Arg Asp Leu Val Lys
                245                 250                 255

Gln Met His Pro Gly Ser Val Ile Val Asp Val Ala Val Asp Gln Gly
            260                 265                 270

Gly Cys Val Glu Thr Leu His Pro Thr Ser His Thr Ser Pro Val Tyr
        275                 280                 285

Ile Asp Glu Gly Val Val His Tyr Gly Val Pro Asn Met Pro Gly Ala
    290                 295                 300

Val Pro Trp Thr Ser Thr Gln Ala Leu Asn Asn Ser Thr Leu Pro Tyr
305                 310                 315                 320

Ala Val Gln Leu Ala Asn Leu Gly Ile Lys Ala Leu Asp Val Asn Pro
                325                 330                 335

Ala Leu Ala Lys Gly Leu Asn Val Gln Asn His Arg Leu Ile His Pro
            340                 345                 350

Ala Val Gln Glu Val Phe Pro Asp Leu Val Ser
        355                 360

<210> 52
<211> 378
<212> PRT
<213> Artificial Sequence

<220>
<223> Modified Alanine Dehydrogenase

<400> 52

Met Ile Ile Gly Val Pro Lys Glu Ile Lys Asn Asn Glu Asn Arg Val
1               5                   10                  15

Ala Leu Thr Pro Gly Gly Val Ser Gln Leu Ile Ser Asn Gly His Arg
            20                  25                  30

Val Leu Val Glu Thr Gly Ala Gly Leu Gly Ser Gly Phe Glu Asn Glu
        35                  40                  45

Ala Tyr Glu Ser Ala Gly Ala Glu Ile Ile Ala Asp Pro Lys Gln Val
    50                  55                  60

Trp Asp Ala Glu Met Val Met Lys Val Lys Glu Pro Leu Pro Glu Glu
65                  70                  75                  80

Tyr Val Tyr Phe Arg Lys Gly Leu Val Leu Phe Thr Tyr Leu His Leu
                85                  90                  95

Ala Ala Glu Pro Glu Leu Ala Gln Ala Leu Lys Asp Lys Gly Val Thr
            100                 105                 110

Ala Ile Ala Tyr Glu Thr Val Ser Glu Gly Arg Thr Leu Pro Leu Leu
        115                 120                 125

Thr Pro Met Ser Glu Val Ala Gly Arg Met Ala Ala Gln Ile Gly Ala
    130                 135                 140

Gln Phe Leu Glu Lys Pro Lys Gly Gly Lys Gly Ile Leu Leu Ala Gly
145                 150                 155                 160

Val Pro Gly Val Ser Arg Gly Lys Val Thr Ile Ile Gly Gly Gly Val
                165                 170                 175

Val Gly Thr Asn Ala Ala Lys Met Ala Val Gly Leu Gly Ala Asp Val
            180                 185                 190

Thr Ile Ile Ala Leu Asn Ala Asp Arg Leu Arg Gln Leu Asp Asp Ile
        195                 200                 205

Phe Gly His Gln Ile Lys Thr Leu Ile Ser Asn Pro Val Asn Ile Ala
    210                 215                 220

Asp Ala Val Ala Glu Ala Asp Leu Leu Ile Cys Ala Val Leu Ile Pro
225                 230                 235                 240

Gly Ala Lys Ala Pro Thr Leu Val Thr Glu Glu Met Val Lys Gln Met
                245                 250                 255

Lys Pro Gly Ser Val Ile Val Asp Val Ala Ile Asp Gln Gly Gly Ile
            260                 265                 270

Val Glu Thr Val Asp His Ile Thr Thr His Asp Gln Pro Thr Tyr Glu
        275                 280                 285

Lys His Gly Val Val His Tyr Ala Val Ala Asn Met Pro Gly Ala Val
    290                 295                 300

Pro Arg Thr Ser Thr Ile Ala Leu Thr Asn Val Thr Val Pro Tyr Ala
305                 310                 315                 320

Leu Gln Ile Ala Asn Lys Gly Ala Val Lys Ala Leu Ala Asp Asn Thr
                325                 330                 335

Ala Leu Arg Ala Gly Leu Asn Thr Ala Asn Gly His Val Thr Tyr Glu
            340                 345                 350

Ala Val Ala Arg Asp Leu Gly Tyr Glu Tyr Val Pro Ala Glu Lys Ala
        355                 360                 365

Leu Gln Asp Glu Ser Ser Val Ala Gly Ala
    370                 375

<210>  53
<211>  1038
<212>  DNA
<213>  Saccharomyces eubayanus

<400>  53
atgtccgtca ctgaattgag aaacgatatc cataagttag ataccggtta tggtctgatg      60

agtttgacct ggagagctga gcccattcct caatcgcaag ctttcgaagc gatgcacaga     120

gctgttgagt tggccagaga acgtggacac aaggccttct tcaacgttgg tgagttttac     180

ggcccggact tcgttaactt aatatttgtt cgcgacttct ttgcaaagta ccctgatttg     240

agaaagcatg tagtcatcag ttgtaaaggt ggtatggagg ttagcactct gacccccaat     300

ggtagccatg atgcagtcat taaaagtgtc aagaactcgg tcagcgccat tggtggctac     360

attgacatct ttgaggttgc caggcttgat acctctctat gtgccaaagg cgaagtctat     420

ccatacgaat cgttcgaagc ccttgctgaa atgatctccg aaggcgttat tggcggtatt     480

tctttgagtg aagttactga agagcaaatc agagctattt acaaggactg gggcaagttc      540

ttgacctgtg ttgagctcga agtttctttg ttcagtaccg atattttcca taacggaatt     600

gccaagacct gtgccgaatt gggattgact gtcatctgct actctcccct aggtagaggg     660

ctattgacag gccaattgaa gtctaacgct gacatcccag aaggcgactt cagaaaggca     720

cttaagaggt ttagtgatga atctttgaag aaaaacctgg atttggtcag gttcctacag     780

gaagaaatcg tcggcaagcg ttccaaggac aactctatta ctctgcctca attggctttg     840

ggctggatca aacactggaa ctatgttccg gaatacaaag gcgccaaatt catccccatt     900

cccagtggtt cttctatctc taaggtcaat gaaaactttg atgaaaagaa aacacaactt     960

accgacaaag agttcaaagc cattaacgac ttcttggcta ctttccacac tgttggcgat 1020

aggtacgaat tcaattga 1038

<210> 54
<211> 1050
<212> DNA
<213> Torulaspora delbrueckii

<400> 54
atggtcgaac aatctgcaat caacaagcta aagcaagatc tctctgcgat tgacaccgga 60

tatgggctga tgtcgcttac gtggagggct gagcccattc cagagaccca ggctcacgaa 120

actatgaaaa gagtcgttga attggctcaa tctaaaggcc ataaggcatt tttcaactgt 180

ggtgagtttt atggacccaa tcggatcaat ctgacctaca tcagggattt ttttgtcaag 240

aatccagaat tgaggaagta tgtgatcatc agttgtaagg gtggttgcga ctgtgaaacc 300

cttactccca agggaaaaca tgacgatgtt atcaaaagtg tggaggcatc agttgcagcc 360

attggtggtt ttattgacat ctttgaagtc gctagattgg atttgagtct atgcactaat 420

ggcgaaacat acccttatga gtcctttgaa gcattggccg agatggttga caatggtgcc 480

atcggtgcca ttagtttgag tgaggtcact gctgagcaga tccgtggaat ttccaaggac 540

tggcacaagt atttggtcag tgtggaggtt gaactatcga tgttcaccaa agcgattttg 600

accaatggtg tcgttaaagc aaataatgac ctaaacttgg tcactatttt gtactctcca 660

ttgggtagag gattgctgac tggtgcagtt actagtagta aagatatacc agctggtgat 720

ttcagaaacc tactgaagag atttgatgat gaaagtttga gacaaaacct gacattactt 780

gacttcttaa gggacgaaat catcgctaag agacctgcaa ataacgagat tacactacca 840

caagtagccc ttggttggga caagtattgg aacaagacag gcgagtaccc caacactcac 900

tttttaccaa tcccaagtgg ttccagtgta aaaaaactcg aggagaactt tgatgaagcc 960

aagactcaaa tcaccgctga ggagtttaga aaaatcaatg atttcttgaa gaacttcaag 1020

actgtaggtg acgcttacga gatgagatga 1050

<210> 55
<211> 1026
<212> DNA
<213> Zygosaccharomyces bailii

<400> 55
atgtcaaaga cgggtgaatt aaaaagacaa ttgaaccaat tgcataccgg ttatgggttg 60

atgagtttga cttggagggc tgatccagtt ccccaagaac aagcctttga agccatggag 120

cgagtagtca gtcttgcgca ggctagcggc aacaaggctt tcttcaatgt tggtgagttc 180

tacggtcccg actattccaa tttggagctt gtaaaagcat tcttcgaggc caagccagag 240

ttacgtgagc acgttctgat cagttgtaag ggtggcgtcg ataataccaa gcttttgcct 300

aagggaaaat acgcagacgt tctttcgagt attgagcaat gcgtgaaaca tttgggcacg 360

cacttagaca tttttgaagt ggcccggttg gacaagtctc ttggtggcga atatcctcgt 420

gaatcatttg aggccatggc atcgatggta gacaaaggta tcatcgatgg tatatctcta 480

agcgaagtaa cagcggatga gattcgagca ataggacatg attgggcgaa ataccttgtc 540

tgtgtagaag tcgaattttc aatgttcagc cctcagatcc tgcacaatgg ggtattggat 600

gcatgcaatg acctgggatt aatcgtcatt gcctattctc ccttgggcag aggcttactt 660

accggtacca tcacgaagga ttcagccttt aaagatttcc gttccagttt gaagcgtttc 720

cagaaagatt ccctgcaaaa gaacttggcg ttgaccgaat ttctgcagga tcagatcgtg 780

gaccaaagaa gttcaacaaa cccgatttcc ttgccacagg tggcacttgg ctgggtcaaa 840

agtctgaata atgggaagta tccccacact cacattgttc ccatccccag cggatctgac 900

aagagaaaag tggaggagaa ctttgacgaa tccagggcta agctcaccgt ttctgagatt 960

gaaaaaatca acgatttctt gaagtcattc aacgttgtcg gcgacagata cgagtgggtt 1020

tcttag 1026

<210> 56
<211> 963
<212> DNA
<213> Aspergillus oryzae

<400> 56
atgccttctc ttgttggacg ggatgttggc catactggct atgggttgat gagaatgacc 60

tgggtgcctc aaccaccccc gcaagaacaa tgcttcgagg ctctgaacac cgccctcgcc 120

catggctcca atttctggaa cgccggtgaa ctctacggca cacccgaata taactctttg 180

catctgctcc atagttactt cgcccagcac ccagagaatg cggacaaggt tgttcttagc 240

attaagggtg ggttgaagcc gggccagctg gtgcctgatg gttccgaggc caatattcgg 300

cgcagtgtgg atgagtgtct tcgtgtgctg gatgggaaga agaagattga tatctttgag 360

tgtgcgagac aggatcccaa gactactgta gagcagacgg ttactgttct tgcgcagttg 420

gttaaggagg ggaagattgg gggcattggg ttgagtgagg ttgatgcgga gactattcgg 480

agagcacaca aggtgcaccc aattgcagct gtagaggttg agatgtctct gttcgacctg 540

accatcctcc agaacgacgt cgcgaaagta tgcgcagaac tcaacatccc cattgtggca 600

tattcgccat tgggacgggg tgtcttggcc ggcgctttca ccacagccgc cgacatcccc 660

gatggagatt tccgcaagac gttgcccaag ttccaagatg aggcaatgaa gcagaacatc 720

aagctagtca atgaggtcaa tgacctcgcc gctcggaagg gcgttgctcc cgttcagatc 780

gcactcgcat gggtcctaac tcttagcggc aagcctggca tgcctacgat tattcctatc 840

cctggtggta ccacgagcgc caaggtggcg cagaacttgc aagcgcctcg tctgactgat 900

gcagagatgg cagagattga tgcaattttg aagcggaatg agattgttgg gactcgatac 960

tga 963

<210> 57
<211> 1032
<212> DNA
<213> Kluyveromyces marxianus

<400> 57
atgccttccc catcagatta tcgtaaggaa ttgcttgacg tcgaagttgg ttatggattg 60

atgagtttga catggaggaa agatccactt cctgctgaaa aggtgtttcc taccatgaag 120

aaggtggtag aaaaggcggg caataagaag gcattgttca atgtgggtga attctatggt 180

cctcatttgg ctaattacaa gttgctacag tcgttcttcg ataagtaccc tgaggatcgc 240

aagaaagtta ttatcagtgc taagggaggt gtgaatgtcg aaacattaca acccaccgga 300

gatgccgaca gcatttctgc ttcaattgag aatgcattga aggtgtttgg tggatatcta 360

gacatttacg agccagctag aattgatctt gcactagcgg agaagaacgg tgagaagttg 420

tttcctcgtg agacttttga cactatcgtc aagtacatca aggatggtaa agttggcggt 480

ttctcgttaa gtgaagttac aaacgagcaa atcagagcta tttacaagga atacggcgag 540

tatctggctt gtgttgaagt tgaactctct atagcttcgc cagaaattat atccaacggg 600

attttggaca catgcaatga atttggaatt cctgtcgttg catatgcccc actaggaaga 660

ggtttactaa cgggtagctt gaaaagcact gctgatatcc ctgaaggtga tttcaggggc 720

caattgaaaa ggttccagga tgatgccatt gcacataacc tgactttggt aaaattcctt 780

caagacgaaa ttgctgcaaa gagatccgac aacccttctc tccctcaagt ggccattggc 840

tgggtccgtg gactcagaag ggagtatcca aaaacaaata ttataccaat tccatcaggg 900

tcttcggttg aaaaggtaag tgtcaacttt gatcacattg atttatcaga cgatgagatg 960

agcaagataa aagacttttt aaagtcattt aaaatcgctg gtgatagata tgagttcgcg 1020

caagcacatt aa 1032

<210> 58
<211> 1050
<212> DNA
<213> Candida albicans

<400> 58
atgtctttta aacctgtcga aatatcggga aagtttggat ttgggacaat gtccatgact 60

tggacaccaa cgccaccacc agcacaacaa tccattgata ccttgaaatt tgtgacatca 120

catccaaaat ttggaactaa attgatcaat ggtggtgaat tttatggtcc agattttgct 180

aatttgaaat tattaaaaca atttttagaa gaaaatgatc ctgaagaaaa taaacaattg 240

attatatcta ttaaaggagg tgctgataat gaaacattaa aaccaaatgg taccaaagag 300

tttgtttcaa aatcaattga aaacattgta tcatttttcc ctaaacaaaa acaaaacaga 360

ccaaaattat tatttgagat ggccagagtt gatccttctg ttccctatgg agaaactatt 420

ggttatatca gtgaatatgt gaaactgggt gtgattgatg gtatttcttt atctgaagtt 480

ggtaaagaat ccattcaggc tgctttgaaa gtatttccaa tatcatgtgt tgaattagaa 540

ttgtcattgt tttcacaaga agttattacc actggtatat tggaagaatt gtcaaagcac 600

aacttgccac tcattgctta ttcaccattg tgtcgtggtc ttttaacaga ttatgcagtt 660

gagaattcag atacattttt ggcatcaatc ccacaaggtg atattagaca tcatttggac 720

aaatttcaac cagacacttt taacaaaaac ttgcccgctt taaaggaatt atacaagttt 780

gctcatgaag ttaaaaacac aactttggaa tctttggcat tatcatggat tgttacagtt 840

tcggaagcaa gaaatttccg aggcattgaa aaagtgacca gaattttacc aattccttct 900

ggatcaacta aaaagagagt tgagtcaaat tttggaagtt taatagagtt gactgatgat 960

gatttacaat caatcgatga aatttttgcc aaatatccaa ttagtgggtt gagatacaat 1020

caatttctcg aaggaactct tttccaatag 1050

<210> 59
<211> 990
<212> DNA
<213> Yarrowia lipolytica

<400> 59
atgacattca acaaaatcac acccacttcc aagggtttcg gactcatggg cttcacatgg 60

agagaaaccg agacccccga tgagattgcc ttccccacca tgaagcgagc cattgaaaag 120

ggcattcttg tgtggaatag cgccgagttc tatggtacca aggacccact aggcaacctc 180

aagttgattc gacgatactt tgagaagtac cccgaagacg ccgacaaggt gtgtctgatg 240

gtcaagggag gtctgggacc caacttggtg cccaatggct ccaaggagtt tgtgcagaag 300

tccattgaca ctgttctgga gactctggga ggctctgttc ctgtgagcat cttccagtgt 360

gcccgtgtgg accccaatac ccccattgag gagacggtcg ctgccatcaa ggagtatgtt 420

gatgctggca aaattggagc tctgggttta tctgaggtcg gggctgccac cattgagaag 480

gtccactctc tcagtcccat tgctgctgtt gagaatgagc tatctctgtg ggccaccgat 540

atcttcacca acggtgttgc tgaagtctgt gccaagtaca acattcccat cactgcctat 600

gctcctctgg gctctggcgt gcttactggc cggttcaaga gcacccagga tgtgcctgag 660

ggagacgccc gaaagcacat ggtcaagtac caggaggaaa acttgaaaca gaatctcgtt 720

ctcgttgacc gacttaagga ggtggccacc aaggccggta ttcctcttcc tcagatggcc 780

attgcatggg tcaaggctca atctaaccac aatggccatg gaaccatcat ccctatccca 840

ggtaacacca ctgttgctcg gctcgaggag aaccacaatg atctcactct gtcggccgag 900

actctcaagg agattgatga tgttctgaag agtactaaga ttgttggaga tcgaggattt 960

actcttggtg gagagtttat gaatggttaa 990

<210> 60
<211> 1179
<212> DNA
<213> Mesorhizobium sp. YR577

<400> 60
atgcgatatc ccgaccacac tgagccggtg atcacactca ccgccggtcc cgtcaacgcc 60

tatcccgatg tgctacgggg gctcgggcgc acggtgcttt acgactacga cccggcgttc 120

cagctcttct acgagaaggt catcggcaag gcgcagaagg ccatgcgtct gtcgaataag 180

ccggtcgtcc tgcatggcga gccggtgatg ggtctggagg ccgccgcggc gtcgctcatc 240

tctagggatg atgtggtgct caatctggca tcgggtgtct acggcaaggg gttcggctat 300

tgggcgaagc gttattcgcc gaacctgctg gagatcgagg ttccttacaa tgaggcgatc 360

gatccgcagt ccgtcgcgga catgctgaaa tcgcatcccg aaatcaccgt cgtttcggtc 420

tgccatcacg acacgccgtc aggcacgatc aacccgatcg atgagatcgg agcgatcgtt 480

tcggcgcatg gcggctatct gatcgtcgac gccgtttcgt ccttcggcgg catgaaaacg 540

catcccgagg attgcaaggc cgacatctat gtgaccgggc cgaacaagtg ccttggcgca 600

cctcccggac tgacgctgat gagcgtgagc gaccgtgcct ggaccaggat gaaagcgaac 660

tctgcagcac ctcgcgcatc ggttctgagc attctcgatt gggagcatgc atggtccaag 720

gaaaagccgt ttccgtttac gccgtccgtc gccgaaatga acggcctgga tgtcgcgctc 780

gacctttatc tcaacgaggg gccggaagcg gtgtgggcgc ggcacgccct gaccgccaag 840

gcgacacgcg ccggggctca ggcgatgggc ctgtcgatct gggctgcaaa tgaaaagatc 900

gcctcaccga caacaaccgc catccgcacg ccggacgggg tcgatgaaaa ggcgctgcgt 960

caggccgcgc gtgagcgtta cggcgtggtg ttttcttccg gccggggcga aacgctcggc 1020

aaactgacgc gcattggcca catggggcca acggcccagc cgatctacgc gatcgcagcg 1080

ctgacggctt tgggcggtgc gctgaatgcg gccggccgga agcttcaggt tggcaagggt 1140

atcgaggcag cactcgccgt gatcgatgcg gatgcctga 1179

<210> 61
<211> 1179
<212> DNA
<213> Pseudaminobacter salicylatoxidans

<400> 61
atgcgatata aagacaatgc cgacccggtg atcacgctca cctcggggcc cgtcaacgcc 60

tatgcggatg tgctgcgcgg gctgagccgc actgtgctct acgactatga tccggcgttc 120

cagctcttct atgaaaacgt ggtggccaag gcgcagaagg ccatgcgtct ttccacccgg 180

ccggtgctgt tgcagggtga gccggttatg ggactggagg cagcggcagc ttcactgatt 240

tccagcgagg atgtcgtcct gaacctcgcc tctggcgtct atggcaaggg cttcggctac 300

tgggcaaagc gctattcgcc gaacctgctc gagatcgaaa ccccctataa cgaatccatc 360

gacccgcagg cggttgccga tatgcttaag aagcatccgg aaatcaccgt tgtctcggtg 420

tgccaccacg acacaccgtc gggcacgatc aacccgatcg acgaaattgg cgcgatcgta 480

tcggcgcacg gggcctattt catcgtcgat gccgtatctt cattcggagg catgaagacc 540

catcccgaag actgcaaggc cgacctctat gtcactggcc cgaacaagtg cctgggcgcg 600

cctccgggac tgaccatgat gggcgtcagc gacagggcct gggccaggat gaaagccaat 660

ccatccgcac cgcgcgcctc ggtgctgagc attctcgact gggaggatgc ctggtcgcac 720

gaaaagccgt ttccgttcac accctctgtg gcggagatca acggcctcga cgtcgcgctg 780

gacctctatc tgaacgaggg gccggaagtg gtgtgggcgc gccatgcgct gacggcaaaa 840

gccacgcgtg cgggcgcatt ggccatgggg ttgtcgctct gggcggctag cgagaagatt 900

gcctcgccca ccacgacgtc gatccgcacg ccgcaggggg tggacgaagc cgcgcttcgg 960

caggcggtgc gagagcgcta tggcgtagtg ttttcatccg gccgtggcga gacgttgggc 1020

aagcttaccc gcatcggcca catgggcccc acggcgcagc cgatttacgc agtcgcggcg 1080

ctgaccgcgc tgggtggcgc gttgaacgcg atgggccaga agctggccgt cggcagaggc 1140

atcgacgccg ccctcgcggt gatcgacgcc gatgcatga 1179

<210> 62
<211> 1179
<212> DNA
<213> Bauldia litoralis

<400> 62
atgcgctatg cactcgatgc cgatcccgtc atcacgctga cctcggggcc ggtcaacgcc 60

tatccggatg tcctgcgggc gttggcgcgc acggttcttt acgactacga cccggccttc 120

cagctccatt acgagcaggt gatcggcaag atgcagacgg cgatgcggac gtcgaaccgt 180

ccggtcatcc tgcatggcga gccggtgctc ggactcgagg cggcggcggc ctcgctgatc 240

accgccgacg atactgtgct caacctggcg tcgggcgtct acggcaaggg gttcggctac 300

tgggcagcgc gctattcacc caaccagctc gagatcgagg ttccctacaa ccaggcgatc 360

gacccgcagc aggtcgccga catgctgaag gccaatccgg ccatttcggt tgtcgccgtc 420

tgccatcacg acacgccgtc ggggaccatc aacccgatcg acgagatagg cgccatcgtc 480

gccgaccatg gcgcctacct gatcgtcgac gcggtgtcct ccttcggcgg gatgaagacc 540

cacccggagg attgcaaggc cgacatctac gtcaccggcc cgaacaaatg cctcggcgcg 600

ccgccggccc tgaccatgct cggcgtcagc gaacgggcgt ggcagaagat gaaggacaat 660

ccggccgcgc cgcgcgcctc gatgctcagc atcctcgact gggagaatgc ctggtcgcgc 720

gacgaggcct ttcccttcac gccgtcggtg gccgaggtca acggtctcga cgtcgcgctc 780

gacctctacc ttgccgaggg accggaggcc gtctgggcgc gccacgcgct gaccgccgcg 840

gcgacgcggg ccggcatcct ggcgatgggc ctcgacctgt gggcggcgga cgcggccatc 900

gcctcgccga cgacgaccgc ggtgcggacg ccggagggca tcgatgaagc gaagctgcgc 960

gcgacggtcc gcgaacgcta tggcgtggtg atgtcgtccg ggcgcggcga aacgctcggc 1020

aagctcacgc ggatcggcca catgggcccg acggcgcagc cgatctatgc gatcgccgcg 1080

ctgaccgcct ttggcggcgc tctccaggcg ctcggccatc ccgcgccggt cggcaaggcg 1140

atcgacgcgg cgctcgcggt catcgacgcc gccgcctga 1179

<210> 63
<211> 1176
<212> DNA
<213> Skermanella stibiiresistens

<400> 63
atgacagtcg agcgtatcgt cgagcctgct ttcactctca gttccggtcc cgtcgacgcg 60

tatcccgccg tgctcagggc actgtcgcgg acggtgctct acgacttcga tcccgtgttc 120

cagcacttct acgaggcggt gacggataag gccaaggtgg cactccgcac cgcggcgcca 180

cccgtgatcc tccatggcga gccggtgctg gggctggagg ccgcggccgc ctcgctgatc 240

gcccgcgacg atgtcgtgct caacctagcg tccggcgtct atggcaaggg cttcggtttc 300

tgggccaaac ggtactgcgc cgagttggtc gagatcgagg tgccttatga cgacgcgatc 360

gacccggcgg cggtcgaggc cgccttcagg gaacgccccg acatccgtgt ggtctcggtc 420

tgccatcatg acacgccgtc tggcacgctg aacccaatcg acgagatcgg ccgtatcgtg 480

gcggcccatg gtgcctatct gatcgtggac gccgtttcct cgttcggggg gatggacgtc 540

catcccgagg cctgccaagc cgacatcttc gtgacagggc cgaacaagtg cctgggctgc 600

ccgcctggtc tgacgctgct ggcggtcagt ggccgcgcct gggacaagat gaaggccaat 660

ccggacgcgc cgcgcgcctc gatcctcagc atcctggatt gggagaacgc gtggcggcgt 720

gaccagccgt ttcccttcac cccatcgatt tccgaagtga acgcgctgga cgcggctctg 780

gacctgtacc tggaggaggg gccggaagcc gtctggcgcc gtcacgcgct cacatccaag 840

gcctgccgcg acggcatccg cgccatgggc atgacgatat ggcccgcgcg ggaggcgatc 900

gcctctccga ccaccacggc ggtgcgggtg cccgacggga tcgacgcgga tgccctgttg 960

gcgacggcgc gggatggctt cggcgtcagc ttctcgtccg ggcggggcga gaccaagggc 1020

aagctgatcc gcatcggaca catgggccgc accgcccaac cactctacgc cgtcgtcggg 1080

ctggcggcgc tgggcggtgc gttgcggcgg ctgggagcca atgtcgacgc cggcgccggc 1140

atggaggcgg ctctcgccgt gatctcaaac caataa 1176

<210> 64
<211> 1185
<212> DNA
<213> Rhizobium sp. AC44/96

<400> 64
atgaaccaga cgaaaattgc cgatccaatc tttaccttga ccacggggcc ggtcgatgcc 60

tatccagcgg tgctgcgcgc cctgtcccgc ccggtgctct acgactacga tccggcgttc 120

cttgcgttct acgaagccgt caatgccaag gtacagcgcg ccttccacac gaaaacccca 180

cccgtgatcc tccagggcga gcccgtgctg gggctcgaag cggctgcggc ttcgctgatc 240

gccaagaaag acgtggtgct taacctcgtt tccggcgtct acggcaaggg gttcggcttt 300

tgggcagcgc gctatgcgcg ggagctggtc gaattggaag ttccctataa cgatgtcctg 360

acggccgatg cggtggccga catgttgaaa aagcgtccgg atatcgccgt ggtcgcgctc 420

tgccaccatg ataccccgtc aggcaccgtc aatcccgtgg ccgaaatcgg tgcagtggtg 480

agagcggcgg gaaagctctt catcgtcgat tccgtatcgg cctttggcgg catggacgtg 540

cttccggaga cggcttgcgc cgatattttc gtaaccggcc ccaataaatg tctcggctgc 600

ccgcccggtt tgtcgcttct gcacgtcagc gaggctgcct gggagaagat cgccgccaat 660

cccgatgccc cgacagcttc gatgctttcg atcagtgatt ggaaacgagc tcacgaggct 720

ggccaaccgt tcccgtttac cccctcggtt tccgaaatca acgccctcga cgccgccatg 780

gatctctatc tggaggaagg cgagcaggca gtatgggcgc gccatgcgct gacagccaag 840

gcatgccggg caggcgtgca agcagccggc ctgaagctct gggcggcacg ggaagacatc 900

gcctcgccaa cctgcactgc cgtggcaatc cccgatggga tcgacgaagc aaagctgcgg 960

gcgtcgatcc gtgatcgcta cggcgttgtc ttttcttctg gccgtgccga gacgctgggc 1020

aagcttacgc gcatcggcca tatgggcccg actgcacgtc cgacctattc actcgtctcg 1080

gttgcagcca tcgttggcgg gctcaaggcc gccggcgtca aggaccttga tgtggaggct 1140

ggagttgcgg cggcaatgaa ggtcatagac agtgcagaaa agtga 1185

<210> 65
<211> 1173
<212> DNA
<213> Erwinia toletana

<400> 65
atgatgcgtt ccgcctttgc ccagccgtta tttaccctga ctaccggtcc ggttgatgtc 60

tatccggcgg tgtcgcgggc attatcgatg ccggtctggt acgactacga cccggctttt 120

cagaactgct ttgaacgcgt cagcctgaag gccgcacagg cgctggaaag cgccacgccg 180

ccgctgatct tacagggtga accggtactg gcgctggaag ccgcggcggc atcgctgctt 240

agcgctgagg atatggtgct gaatctggtt tccggcgtat atgccgccgg tttctctgag 300

tgggcgcggc gctattcgcg tcaggttgag gagctgcgcg ttgaatttaa tcaggtgatt 360

gatcctgtcg cggttgatgc atggcttagt cgtcatccgc agattaccgt ggtggtggtt 420

tgccaccatg acacgccgtc cggcacccta aacccactgg atgaaattgg gcgggtggtg 480

aaaaaacatg gcaaattgct gctggtggat gcggtttctt cgtttgccgg tgtggcggtg 540

acggcggcca gttgccaggc ggacctgttt attaccggcc ccaataaatg tctcggctgt 600

ccgccgggct taagcctggt ggcggtatct gacgctgcct ggcggaaaat cgaggccaat 660

gacgctgcgc cgcgtgcgtc ggtactcagc ctgctcgact ggcgtgatgc ctggtcagcg 720

gaaaaacctt ttccgtatac cccatcagtg gcggagatca acgggctgga ggcggcgctg 780

gacggttatt tgcaggaggg gccagcgcag gtctgggcgc gccacaggct aaccgcacag 840

gcatttcgcg ccgggattca ggcgatggga cttagcctgt gggcggcgga tgaaagcatt 900

gcttcaccaa cggcaactgc gatacgcgtg cccgacgggg taaatgaagc gcaatggcga 960

ctgcgcgcgc gtgagtgtta tggcgtgatg ttttcgtcag ggcgcggtga aacgctgggc 1020

aaggtattgc gtgtcggcca tatggggccg actgcgcaac cgatgtttgc gattattgcg 1080

ctgaccgcac tggcgggggc acttaatcag ctacgcgagc cgcagctgga tattggcgct 1140

ggcgtggcgg cggcaatggc ggtgattggc taa 1173

<210> 66
<211> 1161
<212> DNA
<213> Herbiconiux ginsengi

<400> 66
atggcacacc ccgacttcac gctctcggcc gggccggtca ccgtcaccgc ccgcacgctc 60

gcgggcctcg gctcgccgat cctctaccac tacgaccccg aattcctcgc gacgttccgg 120

cgcacgcagg gcaaggtggg gcagatgttc cagaccgaca acgacatcat cctcatgcag 180

ggtgaggcca tcgtcggtct cgagggcgcc ttgcgctcgc tgatcacgcc cgggatgcac 240

gtgctgaacc tcgtgcaggg cgtgttcggc aagggcaccg gctactggat cgccgacttc 300

ggcgccgtgc tgcacgagat cgaggtcggc tacgacgacg cggtctcgcc ggcacaggtc 360

gaggagtacc tcgacgcgca tccggagatc cagatggtgt gcctggtggc ctccgagacc 420

ccgtcgggca cggtgacgga tgtcgcggcg atcggcccga tgtgccggga ccgcggcatc 480

ctgacctaca tcgacactgt ctcgggcgtg ctcggcatgc cctggaagac cgacgagtgg 540

ggcctggacc tctgcgtcgc cggcgctcag aaatgcctcg ggggccctcc tggcgtggcg 600

ctcatctcgg tgagccagaa ggcgtgggac gtgatgtacg cgaacccggc cgccccgcgt 660

gactcctatc tctcgctgat cgactggaag gagaagtggc tcggtgaggg ccgctttccg 720

tacacgccgt cggtgagcga catgaacggg ctcgaagcgg cgctcgacca ggcgctggag 780

gaggggatcg acgcggtcgt cgcgcgtcac gaggccgccg ccgccgtgac ccgcgccggc 840

gcccgagcca tgggtctcga gctctgggcg aagagcgagg agatcgcgtc ggcctgcgtc 900

acctccatcc gcctgcccga cgacatcgac aacgccgtgg tgcgcgacca cgcccgcgag 960

gtctacgggg tgatgctctc gcacgggcag ggtgccggca acctcgtgcg gctctcgcac 1020

atgggcccga cagccggtgg cctccactcg gtggtgggcc tcgccgcgct cggccgcacc 1080

ctcgccgatc tgggcatgtc cgtcgacatc ggagcgggac tggaggcggc gctcgcgctg 1140

ctcagtaccc agcggcggtg a 1161

<210> 67
<211> 1116
<212> DNA
<213> Artificial Sequence

<220>
<223> Modified Nucleotide Sequence for Alanine Dehydrogenase

<400> 67
atgattattg gcgtacccaa ggaaatcaaa aaccatgaat accgggtcgg catgactccc 60

gccggagtgc aggagctgac ggcccgggga cacaaggtgc tggtgcagca gcagggaggc 120

gaggcgatcg ggctgtccga cgccgagtac caggcagccg gtgccgaact ggtggccagc 180

gcggcggaga tcttcgcccg ggccgagatg atagtgaagg tgaaggagcc ccagccggag 240

gagtgcgagc tgctcagacc gggtcagctg ctgttcactt acctgcacct ggcgccggat 300

ccggtgcaga cccagctgct ggtgcactcg ggggcggtgg ccatcgccta cgagacggta 360

acagacgatc gcggcggcct gccgctgctg gcgcccatgt ccgaggtggc ggggcggatg 420

gcgatccagg ccggcgccca cagcctggag aaggcgcagg ggggcaacgg caccctgctg 480

ggcggggtgc cggcagtggc gcccgccaag gtggtggtgc tgggcggtgg cgtggtcggc 540

gtcaactcgg cgcgcatggc gctgggactg ggggccgacg tcaccatcct ggctcgctcc 600

ctgccgcgac tgcgggaact ggacagcctg tacggcccgg ccctcaagac cctctactcc 660

actcgcacca acatagaggc gtgcatcgcc caggctgacc tggtgatcgg tgccgtgctg 720

atcccggggg cggcggcgcc caagctgctg acccgcgaca tgctcaagct gatgcgcaag 780

ggctcggtgc tggtggacgt ggccatcgat cagggcggct gtttcgagac ctcccacccg 840

accacccacg aggagcccac ctacgtggtg gacggcatca tccactactg tgtggccaac 900

atgccgggcg gggtggcgcg cacctccacc ttcgcgctga ccaacgccac cctgccgttc 960

gtgctggcgc tggccgacaa gggctatgtc agggcgctgc aggaggatcg ccacctgcgg 1020

gccggtctga acgtgttcaa gggcaagatc acccaggcgg cggtggcgca ggcgctcggc 1080

tacgagtacg tcagcgccga ggtggttctg gactga 1116

<210> 68
<211> 1119
<212> DNA
<213> Artificial Sequence

<220>
<223> Modified Nucleotide Sequence for Alanine Dehydrogenase

<400> 68
atgcgtgtcg gttgcccgaa ggaaatcaag aatcatgaat atcgtgtcgg cctgacacct 60

gcgtccgttc gtgagtacgt tgcccatggc catgaggtcc gggtcgaaac caaggcaggc 120

gccggcattg gcgccgatga tgccgcctat gtggccgctg gcgcaaagat tgccgcctcg 180

gccaaggaga tcttcgagaa gtgcgacatg atcgtaaagg tcaaggagcc gcagccctcc 240

gagtgggcgc agcttcgcga tggccagatt ctctatacct atcttcacct cgcgcccgat 300

ccggaacaga ccaagggcct gcttgcctcc ggcgtcaccg ctgttgccta tgagactgtg 360

acggatgatc gcggcggcct gccgctcctt gctccgatgt ccgaggtcgc cggacgtctg 420

tcgatccagg cgggggcgac cgcattgcag aaggccaatg gcggccttgg catcttgctc 480

ggaggcgtgc ctggcgtgtt gccggccaag gtcacggtga tcggcggcgg tgtggtcggc 540

ttgcacgcgg ccaggatggc ggcaggcctc ggtgccgacg tcagcatcct cgcaaagtct 600

ctaccgcgcc tgcgccagct cgatgatatc ttcggcggtc gtgtccatac gcgctattcc 660

agcattcagg ccttggaaga ggaggtattc tcggccgacc tcgtcatcgg cgctgtgctc 720

attccgggcg ctgccgcacc gaagcttgtc acccgtgaaa tgctgtcggg catgaagcga 780

ggctctgtca tcgtcgacgt cgccatcgac caaggtggct gctttgagac gtcgcatgca 840

acgacgcatt ccgagccgac ctacgttgtc gatgacgtgg tgcattattg cgttgccaac 900

atgcctggcg ctgtgccggt cacttcggcg catgccctga acaatgcgac gctcgtttat 960

ggcctggcgc ttgcagaccg cggactgcgc gcgatcgcgg aagatcgcca cctgaggaac 1020

ggcctcaacg tccataaggg ccgcgtcacc aacaagccgg tggccgaagc gctgggctat 1080

gaatcctaca ctccggaaag cgtactgaac gttgcgtaa 1119

<210> 69
<211> 1122
<212> DNA
<213> Artificial Sequence

<220>
<223> Modified Nucleotide Sequence for Alanine Dehydrogenase

<400> 69

atgcgaatcg gcgtacccaa ggaaatcaag aatcacgagt accgtgttgg ccttacgccg 60

cagtccgtgg ccgaactgac cacgctgggg catgaggtgt ggatcgagac cctggccggc 120

gccgccatcg gttttgccga tgaggattac cgtaaggccg gggcgcagat cgctccgtgc 180

gccggcgagg tgttccagca ggcgcagctg atcgtcaagg tcaaggagcc tctggcggtg 240

gaacgggcca agctgcgcga gcagcatacg ctgttcacct acctgcacct tgcgccggac 300

cggccgcaga ccgacgagct gatggccagc ggcgccacct gtatcgccta cgagacggtg 360

accgatgtgc agggccgcct gccgttgttg gcgccgatgt cggaagtggc tgggcgcatg 420

tcgatccagg ccggtgccgg ttgcctggaa aaggcgcgtg gcggtcgtgg cgtactgctc 480

ggcggcgtgc ctggggtggc gccgggcaag gtggtgatcc ttggcggtgg tgtggttggc 540

agtcatgccc tggccatggc cgtgggcctg ggcgccgatg tcacggtgct ggccaagagt 600

gtcgatgccc tgcgccggct cgatgcgcag tacggcaacc gcatcaccac gctctattcc 660

acacgcgccg cagtgcagga gcaggtgttg gcggccgact tggtcatcgg cggcgtgttg 720

attcccgggg cggcagcgcc caagttgatc agcgcggaca tggtgcgcca gatgaaggcc 780

ggcgcggtgt tggtggatgt ggccatcgac cagggcggct gcgctgaaac ctcgcgggcc 840

accactcatg ccgagcccac ctacgtggtc gacgatgtgg tgcattactg cgtggccaat 900

atgcccggtg cggtggcccg tacctcgacg ttggcgctga acaacgccac tttgccattc 960

gtcgtggcgc tggcgcagaa gggcacgcgg cgagcgctgg aagacgatcc gcacctgctg 1020

gccggtctca atgtcgcgcg cggcgccatc acctgcgcca gtgtcgccga ggctcacggg 1080

ctacccttcc agccgccggc cagcgtgctc gagcggctct ag 1122

<210> 70
<211> 1116
<212> DNA
<213> Artificial Sequence

<220>
<223> Modified Nucleotide Sequence for Alanine Dehydrogenase

<400> 70
atgcgcgtcg gtgtgcccaa ggagatcaag atgcaggaat atcgcgtcgg gctcacccca 60

ggcgccgtcc gcgaatatgt cgcggccggg caccaggtga cggtcgagac cggcgccggc 120

agcggcatcg gcgcgtctga cgaggtgtac cagcgggcag gggccgcgat cgcggagaac 180

gcccgcgaca tcttcgacag ctccgacatg atcgtgaagg tgaaggagcc gcagaaaagc 240

gaatgggccc agcttcgcga aagtcagatc ctctttacct acctccatct tgcgccggat 300

cccgaacagg ccaagggcct gctcacctcg ggttgcactg cggtcgccta tgagaccgtc 360

accgacgcgg ccggtcacct ccccctgctt gcgccgatga gcgaagtcgc cggccgcctc 420

gccatcgaag ccgccggcgc cgcgctcaag cgatcagccg gcggccgcgg ggtgctgctc 480

ggcggtgtgc ccggcgtgca gccggcgcgc gtcgtcgtgc tcggcggcgg cgtggtggga 540

acgcaggccg cgcgcatggc ggcgggtctc ggtgcggaag tcactgtgat cgcacgctcg 600

gttccccgtc tgcgcgagct ggacgatgtc tttctcgggc gggtgcgcac ccgcttctcg 660

accatcgagg cagtcgagga cgaggtgttc gccgccgacg tcgtgatcgg ggcagtgctg 720

gtgccgggcg ccagtgcacc aaagctcgtc acgcgcggga tgctcaagtc gatgcggccg 780

ggcgccgtgc tggtcgacgt cgcgatcgac cagggcggct gcttcgagac ctcgcatccg 840

acaacgcacg ccgatccgac ctatcaggtc gacggcgtcg tgcattattg cgtcgccaac 900

atgccgggcg cggtgcccgt gacgtcgagc cagggactga acaacgcgac gctgccgttc 960

ggcctgatgc tcgcgaacaa gggctttgcc gcggtcctgg aaaatccgca tttgcgcaac 1020

ggactgaacg tgcatcgtgg ccgcataacc aacaaggcgg ttgccgagag ccttgggctg 1080

gaatttaccc cggtcgagag cggcctcgcc gcgtaa 1116

<210> 71
<211> 1113
<212> DNA
<213> Artificial Sequence

<220>
<223> Modified Nucleotide Sequence for Alanine Dehydrogenase

<400> 71
atgaaggtcg gagttccaaa ggaaatcaag gcgcacgaat atcgcgtggg ccttacccca 60

ggagctgccc gtgaatacgt ggcagcgggc caccgcgtga tgatcgagac caatgccggc 120

gctggcattg gtgcaaccga tggcgattat cgcaatgccg gcgcaacgat cttaacttcg 180

gcagctgagg tattcgcatc gagcgagatg atcgtaaagg tcaaggagcc tcagccggcc 240

gaatggtccc agctgcgtga ggatcaaatc ctgttcacct atttgcactt ggccccggac 300

ccggaacagg ctgccggcct cttgaaatct ggatgcattg caatcgccta tgagaccgta 360

actgatgctc atggtggtct tccactgctg gcgccgatga gcgaggtcgc gggcaggctt 420

tcgatcgaag cggcgggtag cgctctaaag cggagtacag gggggcgagg gctgctgatc 480

ggtggggtgc ccggggttca gcctgcccga atcgtggtga ttggaggtgg ggtcgtcggt 540

acgcacgccg cgcgcatggc ggctggtctg ggcgcagagg tgaccatcat cgcacgttcg 600

atcatccggc tccgtgagct ggacgagcta ttcgaaggac gggttcgcac taggttttcg 660

accatcgaat ccgtggagga agaggtattt gcggcggacg tggtgattgg tgcggtgctc 720

gtcccagggg caagcgctcc gaagctggtc cgccggagca tgctgagctc aatgcgcaag 780

agagccgtac tcgtggacgt cgctatcgat cagggcggct gctttgagac atcgcgccca 840

accactcacg ctgatccaac atacgaagta gatggcatca ttcattattg tgtggccaat 900

atgccgggtg ccgtgccgct aacctcgagc caggcactga ataacgcgac cctgccgttt 960

ggtttggctc tggccaacaa gggtttttcc gccgtactcg agaatccaca tctgcgcgca 1020

ggcctcaacg tccatcgggg ccggctaaca tacaaggcgg tggccgagag cctcgggctg 1080

cccttctccc cgatcgaaca ggctgcggcc tga 1113

<210> 72
<211> 1116
<212> DNA
<213> Artificial Sequence

<220>
<223> Modified Nucleotide Sequence for Alanine Dehydrogenase

<400> 72
atgaaggtcg gcatcccccg cgaggtcaag aaccacgagt accgcgtggc catcacgccc 60

gccggcgtgc atgagctggt ccgcaacgga cacgaggtct acatcgagga caacgccggt 120

ctcggctcct cgatccccaa cgaggagtac gtggccgccg gcgccaccat cctccccacc 180

gccgacgagg tgtgggccac cgccgacctg ctgctgaagg tcaaggagcc gatctcctcc 240

gagtaccacc ggctgcggaa gggccagacc ctcttcacgt acctgcacct ggcggccgac 300

cgggccggta ccgacgcgtt gatcgcctcg ggcaccaccg cgatcgcgta cgagaccgtg 360

cagctgggca acggcgcgct gccgctgctc gccccgatgt ccgaggtcgc gggccgcctg 420

gccccgcagg tcggctcgta ccacctgatg cgtccggccg gcggccgcgg cgtgctgccc 480

ggcggcgtgc ccggcaccca cccggccaag gccgtcgtca tcggcggtgg cgtctccggc 540

tggcacgcgg ccaccatcgc catcggcatg ggctacgacg tgaccctgct ggcacgcgac 600

atcaacaagc tgcgcgaggc cgaccggatc ttcggcaccc agatcaaggc catcatgtcc 660

aactcgttcg agctggagaa ggccgtcctg gacgccgacc tggtcatcgg cgccgtgctg 720

atcccgggcg ccaaggcccc gaagctggtc accaacgagc tggtctcccg catgaagccg 780

ggctccgtgc tcgtcgacat cgccatcgac cagggcggct gcttcgagga ctcgcacccg 840

accacgcacg acgacccgac cttccaggtc cacaactcgg tcttctactg cgtggccaac 900

atgccgggcg ccgtcccgaa cacctccacc tacgcgctga ccaacgccac cctgccgtac 960

gtgctggagc tggccaaccg tggctggaag gacgcgctgc gccgcgaccc ggcgctcgcg 1020

aagggcctga acgtccacga gggtcagatc accttcccgg ccgtggccga cgccttcggc 1080

ctggagtcgg tcacgctgga cagcgtgctc gcctga 1116

<210> 73
<211> 1092
<212> DNA
<213> Artificial Sequence

<220>

<223>  Modified Nucleotide Sequence for Alanine Dehydrogenase

<400>  73
atggaaatcg gcgttcctaa ggaaactaag gatcaagagt ttcgtgtagg gttaagtcct      60

tctagtgtgc gggtactgcg agaaaatggt catagtatct tcgtccaaac acaggcgggt     120

actggtgctg gatttacaga tgaagactat atcagtgcgg gggcagaaat tgtctctaca     180

ctggaagccg cttggaatcg ggagttagtg atcaaggtta aagaaccgtt ggtaagcgag     240

tataatcttc tgcaaaaggg acagttatta tttacttatt tacatttggc agccgatcgc     300

aaattaacag aacatttact agattgtggc attagtgcga tcgcttacga aacagtagaa     360

caagctggag tgaatagatt acccttgctt accccgatga gcattatcgc tggtcggtta     420

gcggtacaat ttggcgctag atttctagaa cgtcaacaag gtggtaaagg cgtacttctc     480

ggcggtgtac ccggtgtcaa agctggcaaa gtagtaattt taggtggtgg tgtcgtcggc     540

acagaagccg ctaaaattgc ggtgggtatg ggtgcgatcg tacaaatttt agcagtcaac     600

gttgaacgtc tttcttactt agaaactctt tttggttcta gagttgaact gctgtatagc     660

aactctgctc atattgaaac cgcagttaaa gaagccaatt tactcattgg tgcggtttta     720

attcctggca ggagagcgcc aattttagta tcccgtgatt tggttaaaca aatgcatccc     780

ggttcagtga ttgtggatgt tgcagttgat caaggtggat gtgtagaaac attacaccct     840

acatctcaca ccagtccggt atatattgat gagggtgtgg tacattatgg tgttcctaat     900

atgccagggg ctgtaccctg gacatccact caagctctca acaacagcac tttgccctat     960

gctgtgcagt tggctaattt gggaattaag gctttggatg ttaacccagc tttggctaag    1020

ggtttgaatg tgcagaatca tcgtttaata catccggctg tacaagaggt tttccctgat    1080

ttggtgagtt aa                                                        1092


<210>  74
<211>  1137
<212>  DNA
<213>  Artificial Sequence

<220>
<223>  Modified Nucleotide Sequence for Alanine Dehydrogenase

<400>  74
atgatcatag gggttcctaa agagataaaa aacaatgaaa accgtgtcgc attaacaccc      60

gggggcgttt ctcagctcat ttcaaacggc caccgggtgc tggttgaaac aggcgcgggc     120

cttggaagcg gatttgaaaa tgaagcctat gagtcagcag gagcggaaat cattgctgat     180

ccgaagcagg tctgggacgc cgaaatggtc atgaaagtaa aagaaccgct gccggaagaa     240

tatgtttatt ttcgcaaagg acttgtgctg tttacgtacc ttcatttagc agctgagcct     300

gagcttgcac aggccttgaa ggataaagga gtaactgcca tcgcatatga aacggtcagt     360

gaaggccgga cattgcctct tctgacgcca atgtcagagg ttgcgggcag aatggcagcg 420

caaatcggcg ctcaattctt agaaaagcct aaaggcggaa aaggcattct gcttgccggg 480

gtgcctggcg tttcccgcgg aaaagtaaca attatcggag gaggcgttgt cgggacaaac 540

gcggcgaaaa tggctgtcgg cctcggtgca gatgtgacga tcattgcctt aaacgcagac 600

cgcttgcgcc agcttgatga catcttcggc catcagatta aaacgttaat ttctaatccg 660

gtcaatattg ctgatgctgt ggcggaagcg gatctcctca tttgcgcggt attaattccg 720

ggtgctaaag ctccgactct tgtcactgag gaaatggtaa aacaaatgaa acccggttca 780

gttattgttg atgtagcgat cgaccaaggc ggcatcgtcg aaactgtcga ccatatcaca 840

acacatgatc agccaacata tgaaaaacac ggggttgtgc attatgctgt agcgaacatg 900

ccaggcgcag tccctcgtac atcaacaatc gccctgacta acgttactgt tccatacgcg 960

ctgcaaatcg cgaacaaagg ggcagtaaaa gcgctcgcag acaatacggc actgagagcg 1020

ggtttaaaca ccgcaaacgg acacgtgacc tatgaagctg tagcaagaga tctaggctat 1080

gagtatgttc ctgccgagaa agctttacag gatgaatcat ctgtggcggg tgcttaa 1137

<210> 75
<211> 1055
<212> DNA
<213> Artificial Sequence

<220>
<223> Artificial Fragment for Pyridoxine Dehydrogenase

<400> 75
acaaaaagga taaaacaatg tccgtgactg aactgcgtaa tgatatccat aaattagata 60

ccggttatgg tctgatgagc ctgacctggc gtgcagagcc gattcctcag tcgcaggcat 120

ttgaagcgat gcaccgtgct gttgagctgg cccgcgaacg tggacacaaa gcctttttca 180

acgttggtga gttttatggc ccggattttg tgaatttaat atttgttcgc gacttctttg 240

caaagtaccc tgatttgcgt aaacatgtag tgatcagttg taaaggtggt atggaggtta 300

gcacgctgac cccgaatgga agccatgatg cagtcattaa aagtgtgaag aactcggtca 360

gcgcgattgg tggctatatt gacatatttg aggtggcccg ccttgataca tctctgtgtg 420

cgaaagggga agtatatcca tacgaatcgt tcgaagccct tgctgaaatg atctccgaag 480

gcgttattgg cggtatttca ctgagtgaag ttactgaaga gcaaatcaga gcaatttata 540

aagattgggg gaagttttta acctgcgtgg agctggaagt ttctttattc agcacggata 600

tttttcataa tggaattgcg aaaacctgtg ccgaattggg actgacggtc atatgctact 660

caccgctggg tcgcgggctg ttaacaggcc agcttaagtc taacgctgac atcccagaag 720

gcgacttcag aaaagcactt aagcgcttta gtgatgaatc attgaagaaa aatctggatc 780

tggtacggtt tctgcaggaa gagatcgtcg gaaaacgttc caaagataac tctattactc 840

tgcctcagtt agctttaggc tggataaaac actggaatta tgttccggaa tataaaggcg 900

cgaaattcat cccaattccc agtggttctt caatatcaaa agtaaatgaa aactttgatg 960

aaaagaaaac acaacttacg gacaaagagt ttaaagccat taacgacttc ttggctacat 1020

ttcatactgt gggggatcgg tacgagttca attaa 1055

<210> 76
<211> 1067
<212> DNA
<213> Artificial Sequence

<220>
<223> Artificial Fragment for Pyridoxine Dehydrogenase

<400> 76
acaaaaagga taaaacaatg gtggaacagt ctgcaattaa taaactgaaa caagatctgt 60

ctgcgattga taccggctat gggctgatgt cgcttacgtg gcgtgcggaa ccgattcctg 120

agacccaggc tcatgaaaca atgaaacgtg tcgttgaact ggcgcagtct aaaggccata 180

aggcattttt caactgtggt gaattttatg gaccgaatcg gatcaatctg acctatattc 240

gtgatttttt tgtgaaaaat ccagaattac gcaagtatgt gatcataagc tgtaaaggtg 300

gctgcgattg tgaaaccctt actcccaagg gaaaacatga cgatgttatc aaaagcgtgg 360

aggcatcagt tgcagccatt ggtggcttta ttgatatatt tgaagtcgct cgcttggatc 420

tgtcactgtg cacgaatggc gaaacatacc cttatgaatc ctttgaagca ttagccgaga 480

tggttgacaa tggtgccatc ggcgccatta gtttgtcaga ggtgactgcg gagcagatac 540

gtggaatttc caaagactgg cacaagtatc ttgtcagtgt ggaggttgaa ctgtcgatgt 600

tcaccaaagc gattttaacc aatggggtag ttaaagcaaa taatgatctg aacttggtca 660

cgattctgta ctctccgtta ggtcgcggat tgctgactgg tgcagttacg agctcaaaag 720

atataccagc tggggatttc cgtaatctgc tgaaacgttt tgatgatgaa agtttaagac 780

aaaacctgac attacttgac ttcttacgcg acgaaattat cgcgaagcgc cctgcaaata 840

acgaaattac actgccgcag gtagcccttg gttgggacaa atattggaac aagacaggcg 900

agtaccccaa cactcacttt ttaccaatcc cgagcggttc cagtgtaaaa aaactggaag 960

agaactttga tgaagccaaa acgcaaataa ccgctgaaga gtttcggaaa atcaatgatt 1020

tcttgaagaa cttcaaaact gtaggggacg cttacgagat gagataa 1067

<210> 77
<211> 1043
<212> DNA
<213> Artificial Sequence

<220>
<223> Artificial Fragment for Pyridoxine Dehydrogenase

<400>  77
acaaaaagga taaaacaatg tcaaaaacgg gtgaattaaa acgccagctg aatcagctgc      60

ataccggtta tgggttgatg agcctgactt ggcgcgctga tccagttccg caagaacaag     120

catttgaagc catggagcgt gtggtcagtt tagcgcaggc tagcggcaac aaggcttttt     180

tcaatgttgg tgaattttat ggtccggatt attccaatct ggaacttgta aaagcatttt     240

tcgaggccaa accagaatta cgtgagcatg ttctgattag ctgtaagggt ggcgtggata     300

ataccaaatt attgcctaag ggaaaatacg cagatgttct ttcgagtatt gaacaatgcg     360

tgaaacatct gggcacgcac ttagacattt ttgaagtggc gcggctggat aaatctcttg     420

gtggcgaata tcctcgtgaa tcatttgagg ccatggcatc gatggtagac aaaggtatta     480

tcgatggtat atctctcagc gaagtaacag cggatgagat tcgtgcaata ggacatgatt     540

gggcgaaata ccttgtgtgt gtagaagtcg aattttcaat gttcagccct cagatcctgc     600

acaatggggt attggatgca tgcaatgacc tgggattaat cgtcattgcc tattctccgc     660

tgggccgcgg cttactcacc ggtacaatca cgaaggattc agcctttaaa gatttccgtt     720

ccagtctgaa acgttttcag aaagattccc tgcaaaagaa cctggcgctg accgaatttt     780

tgcaggatca gatagtggac caacgcagtt caacaaaccc gatttcgctg ccacaggtgg     840

cacttggctg ggtcaaaagt ctgaataatg ggaaatatcc ccatactcac attgttccga     900

tccccagcgg atctgacaaa agaaaagtgg aggagaactt tgacgaatcc cgggctaagc     960

tcaccgtttc tgagattgaa aaaatcaacg atttcttgaa gtcattcaac gttgtcggcg    1020

accgctacga gtgggtttct taa                                            1043

<210>  78
<211>  980
<212>  DNA
<213>  Artificial Sequence

<220>
<223>  Artificial Fragment for Pyridoxine Dehydrogenase

<400>  78
acaaaaagga taaaacaatg ccgtctcttg ttggacgtga tgttggccat accggctatg      60

gtctgatgcg catgacctgg gtgcctcaac caccaccgca agaacaatgc tttgaagctc     120

tgaataccgc cctggcccat ggctcaaatt tttggaacgc tggtgaactg tacggcacac     180

cggaatataa ctctttgcat ctgttacata gttatttcgc ccagcaccca gagaatgcgg     240

acaaagttgt gcttagcatt aaaggtgggc tgaaaccggg ccagctggtg cctgatggtt     300

ccgaagccaa tattcgtcgc tcagtggatg agtgtcttcg tgtgctggat ggtaaaaaga     360

aaattgatat ctttgaatgt gcgagacagg accccaaaac tacagtagag cagacggtta     420

ctgtacttgc gcagttggtt aaagaaggga aaataggtgg aattgggctg agtgaggttg     480

atgcggaaac aattcgtaga gcacacaagg tgcacccaat tgcagctgta gaggttgaaa 540

tgtctctgtt tgacctgacc atcttacaga atgacgtcgc gaaagtatgc gcagaactca 600

acatcccgat agtggcatat tcgccattgg gacggggtgt cctggccggc gctttcacca 660

cagccgctga catccccgat ggagattttc gcaaaacgtt gccgaaattc caagatgaag 720

caatgaaaca gaacatcaag ttagtcaatg aggtcaatga cttagccgct cgcaaaggcg 780

tagctcccgt tcagatagca ctcgcgtggg tcttaactct tagcggaaag ccgggtatgc 840

ctacgattat tccgatccct ggtggcacca cgagcgccaa agtggcgcag aacttacaag 900

cgcctcgtct gacagatgca gaaatggcag agattgatgc aatactgaag cggaatgaaa 960

ttgttgggac tcgctactaa 980

<210> 79
<211> 1049
<212> DNA
<213> Artificial Sequence

<220>
<223> Artificial Fragment for Pyridoxine Dehydrogenase

<400> 79
acaaaaagga taaaacaatg ccgtccccgt cagattatcg taaagaactg cttgatgtgg 60

aagttggtta tggactgatg agtctgacat ggcgcaaaga tcctcttcca gcagaaaaag 120

tgtttccgac catgaaaaag gtggtagaaa aggcgggcaa taaaaaggca ctgtttaatg 180

tgggtgaatt ttatggtcct catctggcga attacaaact gttgcagtct tttttcgata 240

aatatccgga ggatcgcaag aaagttatta tcagtgctaa aggaggcgtg aatgtcgaaa 300

cattacagcc caccggagat gccgacagca tctctgcgtc aattgaaaat gcactgaaag 360

tgtttggtgg gtatctggac atttacgagc cggcccgtat tgatcttgca ctggcggaaa 420

agaatggtga gaaattgttt cctcgtgaga cctttgacac tatcgtaaag tacatcaaag 480

atggcaaagt tggcggtttc agcttaagtg aagttacgaa cgagcaaatc cgtgctattt 540

acaaggaata cggcgagtat ctggcttgtg ttgaagttga actttctata gcgtcgccag 600

aaattatatc caacgggatt ctggacacat gcaatgaatt tggaataccg gtcgttgcct 660

atgcacccct ggggcggggt ttactgacgg gcagcttgaa aagcaccgct gatatccctg 720

aaggtgattt ccgcggccag ttaaaacgct tccaggatga tgccattgca cataacctga 780

ctttggtaaa attccttcaa gacgaaattg ccgcaaaacg ttccgacaac ccgtctctcc 840

ctcaggtggc cattggctgg gtccgtggac tccggcgcga gtatccaaaa acgaatatca 900

tacccattcc atcagggtct tcggttgaaa aggtaagtgt caactttgat cacattgatt 960

tatcagacga tgagatgagc aaaataaaag actttttaaa gtcatttaaa atcgctggtg 1020

atagatatga gttcgcgcaa gcacattaa                                    1049

<210>  80
<211>  1067
<212>  DNA
<213>  Artificial Sequence

<220>
<223>  Artificial Fragment for Pyridoxine Dehydrogenase

<400>  80
acaaaaagga taaaacaatg tcttttaaac ctgttgaaat atcgggcaag tttggctttg      60

ggacaatgtc catgacctgg acaccgacgc cgccaccggc acagcaatcc attgataccc     120

tgaaatttgt gacgagccat ccgaaattcg gcactaaact gatcaatggt ggtgaatttt     180

atggcccaga ttttgccaat ctgaaactgt taaagcagtt cctggaagag aatgatcctg     240

aagagaataa acagttgatt atatctatca aaggaggtgc ggacaatgaa acattaaagc     300

cgaatggtac caaagagttt gtttcaaaaa gcattgaaaa catcgtaagt tttttcccta     360

aacaaaaaca gaaccgtccg aaattactgt ttgagatggc ccgcgttgat ccttctgtgc     420

cctatggaga aaccattggc tatatcagtg aatatgtgaa actgggtgtg attgatggta     480

ttagcctgtc tgaagtcggc aaggaatcca ttcaggccgc tctgaaagta ttcccaatat     540

catgtgttga attagagctg agccttttta gtcaagaagt tatcaccact ggtatattgg     600

aagagctgtc aaagcacaac ctgccgctga ttgcttatag cccgctttgc cgtgggcttt     660

taacggatta tgcagttgag aatagcgata catttttggc atcaatccca cagggtgata     720

tacgtcatca tctggacaaa tttcaaccgg acaccttcaa caaaaacctt cccgcgttaa     780

aggaactgta caagtttgct cacgaagtca aaaacacgac tctggaatct ttggcactta     840

gctggattgt tacagtctcg gaggcgcgca atttccgggg cattgaaaaa gtgacccgta     900

tcttacccat tccttccgga agtacgaaaa agcgcgtaga gtcaaatttt gggagtttaa     960

tagagctgac tgatgacgat ttacagtcga tcgacgaaat tttcgccaaa tacccaatta    1020

gtgggttgag atacaatcag tttctggaag gaactctttt ccaataa                  1067

<210>  81
<211>  1007
<212>  DNA
<213>  Artificial Sequence

<220>
<223>  Artificial Fragment for Pyridoxine Dehydrogenase

<400>  81
acaaaaagga taaaacaatg acatttaata aaatcacacc gacgagtaaa ggttttggac      60

tgatgggctt cacatggaga gaaaccgaaa ccccggatga aattgcattt ccgaccatga     120

aacgtgccat tgaaaagggc attttagtgt ggaatagcgc agagttctat ggcaccaaag     180

atccattagg taacctgaaa ttgattcgtc gctattttga aaagtacccg gaagacgccg 240

acaaagtgtg tctgatggtc aaaggaggtc tggggccgaa cttggtgccg aatggctcca 300

aggagtttgt gcagaaatcc attgatactg ttctggaaac gctgggtggc tctgtacctg 360

tgagcatctt ccagtgtgca cgtgtggacc cgaatacacc catagaagag acggtcgcgg 420

ccatcaaaga gtatgttgat gctggcaaaa ttggagcgct gggtttatca gaagtcgggg 480

ctgcaaccat tgagaaggta cattctctga gtccgatagc ggctgttgaa aatgagttat 540

cactgtgggc caccgatatc tttacaaacg gtgttgcgga agtatgcgca aaatataata 600

ttcccatcac tgcctatgct cctctgggtt ctggcgtact tacgggccgt ttcaaaagca 660

cccaggatgt gccagaaggg gacgcacgca agcacatggt caaataccaa gaggaaaact 720

tgaaacagaa tctggttctc gtagaccgcc ttaaagaagt ggccaccaag gcgggtattc 780

ctcttccaca gatggccatt gcatgggtca aagctcaatc aaaccacaat ggccatggga 840

ccataatccc tatcccaggt aacacaactg ttgcgcggct ggaagagaac cataatgatt 900

taacgctgtc ggcagaaact ctcaaagaga tagatgatgt tctgaagagt acgaaaattg 960

ttggagatcg cgggtttact cttggtggag aatttatgaa tggttaa 1007

<210> 82
<211> 1196
<212> DNA
<213> Artificial Sequence

<220>
<223> Artificial Fragment for Pyridoxamine Pyruvate Transaminase

<400> 82
acaaaaagga taaaacaatg cgttatcctg atcacactga accggtgatt acattaaccg 60

caggtccagt taatgcctat cccgatgtgt tacgcggttt gggtcgcacc gtgctttatg 120

attacgaccc ggcttttcag ttattttatg agaaagtcat tggtaaagcg cagaaggcca 180

tgcgtctgag caataaaccg gttgtcctgc atggcgaacc ggtgatgggt ctggaggccg 240

cagctgcgtc attgatctct agagatgatg tggtacttaa tctggcaagc ggtgtttacg 300

gtaaagggtt cggctattgg gctaagcgtt attcgcctaa cctgttagaa attgaggttc 360

cttacaatga agcgatcgat ccgcagtccg tcgctgacat gctgaaaagt catcccgaaa 420

ttaccgtagt tagtgtctgt catcacgaca ctccatcagg tacgatcaat ccgattgatg 480

agatcggagc gattgtttcg gctcatggcg gatatctgat cgtagatgcc gttagttcat 540

tcggtggcat gaaaacacat cccgaagatt gcaaagccga catatatgtg accggccaaa 600

acaaatgtct tggcgcacct cccggactga cgctgatgag cgtgagcgac cgtgcctgga 660

ccagaatgaa agcgaactct gcagcacctc gcgcaagtgt tctgagcatt ttggattggg 720

agcatgcatg gtccaaggaa aaaccgtttc catttactcc gtctgtcgcc gaaatgaacg 780

gactggatgt tgctttagac ctttatttga acgaagggcc tgaagcggtg tgggctcggc 840

acgcactgac cgccaaagcg acacgcgccg gggctcaagc aatgggctta tcgatctggg 900

ctgcaaatga aaagattgcc tcaccgacaa caaccgcaat acgcacgcca gacggggtcg 960

atgaaaaagc gctgcgtcag gccgctcgtg agcgttacgg agtggtattt tcttccggcc 1020

ggggagaaac tttaggcaaa ctgacgcgca ttggtcacat ggggccaact gcgcagccga 1080

tctacgcgat agcagcgctg acggctttgg gcggtgcgct gaatgccgct ggacggaaac 1140

ttcaagttgg caagggtatt gaggcagcat tagccgtaat agatgcggat gcctaa 1196

<210> 83
<211> 1196
<212> DNA
<213> Artificial Sequence

<220>
<223> Artificial Fragment for Pyridoxamine Pyruvate Transaminase

<400> 83
acaaaaagga taaaacaatg cgttataaag ataatgccga tccggtgatt acactgacca 60

gtggtcctgt taatgcatat gcggatgtgc tgcgtggtct gagccgcact gttttatacg 120

actatgatcc agcatttcag ttattctatg aaaatgtggt ggccaaagca cagaaagcta 180

tgcgtctttc tacccggccg gtactgttgc agggtgaacc tgttatggga ctggaagcag 240

cggcagcttc actgatttcc agcgaagatg tcgttctgaa cttagcctct ggtgtctatg 300

gtaagggctt tggttactgg gcaaaacgct atagtccgaa tctgttagag attgaaaccc 360

cctataacga atctatcgac ccacaagctg ttgccgatat gcttaagaaa catccggaaa 420

ttaccgttgt ctcggtgtgt catcacgata cacctagtgg cacgattaat ccgatcgacg 480

aaattggtgc gatagtatca gcacacgggg cctattttat cgttgatgct gtatcttcat 540

tcggaggcat gaaaacccat cccgaagatt gtaaggccga cctttacgtt actggtccaa 600

acaaatgcct gggcgcgcct ccgggactga caatgatggg ggtcagcgat cgggcctggg 660

ccagaatgaa agctaatcca tccgcacctc gtgcctcggt gctgagcatt ttagactggg 720

aggatgcttg gagtcatgaa aaaccgtttc cattcacacc ttctgtagcg gagataaatg 780

gcttagatgt cgcgctggac ctttacctga acgagggccc ggaagtggtg tgggcacgcc 840

atgcgctgac tgcaaaagcc acgcgtgcag gtgcattggc tatggggttg tcattatggg 900

cggctagcga gaagattgcc agtcccacca caacgtcgat acgcactcca cagggggttg 960

atgaagctgc acttcggcaa gcggtgcgtg agcgctatgg cgtagtgttt tcatccggtc 1020

gtggcgagac gttgggtaaa cttacccgca tcggacacat gggccccacg gcgcagccga 1080

tttacgcagt tgcggctctg actgcgctgg gcggggcttt gaacgcgatg ggacaaaagc 1140

tggccgtcgg cagaggaata gacgccgctt tagcggtaat cgacgccgat gcataa 1196

<210> 84
<211> 1196
<212> DNA
<213> Artificial Sequence

<220>
<223> Artificial Fragment for Pyridoxamine Pyruvate Transaminase

<400> 84
acaaaaagga taaaacaatg cgttatgcac tggatgccga tcctgttatt acactgacca 60

gcggtccggt taatgcatat ccggatgtgc tgcgtgcttt ggcgcgcaca gttctttatg 120

attacgaccc tgcttttcag ttacattatg aacaggtgat cggtaaaatg caaacggcaa 180

tgcggacaag caaccgtccg gtcattctgc atggcgaacc agtgctggga ttagaggcgg 240

ctgcggccag tctgattacc gcagacgata ctgtgttaaa tctggcatct ggtgtttacg 300

gcaaagggtt tggttattgg gcagcgcgtt attcacccaa ccagctggaa atcgaggttc 360

cctacaacca ggctattgat ccgcagcaag tcgccgatat gctgaaggct aatcctgcca 420

tttctgttgt tgcagtatgt catcacgaca ctccgtcagg gaccattaat ccaatcgatg 480

aaataggtgc cattgtcgcc gaccatggag cctatctgat tgttgatgcg gtgagttcct 540

tcggcgggat gaaaacccac ccggaggatt gcaaagcaga catctacgta acaggtccta 600

acaaatgttt aggcgcaccg ccagccctga ccatgttggg tgtcagcgaa agagcgtggc 660

agaagatgaa agataatccg gcagctcctc gcgcctcgat gttaagcatt cttgactggg 720

aaaatgcctg gtctcgtgat gaggcctttc ccttcacgcc gtcagtggct gaagttaacg 780

ggttagacgt agcgctggat ttataccttg ccgagggacc agaagcagtc tgggcacgcc 840

atgcgctgac cgccgctgcg actcgggctg gcatactggc aatgggtttg gacctgtggg 900

cggctgatgc ggccatcgca agtccgacaa cgaccgcagt gagaactcct gagggcattg 960

atgaagcgaa actgcgtgct acagtacgcg aacgttatgg agtggtgatg tcgtccgggc 1020

gcggcgaaac gttgggtaaa cttactcgga taggccacat gggaccaacg gcgcagccta 1080

tctatgcaat tgccgcgctg accgcttttg gtggcgcttt acaagcgttg ggtcatcccg 1140

ctccagtagg aaaggcaatc gacgcggctc ttgcggtcat agatgccgca gcctaa 1196

<210> 85
<211> 1193
<212> DNA
<213> Artificial Sequence

<220>
<223> Artificial Fragment for Pyridoxamine Pyruvate Transaminase

<400> 85

acaaaaagga taaaacaatg acagttgaac gtattgttga acctgctttt actttaagta      60

gcggtcctgt cgatgcgtat ccagccgtgt tacgcgcact gtcacgtacg gttttatacg     120

attttgatcc tgtgttccag cacttttatg aagcagttac tgataaagct aaagtggcac     180

ttcgtaccgc ggcaccacct gtaatcttgc atggcgaacc ggtgctgggt ttagaggccg     240

cggctgccag cctgattgca cgcgacgatg ttgtacttaa tttagcgtct ggtgtctatg     300

gcaagggttt cggtttttgg gccaaacgtt actgtgctga attggttgag attgaagtgc     360

cttatgatga cgcgatcgat ccggcggcgg tcgaggccgc attcagagaa cgccccgaca     420

ttcgtgtagt tagtgtctgc catcatgata cgccgtctgg cactctgaac ccaattgatg     480

aaatcggacg tatagtggca gcccatggtg cttatctgat tgtagacgcc gtttcttcgt     540

ttggtgggat ggatgttcat ccagaggctt gtcaagccga catctttgtg acagggccga     600

ataaatgcct gggctgtccg cctggtttga cgctgctggc ggtcagtggc cgcgcttggg     660

ataaaatgaa ggccaatccg gacgcgccga gagcatcaat tcttagcata ctggattggg     720

aaaacgcgtg gcggcgtgat cagccgtttc ccttcacccc aagtatttcc gaagtgaatg     780

cactggatgc ggctttagac ctgtatctgg aagaggggcc ggaagccgta tggcgccgtc     840

acgcacttac atcaaaagcc tgccgcgatg gcatccgcgc tatgggaatg actatatggc     900

ccgcgcgtga ggcaattgct tctccgacca ccacggcggt gcgggtaccc gacgggattg     960

atgcagatgc tctgttggcg acagcacgtg atggctttgg tgttagcttc tcgtccgggc    1020

ggggcgaaac caaaggaaag ctgatccgca taggacacat gggtcgcacc gcccagccat    1080

tatacgccgt tgtcgggtta gcagcgctgg gcggtgcgtt gagacggctg ggagctaatg    1140

tggacgccgg cgcaggaatg gaggcagctc ttgccgtcat ctcaaaccaa taa           1193

<210>  86
<211>  1202
<212>  DNA
<213>  Artificial Sequence

<220>
<223>  Artificial Fragment for Pyridoxamine Pyruvate Transaminase

<400>  86
acaaaaagga taaaacaatg aatcagacga aaattgcaga tccaattttt accttgacca      60

caggtccggt tgatgcctat ccagcggtgc tgcgtgcact gtctcgcccg gtgttatatg     120

actacgatcc ggcgtttctt gcgttctatg aagccgtcaa tgctaaagta cagcgtgcct     180

ttcatacgaa aaccccacct gtgatcctgc agggtgaacc tgtgctgggg ttagaagcgg     240

ctgcggctag cctgattgcc aagaaagatg tggtgcttaa cctggtttct ggcgtttacg     300

gtaaagggtt cggcttttgg gcagcgcgct atgcgcgtga gctggtcgaa ttggaagttc     360

cctataacga tgtcctgaca gccgatgcgg tagctgacat gttgaaaaag cgtccagata     420

tcgccgtggt tgcgttatgt caccatgata ccccgtcagg taccgtcaat cctgtagccg 480

aaataggtgc agtggtgaga gcggcgggaa aactgtttat tgttgattcc gtaagtgcct 540

ttggtggcat ggacgtgtta ccggaaacgg cttgcgctga tattttcgta actggtccca 600

ataaatgttt aggctgcccg cctggtttgt ctcttctgca cgtcagcgaa gctgcctggg 660

agaagattgc agccaatccc gatgcaccaa cagcttcaat gcttagcatc agtgattgga 720

aaagagctca cgaggctggt caaccgttcc cgtttactcc ttcggtttcc gaaataaacg 780

ccctggacgc tgccatggat ttatatctgg aggaaggcga gcaggcagta tgggcgcgcc 840

atgcgctgac agccaaagca tgtcgggcag gagtgcaagc agccggcctg aagttatggg 900

cggcacggga agacatcgct tcaccaacct gcactgccgt ggcaattccc gatgggattg 960

acgaagcaaa actgcgggcg agtatacgtg atcgctacgg agttgtcttt tctagcggcc 1020

gtgctgagac gctgggtaag ttgacacgca tcgggcatat gggcccgact gcacgtccta 1080

cctattcatt agtctcggtt gcagccattg ttggagggct gaaagcagcc ggcgttaaag 1140

accttgatgt agaggctgga gttgcggcgg caatgaaggt catagacagt gcagaaaaat 1200

aa 1202

<210> 87
<211> 1190
<212> DNA
<213> Artificial Sequence

<220>
<223> Artificial Fragment for Pyridoxamine Pyruvate Transaminase

<400> 87
acaaaaagga taaaacaatg atgcgttccg cctttgccca gccgttattt accctgacta 60

caggtcctgt tgatgtctat ccggcggtga gtcgtgcatt atctatgcct gtctggtatg 120

attacgaccc ggcttttcag aactgctttg aacgcgtcag cctgaaggcc gcacaggctc 180

tggaaagtgc cacgccgcct ctgatcttac agggtgaacc cgtactggca ctggaagccg 240

cagcggcatc actgcttagc gctgaagata tggtgctgaa tctggtttcc ggcgtatatg 300

ccgccggttt ctctgagtgg gcgcggcgtt actctcgtca agttgaagag ctgcgcgttg 360

aatttaatca ggtgattgat cctgtcgctg ttgatgcatg gcttagtcgt catccgcaga 420

ttaccgtggt tgtggtttgc caccatgata cgccttccgg caccttaaac ccactggatg 480

aaattgggcg ggttgtgaaa aaacatggta aattgctgct tgtggatgcg gtttcttcgt 540

ttgccggtgt ggcagtcaca gcggccagtt gccaagctga cctgttcata accggcccca 600

ataaatgttt aggctgtccg ccaggattaa gcctggtggc ggtatctgat gctgcctggc 660

ggaaaatcga ggccaatgac gctgcaccgc gtgcgagcgt attatcgttg ttagactggc 720

gtgatgcctg gtcagctgaa aaaccttttc cctatacacc atcagtagcg gagatcaacg 780

gactggaagc agcgctggac ggttacttgc aggaggggcc agctcaggtc tgggcgcgcc 840

acagattaac cgcacaagca ttccgcgccg ggattcaggc aatgggactt agcttgtggg 900

cggctgatga aagtattgct tcaccaacgg caactgcgat acgcgttccc gacggagtaa 960

atgaagccca atggagactg cgcgcgcgtg agtgttatgg cgtgatgttt tcgtcagggc 1020

gcggtgaaac gctgggcaag gtattgcgtg tcggtcatat gggaccgact gcccaaccaa 1080

tgttcgcgat tatcgctctt acagcactgg cgggggcact taaccagtta agagagccgc 1140

aactggatat tggcgctggc gttgcagccg caatggctgt gataggttaa 1190

<210> 88
<211> 1178
<212> DNA
<213> Artificial Sequence

<220>
<223> Artificial Fragment for Pyridoxamine Pyruvate Transaminase

<400> 88
acaaaaagga taaaacaatg gcacatcctg attttacact gagcgccggt ccggttaccg 60

ttaccgcacg cacgctggcg ggtttaggca gtccgattct gtatcattat gaccctgaat 120

ttttagcaac atttcgtcgc acgcagggta aagtggggca gatgttccaa accgataatg 180

acattatcct gatgcagggt gaagccattg ttggtcttga aggcgctttg cgttctctga 240

taacgcccgg gatgcatgtt ctgaacttag tgcagggagt atttggcaaa ggtaccggct 300

actggattgc cgatttcgga gctgtgctgc acgagatcga agttggctat gatgacgcgg 360

tctcaccggc acaagttgaa gagtatcttg atgcacatcc agaaattcag atggtttgtc 420

tggtggcctc cgagaccccg agtggtacag taacggatgt cgcggcaatt ggcccaatgt 480

gtcgggatcg tggaatcctg acttatatag atactgtttc tggcgtgttg ggtatgccct 540

ggaaaacaga cgaatggggc ctggatttat gcgtcgcagg agctcagaaa tgtctggggg 600

gccctcctgg tgttgcgctt atttcagtga gccaaaaggc atgggatgta atgtacgcga 660

atccggccgc tccacgtgac tcatatttaa gtctgatcga ttggaaagag aaatggctgg 720

gtgaaggccg ctttccgtat acgccatctg tgagcgacat gaacgggctt gaagcagcgt 780

tagatcaggc actggaagag gggattgacg cggttgtcgc acgtcacgaa gccgctgccg 840

ctgtgacccg cgccggagct agagccatgg gtctggaact ttgggcgaag agcgaagaga 900

tagcatcggc ttgcgttact tccattcgtc tgcccgatga catcgataat gccgtggtac 960

gcgatcatgc tcgtgaagtc tacggggtga tgttatcaca cgggcagggt gccggcaact 1020

tggtacggtt atctcacatg ggtccgacag ctggtggcct gcattcggtg gtaggacttg 1080

ccgcgttagg ccgcaccttg gctgacctgg gaatgtccgt cgatataggt gcgggactgg 1140

aggcggcatt agcgctgttg agtactcaaa gacggtaa                              1178

<210>  89
<211>  1133
<212>  DNA
<213>  Artificial Sequence

<220>
<223>  Artificial Fragment for Alanine Dehydrogenase

<400>  89
acaaaaagga taaaacaatg attattggtg tacctaaaga aattaaaaat catgaatatc      60

gtgtcggcat gactccagca ggagttcagg aactgacggc ccgtggacat aaagtgctgg     120

ttcagcaaca gggaggtgaa gcgattggac tgagtgatgc cgagtatcaa gcagccggtg     180

ctgaattagt ggccagcgca gcggaaatct ttgctcgtgc cgagatgata gtaaaggtta     240

aagaacccca gccggaagag tgcgaactgt tgagaccggg tcaattactg tttacttacc     300

ttcatctggc acctgatccg gtgcagaccc agctgttagt tcactcaggg gcggtggcta     360

ttgcctatga gacggtaaca gacgatcgcg gcggtttgcc tctgttagca ccaatgtctg     420

aagtcgcggg gcggatggca attcaggctg gcgcccatag cctggaaaaa gcgcaagggg     480

gtaacggcac actgttaggt ggggttccgg cagtggcgcc cgctaaggtt gtggtacttg     540

gcggtggcgt tgtcggcgtc aattcagcgc gtatggcact gggattaggg gccgatgtca     600

ccatcctggc tcgctccctt cctcgtctgc gggaattaga cagcctgtac ggtccggctc     660

ttaaaacgtt gtatagtact cgtaccaaca tagaggcgtg cattgcccag gctgatctgg     720

tgattggtgc cgttcttatc cctggggcag cggcaccaaa actgttaaca cgcgacatgt     780

tgaagctgat gcgtaaaggc tcggtgcttg tagatgttgc catagatcaa ggtggctgtt     840

tcgaaacctc tcacccgacg acccatgagg aacccacata tgtggttgac ggtattatcc     900

attactgtgt ggctaatatg ccaggcgggg tagcgcgcac ctccactttt gcactgacca     960

atgccacatt gccgtttgtt ttagcgctgg ctgataaagg atatgtcaga gcacttcagg    1020

aggatcgcca cctgcgggcc ggtctgaacg tgttcaaagg caagatcacg caagcggcag    1080

ttgcgcaggc attgggatat gaatacgtca gcgctgaggt agttttagac taa           1133

<210>  90
<211>  1136
<212>  DNA
<213>  Artificial Sequence

<220>
<223>  Artificial Fragment for Alanine Dehydrogenase

<400>  90
acaaaaagga taaaacaatg cgtgttggtt gtccgaaaga aattaaaaat catgaatatc      60

gtgttggcct gacacctgcg agcgttcgtg aatacgttgc ccatggtcat gaggtccggg 120

ttgaaaccaa ggcaggcgca ggtattggag ccgatgatgc cgcttatgtg gccgctggcg 180

caaaaattgc cgcttctgcc aaagagatct ttgaaaagtg cgacatgatt gtaaaagtta 240

aagagccgca gcccagcgag tgggcgcagt tacgcgatgg tcagattctg tatacctatc 300

ttcacttagc gcccgatccg gaacagacca agggcctgct tgcatcaggt gtcaccgctg 360

ttgcctatga aactgtgacg gatgatcgtg gaggcctgcc tctgcttgct ccgatgtcag 420

aggtcgcagg acgtctgagt atccaggcag gggcgaccgc attgcaaaaa gccaatggtg 480

gccttggtat tttgttagga ggggtgcctg gcgtgttgcc agctaaagtt acagtgatcg 540

gtggcggtgt ggtcgggttg cacgcggcca gaatggcggc aggactgggt gcagacgtta 600

gcattttagc aaagtcttta ccgcgcctgc ggcagctgga tgatatcttt ggcggtcgtg 660

tacatacgcg ctattccagc attcaagcct tggaagagga agtattcagt gcagacttag 720

taataggtgc tgtgttgatt ccaggcgctg ccgcaccgaa acttgtcact cgtgaaatgc 780

tgtcgggtat gaaaagaggg tctgtgatcg tagatgtcgc tatagaccaa ggtgggtgtt 840

ttgagacaag tcatgcaaca acgcattcag agccaaccta cgttgtagat gacgtggtgc 900

attattgcgt tgccaatatg cctggcgctg tgccggtcac ttcggcgcat gccctgaaca 960

atgcgacgtt agtttatggg ctggcgcttg cagaccgcgg actgcgggcg atagcggaag 1020

atcgccacct gagaaatggc ttaaacgtcc acaaggacgc cgtaacaaac aaaccagtgg 1080

ctgaagcgct ggggtacgaa tcctacactc ccgaaagcgt actgaacgtt gcgtaa 1136

<210> 91
<211> 1139
<212> DNA
<213> Artificial Sequence

<220>
<223> Artificial Fragment for Alanine Dehydrogenase

<400> 91
acaaaaagga taaaacaatg cgtatcggtg tacctaaaga aattaaaaat catgaatatc 60

gtgttggcct tacgccgcag tctgtggcag aactgaccac gctggggcat gaggtttgga 120

ttgaaaccct ggccggtgca gccattggtt ttgcagatga ggattaccgt aaagccgggg 180

cgcagattgc tccgtgtgca ggcgaagtgt ttcagcaagc gcagttaatc gtcaaagtaa 240

aggagcctct ggcggttgaa cgtgccaaac tgcgcgaaca acatacgctg ttcacatatc 300

tgcaccttgc tccggatcgg ccgcagaccg acgagctgat ggcaagcgga gccacatgta 360

ttgcatatga aactgtgacc gatgttcagg gccgtctgcc attattggcg ccgatgtcag 420

aagtggctgg gcgcatgtct atacaagccg gtgcaggttg cctggaaaaa gcgcgtggtg 480

gtcgtggcgt actgttagga ggcgttcctg gggtggcgcc gggaaaggtt gtgattcttg 540

gcggtggtgt agttggaagc catgccctgg caatggccgt gggcctgggt gctgatgtca 600

cggtactggc caaaagtgtc gatgcactgc gcagacttga tgctcagtac ggcaatcgca 660

tcaccacttt atattccaca cgcgccgcag tgcaggagca agttttagcg gctgacttgg 720

tcataggtgg cgtgttaatt cccggggcgg cagctccaaa attgattagc gcggacatgg 780

ttcgccagat gaaagccgga gcggtgttag ttgatgtggc aatcgaccaa ggcggatgtg 840

ctgaaacctc gcgggccaca actcatgcag aacccaccta tgttgtcgat gacgttgtac 900

attactgcgt agccaatatg cccggtgctg tggcacgtac atcaacgttg gcgctgaaca 960

acgccacttt gccatttgtc gttgcgctgg ctcagaaggg cactcggaga gcgctggaag 1020

acgatcctca cctgctggct ggtcttaatg tcgcgcgcgg agcaataaca tgcgccagtg 1080

tcgcagaggc tcacgggtta ccattccaac cgcctgctag cgtattagag agactttaa 1139

<210> 92
<211> 1133
<212> DNA
<213> Artificial Sequence

<220>
<223> Artificial Fragment for Alanine Dehydrogenase

<400> 92
acaaaaagga taaaacaatg cgtgttggtg tgcctaaaga aattaaaatg caggaatatc 60

gtgttggtct gaccccaggt gcagtccgcg aatatgttgc agccgggcac caggtgacgg 120

ttgagaccgg cgcaggtagc ggcattggag cgtctgatga agtgtaccaa cgggcagggg 180

ccgcgattgc tgaaaacgca cgtgacatct ttgatagctc cgacatgatt gtgaaagtga 240

aagagccgca gaaaagcgaa tgggcccagc ttcgcgaaag tcaaatcctg tttacatact 300

tacatcttgc gcctgatccc gaacaggcaa aaggcctgtt aacctctggt tgtactgcgg 360

tcgcctatga gacagttacc gatgcagctg gtcacttacc tctgcttgcg ccgatgagcg 420

aagttgccgg tcgtctggca attgaagccg caggcgccgc gttaaagcgt tcagctggag 480

gccgcggggt gctgttaggt ggtgtgcccg gcgtacagcc ggctcgtgtc gttgtgttag 540

gaggcggtgt agtgggaacg caagccgctc gcatggcggc gggtcttggt gctgaagtta 600

ctgtaatcgc acgttcggtt cctcgtctgc gcgaactgga cgatgtcttt ttagggcggg 660

tgagaacacg cttctctacc attgaggcag ttgaagatga ggtatttgct gccgacgttg 720

tgatcggggc agtgctggta ccgggcgcta gtgcaccaaa attggtcacg agagggatgt 780

taaagtcaat gcggcctggt gccgtgctgg ttgatgttgc gatagaccag ggcggatgtt 840

tcgagacttc gcatccgaca acgcacgctg atccgaccta tcaggtcgat ggcgttgtac 900

attattgcgt cgccaacatg ccaggtgcgg tgcccgtaac gtcaagccaa ggactgaata 960

atgcgacact gccgttcggc ctgatgttgg cgaataaagg atttgccgct gttctggaaa 1020

atccgcattt gcgcaatgga ctgaacgtac atcgtggcag aataactaac aaggcagttg 1080

ctgagagtct tgggctggaa tttaccccag tcgagtccgg tttggccgcg taa 1133

<210> 93
<211> 1130
<212> DNA
<213> Artificial Sequence

<220>
<223> Artificial Fragment for Alanine Dehydrogenase

<400> 93
acaaaaagga taaaacaatg aaagttggag ttcctaaaga aattaaagca catgaatatc 60

gtgttggtct taccccagga gctgcccgtg aatacgtggc agcgggccat cgcgtgatga 120

tcgaaaccaa tgcaggtgct ggcattggtg caaccgatgg cgattatcgc aatgccggcg 180

caacgatttt aacttcagca gctgaggtat ttgcatctag cgaaatgatc gttaaggtca 240

aagagcctca gccggcagaa tggtcccagc tgcgtgagga tcaaattctg ttcacctatt 300

tgcacttggc accggacccg gaacaggctg ccggcctgtt gaaatctgga tgtattgcaa 360

tcgcctatga gacagtaact gatgctcatg gtggtcttcc actgctggcg ccgatgagcg 420

aagtcgcggg cagactttca atagaagcgg cgggtagcgc tttaaaacgt agtacaggag 480

ggcgtggact gctgatcggt ggggttcccg gggttcagcc tgcccgtatt gtggtgattg 540

gaggtggggt tgtcggtacg cacgccgcgc gcatggcggc tggtctgggc gcagaggtta 600

ccatcatagc acgttctatc attcggttac gtgaactgga cgaattattc gaaggacgtg 660

ttcgcactag attttcgacg atcgaatccg tggaggaaga ggtatttgcg gcggacgtgg 720

ttattggtgc ggtgcttgtc cctggggcaa gtgctccgaa gctggttcgc cggagcatgc 780

tgagttcaat gcgcaaaaga gccgtattgg tggacgtcgc tatagatcaa ggcggctgct 840

ttgaaacatc tcgcccaacc actcacgctg atcctacata cgaagtagat ggcattattc 900

attattgtgt tgccaatatg ccgggtgccg tgcccttaac gtcgagccag gcactgaata 960

acgcgaccct gccgtttggt ttggctctgg ccaacaaagg tttttccgcc gtacttgaga 1020

atccacatct gcgcgcaggc ttaaacgtcc atcgtggccg gttaacatac aaggcggtgg 1080

ccgagagtct tgggctgccc ttctcaccga tagaacaagc tgcggcctaa 1130

<210> 94
<211> 1133
<212> DNA
<213> Artificial Sequence

<220>
<223> Artificial Fragment for Alanine Dehydrogenase

<400>  94
acaaaaagga taaaacaatg aaagttggta ttcctcgtga agttaaaaat catgaatatc      60

gcgtggcaat cacgccagcc ggcgtgcatg agctggtccg taatggacat gaagtttata     120

ttgaagataa tgcgggttta ggaagcagta tccctaacga agagtacgtg gcagccggtg     180

ctaccatttt acccaccgca gatgaagtat gggccacagc tgacttactg ctgaaagtca     240

aggagccgat cagctctgaa tatcaccgtc ttcggaaagg gcagacttta tttacgtatc     300

tgcatctggc ggcagatcgt gccggtacag atgcgttgat tgcttcaggc accaccgcga     360

tagcgtacga aacagtgcag ttaggtaatg gagcgctgcc tcttttagca ccgatgagtg     420

aggttgcggg ccgcctggcc ccacaagttg gttcttatca tctgatgcgt ccggctggcg     480

ggcgtggtgt acttcccgga ggcgtgcccg ggacacaccc tgcaaaagcc gttgtaattg     540

gcggtggtgt ctccggatgg catgcggcta ctatcgcaat tggtatgggc tatgacgtga     600

ccctgttggc acgcgatata aacaaactga gagaagccga tcggattttc gggacccaga     660

tcaaggctat tatgtcaaat agctttgaac tggagaaagc agttcttgac gccgatctgg     720

ttataggagc tgtgttgatt ccgggcgcaa aagccccaaa actggtcaca aacgaactgg     780

tttctcgcat gaagccgggt tcagtgttag tagatatcgc tattgaccag ggcgggtgtt     840

ttgaagattc gcatccgact acgcacgatg acccaacgtt ccaggttcat aatagtgttt     900

tttactgcgt agcaaacatg ccgggagccg tccctaatac ctccacctat gcgcttacta     960

acgctacact gccgtacgtg ttggagctgg caaaccgtgg gtggaaagat gcgctgcgca    1020

gagatccagc gttagcgaag ggccttaatg ttcacgaagg tcaaataact tttccggccg    1080

tagctgatgc attcggcctg gagtcggtca cgttggacag cgtgttagcc taa          1133


<210>  95
<211>  1109
<212>  DNA
<213>  Artificial Sequence

<220>
<223>  Artificial Fragment for Alanine Dehydrogenase

<400>  95
acaaaaagga taaaacaatg gaaatcggcg ttccgaaaga aaccaaagat caggagtttc      60

gtgttgggct gagtccttct agcgtgcgcg tactgcgtga aaatggtcat agtatcttcg     120

tccaaacaca ggcgggtact ggtgcaggat ttacggatga agactatatc agcgcggggg     180

cagagattgt ctcaacactg gaagccgctt ggaatcggga gttagtgatc aaggttaaag     240

aaccgttggt gagcgagtat aaccttctgc agaagggaca gctgttattt acctatttac     300

atctggcagc cgatcgcaaa ttaacggaac acttactgga ttgtggcatt agtgcgatcg     360

catacgaaac agtagaacaa gccggagtga atcgcttacc attgcttacc ccgatgagca     420

ttatcgccgg tcggctggcg gttcagtttg gcgctagatt ccttgagcgt cagcagggtg 480

gtaaaggcgt acttctgggc ggtgttcccg gcgtcaaagc aggcaaagtg gtaattttag 540

gtggtggtgt cgtcggcacg gaagccgcta aaattgcggt gggcatgggt gcgatcgtac 600

aaattctggc tgtcaacgtt gagcgtcttt cttacttaga aactcttttt ggatcacgcg 660

ttgaactgct gtatagcaac tctgcacata ttgaaaccgc agttaaagaa gccaatctgc 720

tgataggtgc ggttttaatt cctggccgca gagcgccaat actggtctcc cgtgatctgg 780

ttaaacaaat gcatccgggg tcagtgattg tggacgttgc agttgatcag ggtggatgcg 840

tagaaacatt acaccctacg tctcacacct caccggtgta tatagacgag ggcgtggtac 900

attatggtgt tccgaatatg ccaggggctg tcccctggac atccacccaa gccctgaaca 960

acagcacttt gccgtacgct gtgcagctgg caaatttggg aattaaggcc ttggatgtta 1020

acccagcttt ggcaaagggg cttaatgtgc agaatcatcg tctgatacac ccggccgtac 1080

aagaggtttt ccctgacttg gtgagttaa 1109

<210> 96
<211> 1154
<212> DNA
<213> Artificial Sequence

<220>
<223> Artificial Fragment for Alanine Dehydrogenase

<400> 96
acaaaaagga taaaacaatg attatagggg ttccgaaaga gataaaaaac aatgaaaatc 60

gtgtggcatt aacccccggg ggtgtttctc agctgattag caatggccac cgggtgctgg 120

ttgaaacagg tgccggcctt ggtagcggat ttgaaaatga agcctatgag agcgcaggtg 180

cggaaatcat tgccgatccg aagcaggtgt gggacgccga aatggtcatg aaagtaaaag 240

aaccgctgcc ggaagaatat gtttattttc gtaaaggact ggtgctgttt acctaccttc 300

atttagcagc tgagcctgag ctggcacagg ccttgaagga taaaggagta actgccatcg 360

catatgaaac ggtgagtgaa ggtcggacat tgcctcttct gacgccaatg tcagaggttg 420

cgggccgtat ggcagcgcag ataggtgctc aattcttaga aaagcctaaa ggcggaaaag 480

gtattctgtt ggccggggtg cctggcgttt cccgcggaaa agtaacaatt atcggaggag 540

gtgttgtcgg gaccaacgcc gcgaaaatgg ctgtcggcct gggtgcagat gtgacgatca 600

ttgccttaaa tgcagaccgc ttgcgccagc ttgatgacat attcggccat cagattaaaa 660

cgttaatttc taatccggtc aatattgctg atgctgtggc ggaagcggat ctgctgattt 720

gcgccgtatt aattccgggt gctaaagctc cgacccttgt cactgaggaa atggtaaaac 780

aaatgaagcc cggttcagtt attgttgatg tcgcgatcga ccaaggcggc atcgtcgaga 840

ctgtggacca tataacaacc catgatcagc caacatatga aaagcacggg gttgtgcatt 900

acgctgtagc gaacatgcca ggcgcagtcc ctcgtacctc gacaatcgcc ctgactaacg     960

ttactgttcc atacgcgctg caaatcgcca acaaaggggc agtaaaggcg ctggcagaca     1020

atacggcact gagagcgggt ttaaataccg caaacgggca cgtgacctat gaagctgtag     1080

cacgtgatct gggctatgag tacgttcccg ccgagaaagc gttacaggat gaaagtagcg     1140

tggctggtgc ttaa                                                       1154

<210>  97
<211>  7
<212>  PRT
<213>  Artificial Sequence

<220>
<223>  Specific Amino Acid Residues

<220>
<221>  misc_feature
<222>  (2)..(2)
<223>  Xaa is Val, Cys, Ile, Ala, Met, Ser, Gly or Leu

<220>
<221>  misc_feature
<222>  (3)..(3)
<223>  Xaa is Gly or Ala

<220>
<221>  misc_feature
<222>  (5)..(5)
<223>  Xaa is Phe or Leu

<400>  97

Asn Xaa Xaa Glu Xaa Tyr Gly
1               5

<210>  98
<211>  6
<212>  PRT
<213>  Artificial Sequence

<220>
<223>  Specific Amino Acid Residues

<220>
<221>  misc_feature
<222>  (1)..(1)
<223>  Xaa is Ile, Val, Phe or Leu

<220>
<221>  misc_feature
<222>  (2)..(2)
<223>  Xaa is Ser, Thr, Asn, Cys or Met

<220>
<221>  misc_feature

<222>  (3)..(3)
<223>  Xaa is Cys, Val, Ala, Ile, Trp or Phe

<220>
<221>  misc_feature
<222>  (6)..(6)
<223>  Xaa is Gly, Ala, Ser or Cys

<400>  98

Xaa Xaa Xaa Lys Gly Xaa
1               5


<210>  99
<211>  6
<212>  PRT
<213>  Artificial Sequence

<220>
<223>  Specific Amino Acid Residues


<220>
<221>  misc_feature
<222>  (1)..(1)
<223>  Xaa is Leu, Met, Ile or Val

<220>
<221>  misc_feature
<222>  (2)..(2)
<223>  Xaa is His or Gln

<220>
<221>  misc_feature
<222>  (3)..(3)
<223>  Xaa is Gly, Cys or Ala

<220>
<221>  misc_feature
<222>  (4)..(4)
<223>  Xaa is Glu or Asp

<220>
<221>  misc_feature
<222>  (5)..(5)
<223>  Xaa is Pro or Ala

<220>
<221>  misc_feature
<222>  (6)..(6)
<223>  Xaa is Val, Ile, Leu or Ala

<400>  99

Xaa Xaa Xaa Xaa Xaa Xaa
1               5


<210>  100
<211>  9
<212>  PRT
<213>  Artificial Sequence

<220>
<223>  Specific Amino Acid Residues

<220>
<221>  misc_feature
<222>  (1)..(1)
<223>  Xaa is His or Ser

<220>
<221>  misc_feature
<222>  (2)..(2)
<223>  Xaa is Glu or Asp

<220>
<221>  misc_feature
<222>  (8)..(8)
<223>  Xaa is Ile, Val or Leu

<220>
<221>  misc_feature
<222>  (9)..(9)
<223>  Xaa is Asn or Thr

<400>  100

Xaa Xaa Thr Pro Ser Gly Thr Xaa Xaa
1               5

<210>  101
<211>  7
<212>  PRT
<213>  Artificial Sequence

<220>
<223>  Specific Amino Acid Residues

<220>
<221>  misc_feature
<222>  (1)..(1)
<223>  Xaa is Val, Ile or Ala

<220>
<221>  misc_feature
<222>  (3)..(3)
<223>  Xaa is Ala, Thr or Ser

<220>
<221>  misc_feature
<222>  (6)..(6)
<223>  Xaa is Ser, Ala or Gly

<220>
<221>  misc_feature
<222>  (7)..(7)
<223>  Xaa is Phe, Trp or Val

<400>  101

Xaa Asp Xaa Val Ser Xaa Xaa

1                 5

<210>  102
<211>  10
<212>  PRT
<213>  Artificial Sequence

<220>
<223>  Specific Amino Acid Residues

<220>
<221>  misc_feature
<222>  (1)..(1)
<223>  Xaa is Gly or Ser

<220>
<221>  misc_feature
<222>  (2)..(2)
<223>  Xaa is Pro, Ser or Ala

<220>
<221>  misc_feature
<222>  (3)..(3)
<223>  Xaa is Asn, Gly, Ser, Ala or Gln

<220>
<221>  misc_feature
<222>  (6)..(6)
<223>  Xaa is Leu or Met

<220>
<221>  misc_feature
<222>  (8)..(8)
<223>  Xaa is Ala, Ser, Cys or Gly

<220>
<221>  misc_feature
<222>  (9)..(9)
<223>  Xaa is Pro, Thr, Ser or Ala

<400>  102

Xaa Xaa Xaa Lys Cys Xaa Gly Xaa Xaa Pro
1               5                   10

<210>  103
<211>  9
<212>  PRT
<213>  Artificial Sequence

<220>
<223>  Specific Amino Acid Residues

<220>
<221>  misc_feature
<222>  (1)..(1)
<223>  Xaa is Gly or Asp

<220>

<221>  misc_feature
<222>  (2)..(2)
<223>  Xaa is Val or Ile

<220>
<221>  misc_feature
<222>  (3)..(3)
<223>  Xaa is Val, Thr, Ala, Ser, Met, Ile or Leu

<220>
<221>  misc_feature
<222>  (4)..(4)
<223>  Xaa is Phe, Met, Leu, Ile or Val

<220>
<221>  misc_feature
<222>  (6)..(6)
<223>  Xaa is Ser, Gly, Ala, Thr, Ile, Leu or His

<220>
<221>  misc_feature
<222>  (8)..(8)
<223>  Xaa is Arg, Met or Gln

<220>
<221>  misc_feature
<222>  (9)..(9)
<223>  Xaa is Gly, Arg, Ala, Asp, His or Lys

<400>  103

Xaa Xaa Xaa Xaa Ser Xaa Gly Xaa Xaa
1               5


<210>  104
<211>  11
<212>  PRT
<213>  Artificial Sequence

<220>
<223>  Specific Amino Acid Residues


<220>
<221>  misc_feature
<222>  (1)..(1)
<223>  Xaa is Leu or Val

<220>
<221>  misc_feature
<222>  (2)..(2)
<223>  Xaa is Thr, Ile, Val or Leu

<220>
<221>  misc_feature
<222>  (4)..(4)
<223>  Xaa is Ile, Val or Leu

<220>
<221>  misc_feature
<222>  (5)..(5)
<223>  Xaa is Gly or Ser

<220>
<221>  misc_feature
<222>  (9)..(9)
<223>  Xaa is Pro, Ala or Arg

<220>
<221>  misc_feature
<222>  (10)..(10)
<223>  Xaa is Thr, Val or Ser

<400>  104

Xaa Xaa Arg Xaa Xaa His Met Gly Xaa Xaa Ala
1               5                   10


<210>  105
<211>  10
<212>  PRT
<213>  Artificial Sequence

<220>
<223>  Specific Amino Acid Residues


<220>
<221>  misc_feature
<222>  (2)..(2)
<223>  Xaa is Ile, Thr, Ser, Phe, Asn or Val

<220>
<221>  misc_feature
<222>  (4)..(4)
<223>  Xaa is Asn, Met, Ala, Val, Leu, Thr or Asp

<220>
<221>  misc_feature
<222>  (5)..(5)
<223>  Xaa is His, Asn, Leu or Gln

<220>
<221>  misc_feature
<222>  (7)..(7)
<223>  Xaa is Tyr, Asn or Phe

<220>
<221>  misc_feature
<222>  (9)..(9)
<223>  Xaa is Val or Ile

<220>
<221>  misc_feature
<222>  (10)..(10)
<223>  Xaa is Gly or Ala

<400>  105

Glu Xaa Lys Xaa Xaa Glu Xaa Arg Xaa Xaa
1               5                   10


<210>  106

<211>  9
<212>  PRT
<213>  Artificial Sequence

<220>
<223>  Specific Amino Acid Residues


<220>
<221>  misc_feature
<222>  (1)..(1)
<223>  Xaa is Met or Leu

<220>
<221>  misc_feature
<222>  (2)..(2)
<223>  Xaa is Ile, Leu or Val

<220>
<221>  misc_feature
<222>  (3)..(3)
<223>  Xaa is Val, Leu, Ile or Met

<220>
<221>  misc_feature
<222>  (9)..(9)
<223>  Xaa is Gln, Leu, Val, Asn or Ile

<400>  106

Xaa Xaa Xaa Lys Val Lys Glu Pro Xaa
1               5


<210>  107
<211>  8
<212>  PRT
<213>  Artificial Sequence

<220>
<223>  Specific Amino Acid Residues


<220>
<221>  misc_feature
<222>  (2)..(2)
<223>  Xaa is Phe or Tyr

<400>  107

Leu Xaa Thr Tyr Leu His Leu Ala
1               5


<210>  108
<211>  9
<212>  PRT
<213>  Artificial Sequence

<220>
<223>  Specific Amino Acid Residues

<220>
<221>  misc_feature
<222>  (1)..(1)
<223>  Xaa is Val or Ala

<220>
<221>  misc_feature
<222>  (3)..(3)
<223>  Xaa is Val or Ile

<220>
<221>  misc_feature
<222>  (5)..(5)
<223>  Xaa is Ile or Val

<400>  108

Xaa Asp Xaa Ala Xaa Asp Gln Gly Gly
1               5


<210>  109
<211>  11
<212>  PRT
<213>  Artificial Sequence

<220>
<223>  Specific Amino Acid Residues


<220>
<221>  misc_feature
<222>  (1)..(1)
<223>  Xaa is Phe, Leu, Ile, Met, Tyr or Val

<220>
<221>  misc_feature
<222>  (2)..(2)
<223>  Xaa is Phe, Ile, Tyr, Leu, Trp or Val

<220>
<221>  misc_feature
<222>  (4)..(4)
<223>  Xaa is Val, Cys, Ile, Ala, Met, Ser, Gly or Leu

<220>
<221>  misc_feature
<222>  (5)..(5)
<223>  Xaa is Gly or Ala

<220>
<221>  misc_feature
<222>  (7)..(7)
<223>  Xaa is Phe or Leu

<220>
<221>  misc_feature
<222>  (10)..(10)
<223>  Xaa is Pro, Lys, Arg, Gly, Glu, Thr, Ala, Asn or Ser

<220>
<221>  misc_feature
<222>  (11)..(11)

<223>  Xaa is Asp, Asn, His, Lys, Glu, Pro, Leu or Ile

<400>  109

Xaa Xaa Asn Xaa Xaa Glu Xaa Tyr Gly Xaa Xaa
1               5                   10


<210>  110
<211>  11
<212>  PRT
<213>  Artificial Sequence

<220>
<223>  Specific Amino Acid Residues


<220>
<221>  misc_feature
<222>  (1)..(1)
<223>  Xaa is Arg, Lys, Ala, Ser or Asn

<220>
<221>  misc_feature
<222>  (2)..(2)
<223>  Xaa is Lys, Ser, Glu, Gln, Asp or Ala

<220>
<221>  misc_feature
<222>  (3)..(3)
<223>  Xaa is Asp, His, Asn, Tyr, Glu, Lys, Cys, Gln or Arg

<220>
<221>  misc_feature
<222>  (4)..(4)
<223>  Xaa is Val, Thr, Ile, Met or Leu

<220>
<221>  misc_feature
<222>  (5)..(5)
<223>  Xaa is Val, Ile, Leu, Phe, Met or Thr

<220>
<221>  misc_feature
<222>  (6)..(6)
<223>  Xaa is Ile, Val, Phe or Leu

<220>
<221>  misc_feature
<222>  (7)..(7)
<223>  Xaa is Ser, Thr, Asn, Cys or Met

<220>
<221>  misc_feature
<222>  (8)..(8)
<223>  Xaa is Cys, Val, Ala, Ile, Trp or Phe

<220>
<221>  misc_feature
<222>  (11)..(11)
<223>  Xaa is Gly, Ala, Ser or Cys

<400>  110

Xaa Xaa Xaa Xaa Xaa Xaa Xaa Xaa Lys Gly Xaa
1               5                   10

<210>  111
<211>  19
<212>  PRT
<213>  Artificial Sequence

<220>
<223>  Specific Amino Acid Residues

<220>
<221>  misc_feature
<222>  (1)..(1)
<223>  Xaa is Val, Leu, Ile or Met

<220>
<221>  misc_feature
<222>  (2)..(2)
<223>  Xaa is Ile, Leu or Val

<220>
<221>  misc_feature
<222>  (3)..(3)
<223>  Xaa is Leu, Met, Ile or Val

<220>
<221>  misc_feature
<222>  (4)..(4)
<223>  Xaa is His or Gln

<220>
<221>  misc_feature
<222>  (5)..(5)
<223>  Xaa is Gly, Cys or Ala

<220>
<221>  misc_feature
<222>  (6)..(6)
<223>  Xaa is Glu or Asp

<220>
<221>  misc_feature
<222>  (7)..(7)
<223>  Xaa is Pro or Ala

<220>
<221>  misc_feature
<222>  (8)..(8)
<223>  Xaa is Val, Ile, Ala or Leu

<220>
<221>  misc_feature
<222>  (9)..(9)
<223>  Xaa is Leu Met, Pro or Val

<220>
<221>  misc_feature
<222>  (10)..(10)
<223>  Xaa is Gly or Ala

<220>
<221>  misc_feature
<222>  (11)..(11)
<223>  Xaa is Leu or Ile

<220>
<221>  misc_feature
<222>  (12)..(12)
<223>  Xaa is Glu or Gln

<220>
<221>  misc_feature
<222>  (13)..(13)
<223>  Xaa is Ala or Gly

<220>
<221>  misc_feature
<222>  (14)..(14)
<223>  Xaa is Ala or Val

<220>
<221>  misc_feature
<222>  (15)..(15)
<223>  Xaa is Ala or Leu

<220>
<221>  misc_feature
<222>  (16)..(16)
<223>  Xaa is Ala, Leu, His or Tyr

<220>
<221>  misc_feature
<222>  (17)..(17)
<223>  Xaa is Ser, Gly or Ala

<220>
<221>  misc_feature
<222>  (18)..(18)
<223>  Xaa is Leu, Phe, Val or Ala

<220>
<221>  misc_feature
<222>  (19)..(19)
<223>  Xaa is Ile, Phe, Val or Leu

<400>  111

Xaa Xaa Xaa Xaa Xaa Xaa Xaa Xaa Xaa Xaa Xaa Xaa Xaa Xaa Xaa Xaa
1               5                   10                  15

Xaa Xaa Xaa

<210>  112
<211>  21
<212>  PRT
<213>  Artificial Sequence

<220>
<223>  Specific Amino Acid Residues

<220>
<221>  misc_feature
<222>  (1)..(1)
<223>  Xaa is Val, Ile, Leu or Met

<220>
<221>  misc_feature
<222>  (2)..(2)
<223>  Xaa is Val or Ile

<220>
<221>  misc_feature
<222>  (3)..(3)
<223>  Xaa is Ser, Ala, Val, Cys or Phe

<220>
<221>  misc_feature
<222>  (4)..(4)
<223>  Xaa is Val, Ile, Ala, Leu or Thr

<220>
<221>  misc_feature
<222>  (5)..(5)
<223>  Xaa is Cys or Val

<220>
<221>  misc_feature
<222>  (6)..(6)
<223>  Xaa is His, Asn or Ala

<220>
<221>  misc_feature
<222>  (7)..(7)
<223>  Xaa is His or Ser

<220>
<221>  misc_feature
<222>  (8)..(8)
<223>  Xaa is Asp or Glu

<220>
<221>  misc_feature
<222>  (14)..(14)
<223>  Xaa is Ile, Val or Leu

<220>
<221>  misc_feature
<222>  (15)..(15)
<223>  Xaa is Asn or Thr

<220>
<221>  misc_feature
<222>  (16)..(16)
<223>  Xaa is Pro or Asp

<220>
<221>  misc_feature
<222>  (17)..(17)
<223>  Xaa is Ile, Val, Leu or Ala

<220>

<221>  misc_feature
<222>  (18)..(18)
<223>  Xaa is Asp, Asn, Glu, Ala, Gly, Gln, Val, Arg or Pro

<220>
<221>  misc_feature
<222>  (19)..(19)
<223>  Xaa is Ala, Glu, Gln or Asp

<220>
<221>  misc_feature
<222>  (20)..(20)
<223>  Xaa is Ile or Leu

<220>
<221>  misc_feature
<222>  (21)..(21)
<223>  Xaa is Gly or Ala

<400>  112

Xaa Xaa Xaa Xaa Xaa Xaa Xaa Xaa Thr Pro Ser Gly Thr Xaa Xaa Xaa
1               5                   10                  15

Xaa Xaa Xaa Xaa Xaa
            20

<210>  113
<211>  15
<212>  PRT
<213>  Artificial Sequence

<220>
<223>  Specific Amino Acid Residues

<220>
<221>  misc_feature
<222>  (1)..(1)
<223>  Xaa is Gly, Asp or Ala

<220>
<221>  misc_feature
<222>  (2)..(2)
<223>  Xaa is Ala, Gly, Lys, Thr, Gln, Arg or Glu

<220>
<221>  misc_feature
<222>  (3)..(3)
<223>  Xaa is Tyr, Asn, Leu or Phe

<220>
<221>  misc_feature
<222>  (4)..(4)
<223>  Xaa is Leu, Phe, Met or Val

<220>
<221>  misc_feature
<222>  (5)..(5)
<223>  Xaa is Ile, Leu or Tyr

<220>
<221>  misc_feature
<222>  (6)..(6)
<223>  Xaa is Val, Ala or Ile

<220>
<221>  misc_feature
<222>  (8)..(8)
<223>  Xaa is Ala, Ser or Thr

<220>
<221>  misc_feature
<222>  (11)..(11)
<223>  Xaa is Ser, Ala or Gly

<220>
<221>  misc_feature
<222>  (12)..(12)
<223>  Xaa is Phe, Trp or Val

<220>
<221>  misc_feature
<222>  (13)..(13)
<223>  Xaa is Gly, Ala or Leu

<220>
<221>  misc_feature
<222>  (14)..(14)
<223>  Xaa is Gly or Ser

<220>
<221>  misc_feature
<222>  (15)..(15)
<223>  Xaa is Met, Val or Leu

<400>  113

Xaa Xaa Xaa Xaa Xaa Xaa Asp Xaa Val Ser Xaa Xaa Xaa Xaa Xaa
1               5                   10                  15


<210>  114
<211>  24
<212>  PRT
<213>  Artificial Sequence

<220>
<223>  Specific Amino Acid Residues


<220>
<221>  misc_feature
<222>  (1)..(1)
<223>  Xaa is Ala, Ser, Val or Ile

<220>
<221>  misc_feature
<222>  (2)..(2)
<223>  Xaa is Asp, Gly or Ala

<220>
<221>  misc_feature
<222>  (3)..(3)

<223>  Xaa is Ile, Leu, Phe, Val or Met

<220>
<221>  misc_feature
<222>  (4)..(4)
<223>  Xaa is Tyr, Phe, Leu or Cys

<220>
<221>  misc_feature
<222>  (5)..(5)
<223>  Xaa is Val or Ile

<220>
<221>  misc_feature
<222>  (6)..(6)
<223>  Xaa is Thr or Ala

<220>
<221>  misc_feature
<222>  (7)..(7)
<223>  Xaa is Gly or Ser

<220>
<221>  misc_feature
<222>  (8)..(8)
<223>  Xaa is Pro, Ser or Ala

<220>
<221>  misc_feature
<222>  (9)..(9)
<223>  Xaa is Asn, Gly, Ser, Gln or Ala

<220>
<221>  misc_feature
<222>  (12)..(12)
<223>  Xaa is Leu or Met

<220>
<221>  misc_feature
<222>  (14)..(14)
<223>  Xaa is Ala, Ser, Cys or Gly

<220>
<221>  misc_feature
<222>  (15)..(15)
<223>  Xaa is Pro, Thr, Ser or Ala

<220>
<221>  misc_feature
<222>  (17)..(17)
<223>  Xaa is Gly, Ala or Ser

<220>
<221>  misc_feature
<222>  (18)..(18)
<223>  Xaa is Leu or Val

<220>
<221>  misc_feature
<222>  (19)..(19)
<223>  Xaa is Thr, Ser or Ala

<220>

<221>  misc_feature
<222>  (20)..(20)
<223>  Xaa is Met, Ile, Leu, Val or Phe

<220>
<221>  misc_feature
<222>  (21)..(21)
<223>  Xaa is Met, Leu, Val, Ala or Ile

<220>
<221>  misc_feature
<222>  (22)..(22)
<223>  Xaa is Gly, Ala, His or Ser

<220>
<221>  misc_feature
<222>  (23)..(23)
<223>  Xaa is Val, Ile or Ala

<400>  114

Xaa Xaa Xaa Xaa Xaa Xaa Xaa Xaa Xaa Lys Cys Xaa Gly Xaa Xaa Pro
1               5                   10                  15

Xaa Xaa Xaa Xaa Xaa Xaa Xaa Ser
            20

<210>  115
<211>  10
<212>  PRT
<213>  Artificial Sequence

<220>
<223>  Specific Amino Acid Residues

<220>
<221>  misc_feature
<222>  (1)..(1)
<223>  Xaa is Tyr, Phe, His or Ser

<220>
<221>  misc_feature
<222>  (2)..(2)
<223>  Xaa is Gly or Asp

<220>
<221>  misc_feature
<222>  (3)..(3)
<223>  Xaa is Val or Ile

<220>
<221>  misc_feature
<222>  (4)..(4)
<223>  Xaa is Val, Thr, Ala, Ser, Met, Ile or Leu

<220>
<221>  misc_feature
<222>  (5)..(5)
<223>  Xaa is Phe, Met, Leu, Ile or Val

<220>
<221>  misc_feature
<222>  (7)..(7)
<223>  Xaa is Ser, Gly, Ala, Thr, Ile, Leu or His

<220>
<221>  misc_feature
<222>  (9)..(9)
<223>  Xaa is Arg, Met or Gln

<220>
<221>  misc_feature
<222>  (10)..(10)
<223>  Xaa is Gly, Arg, Ala, Asp, His or Lys

<400>  115

Xaa Xaa Xaa Xaa Xaa Ser Xaa Gly Xaa Xaa
1               5                   10


<210>  116
<211>  16
<212>  PRT
<213>  Artificial Sequence

<220>
<223>  Specific Amino Acid Residues


<220>
<221>  misc_feature
<222>  (1)..(1)
<223>  Xaa is Leu, Gln, Lys, Ala, Phe, Tyr or Trp

<220>
<221>  misc_feature
<222>  (2)..(2)
<223>  Xaa is Gly, Asn, His or Asp

<220>
<221>  misc_feature
<222>  (3)..(3)
<223>  Xaa is Lys, Arg or Asn

<220>
<221>  misc_feature
<222>  (4)..(4)
<223>  Xaa is Leu or Val

<220>
<221>  misc_feature
<222>  (5)..(5)
<223>  Xaa is Thr, Ile, Val or Leu

<220>
<221>  misc_feature
<222>  (7)..(7)
<223>  Xaa is Ile, Val or Leu

<220>
<221>  misc_feature
<222>  (8)..(8)

<223>  Xaa is Gly or Ser

<220>
<221>  misc_feature
<222>  (12)..(12)
<223>  Xaa is Pro, Ala or Arg

<220>
<221>  misc_feature
<222>  (13)..(13)
<223>  Xaa is Thr, Val or Ser

<220>
<221>  misc_feature
<222>  (15)..(15)
<223>  Xaa is Gln, Arg, Glu, Lys, His, Tyr or Gly

<220>
<221>  misc_feature
<222>  (16)..(16)
<223>  Xaa is Pro or Gly

<400>  116

Xaa Xaa Xaa Xaa Xaa Arg Xaa Xaa His Met Gly Xaa Xaa Ala Xaa Xaa
1               5                   10                  15


<210>  117
<211>  23
<212>  PRT
<213>  Artificial Sequence

<220>
<223>  Specific Amino Acid Residues


<220>
<221>  misc_feature
<222>  (2)..(2)
<223>  Xaa is Val, Ile, Leu or Cys

<220>
<221>  misc_feature
<222>  (4)..(4)
<223>  Xaa is Thr, Lys or Arg

<220>
<221>  misc_feature
<222>  (6)..(6)
<223>  Xaa is Ile, Val, Thr, Ser, Phe or Asn

<220>
<221>  misc_feature
<222>  (8)..(8)
<223>  Xaa is Asn, Met, Ala, Asp, Val, Leu or Thr

<220>
<221>  misc_feature
<222>  (9)..(9)
<223>  Xaa is His, Asn, Gln or Leu

<220>

<221>  misc_feature
<222>  (11)..(11)
<223>  Xaa is Tyr, Asn or Phe

<220>
<221>  misc_feature
<222>  (13)..(13)
<223>  Xaa is Val or Ile

<220>
<221>  misc_feature
<222>  (14)..(14)
<223>  Xaa is Gly or Ala

<220>
<221>  misc_feature
<222>  (15)..(15)
<223>  Xaa is Met, Leu or Ile

<220>
<221>  misc_feature
<222>  (16)..(16)
<223>  Xaa is Val, Thr, Ile or Ser

<220>
<221>  misc_feature
<222>  (18)..(18)
<223>  Xaa is Ser, Ala, Thr, Gln, His, Asn, Leu or Gly

<220>
<221>  misc_feature
<222>  (19)..(19)
<223>  Xaa is Ser, Ala, Asn, Gly or Val

<220>
<221>  misc_feature
<222>  (20)..(20)
<223>  Xaa is Val or Ala

<220>
<221>  misc_feature
<222>  (21)..(21)
<223>  Xaa is Arg, Lys, Asn, Gln, Ser, Ala, Leu or His

<220>
<221>  misc_feature
<222>  (22)..(22)
<223>  Xaa is Glu, Gln, Asp, Val or Ala

<220>
<221>  misc_feature
<222>  (23)..(23)
<223>  Xaa is Leu, Ala, Val, Phe or Tyr

<400>  117

Gly Xaa Pro Xaa Glu Xaa Lys Xaa Xaa Glu Xaa Arg Xaa Xaa Xaa Xaa
1               5                   10                  15


Pro Xaa Xaa Xaa Xaa Xaa Xaa
            20

<210>  118
<211>  16
<212>  PRT
<213>  Artificial Sequence

<220>
<223>  Specific Amino Acid Residues



<220>
<221>  misc_feature
<222>  (1)..(1)
<223>  Xaa is Asp, Glu, Gln or Lys

<220>
<221>  misc_feature
<222>  (2)..(2)
<223>  Xaa is Met or Leu

<220>
<221>  misc_feature
<222>  (3)..(3)
<223>  Xaa is Ile, Leu or Val

<220>
<221>  misc_feature
<222>  (4)..(4)
<223>  Xaa is Val, Leu, Ile or Met

<220>
<221>  misc_feature
<222>  (10)..(10)
<223>  Xaa is Gln, Leu, Ile, Val or Gln

<220>
<221>  misc_feature
<222>  (11)..(11)
<223>  Xaa is Ala, Thr, Ser, Pro, Met, Lys, Arg, Gln, Val, Ile or Glu

<220>
<221>  misc_feature
<222>  (12)..(12)
<223>  Xaa is Val, Ile, Glu, Asn, Gln, Thr, Ala, Asp, His, Met, Asn,
       Val, Ala, Ser, Ile, Asp, Gly, Trp or Lys

<220>
<221>  misc_feature
<222>  (14)..(14)
<223>  Xaa is Arg, Cys, Tyr or Trp

<220>
<221>  misc_feature
<222>  (15)..(15)
<223>  Xaa is Ala, Glu, Arg, Gln, Lys, Thr, Ser, Met, Asn, Val, Pro,
       Gly, Cys or His

<220>
<221>  misc_feature
<222>  (16)..(16)
<223>  Xaa is Met, Leu, Lys, Arg, Gln, Glu, Trp, Phe or Tyr

<400>  118

Xaa Xaa Xaa Xaa Lys Val Lys Glu Pro Xaa Xaa Xaa Glu Xaa Xaa Xaa
1               5                   10                  15

<210>  119
<211>  27
<212>  PRT
<213>  Artificial Sequence

<220>
<223>  Specific Amino Acid Residues

<220>
<221>  misc_feature
<222>  (1)..(1)
<223>  Xaa is Leu or Phe

<220>
<221>  misc_feature
<222>  (2)..(2)
<223>  Xaa is Arg, Lys, Cys, Ser, His, Gly or Gln

<220>
<221>  misc_feature
<222>  (3)..(3)
<223>  Xaa is His, Glu, Pro, Ser, Asp, Arg, Lys, Gln or Ala

<220>
<221>  misc_feature
<222>  (4)..(4)
<223>  Xaa is Asp, Gly, Gln, His, Glu, Ser, Asn or Met

<220>
<221>  misc_feature
<222>  (5)..(5)
<223>  Xaa is Gln or His

<220>
<221>  misc_feature
<222>  (6)..(6)
<223>  Xaa is Ile, Leu, Val, Thr, Cys or Ala

<220>
<221>  misc_feature
<222>  (8)..(8)
<223>  Xaa is Phe or Tyr

<220>
<221>  misc_feature
<222>  (15)..(15)
<223>  Xaa is Pro or Ala

<220>
<221>  misc_feature
<222>  (16)..(16)
<223>  Xaa is Asp, Ser, Asn, His or Glu

<220>
<221>  misc_feature
<222>  (17)..(17)
<223>  Xaa is Leu, Met, Pro, Arg, Val, Gln, Glu or Lys

<220>
<221>  misc_feature
<222>  (18)..(18)
<223>  Xaa is Pro, Ala, Val, Gln, Lys, Glu, Asp, Thr, Asn, Arg, Ser or
      Met

<220>
<221>  misc_feature
<222>  (19)..(19)
<223>  Xaa is Gln, Cys or Leu

<220>
<221>  misc_feature
<222>  (20)..(20)
<223>  Xaa is Thr, Ala or Val

<220>
<221>  misc_feature
<222>  (21)..(21)
<223>  Xaa is Glu, Ile, Gln, Ala, Arg, Lys, Thr, Asp or Asn

<220>
<221>  misc_feature
<222>  (22)..(22)
<223>  Xaa is Glu, Asp, Leu, Ala, Gly, His, Tyr or Ser

<220>
<221>  misc_feature
<222>  (24)..(24)
<223>  Xaa is Ile, Met, Val, Leu, Thr or Lys

<220>
<221>  misc_feature
<222>  (25)..(25)
<223>  Xaa is Thr, Lys, Ser, Asp, His, Glu, Ala, Asn, Arg, Gly or Gln

<220>
<221>  misc_feature
<222>  (26)..(26)
<223>  Xaa is Ser, Gly, Cys, Ala or Lys

<220>
<221>  misc_feature
<222>  (27)..(27)
<223>  Xaa is Gly, Lys, Arg, Gln or Asp

<400>  119

Xaa Xaa Xaa Xaa Xaa Xaa Leu Xaa Thr Tyr Leu His Leu Ala Xaa Xaa
1               5                   10                  15

Xaa Xaa Xaa Xaa Xaa Xaa Leu Xaa Xaa Xaa Xaa
            20                  25

<210>  120
<211>  17
<212>  PRT
<213>  Artificial Sequence

<220>

<223>  Specific Amino Acid Residues

<220>
<221>  misc_feature
<222>  (1)..(1)
<223>  Xaa is Gly, Arg or Ser

<220>
<221>  misc_feature
<222>  (2)..(2)
<223>  Xaa is Ser, Ala or Gly

<220>
<221>  misc_feature
<222>  (3)..(3)
<223>  Xaa is Ala or Val

<220>
<221>  misc_feature
<222>  (4)..(4)
<223>  Xaa is Ile, Leu, Met or Val

<220>
<221>  misc_feature
<222>  (5)..(5)
<223>  Xaa is Val or Ala

<220>
<221>  misc_feature
<222>  (7)..(7)
<223>  Xaa is Val or Ile

<220>
<221>  misc_feature
<222>  (9)..(9)
<223>  Xaa is Ile or Val

<220>
<221>  misc_feature
<222>  (14)..(14)
<223>  Xaa is Cys or Ile

<220>
<221>  misc_feature
<222>  (15)..(15)
<223>  Xaa is Val, Ile, Ala, Phe, Ser or Cys

<220>
<221>  misc_feature
<222>  (16)..(16)
<223>  Xaa is Glu or Ala

<220>
<221>  misc_feature
<222>  (17)..(17)
<223>  Xaa is Thr or Asp

<400>  120

Xaa Xaa Xaa Xaa Xaa Asp Xaa Ala Xaa Asp Gln Gly Gly Xaa Xaa Xaa
1               5                   10                  15

Xaa